(19) Europäisches Patentamt
European Patent Office
Office européen des brevets

(11) EP 2 582 709 B1

(12) **EUROPÄISCHE PATENTSCHRIFT**

(45) Veröffentlichungstag und Bekanntmachung des Hinweises auf die Patenterteilung:
**24.01.2018 Patentblatt 2018/04**

(21) Anmeldenummer: **11725479.7**

(22) Anmeldetag: **17.06.2011**

(51) Int Cl.:
***A61K 31/437*** *(2006.01)* ***A61K 31/444*** *(2006.01)*
***A61K 31/4545*** *(2006.01)* ***A61K 31/5377*** *(2006.01)*
***A61K 31/5383*** *(2006.01)* ***A61K 45/06*** *(2006.01)*
***A61K 31/496*** *(2006.01)* ***C07D 513/04*** *(2006.01)*

(86) Internationale Anmeldenummer:
**PCT/EP2011/060122**

(87) Internationale Veröffentlichungsnummer:
**WO 2011/157827 (22.12.2011 Gazette 2011/51)**

(54) **AZOLOPYRIDIN-3-ON-DERIVATE ALS INHIBITOREN VON LIPASEN UND PHOSPHOLIPASEN**

AZOLOPYRIDIN-3-ONE DERIVATIVES AS INHIBITORS OF LIPASES AND PHOSPHOLIPASES

DÉRIVÉS D'AZOLOPYRIDIN-3-ONE EN TANT QU'INHIBITEURS DE LIPASES ET DE PHOSPHOLIPASES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**

(30) Priorität: **18.06.2010 EP 10305655**

(43) Veröffentlichungstag der Anmeldung:
**24.04.2013 Patentblatt 2013/17**

(73) Patentinhaber: **SANOFI**
**75008 Paris (FR)**

(72) Erfinder:
- **PETRY, Stefan**
  **65926 Frankfurt am Main (DE)**
- **TENNAGELS, Norbert**
  **65926 Frankfurt am Main (DE)**
- **BARINGHAUS, Karl-Heinz**
  **65926 Frankfurt am Main (DE)**

(74) Vertreter: **Essler, Frank et al**
**Sanofi-Aventis Deutschland GmbH**
**Global Intellectual Property Department**
**Industriepark Höchst**
**Gebäude K 703**
**65926 Frankfurt am Main (DE)**

(56) Entgegenhaltungen:
**WO-A1-2004/094394 US-A- 4 512 985**
**US-A- 4 512 985**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 582 709 B1

SEQUENCE LISTING

<110> SANOFI

<120> Azolopyridin-3-on-derivate als Inhibitoren von Lipasen und Phospholipasen

<130> DE2010/120

<140> PCT/EP2011/060122
<141> 2011-06-17

<150> EP 10305655.2
<151> 2010-06-18

<160> 2

<170> PatentIn version 3.3

<210> 1
<211> 16
<212> PRT
<213> Artificial

<220>
<223> AOD 9604 from Metabolic Pharmaceuticals

<400> 1

```
Tyr Leu Arg Ile Val Gln Cys Arg Ser Val Glu Gly Ser Cys Gly Phe
1               5                   10                  15
```

<210> 2
<211> 30
<212> PRT
<213> Artificial

<220>
<223> Taspoglutide from Ipsen Pharma

<220>
<221> misc_feature
<222> (2) .. (2)
<223> Xaa is 2-Methylanayl

<220>
<221> misc_feature
<222> (29) .. (29)
<223> Xaa is 2-Methylanayl

<400> 2

```
His Xaa Glu Gly Thr Phe Thr Ser Asp Val Ser Ser Tyr Leu Glu Gly
1               5                   10                  15

Gln Ala Ala Lys Glu Phe Ile Ala Trp Leu Val Lys Xaa Arg
            20                  25                  30
```

**Beschreibung**

Die vorliegende Erfindung betrifft Azolopyridin-3-on-derivate der allgemeinen Formel I, deren pharmazeutisch anwendbare Salze und deren Verwendung als Arzneistoffe.

Strukturähnliche Indazolverbindungen mit pharmazeutischer Wirkung sind aus der WO 2004/093872 bekannt. Strukturähnliche Benzisoxazolverbindungen mit pharmazeutischer Wirkung sind aus der WO 2004/094393 bekannt. US 4,512,985 beschreibt Isothiazolopyridin-3-on-Verbindungen zur Aknebehandlung. Strukturähnliche Azolopyridinverbindungen mit hemmender Wirkung auf die endotheliale Lipase sind aus der WO 2007/110216 bekannt. WO 2004/094394, WO 2004/094393, WO 2004/093872, WO 2007/045393, WO 2007/110215, WO 208/122352 und WO 2008/122357 beschreiben Verbindungen mit hemmender Wirkung auf die endotheliale Lipase.

Aufgabe der vorliegenden Erfindung ist die Bereitstellung von alternativen Verbindungen, die eine Hemmung der endothelialen Lipase bewirken.

Gegenstand der Erfindung sind Azolopyridin-3-on-derivate der allgemeinen Formel I

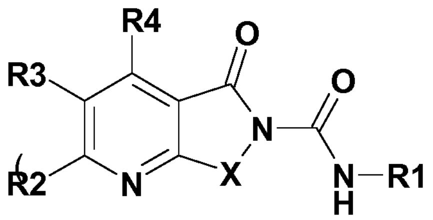

(I)

wobei bedeuten:

X S oder $SO_2$;

R1

ein Rest der Formel Ia

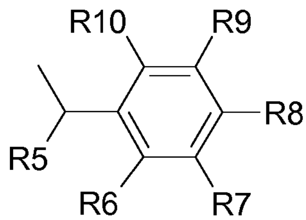

,

worin

R5 Wasserstoff, $(C_1-C_3)$-Alkyl;

R6, R7, R8, R9, R10
unabhängig voneinander Wasserstoff, OH, CN, $OCF_3$, $OCHF_2$, O-$(C_1-C_6)$-Alkyl, O-$(C_1-C_4)$-Alkoxy-$(C_1-C_4)$-alkyl, S-$(C_1-C_6)$-Alkyl, $(C_2-C_4)$-Haloalkyl, O-$(C_2-C_4)$-Haloalkyl, $(C_2-C_6)$-Alkenyl, $(C_2-C_6)$-Alkinyl, N(R11)(R12), $SO_2-CH_3$, $SO_2$-N(R13)(R14), $SF_5$, $SCF_3$, COOH, COO-$(C_1-C_6)$-Alkyl, CON(R15)(R16), N(R17)CO(R18), N(R19)$SO_2$(R20), CO(R21), $(CR22R23)_x$-O(R24), $(CR22R23)_x$-CO-O(R24), O-$(CR22R23)_x$-CO-O(R24), $(CR22R23)_x$-N(R25)(R26), O-$(CR22R23)_x$-N(R25)(R26), $(CR22R23)_x$-CON(R25)(R26), O-$(CR22R23)_x$-CON(R25)(R26), O-CO-N(R25)(R26), O-CO-$(C_1-C_6)$-Alkylen-CO-O-$(C_1-C_6)$-Alkyl, O-CO-$(C_1-C_6)$-Alkylen-CO-OH, O-CO-$(C_1-C_6)$-Alkylen-CO-N(R27)(R28);

x unabhängig voneinander 0, 1, 2, 3, 4, 5, 6;
oder

R7 oder R8 ist
$(O)_y-(CH_2-)_{y'}-(O)_{y''}-(CH_2)_{y'''}-R100$;

y,y" unabhängig voneinander 0,1;

y', y''' unabhängig voneinander 1, 2, 3, 4, 5, 6;

R100 ein 4 bis 10 gliedriger mono- oder bicyclischer gesättigter, teilweise ungesättigter oder aromatischer Ring welcher 1 bis 4 Heteroatome, ausgewählt aus der Gruppe Sauerstoff, Stickstoff oder Schwefel enthalten kann, wobei das Ringsystem zusätzlich ein oder mehrfach durch F, Cl, Br, I, OH, $CF_3$, $NO_2$, CN, $OCF_3$, O-($C_1$-$C_6$)-Alkyl, O-($C_1$-$C_4$)-Alkoxy-($C_1$-$C_4$)-alkyl, S-($C_1$-$C_6$)-Alkyl, ($C_1$-$C_6$)-Alkyl, ($C_2$-$C_4$)-Haloalkyl, ($C_2$-$C_6$)-Alkenyl, ($C_3$-$C_8$)-Cycloalkyl, O-($C_3$-$C_8$)-Cycloalkyl, ($C_2$-$C_6$)-Alkinyl, N(R29)(R30), $SO_2$-$CH_3$, $SF_5$, COOH, COO-($C_1$-$C_6$)-Alkyl, CON(R31)(R32), N(R33)CO(R34), N(R35)$SO_2$(R36), CO(R37), $(CR38R39)_{x'}$-O(R40), $(CR38R39)_{x'}$-CO-O(R40), O-$(CR22R23)_{x'}$-CO-O(R40), $(CR22R23)_{x'}$,-N(R41)(R42), O-$(CR38R39)_{x'}$-N(R41)(R42), $(CR38R39)_{x'}$-CON(R41)(R42), O-$(CR38R39)_{x'}$-CON(R41)(R42), O-CO-N(R41)(R42), O-CO-($C_1$-$C_6$)-Alkylen-CO-O-($C_1$-$C_6$)-Alkyl, O-CO-($C_1$-$C_6$)-Alkylen-CO-OH, O-CO-($C_1$-$C_6$)-Alkylen-CO-N(R43)(R44) substituiert sein kann;

x' 0, 1, 2, 3, 4, 5, 6;
oder

R7 und R8 oder R8 und R9 oder R9 und R10
zusammen mit dem sie tragenden Kohlenstoffatomen bilden ein monocyclisches, 5 bis 7 gliederiges gesättigtes, teilweise ungesättigtes oder aromatisches Ringsystem, dessen einzelne Glieder durch -CHR45-, -CR46R47-, =(C-R46)-, O, N oder S substituiert sein können; mit der Maßgabe, dass zwei Einheiten aus der Reihe -O-, N oder -S- nicht benachbart sein dürfen;

R11, R12, R13, R14, R15, R16, R17, R18, R19, R20, R21, R22, R23, R24, R25, R26, R27, R28, R29, R30, R31, R32, R33, R34, R35, R36, R37, R38, R39, R40, R41, R42, R43, R44

unabhängig voneinander Wasserstoff, ($C_1$-$C_6$)-Alkyl;

bedeuten;
oder

R11 und R12, R13 und R14, R15 und R16, R25 und 26, R27 und R28, R29 und R30, R31 und R32, R41 und 42, R43 und 44
bilden unabhängig voneinander optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-($C_1$-$C_6$)-Alkyl, Sauerstoff und Schwefel beinhalten kann;

R45, R46, R47 gleich oder verschieden, F, Cl, Br, I, OH, $CF_3$, $NO_2$, CN, $OCHF_2$, $OCF_3$, $SF_5$, O-($C_1$-$C_6$)-Alkyl, O-($C_1$-$C_4$)-Alkoxy-($C_1$-$C_4$)-Alkyl, S-($C_1$-$C_6$)-Alkyl, ($C_1$-$C_6$)-Alkyl, ($C_2$-$C_4$)-Haloalkyl, O-($C_2$-$C_4$)-Haloalkyl, ($C_2$-$C_6$)-Alkenyl, ($C_3$-$C_8$)-Cycloalkyl, O-($C_3$-$C_8$)-Cycloalkyl, ($C_2$-$C_6$)-Alkinyl, N(R134)(R135), $SO_2$-$CH_3$, COOH, COO-($C_1$-$C_6$)-Alkyl, CON(R136)(R137), N(R138)CO(R139), N(R140)$SO_2$(R141), CO(R142), $(CR143R144)_{x''}$-O(R145), $(CR143R144)_{x''}$-CO-O(R145), O-$(CR143R144)_{x''}$-CO-O(R145), $(CR143R144)_{x''}$-N(R146)(R147), O-$(CR143R144)_{x''}$-N(R146)(R147), $(CR143R144)_{x''}$-CON(R146)(R147), O-$(CR143R144)_{x''}$-CON(R146)(R147), O-CO-N(R146)(R147), O-CO-($C_1$-$C_6$)-Alkylen-CO-O-($C_1$-$C_6$)-Alkyl, O-CO-($C_1$-$C_6$)-Alkylen-CO-OH, O-CO-($C_1$-$C_6$)-Alkylen-CO-N(R148)(R149);

x" unabhängig voneinander 1, 2, 3, 4, 5, 6;

R134, R135, R136, R137, R138, R139, R140, R141, R142, R143, R144, R145, R146, R147, R148, R149 gleich oder verschieden Wasserstoff, ($C_1$-$C_6$)-Alkyl;

ein Rest der Formel Ib

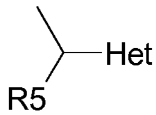

Ib,

worin

R5 Wasserstoff, $(C_1-C_3)$-Alkyl;

Het ein 4 bis 10 gliedriger mono oder bicyclischer aromatischer Ring welcher 1 bis 4 Heteroatome, ausgewählt aus der Gruppe Sauerstoff, Stickstoff oder Schwefel enthält, wobei das Ringsystem zusätzlich ein oder mehrfach durch unabhängig voneinander F, Cl, Br, I, OH, $CF_3$, $NO_2$, CN, $OCF_3$, $OCHF_2$, O-$(C_1-C_6)$-Alkyl, O-$(C_1-C_4)$-Alkoxy-$(C_1-C_4)$-alkyl, S-$(C_1-C_6)$-Alkyl, $(C_1-C_6)$-Alkyl, $(C_2-C_4)$-Haloalkyl, O-$(C_2-C_4)$-Haloalkyl, $(C_2-C_6)$-Alkenyl, $(C_2-C_6)$-Alkinyl, N(R48)(R49), $SO_2-CH_3$, $SO_2$-N(R50)(R51), $SF_5$, $SCF_3$, COOH, COO-$(C_1-C_6)$-Alkyl, CON(R52)(R53), N(R54)CO(R55), N(R56)$SO_2$(R57), CO(R58), $(CR59R60)_{x'''}$-O(R61), $(CR59R60)_{x'''}$-CO-O(R61), O-$(CR59R60)_{x'''}$-CO-O(R61), $(CR59R60)_{x'''}$-N(R62)(R63), O-$(CR59R60)_{x'''}$-N(R62)(R63), $(CR59R60)_{x'''}$-CON(R62)(R63), O-$(CR59R60)_{x'''}$-CON(R62)(R63), O-CO-N(R62)(R63), O-CO-$(C_1-C_6)$-Alkylen-CO-O-$(C_1-C_6)$-Alkyl, O-CO-$(C_1-C_6)$-Alkylen-CO-OH, O-CO-$(C_1-C_6)$-Alkylen-CO-N(R64)(R65);

oder $(O)_y-(CH_2-)_{y'}-(O)_{y''}-(CH_2)_{y'''}$-R101 substituiert sein kann,

x''' unabhängig voneinander 1, 2, 3, 4, 5, 6;

y, y'' unabhängig voneinander 0,1;

y', y''' unabhängig voneinander 0, 1, 2, 3, 4, 5, 6;

R101 ein 4 bis 10 gliedriger mono oder bicyclischer gesättigter, teilweise ungesättigter oder aromatischer Ring welcher 1 bis 4 Heteroatome, ausgewählt aus der Gruppe Sauerstoff, Stickstoff oder Schwefel enthalten kann, wobei das Ringsystem zusätzlich ein oder mehrfach durch, F, Cl, Br, I, OH, $CF_3$, $NO_2$, CN, $OCHF_2$, $OCF_3$, O-$(C_1-C_6)$-Alkyl, O-$(C_1-C_4)$-Alkoxy-$(C_1-C_4)$-alkyl, S-$(C_1-C_6)$-Alkyl, $(C_1-C_6)$-Alkyl, $(C_2-C_4)$-Haloalkyl, $(C_2-C_6)$-Alkenyl, $(C_3-C_8)$-Cycloalkyl, O-$(C_3-C_8)$-Cycloalkyl, $(C_2-C_6)$-Alkinyl, N(R66)(R67), $SO_2-CH_3$, $SF_5$, COOH, COO-$(C_1-C_6)$-Alkyl, CON(R68)(R69), (C), N(R70)CO(R71), N(R72)$SO_2$(R73), CO(R74), $(CR75R76)_{x''''}$-O(R77R75R76)$_{x''''}$-CO-O(R77), O-$(CR75R76)_{x''''}$-CO-O(R77), $(CR75R76)_{x''''}$-N(R78)(R79), O-$(CR75R76)_{x''''}$-N(R78)(R79), $(CR75R76)_{x''''}$-CON(R78)(R79), O-$(CR75R76)_{x''''}$-CON(R78)(R79), O-CO-N(R78)(R79), O-CO-$(C_1-C_6)$-Alkylen-CO-O-$(C_1-C_6)$-Alkyl, O-CO-$(C_1-C_6)$-Alkylen-CO-OH, O-CO-$(C_1-C_6)$-Alkylen-CO-N(R80)(R81) substituiert sein kann;

x'''' unabhängig voneinander 1, 2, 3, 4, 5, 6;

R48, R49, R50, R51, R52, R53, R54, R55, R56, R57, R58, R59, R60, R61, R62, R63, R64, R65, R66, R67, R68, R69, R70, R71, R72, R73, R74, R75, R76, R77, R78, R79, R80, R81

unabhängig voneinander Wasserstoff, $(C_1-C_6)$-Alkyl;

oder

R48 und R49, R50 und R51, R52 und R53, R62 und R63, R64 und R65, R66 und R67, R68 und R69, R78 und R79, R80 und R81
bilden unabhängig voneinander optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-$(C_1-C_6)$-Alkyl, Sauerstoff und Schwefel beinhalten kann;

ein Rest der Formel Ic

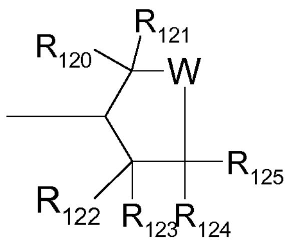

Ic,

worin

W, -C(R126)(R127)-, -C(R126)(R127)-C(R128)(R129)-, -C(R126)(R127)-O-;

R120, R121, R123, R125, R126, R127, R128, R129

gleich oder verschieden Wasserstoff, F, Cl, Br, I, OH, $CF_3$, $NO_2$, CN, $OCHF_2$, $OCF_3$, $SF_5$, O-($C_1$-$C_6$)-Alkyl, O-($C_1$-$C_4$)-Alkoxy-($C_1$-$C_4$)-alkyl, S-($C_1$-$C_6$)-Alkyl, ($C_1$-$C_6$)-Alkyl, ($C_2$-$C_4$)-Haloalkyl, O-($C_2$-$C_4$)-Haloalkyl, ($C_2$-$C_6$)-Alkenyl, ($C_3$-$C_8$)-Cycloalkenyl, ($C_2$-$C_6$)-Alkinyl, N(R90)(R91), $SO_2$-$CH_3$, COOH, COO-($C_1$-$C_6$)-Alkyl, CON(R92)(R93), N(R94)CO(R95), N(R96)$SO_2$(R97), CO(R98), $(CR99R102)_z$-O(R103), $(CR99R76)_z$-CO-O(R103), O-$(CR99R102)_z$-CO-O(R103), $(CR99R102)_z$-N(R104)(R105), O-$(CR99R102)_z$-N(R104)(R105), $(CR99R102)_z$-CON(R104)(R105), O-$(CR99R102)_z$-CON(R104)(R105), O-CO-N(R104)(R105), O-CO-($C_1$-$C_6$)-Alkylen-CO-O-($C_1$-$C_6$)-Alkyl, O-CO-($C_1$-$C_6$)-Alkylen-CO-OH, O-CO-($C_1$-$C_6$)-Alkylen-CO-N(R106)(R107);

z unabhängig voneinander 1, 2, 3, 4, 5, 6;

R90, R91, R92, R93, R94, R95, R96, R97, R98, R99, R102, R103, R104, R105, R106, R107

gleich oder verschieden Wasserstoff, ($C_1$-$C_6$)-Alkyl;

R122 und R124, zusammen mit dem sie tragenden Kohlenstoffatomen bilden ein monocyclisches, 5 oder 6 gliederiges gesättigtes, teilweise ungesättigtes oder ein aromatisches Ringsystem, dessen einzelne Glieder durch -CHR130-, - CR131R132-, =(C-R133)- substituiert sein können;

R130, R131, R132, R133 gleich oder verschieden, F, Cl, Br, I, OH, $CF_3$, $NO_2$, CN, $OCHF_2$, $OCF_3$, $SF_5$, O-($C_1$-$C_6$)-Alkyl, O-($C_1$-$C_4$)-Alkoxy-($C_1$-$C_4$)-Alkyl, S-($C_1$-$C_6$)-Alkyl, ($C_1$-$C_6$)-Alkyl, ($C_2$-$C_4$)-Haloalkyl, O-($C_2$-$C_4$)-Haloalkyl, ($C_2$-$C_6$)-Alkenyl, ($C_2$-$C_6$)-Alkinyl, N(R160)(R161), $SO_2$-$CH_3$, COOH, COO-($C_1$-$C_6$)-Alkyl, CON(R162)(R163), N(R164)CO(R165), N(R166)$SO_2$(R167), CO(R168), $(CR169R170)_{z'}$-O(R171), $(CR169R170)_{z'}$-CO-O(R77), O-$(CR169R170)_{z'}$-CO-O(R171), $(CR169R170)_{z'}$-N(R172)(R173), O-$(CR169R170)_{z'}$-N(R172)(R173), $(CR169R170)_{z'}$-CON(R172)(R173), O-$(CR169R170)_{z'}$-CON(R172)(R173), O-CO-N(R172)(R173), O-CO-($C_1$-$C_6$)-Alkylen-CO-O-($C_1$-$C_6$)-Alkyl, O-CO-($C_1$-$C_6$)-Alkylen-CO-OH, O-CO-($C_1$-$C_6$)-Alkylen-CO-N(R172)(R173);

z' unabhängig voneinander 1, 2, 3, 4, 5, 6;

R160, R161, R162, R163, R164, R165, R166, R167, R168, R169, R170, R171, R172, R173

gleich oder verschieden Wasserstoff, ($C_1$-$C_6$)-Alkyl,

oder

R160 und R161, R162 und R163, R172 und R173 bilden unabhängig voneinander optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-($C_1$-$C_6$)-Alkyl, Sauerstoff und Schwefel beinhalten kann;

R2, R3, R4 gleich oder verschieden Wasserstoff, F, Cl, Br, I, OH, $CF_3$, $NO_2$, CN, $OCHF_2$, $OCF_3$, $SF_5$, O-($C_1$-$C_6$)-Alkyl, O-($C_1$-$C_4$)-Alkoxy-($C_1$-$C_4$)-Alkyl, S-($C_1$-$C_6$)-Alkyl, ($C_1$-$C_6$)-Alkyl, ($C_2$-$C_4$)-Haloalkyl, O-($C_2$-$C_4$)-Haloalkyl, ($C_2$-$C_6$)-Al-

kenyl, ($C_3$-$C_8$)-Cycloalkyl, O-($C_3$-$C_8$)-Cycloalkyl, ($C_3$-$C_8$)-Cycloalkenyl, Aryl, ($C_2$-$C_6$)-Alkinyl, N(R200)(R201), $SO_2$-$CH_3$, COOH, COO-($C_1$-$C_6$)-Alkyl, CON(R202)(R203), N(R204)CO(R205), N(R206)$SO_2$(R207), CO(R208), $(CR209R210)_{z''}$-O(R211), $(CR209R210)_{z''}$-CO-O(R211), O-$(CR209R210)_{z''}$-CO-O(R211), $(CR209R210)_{z''}$-N(R212)(R213), O-$(CR209R210)_{z''}$-N(R212)(R213), $(CR209R210)_{z''}$-CON(R212)(R213), O-$(CR209R210)_{z''}$-CON(R212)(R213), O-CO-N(R212)(R213), O-CO-($C_1$-$C_6$)-Alkylen-CO-O-($C_1$-$C_6$)-Alkyl, O-CO-($C_1$-$C_6$)-Alkylen-CO-OH, O-CO-($C_1$-$C_6$)-Alkylen-CO-N(R212)(R213);

z" unabhängig voneinander 1, 2, 3, 4, 5, 6;

R200, R201, R202, R203, R204R205, R206, R207, R208, R209, R210, R211, R212, R213

gleich oder verschieden Wasserstoff, ($C_1$-$C_6$)-Alkyl;

oder

R200 und R201, R202 und R203, R212 und R213

bilden unabhängig voneinander optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-($C_1$-$C_6$)-Alkyl, Sauerstoff und Schwefel beinhalten kann;
die tautomeren Formen der Verbindungen sowie deren physiologisch verträgliche Salze und N-Oxide;
mit der Maßgabe, dass die Verbindungen mit R2, R3, R4 = Wasserstoff, X = S und R1 = Heptyl, Nonyl oder Cyclohexyl ausgenommen sind.

Die Verbindungen der Formel I zeichnen sich dadurch aus, dass sie gegenüber strukturell ähnlichen Verbindungen mit hemmender Wirkung auf die endotheliale Lipase eine verbesserte Löslichkeit in wässrigen Medien (insbesondere in physiologisch relevanten Puffersystemen) bei gleichzeitiger hoher Aktivität aufweisen. Des Weiteren weisen bevorzugte erfindungsgemäße Verbindungen gegenüber Verbindungen des Standes der Technik eine verbesserte metabolische Stabilität auf. Auch wurde die chemische Stabilität in Blutplasma signifikant verbessert.
Die Alkyl-, Alkenyl- und Alkinylreste in den Substituenten R1 bis R213 können sowohl geradkettig, und/oder verzweigt sein. Dies trifft auch zu, sofern die Alkyl-, Alkenyl- und Alkinylreste Teil einer anderen Gruppe sind, z.B. Teil einer Alkoxygruppe (wie ($C_1$-$C_4$)-Alkoxy-($C_1$-$C_4$)-alkyl)).
Geeignete Halogene sind Fluor, Chlor, Brom und Iod, bevorzugt Fluor, Chlor und Brom, besonders bevorzugt Fluor.

Beispiele für Alkylgruppen sind: Methyl, Ethyl, Propyl, Butyl, Pentyl, Hexyl, Heptyl und Octyl. Dabei sind sowohl die n-Isomere dieser Reste als auch verzweigte Isomere wie Isopropyl, Isobutyl, Isopentyl, sec-Butyl, tert-Butyl, Neopentyl, 3,3-Dimethylbutyl usw. mit umfasst.
Haloalkyl ist ein durch Halogen ein, mehrfach oder vollständig substituiertes Alkyl. Bevorzugte Halogene sind Fluor und Chlor.
Beispiele für mit Halogen substituierte Alkylgruppen sind fluorierte Alkylgruppen wie $CF_3$, $CHF_2$, $CH_2F$, 3-Fluorprop-1-yl, 2,2,1,1-Tetrafluorethyl. Dabei können die zusätzlichen Substituenten in jeder beliebigen Position des Alkylrestes auftreten. Bevorzugt sind die Alkylreste -soweit nicht anders definiert- unsubstituiert.

Unter Cycloalkyl ist im Sinne der vorliegenden Anmeldung Cycloalkyl sowie Cycloalkylalkyl- (Alkyl, das wiederum mit Cycloalkyl substituiert ist) zu verstehen, wobei Cycloalkyl mindestens 3 Kohlenstoffatome aufweist. Beispiele für Cycloalkylreste sind: Cyclopropyl, Cyclobutyl, Cyclopentyl, Cyclohexyl, Cycloheptyl, Cyclooctyl, Cyclononyl und Cyclodecyl. Gegebenenfalls kann es sich auch um polycyclische Ringsysteme handeln, wie Decalinyl, Norbornanyl, Bornanyl oder Adamantanyl. Die Cycloalkylreste können unsubstituiert oder gegebenenfalls mit einem oder mehreren weiteren Resten substituiert sein, wie vorstehend bei den Alkylresten beispielhaft aufgeführt. Bevorzugt sind die Cycloalkylreste -soweit nicht anders definiert- unsubstituiert.

Beispiele für Alkenyl- und Alkinylgruppen sind: Vinyl, 1-Propenyl, 2-Propenyl (Allyl), 2-Butenyl, 2-Methyl-2-propenyl, 3-Methyl-2-butenyl, Ethinyl, 2-Propinyl (Propargyl), 2-Butinyl oder 3-Butinyl.

Unter Cycloalkenyl sind im Sinne der vorliegenden Anmeldung Cycloalkenylreste sowie Cycloalkenyl-Alkylreste (Alkyl, das mit Cycloalkenyl substituiert ist) zu verstehen, die mindestens drei Kohlenstoffatome enthalten. Beispiele für Cycloalkenyl sind: Cyclopentenyl, Cyclohexenyl, Cycloheptenyl und Cyclooctenyl.

Die Alkenylreste und Cycloalkenylreste können ein bis drei konjugierte oder nicht konjugierte Doppelbindungen (also auch Alk-dienyl- sowie Alk-trienylreste), vorzugsweise eine Doppelbindung in einer geraden oder verzweigten Kette aufweisen. Für Alkinylreste gilt das gleiche für die Dreifachbindungen. Die Alkenyl- und Alkinylreste können unsubstituiert oder gegebenenfalls mit einem oder mehreren weiteren Resten substituiert sein, wie vorstehend bei den Alkylresten beispielhaft aufgeführt. Bevorzugt sind die Alkenyl- und Alkinylreste -soweit nicht anders definiertunsubstituiert.

Unter einem Arylrest wird ein Phenyl- oder Naphthylrest verstanden.

Unter einer polycyclischen Gruppe (bi-, tri- oder spirocyclisches Ringgerüst) ist im Sinne der vorliegenden Anmeldung eine Gruppe zu verstehen, die von Spiranen, kondensierten Ringsystemen oder Brücken-Ringsystemen abgeleitet ist. Die Spirane zeichnen sich dadurch aus, dass zwei Ringe nur ein Kohlenstoffatom gemeinsam aufweisen und die Ringebenen der beiden Ringe senkrecht aufeinander stehen. Bei den kondensierten Ringsystemen sind zwei Ringe so miteinander verknüpft, dass sie zwei Atome gemeinsam aufweisen. Bei dieser Art der Verknüpfung handelt es sich um eine "*ortho*-Kondensation". Bei den Brücken-Ringsystemen handelt es sich um Ringsysteme, die eine Brücke aus Kohlenstoff- und/oder Heteroatomen zwischen zwei nicht benachbarten Atomen eines Rings aufweisen.

Beispielhafte "Ringsysteme mit Heteroatomen", "Heterocyclische Ringe" bzw. "Heterocyclische Reste" sind Azocinyl, Benzimidazolyl, Benzofuryl, Benzothienyl, Benzothiophenyl, Benzoxazolyl, Benzthiazolyl, Benztriazolyl, Benztetrazolyl, Benzisoxazolyl, Benzisothiazolyl, Benzimidazalinyl, Carbazolyl, 4aH-Carbazolyl, Carbolinyl, Chinazolinyl, Chinolinyl, 4H-Chinolizinyl, Chinoxalinyl, Chinuclidinyl, Chromanyl, Chromenyl, Cinnolinyl, Decahydrochinolinyl, 2H,6H-1,5,2-Dithiazinyl, Dihydrofuro[2,3-b]-Tetrahydrofuran, Furyl, Furazanyl, Imidazolidinyl, Imidazolinyl, Imidazolyl, 1H-Indazolyl, Indolinyl, Indolizinyl, Indolyl, 3H-Indolyl, Isobenzofuranyl, Isochromanyl, Isoindazolyl, Isoindolinyl, Isoindolyl, Isochinolinyl (Benzimidazolyl), Isothiazolyl, Isoxazolyl, Morpholinyl, Naphthyridinyl, Octahydroisochinolinyl, Oxadiazolyl, 1,2,3-Oxadiazolyl, 1,2,4-Oxadiazolyl, 1,2,5-Oxadiazolyl, 1,3,4-Oxadiazolyl, Oxazolidinyl, Oxazolyl, Oxazolidinyl, Pyrimidinyl, Phenanthridinyl, Phenanthrolinyl, Phenazinyl, Phenothiazinyl, Phenoxathiinyl, Phenoxazinyl, Phthalazinyl, Piperazinyl, Piperidinyl, Pteridinyl, Purynyl, Pyranyl, Pyrazinyl, Pyroazolidinyl, Pyrazolinyl, Pyrazolyl, Pyridazinyl, Pyridooxazole, Pyridoimidazole, Pyridothiazole, Pyridinyl, Pyridyl, Pyrimidinyl, Pyrrolidinyl, Pyrrolinyl, 2H-Pyrrolyl, Pyrrolyl, Tetrahydrofuranyl, Tetrahydroisochinolinyl, Tetrahydrochinolinyl, 6H-1,2,5-Thiadazinyl, Thiazolyl, 1,2,3-Thiadiazolyl, 1,2,4-Thiadiazolyl, 1,2,5-Thiadiazolyl, 1,3,4-Thiadiazolyl, Thienyl, Triazolyl, Tetrazolyl und Xanthenyl.

Pyridyl steht sowohl für 2-, 3- als auch 4-Pyridyl. Thienyl steht sowohl für 2- als auch 3-Thienyl. Furyl steht sowohl für 2- als auch 3-Furyl.

Umfasst sind weiterhin die entsprechenden N-Oxide dieser Verbindungen, also z.B. 1-Oxy-2-, 3- oder 4-pyridyl.

Unter einem "chemisch sinnvollen Rest" ist im Sinne der vorliegenden Erfindung ein Rest zu verstehen, der bei Raumtemperatur und Normaldruck stabil ist. Bevorzugt sind im Sinne der vorliegenden Erfindung unter einem "chemisch sinnvollen Rest" in der Definition der Gruppe A in den Verbindungen der Formel I Gruppen zu verstehen, die keine Heteroatom-Heteroatom-Bindungen zwischen den einzelnen Gliedern der Gruppen aufweisen.

Unter einem "nicht aromatischen" Ring ist im Sinne der vorliegenden Anmeldung bevorzugt ein Ring zu verstehen, der gesättigt oder teilweise ungesättigt ist. Dabei weist ein teilweise ungesättigter Ring gemäß der vorliegenden Anmeldung eine oder gegebenenfalls mehrere Doppelbindungen auf, wobei der teilweise ungesättigte Ring jedoch nicht aromatisch ist. Von dem Ausdruck "nicht aromatisch" sind im Sinne der vorliegenden Anmeldung auch "nicht heteroaromatische" Ringe umfasst.

Die Verbindungen der Formel I können ein oder mehrere Assymmetriezentren enthalten. Daher können die Verbindungen der Formel I in Form ihrer Racemate, Enantiomeren angereicherten Mischungen, reinen Enantiomere, Diastereomere und Diastereomer-Mischungen vorliegen. Die vorliegende Erfindung umfasst alle diese isomeren Formen der Verbindungen der Formel I. Diese Isomeren Formen können, wenn auch zum Teil nicht expressis verbis beschrieben, nach bekannnten Methoden erhalten werden.

Pharmazeutisch verträgliche Salze sind aufgrund ihrer höheren Wasserlöslichkeit gegenüber den Ausgangs- bzw. Basisverbindungen besonders geeignet für medizinische Anwendungen. Diese Salze müssen ein pharmazeutisch verträgliches Anion oder Kation aufweisen. Geeignete pharmazeutisch verträgliche Säureadditionssalze der erfindungsgemäßen Verbindungen sind zum Beispiel Salze anorganischer Säuren sowie organischer Säuren. Solche pharmazeutisch verträgliche Anionen oder Kationen sind in J. Pharm. Sci., Bd. 94, Nr. 10, 2111-2120, 2005 beschrieben.

Salze mit einem nicht pharmazeutisch verträglichen Anion gehören ebenfalls in den Rahmen der Erfindung als nützliche Zwischenprodukte für die Herstellung oder Reinigung pharmazeutisch verträglicher Salze und/oder für die Verwendung in nichttherapeutischen, zum Beispiel in-vitro-Anwendungen.

Der hier verwendete Begriff "physiologisch funktionelles Derivat" bezeichnet jedes physiologisch verträgliche Derivat einer erfindungsgemäßen Verbindung der Formel I, z.B. einen Ester, der bei Verabreichung an einen Säuger, wie z.B. den Menschen, in der Lage ist, (direkt oder indirekt) eine Verbindung der Formel I oder einen aktiven Metaboliten hiervon zu bilden.

Zu den physiologisch funktionellen Derivaten zählen auch Prodrugs der erfindungsgemäßen Verbindungen, wie zum Beispiel in H. Okada et al., Chem. Pharm. Bull. 1994, 42, 57-61 beschrieben. Solche Prodrugs können in vivo zu einer erfindungsgemäßen Verbindung metabolisiert werden. Diese Prodrugs können selbst wirksam sein oder nicht.

Die erfindungsgemäßen Verbindungen können auch in verschiedenen polymorphen Formen vorliegen, z.B. als amorphe und kristalline polymorphe Formen. Alle polymorphen Formen der erfindungsgemäßen Verbindungen gehören in den Rahmen der Erfindung und sind ein weiterer Aspekt der Erfindung.

Nachfolgend beziehen sich alle Verweise auf "Verbindung(en) gemäß Formel I" auf Verbindung(en) der Formel

I, wie vorstehend beschrieben, sowie ihre Salze, Racemate, racemischen Mischungen und reinen Enantiomere sowie auf ihre Diastereomere und Mischungen davon, sowie Solvate und physiologisch funktionelle Derivate wie hierin beschrieben.
Können Reste oder Substituenten mehrfach in den Verbindungen der Formel I auftreten, so können sie alle unabhängig voneinander die angegebenen Bedeutungen haben und gleich oder verschieden sein.

Bevorzugt weisen die Symbole in der Formel I unabhängig voneinander die folgenden Bedeutungen auf:

X ist bevorzugt S .

In einer weiteren Ausführungsform ist

R1 ein Rest der Formel Ia

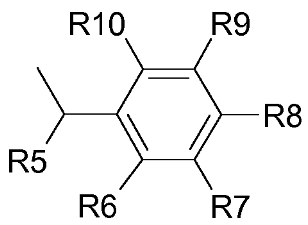

,

worin

R5 Wasserstoff, $(C_1-C_3)$-Alkyl;

R6, R7, R8, R9, R10
unabhängig voneinander Wasserstoff, OH, CN, $OCF_3$, $OCHF_2$, O-$(C_1-C_6)$-Alkyl, O-$(C_1-C_4)$-Alkoxy-$(C_1-C_4)$-alkyl, S-$(C_1-C_6)$-Alkyl, $(C_2-C_4)$-Haloalkyl, O-$(C_2-C_4)$-Haloalkyl, $(C_2-C_6)$-Alkenyl, $(C_2-C_6)$-Alkinyl, N(R11)(R12), $SO_2$-$CH_3$, $SO_2$-N(R13)(R14), $SF_5$, $SCF_3$, COOH, COO-$(C_1-C_6)$-Alkyl, CON(R15)(R16), N(R17)CO(R18), N(R19)$SO_2$(R20), CO(R21), $(CR22R23)_x$-O(R24), $(CR22R23)_x$-CO-OR24), O-$(CR22R23)_x$-CO-O(R24), $(CR22R23)_x$-N(R25)(R26), O-$(CR22R23)_x$-N(R25)(R26), $(CR22R23)_x$-CON(R25)(R26), O-$(CR22R23)_x$-CON(R25)(R26), O-CO-N(R25)(R26), O-CO-$(C_1-C_6)$-Alkylen-CO-O-$(C_1-C_6)$-Alkyl, O-CO-$(C_1-C_6)$-Alkylen-CO-OH, O-CO-$(C_1-C_6)$-Alkylen-CO-N(R27)(R28);
mit der Massgabe dass mindestens ein Rest R6, R7, R8, R9, R10 von Wasserstoff verschieden ist;

x unabhängig voneinander 0, 1, 2, 3, 4, 5, 6;
oder

R7 oder R8 ist
$(O)_y$-$(CH_2-)_{y'}$-$(O)_{y''}$-$(CH_2)_{y'''}$-R100;

y,y" unabhängig voneinander 0,1;

y', y''' unabhängig voneinander 1, 2, 3, 4, 5, 6;

R100 ein 4 bis 10 gliedriger mono- oder bicyclischer gesättigter, teilweise ungesättigter oder aromatischer Ring welcher 1 bis 4 Heteroatome, ausgewählt aus der Gruppe Sauerstoff, Stickstoff oder Schwefel enthalten kann, wobei das Ringsystem zusätzlich ein oder mehrfach durch F, Cl, Br, I, OH, $CF_3$, $NO_2$, CN, $OCF_3$, O-$(C_1-C_6)$-Alkyl, O-$(C_1-C_4)$-Alkoxy-$(C_1-C_4)$-alkyl, S-$(C_1-C_6)$-Alkyl, $(C_1-C_6)$-Alkyl, $(C_2-C_4)$-Haloalkyl, $(C_2-C_6)$-Alkenyl, $(C_3-C_8)$-Cycloalkyl, O-$(C_3-C_8)$-Cycloalkyl, $(C_2-C_6)$-Alkinyl, N(R29)(R30), $SO_2$-$CH_3$, $SF_5$, COOH, COO-$(C_1-C_6)$-Alkyl, CON(R31)(R32), N(R33)CO(R34), N(R35)$SO_2$(R36), CO(R37), $(CR38R39)_{x'}$-O(R40), $(CR38R39)_{x'}$-CO-O(R40), O-$(CR22R23)_{x'}$-CO-O(R40), $(CR22R23)_{x'}$-N(R41)(R42), O-$(CR38R39)_{x'}$-N(R41)(R42), $(CR38R39)_{x'}$-CON(R41)(R42), O-$(CR38R39)_{x'}$-CON(R41)(R42), O-CO-N(R41)(R42), O-CO-$(C_1-C_6)$-Alkylen-CO-O-$(C_1-C_6)$-Alkyl, O-CO-$(C_1-C_6)$-Alkylen-CO-OH, O-CO-$(C_1-C_6)$-Alkylen-CO-N(R43)(R44) substituiert sein kann;

x' 0, 1, 2, 3, 4, 5, 6;

oder

R7 und R8 oder R8 und R9 oder R9 und R10 zusammen mit dem sie tragenden Kohlenstoffatomen bilden ein monocyclisches, 5 bis 7 gliederiges gesättigtes, teilweise ungesättigtes oder aromatisches Ringsystem, dessen einzelne Glieder durch -CHR45-, -CR46R47-, =(C-R46)-, O, N oder S substituiert sein können; mit der Maßgabe, dass zwei Einheiten aus der Reihe -O-, N oder -S- nicht benachbart sein dürfen;

R11, R12, R13, R14, R15, R16, R17, R18, R19, R20, R21, R22, R23, R24, R25, R26, R27, R28, R29, R30, R31, R32, R33, R34, R35, R36, R37, R38, R39, R40, R41, R42, R43, R44

unabhängig voneinander Wasserstoff, ($C_1$-$C_6$)-Alkyl;

bedeuten;
oder

R11 und R12, R13 und R14, R15 und R16, R25 und 26, R27 und R28, R29 und R30, R31 und R32, R41 und 42, R43 und 44
bilden unabhängig voneinander optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-($C_1$-$C_6$)-Alkyl, Sauerstoff und Schwefel beinhalten kann;

R45, R46, R47 gleich oder verschieden, F, Cl, Br, I, OH, $CF_3$, $NO_2$, CN, $OCHF_2$, $OCF_3$, $SF_5$, O-($C_1$-$C_6$)-Alkyl, O-($C_1$-$C_4$)-Alkoxy-($C_1$-$C_4$)-Alkyl, S-($C_1$-$C_6$)-Alkyl, ($C_1$-$C_6$)-Alkyl, ($C_2$-$C_4$)-Haloalkyl, O-($C_2$-$C_4$)-Haloalkyl, ($C_2$-$C_6$)-Alkenyl, ($C_3$-$C_8$)-Cycloalkyl, O-($C_3$-$C_8$)-Cycloalkyl, ($C_2$-$C_6$)-Alkinyl, N(R134)(R135), $SO_2$-$CH_3$, COOH, COO-($C_1$-$C_6$)-Alkyl, CON(R136)(R137), N(R138)CO(R139), N(R140)$SO_2$(R141), CO(R142), $(CR143R144)_{x''}$-O(R145), $(CR143R144)_{x''}$-CO-O(R145), O-$(CR143R144)_{x''}$-CO-O(R145), $(CR143R144)_{x''}$-N(R146)(R147), O-$(CR143R144)_{x''}$-N(R146)(R147), $(CR143R144)_{x''}$-CON(R146)(R147), O-$(CR143R144)_{x''}$-CON(R146)(R147), O-CO-N(R146)(R147), O-CO-($C_1$-$C_6$)-Alkylen-CO-O-($C_1$-$C_6$)-Alkyl, O-CO-($C_1$-$C_6$)-Alkylen-CO-OH, O-CO-($C_1$-$C_6$)-Alkylen-CO-N(R148)(R149);

x" unabhängig voneinander 1, 2, 3, 4, 5, 6;

R134, R135, R136, R137, R138, R139, R140, R141, R142, R143, R144, R145, R146, R147, R148, R149 gleich oder verschieden Wasserstoff, ($C_1$-$C_6$)-Alkyl;

oder R1 ist bevorzugt ein Rest der Formel Ia

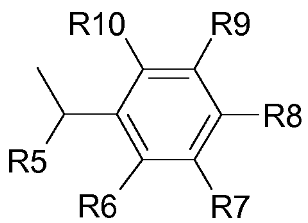

,

worin bedeuten

R5 Wasserstoff, $CH_3$, $CH_2CH_3$;

R6, R7, R8, R9, R10
unabhängig voneinander Wasserstoff, OH, CN, $OCF_3$, $SCF_3$, $OCHF_2$, O-($C_1$-$C_6$)-Alkyl, O-($C_1$-$C_4$)-Alkoxy-($C_1$-$C_4$)-alkyl, S-($C_1$-$C_6$)-Alkyl, N(R11)(R12), $SO_2$-$CH_3$, $SO_2$-N(R13)(R14), COOH, COO-($C_1$-$C_6$)-Alkyl, CO(R21), $(CR22R23)_x$-O(R24), $(CR22R23)_x$-CO-OR24), O-$(CR22R23)_x$-CO-O(R24), $(CR22R23)_x$-N(R25)(R26), O-$(CR22R23)_x$-N(R25)(R26), $(CR22R23)_x$-CON(R25)(R26);

x unabhängig voneinander 0, 1, 2, 3, 4;
oder

R7 oder R8 ist
$(O)_y-(CH_2-)_{y'}-(O)_{y''}-(CH_2)_{y'''}-R100$;

y,y" unabhängig voneinander 0,1;

y', y''' unabhängig voneinander 1, 2, 3, 4, 5, 6;

R100 ein 4 bis 10 gliedriger mono- oder bicyclischer gesättigter, teilweise ungesättigter oder aromatischer Ring welcher 1 bis 4 Heteroatome, ausgewählt aus der Gruppe Sauerstoff, Stickstoff oder Schwefel enthalten kann, wobei das Ringsystem zusätzlich ein oder mehrfach durch F, Cl, Br, I, OH, $CF_3$, $NO_2$, CN, $OCF_3$, O-($C_1$-$C_6$)-Alkyl, O-($C_1$-$C_4$)-Alkoxy-($C_1$-$C_4$)-alkyl, S-($C_1$-$C_6$)-Alkyl, ($C_1$-$C_6$)-Alkyl, ($C_2$-$C_4$)-Haloalkyl, ($C_2$-$C_6$)-Alkenyl, ($C_2$-$C_6$)-Alkinyl, N(R29)(R30), $SO_2$-$CH_3$, $SF_5$, COOH, COO-($C_1$-$C_6$)-Alkyl, CON(R31)(R32), N(R33)CO(R34), N(R35)$SO_2$(R36), CO(R37), $(CR38R39)_x$-O(R40), $(CR38R39)_{x'}$-CO-O(R40), O-$(CR22R23)_{x'}$-CO-O(R40), $(CR22R23)_{x'}$-N(R41)(R42), O-$(CR38R39)_{x'}$-N(R41)(R42), $(CR38R39)_{x'}$-CON(R41)(R42), O-$(CR38R39)_{x'}$-CON(R41)(R42), O-CO-N(R41)(R42), O-CO-($C_1$-$C_6$)-Alkylen-CO-O-($C_1$-$C_6$)-Alkyl, O-CO-($C_1$-$C_6$)-Alkylen-CO-OH, O-CO-($C_1$-$C_6$)-Alkylen-CO-N(R43)(R44) substituiert sein kann;

x' unabhängig voneinander 1, 2, 3, 4, 5, 6;
oder

R7 und R8 oder R8 und R9 oder R9 und R10

zusammen mit dem sie tragenden Kohlenstoffatomen bilden ein monocyclisches, 5 bis 7 gliederiges gesättigtes, teilweise ungesättigtes oder aromatisches Ringsystem, dessen einzelne Glieder durch -CHR45-, -CR46R47-, =(C-R46)-, O, N oder S substituiert sein können; mit der Maßgabe, dass zwei Einheiten aus der Reihe -O-, N oder -S- nicht benachbart sein dürfen;

R11, R12, R13, R14, R15, R16, R17, R18, R20, R21, R22, R23, R24, R25, R26, R27, R28, R29, R30, R31, R32, R33, R34, R35, R36, R37, R38, R39, R40, R41, R42, R43, R44

unabhängig voneinander Wasserstoff, ($C_1$-$C_6$)-Alkyl;

bedeuten;
oder

R11 und R12, R13 und R14, R15 und R16, R25 und 26, R27 und R28, R29 und R30, R31 und R32, R41 und 42, R43 und 44
bilden unabhängig voneinander optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-($C_1$-$C_6$)-Alkyl, Sauerstoff und Schwefel beinhalten kann;

R45, R46, R47 gleich oder verschieden, F, Cl, Br, I, OH, $CF_3$, $OCHF_2$, $OCF_3$, $SF_5$, O-($C_1$-$C_6$)-Alkyl, O-($C_1$-$C_4$)-Alkoxy-($C_1$-$C_4$)-Alkyl, S-($C_1$-$C_6$)-Alkyl, ($C_1$-$C_6$)-Alkyl, $SO_2$-$CH_3$, COOH, COO-($C_1$-$C_6$)-Alkyl, CO(R142), $(CR143R144)_{x''}$-O(R145), $(CR143R144)_{x''}$-CO-O(R145), O-$(CR143R144)_{x''}$-CO-O(R145), $(CR143R144)_{x''}$-N(R146)(R147), O-$(CR143R144)_{x''}$-N(R146)(R147), $(CR143R144)_{x''}$-CON(R146)(R147), O-$(CR143R144)_{x''}$-CON(R146)(R147),;

x" unabhängig voneinander 1, 2, 3, 4;

R134, R135, R136, R137, R138, R139, R140, R141, R142, R143, R144, R145, R146, R147, R148, R149 gleich oder verschieden Wasserstoff, ($C_1$-$C_6$)-Alkyl;

besonders bevorzugt ist ein Rest der Formel Ia

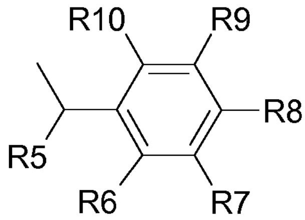

,

worin bedeuten

R5 Wasserstoff, $CH_3$;

R6, R7, R8, R9, R10
unabhängig voneinander Wasserstoff, $OCF_3$, $SCF_3$, $OCHF_2$, O-($C_1$-$C_6$)-Alkyl, O-($C_1$-$C_4$)-Alkoxy-($C_1$-$C_4$)-alkyl, S-($C_1$-$C_6$)-Alkyl, $SO_2$-$CH_3$, $SO_2$-N(R13)(R14), COOH, COO-($C_1$-$C_6$)-Alkyl, $(CR22R23)_x$-O(R24), $(CR22R23)_x$-CO-OR24), O-$(CR22R23)_x$-CO-O(R24), $(CR22R23)_x$-N(R25)(R26), O-$(CR22R23)_x$-N(R25)(R26), $(CR22R23)_x$-CON(R25)(R26);

x unabhängig voneinander 0, 1, 2, 3, 4;
oder

R7 oder R8 ist

R100, -$CH_2$-R100, -$OCH_2$-R100, -$CH_2$-O-R100, -$CH_2$-O-$CH_2$-R100 oder-O-$CH_2CH_2$-R100;

R100 ein 4 bis 7 gliedriger monocyclischer gesättigter, teilweise ungesättigter oder aromatischer Ring welcher 1 bis 3 Heteroatome, ausgewählt aus der Gruppe Sauerstoff, Stickstoff oder Schwefel enthalten kann, wobei das Ringsystem zusätzlich ein oder mehrfach durch F, Cl, Br, OH, $CF_3$, $OCF_3$, O-($C_1$-$C_6$)-Alkyl, O-($C_1$-$C_4$)-Alkoxy-($C_1$-$C_4$)-alkyl, S-($C_1$-$C_6$)-Alkyl, ($C_1$-$C_6$)-Alkyl, $SO_2$-$CH_3$, COOH, COO-($C_1$-$C_6$)-Alkyl, N(R35)$SO_2$(R36), CO(R37), $(CR38R39)_{x'}$-O(R40), $(CR38R39)_{x'}$-CO-O(R40), O-$(CR22R23)_{x'}$-CO-O(R40), $(CR22R23)_{x'}$-N(R41)(R42), O-$(CR38R39)_{x'}$-N(R41)(R42), $(CR38R39)_{x'}$-CON(R41)(R42), O-$(CR38R39)_{x'}$-CON(R41)(R42) substituiert sein kann;

x' unabhängig voneinander 1, 2, 3, 4;
oder

R7 und R8 oder R8 und R9 oder R9 und R10
zusammen mit dem sie tragenden Kohlenstoffatomen bilden -O-$CH_2$-O-, -O-$CH_2$-$CH_2$-O-, -O-$CH_2$-$CH_2$-$CH_2$-O-, -O-$CF_2$-O- oder-N($CH_3$)-N=N-;

R11, R12, R13, R14, R15, R16, R17, R18, R19, R20, R21, R22, R23, R24, R25, R26, R27, R28, R29, R30, R31, R32, R33, R34, R35, R36, R37, R38, R39, R40, R41, R42

unabhängig voneinander Wasserstoff, ($C_1$-$C_6$)-Alkyl;

bedeuten;
oder

R11 und R12, R13 und R14, R15 und R16, R25 und 26, R27 und R28, R29 und R30, R31 und R32, R41 und 42, R43 und 44 bilden unabhängig voneinander optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-($C_1$-$C_6$)-Alkyl, Sauerstoff und Schwefel beinhalten kann.

In einer weiteren Ausführungsform ist

R1 besonders bevorzugt ein Rest der Formel Ia

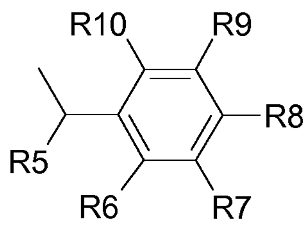

,

worin bedeuten

R5 Wasserstoff, $CH_3$,$CH_2CH_3$;

R6, R7, R8, R9, R10
unabhängig voneinander Wasserstoff, OH, CN, $OCF_3$, $SCF_3$, $OCHF_2$, O-($C_1$-$C_6$)-Alkyl, O-($C_1$-$C_4$)-Alkoxy-($C_1$-$C_4$)-alkyl, S-($C_1$-$C_6$)-Alkyl, N(R11)(R12), $SO_2$-$CH_3$, $SO_2$-N(R13)(R14), COOH, COO-($C_1$-$C_6$)-Alkyl, CO(R21), $(CR22R23)_x$-O(R24), $(CR22R23)_x$-CO-OR24), O-$(CR22R23)_x$-CO-O(R24), $(CR22R23)_x$-N(R25)(R26), O-$(CR22R23)_x$-N(R25)(R26), $(CR22R23)_x$-CON(R25)(R26);
mit der Massgabe dass mindestens ein Rest R6, R7, R8, R9, R10 von Wasserstoff verschieden ist;

x unabhängig voneinander 0, 1, 2, 3, 4;
oder

R7 oder R8 ist
$(O)_y$-$(CH_2-)_{y'}$-$(O)_{y''}$-$(CH_2)_{y'''}$-R100;

y,y" unabhängig voneinander 0,1;

y', y''' unabhängig voneinander 1, 2, 3, 4, 5, 6;

R100 ein 4 bis 10 gliedriger mono- oder bicyclischer gesättigter, teilweise ungesättigter oder aromatischer Ring welcher 1 bis 4 Heteroatome, ausgewählt aus der Gruppe Sauerstoff, Stickstoff oder Schwefel enthalten kann, wobei das Ringsystem zusätzlich ein oder mehrfach durch F, Cl, Br, I, OH, $CF_3$, $NO_2$, CN, $OCF_3$, O-($C_1$-$C_6$)-Alkyl, O-($C_1$-$C_4$)-Alkoxy-($C_1$-$C_4$)-alkyl, S-($C_1$-$C_6$)-Alkyl, ($C_1$-$C_6$)-Alkyl, ($C_2$-$C_4$)-Haloalkyl, ($C_2$-$C_6$)-Alkenyl, ($C_2$-$C_6$)-Alkinyl, N(R29)(R30), $SO_2$-$CH_3$, $SF_5$, COOH, COO-($C_1$-$C_6$)-Alkyl, CON(R31)(R32), N(R33)CO(R34), N(R35)$SO_2$(R36), CO(R37), $(CR38R39)_{x'}$-O(R40), $(CR38R39)_{x'}$-CO-O(R40), O-$(CR22R23)_{x'}$-CO-O(R40), $(CR22R23)_{x'}$-N(R41)(R42), O-$(CR38R39)_{x'}$-N(R41)(R42), $(CR38R39)_{x'}$-CON(R41)(R42), O-$(CR38R39)_{x'}$-CON(R41)(R42), O-CO-N(R41)(R42), O-CO-($C_1$-$C_6$)-Alkylen-CO-O-($C_1$-$C_6$)-Alkyl, O-CO-($C_1$-$C_6$)-Alkylen-CO-OH, O-CO-($C_1$-$C_6$)-Alkylen-CO-N(R43)(R44) substituiert sein kann;

x' unabhängig voneinander 1, 2, 3, 4, 5, 6;
oder

R7 und R8 oder R8 und R9 oder R9 und R10
zusammen mit dem sie tragenden Kohlenstoffatomen bilden ein monocyclisches, 5 bis 7 gliederiges gesättigtes, teilweise ungesättigtes oder aromatisches Ringsystem, dessen einzelne Glieder durch -CHR45-, -CR46R47-, =(C-R46)-, O, N oder S substituiert sein können; mit der Maßgabe, dass zwei Einheiten aus der Reihe -O-, N oder -S- nicht benachbart sein dürfen;

R11, R12, R13, R14, R15, R16, R17, R18, R19, R20, R21, R22, R23, R24, R25, R26, R27, R28, R29, R30, R31, R32, R33, R34, R35, R36, R37, R38, R39, R40, R41, R42, R43, R44

unabhängig voneinander Wasserstoff, ($C_1$-$C_6$)-Alkyl,

bedeuten;
oder

R11 und R12, R13 und R14, R15 und R16, R25 und 26, R27 und R28, R29 und R30, R31 und R32, R41 und

42, R43 und 44 bilden unabhängig voneinander optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-($C_1$-$C_6$)-Alkyl, Sauerstoff und Schwefel beinhalten kann;

R45, R46, R47 gleich oder verschieden, F, Cl, Br, I, OH, $CF_3$, $OCHF_2$, $OCF_3$, $SF_5$, O-($C_1$-$C_6$)-Alkyl, O-($C_1$-$C_4$)-Alkoxy-($C_1$-$C_4$)-Alkyl, S-($C_1$-$C_6$)-Alkyl, ($C_1$-$C_6$)-Alkyl, $SO_2$-$CH_3$, COOH, COO-($C_1$-$C_6$)-Alkyl, CO(R142), $(CR143R144)_{x''}$-O(R145), $(CR143R144)_{x''}$-CO-O(R145), O-$(CR143R144)_{x''}$-CO-O(R145), $(CR143R144)_{x''}$-N(R146)(R147), O-$(CR143R144)_{x''}$-N(R146)(R147), $(CR143R144)_{x''}$-CON(R146)(R147), O-$(CR143R144)_{x''}$-CON(R146)(R147),;

x'' unabhängig voneinander 1, 2, 3, 4;

R134, R135, R136, R137, R138, R139, R140, R141, R142, R143, R144, R145, R146, R147, R148, R149 gleich oder verschieden Wasserstoff, ($C_1$-$C_6$)-Alkyl,

ganz besonders bevorzugt ist ein Rest der Formel Ia

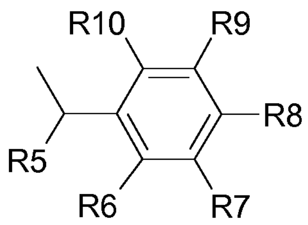

,

worin bedeuten

R5 Wasserstoff, $CH_3$;

R6, R7, R8, R9, R10
unabhängig voneinander Wasserstoff, $OCF_3$, $SCF_3$, $OCHF_2$, O-($C_1$-$C_6$)-Alkyl, O-($C_1$-$C_4$)-Alkoxy-($C_1$-$C_4$)-alkyl, S-($C_1$-$C_6$)-Alkyl, $SO_2$-$CH_3$, $SO_2$-N(R13)(R14), COOH, COO-($C_1$-$C_6$)-Alkyl, $(CR22R23)_x$-O(R24), $(CR22R23)_x$-CO-OR24), O-$(CR22R23)_x$-CO-O(R24), $(CR22R23)_x$-N(R25)(R26), O-$(CR22R23)_x$-N(R25)(R26), $(CR22R23)_x$-CON(R25)(R26);
mit der Massgabe dass mindestens ein Rest R6, R7, R8, R9, R10 von Wasserstoff verschieden ist;

x unabhängig voneinander 0, 1, 2, 3, 4;
oder

R7 oder R8 ist

R100, -$CH_2$-R100, -$OCH_2$-R100, -$CH_2$-O-R100, -$CH_2$-O-$CH_2$-R100 oder-O-$CH_2CH_2$-R100;

R100 ein 4 bis 7 gliedriger monocyclischer gesättigter, teilweise ungesättigter oder aromatischer Ring welcher 1 bis 3 Heteroatome, ausgewählt aus der Gruppe Sauerstoff, Stickstoff oder Schwefel enthalten kann, wobei das Ringsystem zusätzlich ein oder mehrfach durch F, Cl, Br, OH, $CF_3$, $OCF_3$, O-($C_1$-$C_6$)-Alkyl, O-($C_1$-$C_4$)-Alkoxy-($C_1$-$C_4$)-alkyl, S-($C_1$-$C_6$)-Alkyl, ($C_1$-$C_6$)-Alkyl, $SO_2$-$CH_3$, COOH, COO-($C_1$-$C_6$)-Alkyl, N(R35)$SO_2$(R36), CO(R37), $(CR38R39)_{x'}$-O(R40), $(CR38R39)_{x'}$-CO-O(R40), O-$(CR22R23)_{x'}$-CO-O(R40), $(CR22R23)_{x'}$-N(R41)(R42), O-$(CR38R39)_{x'}$-N(R41)(R42), $(CR38R39)_{x'}$-CON(R41)(R42), O-$(CR38R39)_{x'}$-CON(R41)(R42) substituiert sein kann;

x' unabhängig voneinander 1, 2, 3, 4;
oder

R7 und R8 oder R8 und R9 oder R9 und R10 zusammen mit dem sie tragenden Kohlenstoffatomen bilden -O-$CH_2$-O-, -O-$CH_2$-$CH_2$-O-, -O-$CH_2$-$CH_2$-$CH_2$-O-, -O-$CF_2$-O- oder-N($CH_3$)-N=N-;

R11, R12, R13, R14, R15, R16, R17, R18, R20, R21, R22, R23, R24, R25, R26, R27, R28, R29, R30, R31, R32, R33, R34, R35, R36, R37, R38, R39, R40, R41, R42

unabhängig voneinander Wasserstoff, ($C_1$-$C_6$)-Alkyl;

bedeuten;
oder

R11 und R12, R13 und R14, R15 und R16, R25 und 26, R27 und R28, R29 und R30, R31 und R32, R41 und 42, R43 und 44 bilden unabhängig voneinander optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-($C_1$-$C_6$)-Alkyl, Sauerstoff und Schwefel beinhalten kann.

In einer weiteren Ausführungsform ist

R1 bevorzugt ein Rest der Formel Ib

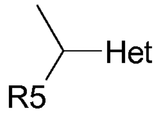

Ib,

worin bedeuten:

R5 Wasserstoff, $CH_3$, $CH_2CH_3$;

;

Het ein 4 bis 10 gliedriger mono oder bicyclischer aromatischer Ring welcher 1 bis 4 Heteroatome, ausgewählt aus der Gruppe Sauerstoff, Stickstoff oder Schwefel enthält, wobei das Ringsystem zusätzlich ein oder mehrfach durch unabhängig voneinander F, Cl, Br, I, OH, $CF_3$, $NO_2$, CN, $OCF_3$, $OCHF_2$, O-($C_1$-$C_6$)-Alkyl, O-($C_1$-$C_4$)-Alkoxy-($C_1$-$C_4$)-alkyl, S-($C_1$-$C_6$)-Alkyl, ($C_1$-$C_6$)-Alkyl, , ($C_2$-$C_6$)-Alkenyl, N(R48)(R49), $SO_2$-$CH_3$, $SO_2$-N(R50)(R51), $SCF_3$, COOH, COO-($C_1$-$C_6$)-Alkyl, N(R54)CO(R55), N(R56)$SO_2$(R57), CO(R58), $(CR59R60)_{x'''}$-O(R61), $(CR59R60)_{x'''}$-CO-O(R61), O-$(CR59R60)_{x'''}$-CO-O(R61), $(CR59R60)_{x'''}$-N(R62)(R63), O-$(CR59R60)_{x'''}$-N(R62)(R63), $(CR59R60)_{x'''}$-CON(R62)(R63), O-$(CR59R60)_{x'''}$-CON(R62)(R63), O-CO-N(R62)(R63), O-CO-($C_1$-$C_6$)-Alkylen-CO-O-($C_1$-$C_6$)-Alkyl, O-CO-($C_1$-$C_6$)-Alkylen-CO-OH, O-CO-($C_1$-$C_6$)-Alkylen-CO-N(R64)(R65); oder $(O)_y$-$(CH_2-)_{y'}$-$(O)_{y''}$-$(CH_2)_{y'''}$-R101 substituiert sein kann,

x''' unabhängig voneinander 1, 2, 3, 4;

y,y" unabhängig voneinander 0,1;

y', y''' unabhängig voneinander 0, 1, 2, 3, 4;

R101 ein 4 bis 10 gliedriger mono oder bicyclischer gesättigter, teilweise ungesättigter oder aromatischer Ring welcher 1 bis 4 Heteroatome, ausgewählt aus der Gruppe Sauerstoff, Stickstoff oder Schwefel enthalten kann, wobei das Ringsystem zusätzlich ein oder mehrfach durch, F, Cl, Br, I, OH, $CF_3$, $NO_2$, CN, $OCHF_2$, $OCF_3$, O-($C_1$-$C_6$)-Alkyl, O-($C_1$-$C_4$)-Alkoxy-($C_1$-$C_4$)-alkyl, S-($C_1$-$C_6$)-Alkyl, ($C_1$-$C_6$)-Alkyl, ($C_2$-$C_4$)-Haloalkyl, ($C_2$-$C_6$)-Alkenyl, ($C_3$-$C_8$)-Cycloalkyl, O-($C_3$-$C_8$)-Cycloalkyl, ($C_2$-$C_6$)-Alkinyl, N(R66)(R67), $SO_2$-$CH_3$, $SF_5$, COOH, COO-($C_1$-$C_6$)-Alkyl, CON(R68)(R69), (C), N(R70)CO(R71), N(R72)$SO_2$(R73), CO(R74), $(CR75R76)_{x''''}$-O$(R77R75R76)_{x''''}$-CO-O(R77), O-$(CR75R76)_{x''''}$-CO-O(R77), $(CR75R76)_{x''''}$-N(R78)(R79), O-$(CR75R76)_{x''''}$-N(R78)(R79), $(CR75R76)_{x''''}$-CON(R78)(R79), O-$(CR75R76)_{x''''}$-CON(R78)(R79), O-CO-N(R78)(R79), O-CO-($C_1$-$C_6$)-Alkylen-CO-O-($C_1$-$C_6$)-Alkyl, O-CO-($C_1$-$C_6$)-Alkylen-CO-OH, O-CO-($C_1$-$C_6$)-Alkylen-CO-N(R80)(R81) substituiert sein kann;

x'''' unabhängig voneinander 1, 2, 3, 4, 5, 6;

R48, R49, R50, R51, R52, R53, R54, R55, R56, R57, R58, R59, R60, R61, R62, R63, R64, R65, R66, R67, R68, R69, R70, R71, R72, R73, R74, R75, R76, R77, R78, R79, R80, R81

unabhängig voneinander Wasserstoff, ($C_1$-$C_6$)-Alkyl;

oder

R48 und R49, R50 und R51, R52 und R53, R62 und R63, R64 und R65, R66 und R67, R68 und R69, R78 und R79, R80 und R81 bilden unabhängig voneinander optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-($C_1$-$C_6$)-Alkyl, Sauerstoff und Schwefel beinhalten kann;

besonders bevorzugt ist ein ein Rest der Formel Ib

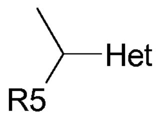

Ib,

worin bedeuten:

R5 Wasserstoff, $CH_3$; ;

Het ausgewählt ist aus der Gruppe Pyridin, Pyrazol, Imidazol, Oxazol, Oxadiazol, Benzothiophen, Imidazo[2,1,b]thiazol, wobei Het zusätzlich ein oder mehrfach durch unabhängig voneinander F, Cl, Br, $CF_3$, $OCF_3$, $OCHF_2$, O-($C_1$-$C_6$)-Alkyl, O-($C_1$-$C_4$)-Alkoxy-($C_1$-$C_4$)-alkyl, S-($C_1$-$C_6$)-Alkyl, ($C_1$-$C_6$)-Alkyl, ($C_2$-$C_6$)-Alkenyl, $SO_2$-$CH_3$, $SO_2$-N(R50)(R51), COOH, COO-($C_1$-$C_6$)-Alkyl, N(R56)$SO_2$(R57), CO(R58), $(CR59R60)_{x'''}$-O(R61), $(CR59R60)_{x'''}$-CO-O(R61), O-$(CR59R60)_{x'''}$-CO-O(R61), $(CR59R60)_{x'''}$-N(R62)(R63), O-$(CR59R60)_{x'''}$-N(R62)(R63), $(CR59R60)_{x'''}$-CON(R62)(R63), O-$(CR59R60)_{x'''}$-CON(R62)(R63), O-CO-N(R62)(R63), O-CO-($C_1$-$C_6$)-Alkylen-CO-O-($C_1$-$C_6$)-Alkyl, O-CO-($C_1$-$C_6$)-Alkylen-CO-OH, O-CO-($C_1$-$C_6$)-Alkylen-CO-N(R64)(R65); oder $(O)_y$-$(CH_2$-$)_{y'}$-$(O)_{y''}$-$(CH_2)_{y'''}$-R101 substituiert sein kann,

x''' unabhängig voneinander 1, 2, 3, 4;

y,y" unabhängig voneinander 0,1;

y', y''' unabhängig voneinander 0, 1, 2;

R101 ein 4 bis 10 gliedriger mono oder bicyclischer gesättigter, teilweise ungesättigter oder aromatischer Ring welcher 1 bis 4 Heteroatome, ausgewählt aus der Gruppe Sauerstoff, Stickstoff oder Schwefel enthalten kann, wobei das Ringsystem zusätzlich ein oder mehrfach durch, F, Cl, Br, I, OH, $CF_3$, $NO_2$, CN, $OCHF_2$, $OCF_3$, O-($C_1$-$C_6$)-Alkyl, O-($C_1$-$C_4$)-Alkoxy-($C_1$-$C_4$)-alkyl, S-($C_1$-$C_6$)-Alkyl, ($C_1$-$C_6$)-Alkyl, ($C_2$-$C_4$)-Haloalkyl, ($C_2$-$C_6$)-Alkenyl, ($C_3$-$C_8$)-Cycloalkyl, O-($C_3$-$C_8$)-Cycloalkyl, ($C_2$-$C_6$)-Alkinyl, N(R66)(R67), $SO_2$-$CH_3$, $SF_5$, COOH, COO-($C_1$-$C_6$)-Alkyl, CON(R68)(R69), (C), N(R70)CO(R71), N(R72)$SO_2$(R73), CO(R74), $(CR75R76)_{x''''}$-O$(R77R75R76)_{x''''}$-CO-O(R77), O-$(CR75R76)_{x''''}$-CO-O(R77), $(CR75R76)_{x''''}$-N(R78)(R79), O-$(CR75R76)_{x''''}$-N(R78)(R79), $(CR75R76)_{x''''}$-CON(R78)(R79), O-$(CR75R76)_{x''''}$-CON(R78)(R79), O-CO-N(R78)(R79), O-CO-($C_1$-$C_6$)-Alkylen-CO-O-($C_1$-$C_6$)-Alkyl, O-CO-($C_1$-$C_6$)-Alkylen-CO-OH, O-CO-($C_1$-$C_6$)-Alkylen-CO-N(R80)(R81) substituiert sein kann;

x'''' unabhängig voneinander 1, 2, 3, 4, 5, 6;

R48, R49, R50, R51, R52, R53, R54, R55, R56, R57, R58, R59, R60, R61, R62, R63, R64, R65, R66, R67, R68, R69, R70, R71, R72, R73, R74, R75, R76, R77, R78, R79, R80, R81

unabhängig voneinander Wasserstoff, ($C_1$-$C_6$)-Alkyl;

oder

R48 und R49, R50 und R51, R52 und R53, R62 und R63, R64 und R65, R66 und R67, R68 und R69, R78 und R79, R80 und R81
bilden unabhängig voneinander optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-($C_1$-$C_6$)-Alkyl, Sauerstoff und Schwefel beinhalten kann.

In einer weiteren Ausführungsform ist

R1 bevorzugt ein Rest der Formel Ic

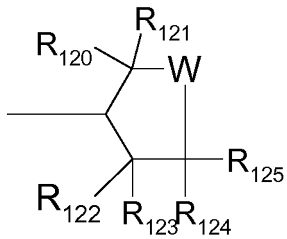
Ic,

worin

W, -C(R126)(R127)-, -C(R126)(R127)-C(R128)(R129)-, -C(R126)(R127)-O-;

R120, R121 ,R123, R125, R126, R127, R128, R129

gleich oder verschieden Wasserstoff, F, Cl, Br, I, OH, $CF_3$, $NO_2$, CN, $OCHF_2$, $OCF_3$, $SF_5$, O-($C_1$-$C_6$)-Alkyl, O-($C_1$-$C_4$)-Alkoxy-($C_1$-$C_4$)-alkyl, S-($C_1$-$C_6$)-Alkyl, ($C_1$-$C_6$)-Alkyl, ($C_2$-$C_4$)-Haloalkyl, O-($C_2$-$C_4$)-Haloalkyl, ($C_2$-$C_6$)-Alkenyl, ($C_3$-$C_8$)-Cycloalkenyl, ($C_2$-$C_6$)-Alkinyl, N(R90)(R91), $SO_2$-$CH_3$, COOH, COO-($C_1$-$C_6$)-Alkyl, CON(R92)(R93), N(R94)CO(R95), N(R96)$SO_2$(R97), CO(R98), $(CR99R102)_z$-O(R103), $(CR99R76)_z$-CO-O(R103), O-$(CR99R102)_z$-CO-O(R103), $(CR99R102)_z$-N(R104)(R105), O-$(CR99R102)_z$-N(R104)(R105), $(CR99R102)_z$-CON(R104)(R105), O-$(CR99R102)_z$-CON(R104)(R105), O-CO-N(R104)(R105), O-CO-($C_1$-$C_6$)-Alkylen-CO-O-($C_1$-$C_6$)-Alkyl, O-CO-($C_1$-$C_6$)-Alkylen-CO-OH, O-CO-($C_1$-$C_6$)-Alkylen-CO-N(R106)(R107);

z unabhängig voneinander 1, 2, 3, 4, 5, 6;

R90, R91, R92, R93, R94, R95, R96, R97, R98, R99, R102, R103, R104, R105, R106, R107

gleich oder verschieden Wasserstoff, ($C_1$-$C_6$)-Alkyl,

R122 und R124, oder R123 und R125 zusammen mit dem sie tragenden Kohlenstoffatomen bilden ein monocyclisches, 5 oder 6 gliederiges gesättigtes, teilweise ungesättigtes oder ein aromatisches Ringsystem, dessen einzelne Glieder durch -CHR130-, -CR131R132-, =(C-R133)- substituiert sein können;

R130, R131, R132, R133 gleich oder verschieden, F, Cl, Br, I, OH, $CF_3$, $NO_2$, CN, $OCHF_2$, $OCF_3$, $SF_5$, O-($C_1$-$C_6$)-Alkyl, O-($C_1$-$C_4$)-Alkoxy-($C_1$-$C_4$)-Alkyl, S-($C_1$-$C_6$)-Alkyl, ($C_1$-$C_6$)-Alkyl, ($C_2$-$C_4$)-Haloalkyl, O-($C_2$-$C_4$)-Haloalkyl, ($C_2$-$C_6$)-Alkenyl, ($C_2$-$C_6$)-Alkinyl, N(R160)(R161), $SO_2$-$CH_3$, COOH, COO-($C_1$-$C_6$)-Alkyl, CON(R162)(R163), N(R164)CO(R165), N(R166)$SO_2$(R167), CO(R168), $(CR169R170)_{z'}$-O(R171), $(CR169R170)_{z'}$-CO-O(R77), O-$(CR169R170)_{z'}$-CO-O(R171), $(CR169R170)_{z'}$-N(R172)(R173), O-$(CR169R170)_{z'}$-N(R172)(R173), $(CR169R170)_{z'}$-CON(R172)(R173), O-$(CR169R170)_{z'}$-CON(R172)(R173), O-CO-N(R172)(R173), O-CO-($C_1$-$C_6$)-Alkylen-CO-O-($C_1$-$C_6$)-Alkyl, O-CO-($C_1$-$C_6$)-Alkylen-CO-OH, O-CO-($C_1$-$C_6$)-Alkylen-CO-N(R172)(R173);

z' unabhängig voneinander 1, 2, 3, 4, 5, 6;

R160, R161, R162, R163, R164, R165, R166, R167, R168, R169, R170, R171, R172, R173

gleich oder verschieden Wasserstoff, $(C_1-C_6)$-Alkyl;

oder

R160 und R161, R162 und R163, R172 und R173 bilden unabhängig voneinander optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-$(C_1-C_6)$-Alkyl, Sauerstoff und Schwefel beinhalten kann;

besonders bevorzugt ein Rest der Formel Ic:

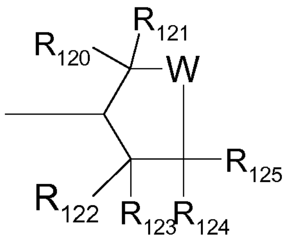

Ic,

worin bedeuten

W, -C(R126)(R127)-, -C(R126)(R127)-C(R128)(R129)-, -C(R126)(R127)-O-;

R120, R121, R123, R125, R126, R127, R128, R129
R120, R121, R126, R127, R128, R129 gleich oder verschieden Wasserstoff, $CH_3$, oxo; gleich oder verschieden Wasserstoff, F, Cl, Br, I, OH, $CF_3$, $NO_2$, CN, $OCHF_2$, $OCF_3$, $SF_5$, O-$(C_1-C_6)$-Alkyl, O-$(C_1-C_4)$-Alkoxy-$(C_1-C_4)$-alkyl, S-$(C_1-C_6)$-Alkyl, $(C_1-C_6)$-Alkyl, $(C_2-C_4)$-Haloalkyl, O-$(C_2-C_4)$-Haloalkyl, $(C_2-C_6)$-Alkenyl, $(C_3-C_8)$-Cycloalkenyl, $(C_2-C_6)$-Alkinyl, N(R90)(R91), $SO_2$-$CH_3$, COOH, COO-$(C_1-C_6)$-Alkyl, CON(R92)(R93), N(R94)CO(R95), N(R96)$SO_2$(R97), CO(R98), $(CR99R102)_z$-O(R103), $(CR99R76)_z$-CO-O(R103), O-$(CR99R102)_z$-CO-O(R103), $(CR99R102)_z$-N(R104)(R105), O-$(CR99R102)_z$-N(R104)(R105), $(CR99R102)_z$-CON(R104)(R105), O-$(CR99R102)_z$-CON(R104)(R105), O-CO-N(R104)(R105), O-CO-$(C_1-C_6)$-Alkylen-CO-O-$(C_1-C_6)$-Alkyl, O-CO-$(C_1-C_6)$-Alkylen-CO-OH, O-CO-$(C_1-C_6)$-Alkylen-CO-N(R106)(R107);

z unabhängig voneinander 1, 2, 3, 4, 5, 6;

R90, R91, R92, R93, R94, R95, R96, R97, R98, R99, R102, R103, R104, R105, R106, R107

gleich oder verschieden Wasserstoff, $(C_1-C_6)$-Alkyl,

R122 und R124 zusammen mit dem sie tragenden Kohlenstoffatomen bilden ein monocyclisches, 6 gliederiges aromatisches Ringsystem, dessen einzelne Glieder durch =(C-R133)- substituiert sein können;

R133 gleich oder verschieden, F, Cl, Br, I, OH, $CF_3$, $NO_2$, CN, $OCHF_2$, $OCF_3$, $SF_5$, O-$(C_1-C_6)$-Alkyl, O-$(C_1-C_4)$-Alkoxy-$(C_1-C_4)$-Alkyl, S-$(C_1-C_6)$-Alkyl, $(C_1-C_6)$-Alkyl, $(C_2-C_4)$-Haloalkyl, O-$(C_2-C_4)$-Haloalkyl, $(C_2-C_6)$-Alkenyl, $(C_2-C_6)$-Alkinyl, N(R160)(R161), $SO_2$-$CH_3$, COOH, COO-$(C_1-C_6)$-Alkyl, CON(R162)(R163), N(R164)CO(R165), N(R166)$SO_2$(R167), CO(R168), $(CR169R170)_{z'}$-O(R171), $(CR169R170)_{z'}$-CO-O(R77), O-$(CR169R170)_{z'}$-CO-O(R171), $(CR169R170)_{z'}$-N(R172)(R173), O-$(CR169R170)_{z'}$-N(R172)(R173), $(CR169R170)_{z'}$-CON(R172)(R173), O-$(CR169R170)_{z'}$-CON(R172)(R173), O-CO-N(R172)(R173), O-CO-$(C_1-C_6)$-Alkylen-CO-O-$(C_1-C_6)$-Alkyl, O-CO-$(C_1-C_6)$-Alkylen-CO-OH, O-CO-$(C_1-C_6)$-Alkylen-CO-N(R172)(R173);

z' unabhängig voneinander 1, 2, 3, 4, 5, 6;

R160, R161, R162, R163, R164, R165, R166, R167, R168, R169, R170, R171, R172, R173

gleich oder verschieden Wasserstoff, $(C_1-C_6)$-Alkyl,

oder

R160 und R161, R162 und R163, R172 und R173 bilden unabhängig voneinander optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-($C_1$-$C_6$)-Alkyl, Sauerstoff und Schwefel beinhalten kann;

ganz besonders bevorzugt ein Rest der Formel Ic:

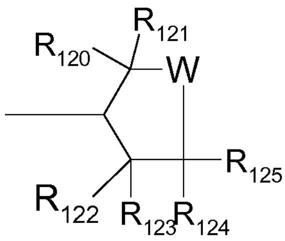

Ic,

worin bedeuten

W, -C(R126)(R127)-, -C(R126)(R127)-C(R128)(R129)-, -C(R126)(R127)-O-;

R120, R121, R123, R125, R126, R127, R128, R129
R120, R121, R126, R127, R128, R129 gleich oder verschieden Wasserstoff, ($C_1$-$C_6$)-Alkyl, oxo, COO-($C_1$-$C_6$)-Alkyl,

R122 und R124 zusammen mit dem sie tragenden Kohlenstoffatomen bilden ein monocyclisches, 6 gliederiges aromatisches Ringsystem, dessen einzelne Glieder durch =(C-R133)- substituiert sein können;

R133 gleich oder verschieden, F, Cl, Br, I, OH, $CF_3$, $NO_2$, CN, $OCHF_2$, $OCF_3$, $SF_5$, O-($C_1$-$C_6$)-Alkyl, O-($C_1$-$C_4$)-Alkoxy-($C_1$-$C_4$)-Alkyl, S-($C_1$-$C_6$)-Alkyl, ($C_1$-$C_6$)-Alkyl, ($C_2$-$C_4$)-Haloalkyl, O-($C_2$-$C_4$)-Haloalkyl, ($C_2$-$C_6$)-Alkenyl, ($C_2$-$C_6$)-Alkinyl, N(R160)(R161), $SO_2$-$CH_3$, COOH, COO-($C_1$-$C_6$)-Alkyl, CON(R162)(R163), N(R164)CO(R165), N(R166)$SO_2$(R167), CO(R168), $(CR169R170)_{z'}$-O(R171), $(CR169R170)_{z'}$-CO-O(R77), O-$(CR169R170)_{z'}$-CO-O(R171), $(CR169R170)_{z'}$-N(R172)(R173), O-$(CR169R170)_{z'}$-N(R172)(R173), $(CR169R170)_{z'}$-CON(R172)(R173), O-$(CR169R170)_{z'}$-CON(R172)(R173), O-CO-N(R172)(R173), O-CO-($C_1$-$C_6$)-Alkylen-CO-O-($C_1$-$C_6$)-Alkyl, O-CO-($C_1$-$C_6$)-Alkylen-CO-OH, O-CO-($C_1$-$C_6$)-Alkylen-CO-N(R172)(R173);

z' unabhängig voneinander 1, 2, 3, 4, 5, 6;

R160, R161, R162, R163, R164, R165, R166, R167, R168, R169, R170, R171, R172, R173

gleich oder verschieden Wasserstoff, ($C_1$-$C_6$)-Alkyl,

oder

R160 und R161, R162 und R163, R172 und R173 bilden unabhängig voneinander optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-($C_1$-$C_6$)-Alkyl, Sauerstoff und Schwefel beinhalten kann.

In einer weiteren bevorzugten Ausführungsform sind

R2, R3, R4 gleich oder verschieden Wasserstoff, F, Cl, $CF_3$, $OCHF_2$, $OCF_3$, O-($C_1$-$C_6$)-Alkyl, O-($C_1$-$C_4$)-Alkoxy-($C_1$-$C_4$)-Alkyl, S-($C_1$-$C_6$)-Alkyl, ($C_1$-$C_6$)-Alkyl, ($C_3$-$C_8$)-Cycloalkyl, O-($C_3$-$C_8$)-Cycloalkyl, Phenyl, N(R200)(R201), $SO_2$-$CH_3$, COO-($C_1$-$C_6$)-Alkyl, CON(R202)(R203), N(R204)CO(R205), N(R206)$SO_2$(R207), CO(R208), $(CR209R210)_{z''}$-O(R211), $(CR209R210)_{z''}$,-CO-O(R211), O-$(CR209R210)_{z''}$,-CO-O(R211), $(CR209R210)_{z''}$-N(R212)(R213), O-$(CR209R210)_{z''}$-N(R212)(R213), $(CR209R210)_{z''}$-CON(R212)(R213), O-CO-($C_1$-$C_6$)-Alkylen-CO-O-($C_1$-$C_6$)-Alkyl;

z" unabhängig voneinander 1, 2, 3;

R200, R201, R202, R203, R204R205, R206, R207, R208, R209, R210, R211, R212, R213

gleich oder verschieden Wasserstoff, ($C_1$-$C_6$)-Alkyl;

oder

R200 und R201, R202 und R203, R212 und R213

bilden unabhängig voneinander optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-($C_1$-$C_6$)-Alkyl, Sauerstoff und Schwefel beinhalten kann;

besonders bevorzugten sind

R2, R3, R4 gleich oder verschieden Wasserstoff, F, Cl, $CF_3$, $OCHF_2$, $OCF_3$, O-($C_{1-}$ $C_6$)-Alkyl, O-($C_1$-$C_4$)-Alkoxy-($C_1$-$C_4$)-Alkyl, S-($C_1$-$C_6$)-Alkyl, ($C_1$-$C_6$)-Alkyl, ($C_3$-$C_8$)-Cycloalkyl, O-($C_3$-$C_8$)-Cycloalkyl, Phenyl, $SO_2$-$CH_3$, COO-($C_1$-$C_6$)-Alkyl, $(CR209R210)_{z''}$-O(R211), $(CR209R210)_{z''}$,-CO-O(R211), O-$(CR209R210)_{z''}$-CO-O(R211), $(CR209R210)_{z''}$-N(R212)(R213);

z" unabhängig voneinander 1, 2, 3;

R209, R210, R211, R212, R213

gleich oder verschieden Wasserstoff, ($C_1$-$C_6$)-Alkyl;

ganz besonders bevorzugt sind

R2 Wasserstoff, -($C_1$-$C_6$)-Alkyl, ($C_3$-$C_8$)-Cycloalkyl oder Phenyl;
R3 Wasserstoff;
R4 Wasserstoff oder -($C_1$-$C_6$)-Alkyl;

Insbsondere besonders bevorzugt sind

R2 Wasserstoff, Methyl oder Phenyl;
R3 Wasserstoff;
R4 Wasserstoff oder Methyl.

Die erfindungsgemäßen Verbindungen der allgemeinen Formel I besitzen eine überraschende hemmende Wirkung auf die endotheliale Lipase (EL). Das antiatheroskleotisch wirksame HDL ist das bevorzugte Substrat für EL. Eine Senkung des HDL-Spiegels führt zur Progression von Atherosklerose und ihrer Folgeerkrankungen wie Koronare Herzerkrankungen und begünstigt darüberhinaus die Entstehung des Metabolischen Syndroms und seiner Folgeerkrankung Diabetes. Eine Hemmung der EL sollte somit generell zur Vorbeugung von atherosklerotischen Erkrankungen führen und indirekt bei Personen mit erhöhtem Risiko für Diabetes die Erkrankungswahrscheinlichkeit verringern.
Weiterhin wurde gefunden, dass die hemmende Wirkung der erfindungsgemäßen Verbindungen der allgemeinen Formel I selektiv gegenüber anderen Lipasen ist.
Gegenüber Verbindungen des Standes der Technik weisen die erfindungsgemäßen Verbindungen verbesserte Eigenschaften auf. Insbesondere konnten die chemische Stabilität in Lösung sowie in Blutplasma und gegenüber Leberenzymen signifikant verbessert werden, was die Eignung der erfindungsgemäßen Verbindungen als Arzneistoffe noch weiter verbessert.
Besonders geeignet sind solche Verbindungen zur Behandlung und/oder Prävention von

1. Dyslipidämien und deren Folgen, wie z.B. Atherosklerose, koronare Herzkrankheit, zerebrovaskuläre Erkrankungen etc, insbesondere solche (aber nicht beschränkt auf), die durch einen oder mehrerer folgender Faktoren charakterisiert sind:

- hohe Plasma-Triglycerid-, hohe postprandiale Plasma-Triglycerid-Konzentrationen

- niedrige HDL-Cholesterin Konzentration
- niedrige ApoA Lipoprotein-Konzentrationen
- hohe LDL-Cholesterin Konzentrationen
- kleine dichte LDL-Cholesterin Partikel
- hohe ApoB Lipoprotein-Konzentrationen

2. Verschiedenen andere Zuständen, die mit dem Metabolischen Syndrom assoziert sein können, sind wie:

- Adipositas (Fettsucht), einschließlich abdominale Adipositas
- Thrombosen, Stadien von Hyperkoagulabilität und Thromboseneigung (arteriell und venös)
- Hoher Blutdruck
- Herzinsuffizienz, wie z.B. (aber nicht beschränkt auf) bei Zustand nach Myokardinfarkt, hypertensive Herzerkrankung oder Kardiomyopathie
- Diabetes mellitus, insbesondere Typ 2 Diabetes einschließlich der Verhinderung der damit verbundenen Folgeerkrankungen (Hyperglykämie, Glucoseintoleranz,
  Verlust der ß-Zellen der Bauchspeicheldrüse, makro- und mikrovaskulärer Erkrankungen

3. weitere Krankheiten oder Zustände bei welchen zum Beispiel entzündliche Reaktionen oder die Zelldifferenzierung eine Rolle spielt sind:

- Atherosklerose wie z.B. (aber nicht beschränkt auf) Koronarsklerose einschl. Angina pectoris oder Herzinfarkt, Hirnschlag
- Vaskuläre Restenose oder Reverschluß
- Chronisch entzündliche Darmerkrankungen, wie z.B. Morbus Crohn und Colitis ulcerosa
- Pankreatitis
- Andere entzündliche Zustände
- Retinopathie
- Fettzell-Tumore (adipose cell tumors)
- Fettzell-Karzinome, wie z.B. Liposarkome
- solide Tumoren und Neoplasien, wie z.B. (aber nicht beschränkt auf) Karzinome des Magen-Darm Traktes, der Leber, der Gallenwege und des Pankreas, endokrine Tumore, Karzinome der Lunge, der Niere und harnableitenden Organe, des Genitaltraktes, Prostata-Karzinome etc.
- akute und chronische myeloprolifeative Erkrankungen und Lymphome
- Angiogenese
- Neurodegenerative Erkrankungen
- Alzheimersche Krankheit
- Multiple Sklerose
- Morbus Parkinson
- Erythemato-squamöse Dermatosen, wie z.B. Psoriasis (Schuppenflechte)
- Akne vulgaris
- Andere Hautkrankheiten und dermatologische Zustände, die durch PPAR moduliert werden
- Ekzeme und Neurodermitis
- Dermatitiden, wie z.B. seborrhoische Dermatitis oder Lichtdermatitis
- Keratitis und Keratosen, wie z.B. seborrhoische Keratosen, senile Keratosen, aktinische Keratose, photo-induzierte Keratosen oder Keratosis follicularis
- Keloide und Keloid-Prophylaxe
- Warzen, einschließlich Kondylomata oder Kondylomata acuminata
- Human papilloma viral (HPV) Infektionen, wie z.B. venerische Papillomata, virale Warzen, wie z.B. Molluscum contagiosum, Leukoplakie
- Papulöse Dermatosen, wie z.B. Lichen planus
- Hautkrebs, wie z.B. Basalzellkarzinome, Melanome oder kutane T-Zell Lymphome
- Lokalisierte, benigne epidermale Tumore, wie z.B. Keratoderma, epidermale Naevi
- Frostbeulen
- Hoher Blutdruck
- Syndrom X
- Syndrom der polyzystischen Ovarien (PCOS)
- Asthma
- Osteoarthritis

- Lupus erythematodes (LE) oder entzündliche rheumatische Erkrankungen, wie z.B. Rheumatoide Arthritis
- Vaskulitis
- Auszehrung (Kachexie)
- Gicht
- Ischämie/Reperfusions Syndrom
- Akutes respiratorisches Distress Syndrom (ARDS) ("Schocklunge")

Galenik

Die Menge einer erfindungsgemäßen Verbindung, die erforderlich ist, um den gewünschten biologischen Effekt zu erreichen, ist abhängig von einer Reihe von Faktoren, z.B. der gewählten spezifischen Verbindung, der beabsichtigten Verwendung, der Art der Verabreichung und dem klinischen Zustand des Patienten. Im allgemeinen liegt die Tagesdosis im Bereich von 0,3 mg bis 100 mg (typischerweise von 3 mg bis 50 mg) pro Tag pro Kilogramm Körpergewicht, z.B. 3-10 mg/kg/Tag. Eine intravenöse Dosis kann z.B. im Bereich von 0,3 mg bis 1,0 mg/kg liegen, die geeigneterweise als Infusion von 10 ng bis 100 ng pro Kilogramm pro Minute verabreicht werden kann. Geeignete Infusionslösungen für diese Zwecke können z.B. von 0,1 ng bis 10 mg, typischerweise von 1 ng bis 10 mg pro Milliliter, enthalten. Einzeldosen können z.B. von 1 mg bis 10 g des Wirkstoffs enthalten. Somit können Ampullen für Injektionen beispielsweise von 1 mg bis 100 mg, und oral verabreichbare Einzeldosisformulierungen, wie zum Beispiel Tabletten oder Kapseln, können beispielsweise von 0.05 bis 1000 mg, typischerweise von 0,5 bis 600 mg enthalten. Zur Therapie der oben genannten Zustände können die Verbindungen gemäß Formel I selbst als Verbindung verwendet werden, vorzugsweise liegen sie jedoch mit einem verträglichen Träger in Form einer pharmazeutischen Zusammensetzung vor. Der Träger muss natürlich verträglich sein, in dem Sinne, dass er mit den anderen Bestandteilen der Zusammensetzung kompatibel ist und nicht gesundheitsschädlich für den Patienten ist. Der Träger kann ein Feststoff oder eine Flüssigkeit oder beides sein und wird vorzugsweise mit der Verbindung als Einzeldosis formuliert, beispielsweise als Tablette, die von 0,05% bis 95 Gew.-% des Wirkstoffs enthalten kann. Weitere pharmazeutisch aktive Substanzen können ebenfalls vorhanden sein, einschließlich weiterer erfindungsgemäßer Verbindungen. Die erfindungsgemäßen pharmazeutischen Zusammensetzungen können nach einer der bekannten pharmazeutischen Methoden hergestellt werden, die im wesentlichen darin bestehen, dass die Bestandteile mit pharmakologisch verträglichen Träger- und/oder Hilfsstoffen gemischt werden.

Erfindungsgemäße pharmazeutische Zusammensetzungen sind solche, die für orale, rektale, topische, perorale (z.B. sublinguale) und parenterale (z.B. subkutane, intramuskuläre, intradermale oder intravenöse) Verabreichung geeignet sind, wenngleich die geeignetste Verabreichungsweise in jedem Einzelfall von der Art und Schwere des zu behandelnden Zustandes und von der Art der jeweils verwendeten Verbindung gemäß Formel I abhängig ist. Auch dragierte Formulierungen und dragierte Retardformulierungen gehören in den Rahmen der Erfindung. Bevorzugt sind säure- und magensaftresistente Formulierungen. Geeignete magensaftresistente Beschichtungen umfassen Celluloseacetatphthalat, Polyvinylacetatphthalat, Hydroxypropylmethylcellulosephthalat und anionische Polymere von Methacrylsäure und Methacrylsäuremethylester.

Geeignete pharmazeutische Zubereitungen für die orale Verabreichung können in separaten Einheiten vorliegen, wie zum Beispiel Kapseln, Oblatenkapseln, Lutschtabletten oder Tabletten, die jeweils eine bestimmte Menge der Verbindung gemäß Formel I enthalten; als Pulver oder Granulate; als Lösung oder Suspension in einer wässrigen oder nicht-wässrigen Flüssigkeit; oder als eine Öl-in-Wasser- oder Wasser-in Öl-Emulsion. Diese Zusammensetzungen können, wie bereits erwähnt, nach jeder geeigneten pharmazeutischen Methode zubereitet werden, die einen Schritt umfasst, bei dem der Wirkstoff und der Träger (der aus einem oder mehreren zusätzlichen Bestandteilen bestehen kann) in Kontakt gebracht werden. Im allgemeinen werden die Zusammensetzungen durch gleichmäßiges und homogenes Vermischen des Wirkstoffs mit einem flüssigen und/oder feinverteilten festen Träger hergestellt, wonach das Produkt, falls erforderlich, geformt wird. So kann beispielsweise eine Tablette hergestellt werden, indem ein Pulver oder Granulat der Verbindung verpresst oder geformt wird, gegebenenfalls mit einem oder mehreren zusätzlichen Bestandteilen. Gepresste Tabletten können durch Tablettieren der Verbindung in frei fließender Form, wie beispielsweise einem Pulver oder Granulat, gegebenenfalls gemischt mit einem Bindemittel, Gleitmittel, inertem Verdünner und/oder einem (mehreren) oberflächenaktiven/dispergierenden Mitteln in einer geeigneten Maschine hergestellt werden. Geformte Tabletten können durch Formen der pulverförmigen, mit einem inerten flüssigen Verdünnungsmittel befeuchteten Verbindung in einer geeigneten Maschine hergestellt werden.

Pharmazeutische Zusammensetzungen, die für eine perorale (sublinguale) Verabreichung geeignet sind, umfassen Lutschtabletten, die eine Verbindung gemäß Formel I mit einem Geschmacksstoff enthalten, üblicherweise Saccharose und Gummi arabicum oder Tragant, und Pastillen, welche die Verbindung in einer inerten Basis wie Gelatine und Glycerin oder Saccharose und Gummi arabicum umfassen.

Geeignete pharmazeutische Zusammensetzungen für die parenterale Verabreichung umfassen vorzugsweise sterile wässrige Zubereitungen einer Verbindung gemäß Formel I, die vorzugsweise isotonisch mit dem Blut des vorgesehenen Empfängers sind. Diese Zubereitungen werden vorzugsweise intravenös verabreicht, wenngleich die Verabreichung auch subkutan, intramuskulär oder intradermal als Injektion erfolgen kann. Diese Zubereitungen können vorzugsweise hergestellt werden, indem die Verbindung mit Wasser gemischt wird und die erhaltene Lösung steril und mit dem Blut isotonisch gemacht wird. Injizierbare erfindungsgemäße Zusammensetzungen enthalten im allgemeinen von 0,1 bis 5 Gew.-% der aktiven Verbindung.

Geeignete pharmazeutische Zusammensetzungen für die rektale Verabreichung liegen vorzugsweise als Einzeldosis-Zäpfchen vor. Diese können hergestellt werden, indem man eine Verbindung gemäß Formel I mit einem oder mehreren herkömmlichen festen Trägern, beispielsweise Kakaobutter, mischt und das entstehende Gemisch in Form bringt.

Geeignete pharmazeutische Zusammensetzungen für die topische Anwendung auf der Haut liegen vorzugsweise als Salbe, Creme, Lotion, Paste, Spray, Aerosol oder Öl vor. Als Träger können Vaseline, Lanolin, Polyethylenglycole, Alkohole und Kombinationen von zwei oder mehreren dieser Substanzen verwendet werden. Der Wirkstoff ist im allgemeinen in einer Konzentration von 0,1 bis 15 Gew.-% der Zusammensetzung vorhanden, beispielsweise von 0,5 bis 2%.

Auch eine transdermale Verabreichung ist möglich. Geeignete pharmazeutische Zusammensetzungen für transdermale Anwendungen können als einzelne Pflaster vorliegen, die für einen langzeitigen engen Kontakt mit der Epidermis des Patienten geeignet sind. Solche Pflaster enthalten geeigneterweise den Wirkstoff in einer gegebenenfalls gepufferten wässrigen Lösung, gelöst und/oder dispergiert in einem Haftmittel oder dispergiert in einem Polymer. Eine geeignete Wirkstoff-Konzentration beträgt ca. 1% bis 35%, vorzugsweise ca. 3% bis 15%. Als eine besondere Möglichkeit kann der Wirkstoff, wie beispielsweise in Pharmaceutical Research, 2(6): 318 (1986) beschrieben, durch Elektrotransport oder Iontophorese freigesetzt werden.

Die Verbindungen der Formel I zeichnen sich durch günstige Wirkungen auf Lipidstoffwechselstörungen aus. Sie beeinflussen das Verhältnis von HDL zu LDL positiv und erhöhen besonders die HDL-Spiegel und sind zur Prävention und Behandlung von Dyslipidämien und Metabolischem Syndrom sowie deren vielfältigen Folgeerkrankungen wie Atherosklerose, koronare Herzerkrankungen, Herzinsuffizienz, Adipositas und Diabetes geeignet.

Kombinationen mit anderen Medikamenten

Die erfindungsgemäßen Verbindungen können allein oder in Kombination mit einer oder mehreren weiteren pharmakologisch wirksamen Substanzen verabreicht werden, die beispielsweise günstige Wirkungen auf Stoffwechselstörungen oder damit häufig assoziierte Erkrankungen haben.

Sie können mit den erfindungsgemäßen Verbindungen der Formel I insbesondere zur synergistischen Wirkungsverbesserung kombiniert werden. Die Verabreichung der Wirkstoffkombination kann entweder durch getrennte Gabe der Wirkstoffe an den Patienten oder in Form von Kombinationspräparaten, worin mehrere Wirkstoffe in einer pharmazeutischen Zubereitung vorliegen, erfolgen. Erfolgt die Gabe der Wirkstoffe durch getrennte Verabreichung der Wirkstoffe, so kann diese gleichzeitig oder nacheinander erfolgen.

Als weitere Wirkstoffe für die Kombinationspräparate sind geeignet:

Alle Antidiabetika, die in der Roten Liste 2007, Kapitel 12 genannt sind; alle Abmagerungsmittel/Appetitzügler, die in der Roten Liste 2007, Kapitel 1 genannt sind; alle Diuretika, die in der Roten Liste 2007, Kapitel 36 genannt sind; alle Lipidsenker, die in der Roten Liste 2007, Kapitel 58 genannt sind. Die meisten der nachfolgend aufgeführten Wirkstoffe sind in USP Dictionary of USAN and International Drug Names, US Pharmacopeia, Rockville 2006, offenbart.

Antidiabetika umfassen Insulin und Insulinderivate, wie z.B. Lantus® (siehe www.lantus.com) oder HMR 1964 oder Levemir® (insulin detemir), Humalog(R) (Insulin Lispro), Humulin(R), VIAject™, SuliXen(R) oder solche, wie sie in WO2005005477 (Novo Nordisk) beschrieben sind, schnell wirkende Insuline (siehe US 6,221,633), inhalierbare Insuline, wie z. B. Exubera ®, Nasulin™, oder orale Insuline, wie z. B. IN-105 (Nobex) oder Oral-lyn™ (Generex Biotechnology) oder Technosphere(R) Insulin (MannKind) oder Cobalamin™ orales Insulin oder Insuline, wie sie in WO2007128815, WO2007128817, WO2008034881, WO2008049711 beschrieben sind oder Insuline, die transdermal verabreicht werden können;

GLP-1-Derivate und GLP-1 Agonisten wie z.B. Exenatide oder spezielle Zubereitungen davon, wie sie z.B. in WO2008061355 beschrieben sind, Liraglutide, Taspoglutide (R-1583), Albiglutide, Lixisenatide oder diejenigen die in WO 98/08871, WO2005027978, WO2006037811, WO2006037810 von Novo Nordisk A/S, in WO 01/04156 von Zealand oder in WO 00/34331 von Beaufour-Ipsen offenbart wurden, Pramlintide Acetat (Symlin; Amylin Pharmaceuticals), AVE-0010, BIM-51077 (R-1583, ITM-077), PC-DAC:Exendin-4 (ein Exendin-4 Analogon, welches kovalent an rekombinantes menschliches Albumin gebunden ist), CVX-73, CVX-98 und CVx-96 (GLP-1 Analoga, welche kovalent an einen monoklonalen Antikörper gebunden sind, der spezifische Bindungsstellen für das GLP-1 Peptid aufweist), CNTO-736 (ein GLP-1 Analogon, welches an eine Domäne gebunden ist, welche den Fc-Teil eines Antikörpers beinhaltet), PGC-GLP-1

(GLP-1 gebunden an einen Nanocarrier), Agonisten wie sie z.B. bei D. Chen et al., Proc. Natl. Acad. Sci. USA 104 (2007) 943 beschrieben sind, solche wie sie in WO2006124529, WO2007124461, WO2008062457, WO2008082274, WO2008101017, WO2008081418, WO2008112939, WO2008112941, WO2008113601, WO2008116294, WO2008116648, WO2008119238 beschrieben sind, Peptide wie z.B. Obinepitide (TM-30338), Amylinrezeptor Agonisten, wie sie z.B. in WO2007104789 beschrieben sind, Analoga des humanen GLP-1, wie sie in WO2007120899, WO2008022015, WO2008056726 beschrieben sind, sowie oral wirksame hypoglykämische Wirkstoffe.

Antidiabetika umfassen auch Agonisten des Glukose-abhängigen insulinotropen Polypeptids (GIP) Rezeptors wie sie z.B. in WO2006121860 beschrieben sind.

Antidiabetika umfassen auch das Glukose-abhängige insulinotrope Polypeptid (GIP) wie auch analoge Verbindungen wie sie z.B. in WO2008021560 beschrieben sind.

Antidiabetika umfassen auch Analoga und Derivate des Fibroblastenwachstumsfaktors 21 (FGF-21, fibroblast growth factor 21).

Die oral wirksamen hypoglykämischen Wirkstoffe umfassen vorzugsweise
Sulfonylharnstoffe,
Biguanidine,
Meglitinide,
Oxadiazolidindione,
Thiazolidindione,
PPAR- und RXR-Modulatoren,
Glukosidase-Inhibitoren,
Hemmstoffe der Glykogenphosphorylase,
Glukagonrezeptor-Antagonisten,
Glukokinaseaktivatoren,
Inhibitoren der Fructose-1,6-bisphosphatase,
Modulatoren des Glukosetransporters-4 (GLUT4),
Inhibitoren der Glutamin-Fructose-6-Phosphat-Amidotransferase (GFAT),
GLP-1-Agonisten,
Kaliumkanalöffner, wie z.B. Pinacidil, Cromakalim, Diazoxid oder solche wie sie bei R. D. Carr et al., Diabetes 52, 2003, 2513.2518, bei J. B. Hansen et al, Current Medicinal Chemistry 11, 2004, 1595-1615, bei T. M. Tagmose et al., J. Med. Chem. 47, 2004, 3202-3211 oder bei M. J. Coghlan et al., J. Med. Chem. 44, 2001, 1627-1653 beschrieben sind, oder diejenigen, die in WO 97/26265 und WO 99/03861 von Novo Nordisk A/S offenbart wurden,
Wirkstoffe, die auf den ATP-abhängigen Kaliumkanal der Betazellen wirken,
Inhibitoren der Dipeptidylpeptidase-IV (DPP-IV),
Insul in-Sensitizer,
Inhibitoren von Leberenzymen, die an der Stimulation der Glukoneogenese und/oder Glykogenolyse beteiligt sind,
Modulatoren der Glukoseaufnahme, des Glukosetransports und der Glukoserückresorption,
Modulatoren der natrium-abhängigen Glukosetransporter 1 oder 2 (SGLT1, SGLT2), Hemmstoffe der 11-beta-Hydroxysteroid-Dehydrogenase-1 (11β-HSD1),
Inhibitoren der Protein-Tyrosin-Phosphatase-1B (PTP-1B),
Nikotinsäurerezeptoragonisten,
Inhibitoren der hormon-sensitiven bzw. endothelialen Lipasen,
Hemmstoffen der Acetyl-CoA Carboxylase (ACC1 und/oder ACC2) oder Inhibitoren der GSK-3 beta.

Weiterhin sind umfasst den Fettstoffwechsel verändernde Verbindungen wie antihyperlipidämische Wirkstoffe und antilipidämische Wirkstoffe,
HMGCoA-Reduktase-Inhibitoren,
Farnesoid X Rezeptor (FXR) Modulatoren,
Fibrate,
Cholesterinresreptionsinhibitoren,
CETP-Inhibitoren,
Gallensäureresorptionsinhibitoren,
MTP-Inhibitoren,
Agonisten des Estrogenrezeptors gamma (ERRγ Agonisten),
Sigma-1 Rezeptorantagonisten,
Antagonisten des Somatostatin 5 Rezeptors (SST5 Rezeptor);
Verbindungen, die die Nahrungsmitteleinnahme verringern und Verbindungen, die die Thermogenese erhöhen.

Bei einer Ausführungsform der Erfindung wird die Verbindung der Formel I in Kombination mit Insulin verabreicht.

Bei einer Ausführungsform wird die Verbindung der Formel I in Kombination mit einem Wirkstoff, der auf den ATP-abhängigen Kaliumkanal der Betazellen wirkt, z.B. Sulfonylharnstoffe, wie z.B. Tolbutamid, Glibenclamid, Glipizid,

Gliclazide oder Glimepirid, verabreicht.

Bei einer Ausführungsform wird die Verbindung der Formel I in Kombination mit einer Tablette verabreicht, die sowohl Glimeprid enthält, welches schnell freigesetzt wird wie auch Metformin enthält, welches über einen längeren Zeitraum freigesetzt wird (wie z.B. in US2007264331, WO2008050987, WO2008062273 beschrieben).

Bei einer Ausführungsform wird die Verbindung der Formel I in Kombination mit einem Biguanid, wie z.B. Metformin, verabreicht.

Bei wieder einer Ausführungsform wird die Verbindung der Formel I in Kombination mit einem Meglitinid, wie z.B. Repaglinide, Nateglinid oder Mitiglinide verabreicht.

Bei einer weiteren Ausführungsform wird die Verbindung der Formel I mit einer Kombination von Mitiglinide mit einem Glitazon, z.B. Pioglitazon Hydrochlorid, verabreicht.

Bei einer weiteren Ausführungsform wird die Verbindung der Formel I mit einer Kombination von Mitiglinide mit einem alpha-Glukosidaseinhibitor verabreicht.

Bei einer weiteren Ausführungsform wird die Verbindung der Formel I in Kombination mit antidiabetischen Verbindungen, wie sie in WO2007095462, WO2007101060, WO2007105650 beschrieben sind, verabreicht.

Bei einer weiteren Ausführungsform wird die Verbindung der Formel I in Kombination mit antihypoglykämischen Verbindungen, wie sie in WO2007137008, WO2008020607 beschrieben sind, verabreicht.

Bei einer Ausführungsform wird die Verbindung der Formel I in Kombination mit einem Thiazolidindion, wie z.B. Troglitazon, Ciglitazon, Pioglitazon, Rosiglitazon oder den in WO 97/41097 von Dr. Reddy's Research Foundation offenbarten Verbindungen, insbesondere 5-[[4-[(3,4-Dihydro-3-methyl-4-oxo-2-chinazolinylmethoxy]-phenyl]methyl]-2,4-thiazolidindion, verabreicht.

Bei einer Ausführungsform der Erfindung wird die Verbindung der Formel I in Kombination mit einem PPAR gamma Agonisten, wie z.B. Rosiglitazon, Pioglitazon, JTT-501, GI 262570, R-483, CS-011 (Rivoglitazon), DRL-17564, DRF-2593 (Balaglitazon), INT-131, T-2384 oder solchen, wie sie in WO2005086904, WO2007060992, WO2007100027, WO2007103252, WO2007122970, WO2007138485, WO2008006319, WO2008006969, WO2008010238, WO2008017398, WO2008028188, WO2008066356, WO2008084303, WO2008089461-WO2008089464, WO2008093639, WO2008096769, WO2008096820, WO2008096829, US2008194617, WO2008099944, WO2008108602, WO2008109334, WO2008126731, WO2008126732 beschrieben sind, verabreicht.

Bei einer Ausführungsform der Erfindung wird die Verbindung der Formel I in Kombination mit Competact™, einer festen Kombination von Pioglitazon Hydrochlorid mit Metformin Hydrochlorid, verabreicht.

Bei einer Ausführungsform der Erfindung wird die Verbindung der Formel I in Kombination mit Tandemact™, einer festen Kombination von Pioglitazon mit Glimeprid, verabreicht.

Bei einer weiteren Ausführungsform der Erfindung wird die Verbindung der Formel I in Kombination mit einer festen Kombination von Pioglitazon Hydrochlorid mit einem Angiotensin II Agonisten, wie z.B. TAK-536, verabreicht.

Bei einer Ausführungsform der Erfindung wird die Verbindung der Formel I in Kombination mit einem PPAR alpha Agonisten bzw. gemischten PPAR alpha/PPAR delta Agonisten, wie z.B. GW9578, GW-590735, K-111, LY-674, KRP-101, DRF-10945, LY-518674, CP-900691, BMS-687453, BMS-711939 oder solchen wie sie in WO2001040207, WO2002096894, WO2005097076, WO2007056771, WO2007087448, WO2007089667, WO2007089557, WO2007102515, WO2007103252, JP2007246474, WO2007118963, WO2007118964, WO2007126043, WO2008006043, WO2008006044, WO2008012470, WO2008035359, WO2008087365, WO2008087366, WO2008087367, WO2008117982 beschrieben sind, verabreicht.

Bei einer Ausführungsform der Erfindung wird die Verbindung der Formel I in Kombination mit einem gemischten PPAR alpha/gamma Agonisten, wie z.B. Naveglitazar, LY-510929, ONO-5129, E-3030, AVE 8042, AVE 8134, AVE 0847, CKD-501 (Lobeglitazon Sulfat), MBX-213, KY-201 oder wie in WO 00/64888, WO 00/64876, WO03/020269, WO2004024726, WO2007099553, US2007276041, WO2007085135, WO2007085136, WO2007141423, WO2008016175, WO2008053331, WO2008109697, WO2008109700, WO2008108735 oder in J.P.Berger et al., TRENDS in Pharmacological Sciences 28(5), 244-251, 2005 beschrieben, verabreicht.

Bei einer Ausführungsform der Erfindung wird die Verbindung der Formel I in Kombination mit einem PPAR delta Agonisten, wie z.B. GW-501516 oder wie sie in WO2006059744, WO2006084176, WO2006029699, WO2007039172-WO2007039178, WO2007071766, WO2007101864, US2007244094, WO2007119887, WO2007141423, US2008004281, WO2008016175, WO2008066356, WO2008071311, WO2008084962, US2008176861 beschrieben sind, verabreicht.

Bei einer Ausführungsform der Erfindung wird die Verbindung der Formel I in Kombination mit einem pan-SPPARM (selective PPAR modulator alpha, gamma, delta), wie z.B. GFT-505 oder solchen wie sie in WO2008035359 beschrieben sind, verabreicht.

Bei einer Ausführungsform wird die Verbindung der Formel I in Kombination mit Metaglidasen oder mit MBX-2044 oder anderen partiellen PPAR gamma Agonisten/Antagonisten verabreicht.

Bei einer Ausführungsform wird die Verbindung der Formel I in Kombination mit einem $\alpha$-Glukosidase-Inhibitor, wie z.B. Miglitol oder Acarbose oder solchen, wie sie z.B. in WO2007114532, WO2007140230, US2007287674,

US2008103201, WO2008065796, WO2008082017 beschrieben sind, verabreicht.
Bei einer Ausführungsform wird die Verbindung der Formel I in Kombination mit einem Hemmstoff der Glykogenphosphorylase, wie z.B. PSN-357 oder FR-258900 oder solchen wie in WO2003084922, WO2004007455, WO2005073229-31, WO2005067932, WO2008062739, WO2008099000, WO2008113760 beschrieben, verabreicht.

Bei einer Ausführungsform wird die Verbindung der Formel I in Kombination mit Glukagon-Rezeptor-Antagonisten, wie z.B. A-770077 oder NNC-25-2504 oder wie in WO2004100875, WO2005065680, WO2006086488, WO2007047177, WO2007106181, WO2007111864, WO2007120270, WO2007120284, WO2007123581, WO2007136577, WO2008042223, WO2008098244 beschrieben, verabreicht.

Bei einer weiteren Ausführungsform wird die Verbindung der Formel I in Kombination mit einer Antisense-Verbindung, z.B. ISIS-325568, verabreicht, welche die Produktion des Glukagonrezeptors inhibiert.

Bei einer Ausführungsform wird die Verbindung der Formel I in Kombination mit Aktivatoren der Glukokinase, wie z. B. LY-2121260 (WO2004063179), PSN-105, PSN-110, GKA-50 oder solchen wie sie z. B. in WO2004072031, WO2004072066, WO2005080360, WO2005044801, WO2006016194, WO2006058923, WO2006112549, WO2006125972, WO2007017549, WO2007017649, WO2007007910, WO2007007040-42, WO2007006760-61, WO2007006814, WO2007007886, WO2007028135, WO2007031739, WO2007041365, WO2007041366, WO2007037534, WO2007043638, WO2007053345, WO2007051846, WO2007051845, WO2007053765, WO2007051847, WO2007061923, WO2007075847, WO2007089512, WO2007104034, WO2007117381, WO2007122482, WO2007125103, WO2007125105, US2007281942, WO2008005914, WO2008005964, WO2008043701, WO2008044777, WO2008047821, US2008096877, WO2008050117, WO2008050101, WO2008059625, US2008146625, WO2008078674, WO2008079787, WO2008084043, WO2008084044, WO2008084872, WO2008089892, WO2008091770, WO2008075073, WO2008084043, WO2008084044, WO2008084872, WO2008084873, WO2008089892, WO2008091770, JP2008189659, WO2008104994, WO2008111473, WO2008116107, WO2008118718, WO2008120754 beschrieben sind, verabreicht.

Bei einer Ausführungsform wird die Verbindung der Formel I in Kombination mit einem Inhibitor der Glukoneogenese, wie sie z. B. in FR-225654, WO2008053446 beschrieben sind, verabreicht.

Bei einer Ausführungsform wird die Verbindung der Formel I in Kombination mit Inhibitoren der Fructose-1,6-bisphosphatase (FBPase) wie z.B. MB-07729, CS-917 (MB-06322) oder MB-07803 oder solchen wie sie in WO2006023515, WO2006104030, WO2007014619, WO2007137962, WO2008019309, WO2008037628 beschrieben sind, verabreicht.

Bei einer Ausführungsform wird die Verbindung der Formel I in Kombination mit Modulatoren des Glukosetransporters-4 (GLUT4), wie z. B. KST-48 (D.-O. Lee et al.: Arzneim.-Forsch. Drug Res. 54 (12), 835 (2004)), verabreicht.

Bei einer Ausführungsform wird die Verbindung der Formel I in Kombination mit Inhibitoren der Glutamin-Fructose-6-Phosphat-Amidotransferase (GFAT), wie sie z. B. in WO2004101528 beschrieben sind, verabreicht.

Bei einer Ausführungsform wird die Verbindung der Formel I in Kombination mit Inhibitoren der Dipeptidylpeptidase-IV (DPP-IV), wie z. B. Vildagliptin (LAF-237), Sitagliptin (MK-0431), Sitagliptin Phosphat, Saxagliptin ((BMS-477118), GSK-823093, PSN-9301, SYR-322, SYR-619, TA-6666, TS-021, GRC-8200 (Melogliptin), GW-825964X, KRP-104, DP-893, ABT-341, ABT-279 oder ein anderes Salz davon, S-40010, S-40755, PF-00734200, BI-1356, PHX-1149, Alogliptin Benzoat, Linagliptin, Melogliptin oder solchen Verbindungen wie sie in WO2003074500, WO2003106456, WO2004037169, WO200450658, WO2005037828, WO2005058901, WO2005012312, WO2005/012308, WO2006039325, WO2006058064, WO2006015691, WO2006015701, WO2006015699, WO2006015700, WO2006018117, WO2006099943, WO2006099941, JP2006160733, WO2006071752, WO2006065826, WO2006078676, WO2006073167, WO2006068163, WO2006085685, WO2006090915, WO2006104356, WO2006127530, WO2006111261, US2006890898, US2006803357, US2006303661, WO2007015767 (LY-2463665), WO2007024993, WO2007029086, WO2007063928, WO2007070434, WO2007071738, WO2007071576, WO2007077508, WO2007087231, WO2007097931, WO2007099385, WO2007100374, WO2007112347, WO2007112669, WO2007113226, WO2007113634, WO2007115821, WO2007116092, US2007259900, EP1852108, US2007270492, WO2007126745, WO2007136603, WO2007142253, WO2007148185, WO2008017670, US2008051452, WO2008027273, WO2008028662, WO2008029217, JP2008031064, JP2008063256, WO2008033851, WO2008040974, WO2008040995, WO2008060488, WO2008064107, WO2008066070, WO2008077597, JP2008156318, WO2008087560, WO2008089636, WO2008093960, WO2008096841, WO2008101953, WO2008118848, WO2008119005, WO2008119208, WO2008120813, WO2008121506 beschrieben sind, verabreicht.

Bei einer Ausführungsform wird die Verbindung der Formel I in Kombination mit Janumet™, einer festen Kombination von Sitagliptin Phosphat mit Metformin Hydrochlorid, verabreicht.

Bei einer Ausführungsform wird die Verbindung der Formel I in Kombination mit Eucreas(R), einer festen Kombination von Vildagliptin mit Metformin Hydrochlorid, verabreicht.

Bei einer weiteren Ausführungsform wird die Verbindung der Formel I in Kombination mit einer festen Kombination von Alogliptin Benzoat mit Pioglitazone verabreicht.

Bei einer Ausführungsform wird die Verbindung der Formel I in Kombination mit einer festen Kombination von eines Salzes von Sitagliptin mit Metformin Hydrochlorid, verabreicht.

Bei einer Ausführungsform wird die Verbindung der Formel I in Kombination mit einer Kombination eines DPP-IV-Inhibitors mit omega-3-Fettsäuren oder omega-3-Fettsäureestern, wie z.B. in WO2007128801 beschrieben, verabreicht.

Bei einer Ausführungsform wird die Verbindung der Formel I in Kombination mit einer festen Kombination von eines Salzes von Sitagliptin mit Metformin Hydrochlorid, verabreicht.

Bei einer Ausführungsform wird die Verbindung der Formel I in Kombination mit einer die Insulinsekretion verstärkende Substanz, wie z. B. KCP-265 (WO2003097064), oder solchen wie sie in WO2007026761, WO2008045484, US2008194617 beschrieben sind, verabreicht.

Bei einer Ausführungsform wird die Verbindung der Formel I in Kombination mit Agonisten des glucose-abhängigen insulinotropischen Rezeptors (GDIR) wie z. B. APD-668 verabreicht.

Bei einer Ausführungsform der Erfindung wird die Verbindung der Formel I in Kombination mit einem ATP-Citrat-Lyase Inhibitor, wie z.B. SB-204990, verabreicht.

Bei einer Ausführungsform wird die Verbindung der Formel I in Kombination mit Modulatoren des natrium-abhängigen Glukosetransporters 1 oder 2 (SGLT1, SGLT2), wie z.B. KGA-2727, T-1095, SGL-0010, AVE 2268, SAR 7226, SGL-5083, SGL-5085, SGL-5094, ISIS-388626, Sergliflozin oder Dapagliflozin oder wie sie z. B. in WO2004007517, WO200452903, WO200452902, PCT/EP2005/005959, WO2005085237, JP2004359630, WO2005121161, WO2006018150, WO2006035796, WO2006062224, WO2006058597, WO2006073197, WO2006080577, WO2006087997, WO2006108842, WO2007000445, WO2007014895, WO2007080170, WO2007093610, WO2007126117, WO2007128480, WO2007129668, US2007275907, WO2007136116, WO2007143316, WO2007147478, WO2008001864, WO2008002824, WO2008013277, WO2008013280, WO2008013321, WO2008013322, WO2008016132, WO2008020011, JP2008031161, WO2008034859, WO2008042688, WO2008044762, WO2008046497, WO2008049923, WO2008055870, WO2008055940, WO2008069327, WO2008070609, WO2008071288, WO2008072726, WO2008083200, WO2008090209, WO2008090210, WO2008101586, WO2008101939, WO2008116179, WO2008116195, US2008242596 oder von A. L. Handion in Expert Opin. Ther. Patents (2005) 15(11), 1531-1540 beschrieben sind, verabreicht.

Bei einer Ausführungsform wird die Verbindung der Formel I in Kombination mit Hemmstoffen der 11-beta-Hydroxysteroid-Dehydrogenase-1 (11-HSD1), wie z. B. BVT-2733, JNJ-25918646, INCB-13739, INCB-20817, DIO-92 ((-)-Ketoconazol) oder solche, wie sie z. B. in WO200190090-94, WO200343999, WO2004112782, WO200344000, WO200344009, WO2004112779, WO2004113310, WO2004103980, WO2004112784, WO2003065983, WO2003104207, WO2003104208, WO2004106294, WO2004011410, WO2004033427, WO2004041264, WO2004037251, WO2004056744, WO2004058730, WO2004065351, WO2004089367, WO2004089380, WO2004089470-71, WO2004089896, WO2005016877, WO2005063247, WO2005097759, WO2006010546, WO2006012227, WO2006012173, WO2006017542, WO2006034804, WO2006040329, WO2006051662, WO2006048750, WO2006049952, WO2006048331, WO2006050908, WO2006024627, WO2006040329, WO2006066109, WO2006074244, WO2006078006, WO2006106423, WO2006132436, WO2006134481, WO2006134467, WO2006135795, WO2006136502, WO2006138508, WO2006138695, WO2006133926, WO2007003521, WO2007007688, US2007066584, WO2007029021, WO2007047625, WO2007051811, WO2007051810 WO2007057768, WO2007058346, WO2007061661, WO2007068330, WO2007070506, WO2007087150, WO2007092435, WO2007089683, WO2007101270, WO2007105753, WO2007107470, WO2007107550, WO2007111921, US2007207985, US2007208001, WO2007115935, WO2007118185, WO2007122411, WO2007124329, WO2007124337, WO2007124254, WO2007127688, WO2007127693, WO2007127704, WO2007127726, WO2007127763, WO2007127765, WO2007127901, US2007270424, JP2007291075, WO2007130898, WO2007135427, WO2007139992, WO2007144394, WO2007145834. WO2007145835, WO2007146761, WO2008000950, WO2008000951, WO2008003611, WO2008005910, WO2008006702, WO2008006703, WO2008011453, WO2008012532, WO2008024497, WO2008024892, WO2008032164, WO2008034032, WO2008043544, WO2008044656, WO2008046758, WO2008052638, WO2008053194, WO2008071169, WO2008074384, WO2008076336, WO2008076862, WO2008078725, WO2008087654, WO2008088540, WO2008099145, WO2008101885, WO2008101886, WO2008101907, WO2008101914, WO2008106128, WO2008110196, WO2008119017, WO2008120655, WO2008127924 beschrieben sind, verabreicht.

Bei einer Ausführungsform wird die Verbindung der Formel I in Kombination mit Inhibitoren der Protein-Tyrosin-Phosphatase-1B (PTP-1B), wie sie z. B. in WO200119830-31, WO200117516, WO2004506446, WO2005012295, WO2005116003, WO2005116003, WO2006007959, DE 10 2004 060542.4, WO2007009911, WO2007028145, WO2007067612-615, WO2007081755, WO2007115058, US2008004325, WO2008033455, WO2008033931, WO2008033932, WO2008033934, WO2008089581 beschrieben sind, verabreicht.

Bei einer Ausführungsform der Erfindung wird die Verbindung der Formel I in Kombination mit einem Agonisten

des GPR109A (HM74A Rezeptor Agonisten; NAR-Agonisten (Nikotinsäurerezeptoragonisten)), wie z.B. Nicotinsäure oder "extended release niacin" in Verbindung mit MK-0524A (Laropiprant) oder MK-0524 oder solchen Verbindungen, wie sie in WO2004041274, WO2006045565, WO2006045564, WO2006069242, WO2006085108, WO2006085112, WO2006085113, WO2006124490, WO2006113150, WO2007017261, WO2007017262, WO2007017265, WO2007015744, WO2007027532, WO2007092364, WO2007120575, WO2007134986, WO2007150025, WO2007150026, WO2008016968, WO2008051403, WO2008086949, WO2008091338, WO2008097535, WO2008099448, US2008234277, WO2008127591 beschrieben sind, verabreicht.

Bei einer anderen Ausführungsform der Erfindung wird die Verbindung der Formel I in Kombination mit einer festen Kombination von Niacin mit Simvastatin verabreicht.

Bei einer anderen Ausführungsform der Erfindung wird die Verbindung der Formel I in Kombination mit Nicotinsäure oder "extended release niacin" in Verbindung mit MK-0524A (Laropiprant) verabreicht.

Bei einer weiteren Ausführungsform der Erfindung wird die Verbindung der Formel I in Kombination mit Nicotinsäure oder "extended release niacin" in Verbindung mit MK-0524A (Laropiprant) und mit Simvastatin verabreicht.

Bei einer Ausführungsform der Erfindung wird die Verbindung der Formel I in Kombination mit Nicotinsäure oder einem anderen Nicotinsäurerezeptoragonisten und einem Prostaglandin DP Rezeptorantagonisten, wie z.B. solchen wie sie in WO2008039882 beschrieben sind, verabreicht.

Bei einer anderen Ausführungsform der Erfindung wird die Verbindung der Formel I in Kombination mit einem Agonisten des GPR116, wie sie z.B. in WO2006067531, WO2006067532 beschrieben sind, verabreicht.

Bei einer Ausführungsform wird die Verbindung der Formel I in Kombination mit Modulatoren des GPR40, wie sie z.B. in WO2007013689, WO2007033002, WO2007106469, US2007265332, WO2007123225, WO2007131619, WO2007131620, WO2007131621, US2007265332, WO2007131622, WO2007136572, WO2008001931, WO2008030520, WO2008030618, WO2008054674, WO2008054675, WO2008066097, US2008176912 beschrieben sind, verabreicht.

Bei einer Ausführungsform wird die Verbindung der Formel I in Kombination mit Modulatoren des GPR119 (G-Protein-gekoppelter Glukose-abhängiger insulinotroper Rezeptor), wie z.B. PSN-119-1, PSN-821, PSN-119-2, MBX-2982 oder solchen wie sie z. B. in WO2004065380, WO2005061489 (PSN-632408), WO2006083491, WO2007003960-62 und WO2007003964, WO2007035355, WO2007116229, WO2007116230, WO2008005569, WO2008005576, WO2008008887, WO2008008895, WO2008025798, WO2008025799, WO2008025800, WO2008070692, WO2008076243, WO200807692, WO2008081204, WO2008081205, WO2008081206, WO2008081207, WO2008081208, WO2008083238, WO2008085316, WO2008109702 beschrieben sind, verabreicht.

Bei einer weiteren Ausführungsform wird die Verbindung der Formel I in Kombination mit Modulatoren des GPR120, wie sie z.B. in EP1688138, WO2008066131, WO2008066131, WO2008103500, WO2008103501 beschrieben sind, verabreicht.

Bei einer Ausführungsform wird die Verbindung der Formel I in Kombination mit Inhibitoren der hormon-sensitiven Lipase (HSL) und/oder Phospholipasen, wie z. B. in WO2005073199, WO2006074957, WO2006087309, WO2006111321, WO2007042178, WO2007119837, WO2008122352, WO2008122357 beschrieben, verabreicht.

Bei einer Ausführungsform wird die Verbindung der Formel I in Kombination mit Inhibitoren der endothelialen Lipase, wie z. B. in WO2006111321, WO2006131233, WO2006131232, WO2006131231, WO2007042178, WO2007045392, WO2007045393, WO2007110216, WO2007110215, WO2008122357, WO2008122352 beschrieben, verabreicht.

Bei einer Ausführungsform wird die Verbindung der Formel I in Kombination mit einem Phospholipase A2 Inhibitor wie z.B. Darapladib oder A-002 oder solchen, wie sie in WO2008048866, WO20080488867 beschrieben sind, verabreicht.

Bei einer Ausführungsform wird die Verbindung der Formel I in Kombination mit Myricitrin, einem Lipase-Inhibitor (WO2007119827), verabreicht.

Bei einer Ausführungsform wird die Verbindung der Formel I in Kombination mit einem Inhibitor der Glykogen Synthase Kinase-3 beta (GSK-3 beta), wie z. B. in US2005222220, WO2005085230, WO2005111018, WO2003078403, WO2004022544, WO2003106410, WO2005058908, US2005038023, WO2005009997, US2005026984, WO2005000836, WO2004106343, EP1460075, WO2004014910, WO2003076442, WO2005087727, WO2004046117, WO2007073117, WO2007083978, WO2007120102, WO2007122634, WO2007125109, WO2007125110, US2007281949, WO2008002244, WO2008002245, WO2008016123, WO2008023239, WO2008044700, WO2008056266, WO2008057940, WO2008077138, EP1939191, EP1939192, WO2008078196, WO2008094992, WO2008112642, WO2008112651, WO2008113469, WO2008121063, WO2008121064 beschrieben.

Bei einer Ausführungsform wird die Verbindung der Formel I in Kombination mit einem Inhibitor der Phosphoenolpyruvatcarboxykinase (PEPCK), wie z.B. solchen, wie in WO2004074288 beschrieben, verabreicht.

Bei einer Ausführungsform wird die Verbindung der Formel I in Kombination mit einem Inhibitor der Phosphoinositidkinase-3 (PI3K), wie z.B. solchen, wie in WO2008027584, WO2008070150, WO2008125833, WO2008125835, WO2008125839 beschrieben, verabreicht.

Bei einer Ausführungsform wird die Verbindung der Formel I in Kombination mit einem Inhibitor der Serum/Glucocorticoid regulierten Kinase (SGK), wie z. B. in WO2006072354, WO2007093264, WO2008009335, WO2008086854 beschrieben, verabreicht.

Bei einer Ausführungsform wird die Verbindung der Formel I in Kombination mit einem Modulator des Glucocorticoidrezeptors, wie z. B. in WO2008057855, WO2008057856, WO2008057857, WO2008057859, WO2008057862, WO2008059867, WO2008059866, WO2008059865, WO2008070507, WO2008124665, WO2008124745 beschrieben, verabreicht.

Bei einer Ausführungsform wird die Verbindung der Formel I in Kombination mit einem Modulator des Mineralocorticoidrezeptors (MR), wie z. B. Drospirenone, oder solchen wie sie in WO2008104306, WO2008119918 beschrieben sind, verabreicht.

Bei einer Ausführungsform wird die Verbindung der Formel I in Kombination mit einem Inhibitor der Protein Kinase C beta (PKC beta), wie z. B. Ruboxistaurin, oder solchen wie sie in WO2008096260, WO2008125945 beschrieben sind, verabreicht.

Bei einer Ausführungsform wird die Verbindung der Formel I in Kombination mit einem Inhibitor der Protein Kinase D, wie z. B. Doxazosin (WO2008088006), verabreicht.

Bei einer weiteren Ausführungsform wird die Verbindung der Formel I in Kombination mit einem Aktivator der AMP-aktivierten Proteinkinase (AMPK), wie sie z. B. in WO2007062568, WO2008006432, WO2008016278, WO2008016730, WO2008083124 beschrieben sind, verabreicht.

Bei einer Ausführungsform wird die Verbindung der Formel I in Kombination mit einem Inhibitor der Ceramidkinase, wie sie z. B. in WO2007112914, WO2007149865 beschrieben sind, verabreicht.

Bei einer weiteren Ausführungsform wird die Verbindung der Formel I in Kombination mit einem Inhibitor der MAPK-interagierenden Kinase 1 oder 2 (MNK1 oder 2), wie sie z.B. in WO2007104053, WO2007115822, WO2008008547, WO2008075741 beschrieben sind, verabreicht.

Bei einer Ausführungsform wird die Verbindung der Formel I in Kombination mit Inhibitoren der "I-kappaB kinase" (IKK Inhibitoren), wie sie z. B. in WO2001000610, WO2001030774, WO2004022057, WO2004022553, WO2005097129, WO2005113544, US2007244140, WO2008099072, WO2008099073, WO2008099073, WO2008099074, WO2008099075 beschrieben sind, verabreicht.

Bei einer anderen Ausführungsform wird die Verbindung der Formel I in Kombination mit Inhibitoren der NF-kappaB (NFKB) Aktivierung, wie sie z. B. Salsalate verabreicht.

Bei einer weiteren Ausführungsform wird die Verbindung der Formel I in Kombination mit Inhibitoren der ASK-1 (apoptosis signal-regulating kinase 1), wie sie z. B. in WO2008016131 beschrieben sind, verabreicht.

Bei einer Ausführungsform der Erfindung wird die Verbindungen der Formel I in Kombination mit einem HMG-CoA-Reduktase Inhibitor wie Simvastatin, Fluvastatin, Pravastatin, Lovastatin, Atorvastatin, Cerivastatin, Rosuvastatin, Pitavastatin, L-659699, BMS-644950 oder solchen, wie sie in US2007249583, WO2008083551 beschrieben sind, verabreicht.

Bei einer weiteren Ausführungsform der Erfindung wird die Verbindung der Formel I in Kombination mit einem Farnesoid X Rezeptor (FXR) Modulatoren, wie z.B. WAY-362450 oder solchen wie in WO2003099821, WO2005056554, WO2007052843, WO2007070796, WO2007092751, JP2007230909, WO2007095174, WO2007140174, WO2007140183, WO2008000643, WO2008002573, WO2008025539, WO2008025540, JP2008214222 beschrieben, verabreicht.

Bei einer anderen Ausführungsform der Erfindung wird die Verbindung der Formel I in Kombination mit einem Liganden des Leber X Rezeptors (liver X receptor; LXR), wie z.B. in WO2007092965, WO2008041003, WO2008049047, WO2008065754, WO2008073825, US2008242677 beschrieben, verabreicht.

Bei einer Ausführungsform der Erfindung wird die Verbindung der Formel I in Kombination mit einem Fibrat, wie z.B. Fenofibrat, Clofibrat, Bezafibrat, oder solchen wie sie in WO2008093655 beschrieben sind, verabreicht.

Bei einer Ausführungsform der Erfindung wird die Verbindung der Formel I in Kombination mit Fibraten, wie z.B. dem Cholinsalz von Fenofibrat (SLV-348), verabreicht.

Bei einer Ausführungsform der Erfindung wird die Verbindung der Formel I in Kombination mit Fibraten, wie z.B. dem Cholinsalz von Fenofibrat und einem HMGCoA Reduktase Inhibitor, wie z.B. Rosuvastatin, verabreicht.

Bei einer weiteren Ausführungsform der Erfindung wird die Verbindung der Formel I in Kombination mit Bezafibrat und Diflunisal verabreicht.

Bei einer weiteren Ausführungsform der Erfindung wird die Verbindung der Formel I in Kombination mit einer festen Kombination von Fenofibrat oder einem Salz davon mit Simvastatin, Rosuvastatin, Fluvastatin, Lovastatin, Cerivastatin, Pravastatin, Pitavastatin oder Atorvastatin verabreicht.

Bei einer weiteren Ausführungsform der Erfindung wird die Verbindung der Formel I in Kombination mit Synordia (R), einer festen Kombination von Fenofibrat mit Metformin, verabreicht.

Bei einer Ausführungsform der Erfindung wird die Verbindung der Formel I in Kombination mit einem Cholesterinresorptionsinhibitor, wie z.B. Ezetimibe, Tiqueside, Pamaqueside, FM-VP4 (sitostanol/campesterol ascorbyl phosphat; Forbes Medi-Tech, WO2005042692, WO2005005453), MD-0727 (Microbia Inc., WO2005021497, WO2005021495) oder mit Verbindungen, wie in WO2002066464, WO2005000353 (Kotobuki Pharmaceutical Co. Ltd.) oder WO2005044256 oder WO2005062824 (Merck & Co.) oder WO2005061451 und WO2005061452 (AstraZeneca AB) und WO2006017257 (Phenomix) oder WO2005033100 (Lipideon Biotechnology AG) oder wie in WO2002050060, WO2002050068, WO2004000803, WO2004000804, WO2004000805, WO2004087655, WO2004097655, WO2005047248, WO2006086562, WO2006102674, WO2006116499, WO2006121861, WO2006122186, WO2006122216, WO2006127893, WO2006137794, WO2006137796, WO2006137782, WO2006137793, WO2006137797, WO2006137795, WO2006137792, WO2006138163, WO2007059871, US2007232688, WO2007126358, WO2008033431, WO2008033465, WO2008052658, WO2008057336, WO2008085300 beschrieben, verabreicht.

Bei einer Ausführungsform der Erfindung wird die Verbindung der Formel I in Kombination mit einem NPC1L1-Antagonisten, wie z.B. solchen, wie sie in WO2008033464, WO2008033465 beschrieben sind, verabreicht.

Bei einer Ausführungsform der Erfindung wird die Verbindung der Formel I in Kombination mit Vytorin™, einer festen Kombination von Ezetimibe mit Simvastatin, verabreicht.

Bei einer Ausführungsform der Erfindung wird die Verbindung der Formel I in Kombination mit einer festen Kombination von Ezetimibe mit Atorvastatin, verabreicht.

Bei einer Ausführungsform der Erfindung wird die Verbindung der Formel I in Kombination mit einer festen Kombination von Ezetimibe mit Fenofibrat verabreicht.

Bei einer Ausführungsform der Erfindung ist der weitere Wirkstoff ein Diphenylazetidinonderivat, wie z.B. in US 6,992,067 oder US 7,205,290 beschrieben.

Bei einer weiteren Ausführungsform der Erfindung ist der weitere Wirkstoff ein Diphenylazetidinonderivat, wie z.B. in US 6,992,067 oder US 7,205,290 beschrieben, kombiniert mit einem Statin, wie z.B. Simvastatin, Fluvastatin, Pravastatin, Lovastatin, Cerivastatin, Atorvastatin, Pitavastatin oder Rosuvastatin.

Bei einer Ausführungsform der Erfindung wird die Verbindung der Formel I in Kombination mit einer festen Kombination von Lapaquistat, einem Squalensynthase-Inhibitor, mit Atorvastatin verabreicht.

Bei einer Ausführungsform der Erfindung wird die Verbindung der Formel I in Kombination mit einem CETP-Inhibitor, wie z.B. Torcetrapib, Anacetrapib oder JTT-705 (Dalcetrapib) oder solchen wie sie in WO2006002342, WO2006010422, WO2006012093, WO2006073973, WO2006072362, WO2007088996, WO2007088999, US2007185058, US2007185113, US2007185154, US2007185182, WO2006097169, WO2007041494, WO2007090752, WO2007107243, WO2007120621, US2007265252, US2007265304, WO2007128568, WO2007132906, WO2008006257, WO2008009435, WO2008018529, WO2008058961, WO2008058967, WO2008059513, WO2008070496, WO2008115442, WO2008111604 beschrieben sind, verabreicht.

Bei einer Ausführungsform der Erfindung wird die Verbindung der Formel I in Kombination mit Gallensäureresorptionsinhibitoren (Inhibitoren des intestinalen Gallensäuretransporters (IBAT)) (siehe z.B. US 6,245,744, US 6,221,897 oder WO00/61568), wie z.B. HMR 1741 oder solchen wie in DE 10 2005 033099.1 und DE 10 2005 033100.9, DE 10 2006 053635, DE 10 2006 053637, WO2007009655-56, WO2008058628, WO2008058629, WO2008058630, WO2008058631 beschrieben, verabreicht.

Bei einer Ausführungsform wird die Verbindung der Formel I in Kombination mit Agonisten des GPBAR1 (G-protein-coupled-bile-acid-receptor-1; TGR5), wie sie z.B. in US20060199795, WO2007110237, WO2007127505, WO2008009407, WO2008067219, WO2008067222, FR2908310, WO2008091540, WO2008097976 beschrieben sind, verabreicht.

Bei einer Ausführungsform wird die Verbindung der Formel I in Kombination mit Inhibitoren des TRPM5 Kanals (TRP-Cation-Channel-M5), wie sie z.B. in WO2008097504 beschrieben sind, verabreicht.

Bei einer Ausführungsform der Erfindung wird die Verbindung der Formel I in Kombination mit einem polymeren Gallensäureadsorber, wie z.B. Cholestyramin, Colesevelam Hydrochlorid, verabreicht.

Bei einer Ausführungsform der Erfindung wird die Verbindung der Formel I in Kombination mit Colesevelam Hydrochlorid und Metformin oder einem Sulfonylharnstoff oder Insulin verabreicht.

Bei einer Ausführungsform der Erfindung wird die Verbindung der Formel I in Kombination mit einem Phytosterole enthaltenden Kaugummi (Reductol™) verabreicht.

Bei einer Ausführungsform der Erfindung wird die Verbindung der Formel I in Kombination mit einem Inhibitor des mikrosomalen Triglycerid-Transfer-Proteins (MTP-Inhibitor), wie z.B. Implitapide , BMS-201038, R-103757, AS-1552133, SLx-4090, AEGR-733 oder solchen wie in WO2005085226, WO2005121091, WO2006010423, WO2006113910, WO2007143164, WO2008049806, WO2008049808, WO2008090198, WO2008100423 beschrieben, verabreicht.

Bei einer weiteren Ausführungsform der Erfindung wird die Verbindung der Formel I in Kombination mit einer Kombinbation eines Cholesterolabsorptionsinhibitors, wie z.B. Ezetimibe, und einem Inhibitor des Triglycerid-Transfer-Proteins (MTP-Inhibitor), wie z.B. Implitapide, wie in WO2008030382 oder in WO2008079398 beschrieben, verabreicht.

Bei einer Ausführungsform der Erfindung wird die Verbindung der Formel I in Kombination mit einem antihypertriglyceridämischen Wirkstoff, wie z.B. solchen wie sie in WO2008032980 beschrieben sind, verabreicht.

Bei einer anderen Ausführungsform der Erfindung wird die Verbindung der Formel I in Kombination mit einem Antagonisten des Somatostatin 5 Rezeptors (SST5 Rezeptor), wie z.B. solchen wie sie in WO2006094682 beschrieben sind, verabreicht.

Bei einer Ausführungsform der Erfindung wird die Verbindung der Formel I in Kombination mit einem ACAT-Inhibitor, wie z.B. Avasimibe, SMP-797 oder KY-382 oder solchen, wie sie in WO2008087029, WO2008087030, WO2008095189 beschrieben sind, verabreicht.

Bei einer weiteren Ausführungsform der Erfindung wird die Verbindung der Formel I in Kombination mit einem Inhibitor der Leber-Carnitin Palmitoyltransferase-1 (L-CPT1), wie sie z.B. in WO2007063012, WO2007096251 (ST-3473), WO2008015081, US2008103182, WO2008074692 beschrieben sind, verabreicht.

Bei einer weiteren Ausführungsform der Erfindung wird die Verbindung der Formel I in Kombination mit einem Modulator der Serin-Palmitoyltransferase (SPT), wie sie z.B. in WO2008031032, WO2008046071, WO2008083280, WO2008084300 beschrieben sind, verabreicht.

Bei einer Ausführungsform der Erfindung wird die Verbindung der Formel I in Kombination mit einem Squalen Synthetase Inhibitor, wie z.B. BMS-188494, TAK-475 (Lapaquistat Acetat) oder wie in WO2005077907, JP2007022943, WO2008003424 beschrieben, verabreicht.

Bei einer Ausführungsform der Erfindung wird die Verbindung der Formel I in Kombination mit ISIS-301012 (Mipomersen), einem Antisense-Oligonukleotid, welches in der Lage ist, das Apolipoprotein B Gen zu regulieren, verabreicht.

Bei einer Ausführungsform der Erfindung wird die Verbindung der Formel I in Kombination mit einem Stimulator des ApoA-1 Gens, wie er z.B. in WO2008092231 beschrieben ist, verabreicht.

Bei einer Ausführungsform der Erfindung wird die Verbindung der Formel I in Kombination mit einem LDL-Rezeptorinducer (siehe US 6,342,512), wie z.B. HMR1171, HMR1586, oder solchen wie in WO2005097738, WO2008020607 beschrieben, verabreicht.

Bei einer anderen Ausführungsform der Erfindung wird die Verbindung der Formel I in Kombination mit einem HDL-Cholesterol-erhöhenden Agens, wie z.B. solchen wie sie in WO2008040651, WO2008099278 beschrieben sind, verabreicht.

Bei einer Ausführungsform der Erfindung wird die Verbindung der Formel I in Kombination mit einem ABCA1 Expressionsverstäker, wie sie z.B. in WO2006072393, WO2008062830 beschrieben, verabreicht.

Bei einer Ausführungsform der Erfindung wird die Verbindung der Formel I in Kombination mit einem Lipoprotein-Lipase Modulator, wie z.B. Ibrolipim (NO-1886), verabreicht.

Bei einer Ausführungsform der Erfindung wird die Verbindung der Formel I in Kombination mit einem Lipoprotein(a) antagonist, wie z.B. Gemcabene (CI-1027) verabreicht.

Bei einer Ausführungsform der Erfindung wird die Verbindung der Formel I in Kombination mit einem Lipase Inhibitor, wie z.B. Orlistat oder Cetilistat (ATL-962), verabreicht.

Bei einer Ausführungsform der Erfindung wird die Verbindung der Formel I in Kombination mit einem Adenosin A1 Rezeptor Agonisten (Adenosin A1 R), wie sie z.B. in EP1258247, EP1375508, WO2008028590, WO2008077050 beschrieben sind, verabreicht.

Bei einer Ausführungsform der Erfindung wird die Verbindung der Formel I in Kombination mit einem Adenosin A2B Rezeptor Agonisten (Adenosin A2B R) wie z.B. ATL-801 verabreicht.

Bei einer anderen Ausführungsform der Erfindung wird die Verbindung der Formel I in Kombination mit einem Modulator der Adenosin A2A und/oder Adenosin A3 Rezeptoren, wie z.B. in WO2007111954, WO2007121918, WO2007121921, WO2007121923, WO2008070661 beschrieben, verabreicht.

Bei einer weiteren Ausführungsform der Erfindung wird die Verbindung der Formel I in Kombination mit einem Agonisten der Adenosin A1/A2B Rezeptoren, wie z.B. in WO2008064788, WO2008064789 beschrieben, verabreicht.

Bei einer Ausführungsform der Erfindung wird die Verbindung der Formel I in Kombination mit einem Adenosin A2B Rezeptor Antagonisten (Adenosin A2B R), wie sie in US2007270433, WO2008027585, WO2008080461 beschrieben sind, verabreicht.

Bei einer Ausführungsform wird die Verbindung der Formel I in Kombination mit Hemmstoffen der Acetyl-CoA Carboxylase (ACC1 und/oder ACC2) wie z. B. solchen wie in WO199946262, WO200372197, WO2003072197, WO2005044814, WO2005108370, JP2006131559, WO2007011809, WO2007011811, WO2007013691, WO2007095601-603, WO2007119833, WO2008065508, WO2008069500, WO2008070609, WO2008072850, WO2008079610, WO2008088688, WO2008088689, WO2008088692, US2008171761, WO2008090944, JP2008179621, US2008200461, WO2008102749, WO2008103382, WO2008121592 beschrieben, verabreicht.

Bei einer anderen Ausführungsform wird die Verbindung der Formel I in Kombination mit Modulatoren der mikrosomalen Acyl-CoA:Glycerol-3-Phosphat-Acyltransferase 3 (GPAT3, beschrieben in WO2007100789) oder mit Modulatoren der mikrosomalen Acyl-CoA:Glycerol-3-Phosphat-Acyltransferase 4 (GPAT4, beschrieben in WO2007100833) verabreicht.

Bei einer weiteren Ausführungsform wird die Verbindung der Formel I in Kombination mit Modulatoren der Xanthin-Oxidoreductase (XOR) verabreicht.

Bei einer anderen Ausführungsform wird die Verbindung der Formel I in Kombination mit Inhibitoren der löslichen Epoxidhydrolase (sEH), wie sie z.B. in WO2008051873, WO2008051875, WO2008073623, WO2008094869, WO2008112022 beschrieben sind, verabreicht.

Bei einer weiteren Ausführungsform wird die Verbindung der Formel I in Kombination mit CART-Modulatoren (siehe "Cocaine-amphetamine-regulated transcript influences energy metabolism, anxiety and gastric emptying in mice" Asakawa, A. et al.: Hormone and Metabolic Research (2001), 33(9), 554-558);

NPY-Antagonisten wie z.B. Naphthalin-1-sulfonsäure-{4-[(4-amino-quinazolin-2-ylamino)-methyl]-cyclohexylmethyl}-amid Hydrochlorid (CGP 71683A) oder Velneperit;

NPY-5 Rezeptorantagonisten wie L-152804 oder die Verbindung "NPY-5-BY" der Firma Banyu oder wie sie z. B. in WO2006001318, WO2007103295, WO2007125952, WO2008026563, WO2008026564, WO2008052769, WO2008092887, WO2008092888, WO2008092891 beschrieben sind;

NPY-4-Rezeptorantagonisten wie sie z. B. in WO2007038942 beschrieben sind;

NPY-2-Rezeptorantagonisten wie sie z. B. in WO2007038943 beschrieben sind;

Peptid YY 3-36 (PYY3-36) oder analoge Verbindungen wie z. B. CJC-1682 (PYY3-36 konjugiert mit humanem Serum Albumin über Cys34) oder CJC-1643 (Derivat des PYY3-36, welches sich in vivo an Serum Albumin konjugiert) oder solche, wie sie in WO2005080424, WO2006095166, WO2008003947 beschrieben sind;

Derivaten des Peptids Obestatin wie sie WO2006096847 beschrieben sind;

CB1 R (Cannabinoid Rezeptor 1) Antagonisten wie z.B. Rimonabant, Surinabant (SR147778), SLV-319 (Ibipinabant), AVE-1625, Taranabant (MK-0364) oder Salze davon, Otenabant (CP-945,598), Rosonabant, V-24343 oder solche Verbindungen wie sie in z. B. EP 0656354, WO 00/15609, WO2001/64632-64634, WO 02/076949, WO2005080345, WO2005080328, WO2005080343, WO2005075450, WO2005080357, WO200170700, WO2003026647-48, WO200302776, WO2003040107, WO2003007887, WO2003027069, US6,509,367, WO200132663, WO2003086288, WO2003087037, WO2004048317, WO2004058145, WO2003084930, WO2003084943, WO2004058744, WO2004013120, WO2004029204, WO2004035566, WO2004058249, WO2004058255, WO2004058727, WO2004069838, US20040214837, US20040214855, US20040214856, WO2004096209, WO2004096763, WO2004096794, WO2005000809, WO2004099157, US20040266845, WO2004110453, WO2004108728, WO2004000817, WO2005000820, US20050009870, WO200500974, WO2004111033-34, WO200411038-39, WO2005016286, WO2005007111, WO2005007628, US20050054679, WO2005027837, WO2005028456, WO2005063761-62, WO2005061509, WO2005077897, WO2006018662, WO2006047516, WO2006060461, WO2006067428, WO2006067443, WO2006087480, WO2006087476, WO2006100208, WO2006106054, WO2006111849, WO2006113704, WO2007009705, WO2007017124, WO2007017126, WO2007018459, WO2007018460, WO2007016460, WO2007020502, WO2007026215, WO2007028849, WO2007031720, WO2007031721, WO2007036945, WO2007038045, WO2007039740, US20070015810, WO2007046548, WO2007047737, WO2007057687, WO2007062193, WO2007064272, WO2007079681, WO2007084319, WO2007084450, WO2007086080, EP1816125, US2007213302, WO2007095513, WO2007096764, US2007254863, WO2007119001, WO2007120454, WO2007121687, WO2007123949, US2007259934, WO2007131219, WO2007133820, WO2007136571, WO2007136607, WO2007136571, US7297710, WO2007138050, WO2007139464, WO2007140385, WO2007140439, WO2007146761, WO2007148061, WO2007148062, US2007293509, WO2008004698, WO2008017381, US2008021031, WO2008024284, WO2008031734, WO2008032164, WO2008034032, WO2008035356, WO2008036021, WO2008036022, WO2008039023, WO2998043544, WO2008044111, WO2008048648, EP1921072-A1, WO2008053341, WO2008056377, WO2008059207, WO2008059335, WO2008062424, WO2008068423, WO2008068424, WO2008070305, WO2008070306, WO2008074816, WO2008074982, WO2008075012, WO2008075013, WO2008075019, WO2008075118, WO2008076754, WO2008081009, WO2008084057, EP1944295, US2008090809, US2008090810, WO2008092816, WO2008094473, WO2008094476, WO2008099076, WO2008099139, WO2008101995, US2008207704, WO2008107179, WO2008109027, WO2008112674, WO2008115705, WO2008118414, WO2008119999, WO200812000, WO2008121257, WO2008127585 beschrieben sind;

Cannabinoid Rezeptor 1 / Cannabinoid Rezeptor 2 (CB1/CB2) modulierende Verbindungen wie z.B. delta-9-Tetrahydrocannabivarin oder solchen wie sie z.B. in WO2007001939, WO2007044215, WO2007047737, WO2007095513, WO2007096764, WO2007112399, WO2007112402, WO2008122618 beschrieben sind;

Modulatoren der FAAH (fatty acid amide hydrolase) wie sie z.B. in WO2007140005, WO2008019357, WO2008021625, WO2008023720, WO2008030532 beschrieben sind;

Inhibitoren der Fettsäuresynthase (fatty acid synthase; FAS), wie sie z.B. in WO2008057585, WO2008059214, WO2008075064, WO2008075070, WO2008075077 beschrieben sind;

Inhibitoren der LCE (long chain fatty acid elongase), wie sie z.B. in WO2008120653 beschrieben sind;

Vanilloid-1-Rezeptor Modulatoren (Modulatoren des TRPV1), wie sie z.B. in WO2007091948, WO2007129188,

WO2007133637, WO2008007780, WO2008010061, WO2008007211, WO2008010061, WO2008015335, WO2008018827, WO2008024433, WO2008024438, WO2008032204, WO2008050199, WO2008059339, WO2008059370, WO2008066664, WO2008075150, WO2008090382, WO2008090434, WO2008093024, WO2008107543, WO2008107544, WO2008110863 beschrieben sind;

Modulatoren, Antagonisten oder inverse Agonisten der Opioidrezeptoren, wie z.B. GSK-982 oder solche wie sie z.B. in WO2007047397, WO2008021849, WO2008021851, WO2008032156, WO2008059335 beschrieben sind;

Modulatoren des "orphan opioid (ORL-1) receptor" wie sie z.B. in US2008249122, WO2008089201 beschrieben sind;

Agonisten des Prostaglandinrezeptors, wie z.B. Bimatoprost oder solchen Verbindungen wie sie in WO2007111806 beschrieben sind;

MC4-Rezeptor Agonisten (Melanocortin-4 Rezeptor Agonisten, MC4R Agonisten wie z.B. 1-Amino-1,2,3,4-tetrahydro-naphthalin-2-carbonsäure [2-(3a-benzyl-2-methyl-3-oxo-2,3,3a,4,6,7-hexahydro-pyrazolo[4,3-c]pyridin-5-yl)-1-(4-chloro-phenyl)-2-oxo-ethyl]-amid; (WO 01/91752)) oder LB53280, LB53279, LB53278 oder THIQ, MB243, RY764, CHIR-785, PT-141, MK-0493 oder solche wie sie in WO2005060985, WO2005009950, WO2004087159, WO2004078717, WO2004078716, WO2004024720, US20050124652, WO2005051391, WO2004112793, WOUS20050222014, US20050176728, US20050164914, US20050124636, US20050130988, US20040167201, WO2004005324, WO2004037797, WO2005042516, WO2005040109, WO2005030797, US20040224901, WO200501921, WO200509184, WO2005000339, EP1460069, WO2005047253, WO2005047251, WO2005118573, EP1538159, WO2004072076, WO2004072077, WO2006021655-57, WO2007009894, WO2007015162, WO2007041061, WO2007041052, JP2007131570, EP-1842846, WO2007096186, WO2007096763, WO2007141343, WO2008007930, WO2008017852, WO2008039418, WO2008087186, WO2008087187, WO2008087189, WO2008087186-WO2008087190, WO2008090357 beschrieben sind;

Orexin-Rezeptor 1 Antagonisten (OX1R Antagonisten), Orexin-Rezeptor 2 Antagonisten (OX2R Antagonisten) oder gemischte OX1R/OX2R Antagonisten (z.B. 1-(2-Methyl-benzoxazol-6-yl)-3-[1,5]naphthyridin-4-yl-harnstoff Hydrochlorid (SB-334867-A) oder solche, wie sie z. B. in WO200196302, WO200185693, WO2004085403, WO2005075458, WO2006067224, WO2007085718, WO2007088276, WO2007116374, WO2007122591, WO2007126934, WO2007126935, WO2008008517, WO2008008518, WO2008008551, WO2008020405, WO2008026149, WO2008038251, US2008132490, WO2008065626, WO2008078291, WO2008087611, WO2008081399, WO2008108991, WO2008107335, US2008249125 beschrieben sind);

Histamin H3 Rezeptor Antagonisten/inverse Agonisten (z. B. 3-Cyclohexyl-1-(4,4-dimethyl-1,4,6,7-tetrahydro-imidazo[4,5-c]pyridin-5-yl)-propan-1-on Oxalsäuresalz (WO 00/63208) oder solche, wie sie in WO200064884, WO2005082893, US2005171181 (z.B. PF-00389027), WO2006107661, WO2007003804, WO2007016496, WO2007020213, WO2007049798, WO2007055418, WO2007057329, WO2007065820, WO2007068620, WO2007068641, WO2007075629, WO2007080140, WO2007082840, WO2007088450, WO2007088462, WO2007094962, WO2007099423, WO2007100990, WO2007105053, WO2007106349, WO2007110364, WO2007115938, WO2007131907, WO2007133561, US2007270440, WO2007135111, WO2007137955, US2007281923, WO2007137968, WO2007138431, WO2007146122, WO2008005338, WO2008012010, WO2008015125, WO2008045371, EP1757594, WO2008068173, WO2008068174, US20080171753, WO2008072703, WO2008072724, US2008188484, US2008188486, US2008188487, WO2008109333, WO2008109336 beschrieben sind);

Histamin H1 / Histamin H3 Modulatoren, wie z. B. Betahistin bzw. seinem Dihydrochlorid;

Modulatoren des Histamin H3 Transporters oder der Histamin H3 / Serotonin Transporter wie sie z.B. in WO2008002816, WO2008002817, WO2008002818, WO2008002820 beschrieben sind;

Histamin H4 Modulatoren wie sie z.B. in WO2007117399 beschrieben sind;

CRF-Antagonisten (z.B. [2-Methyl-9-(2,4,6-trimethyl-phenyl)-9H-1,3,9-triaza-fluoren-4-yl]-dipropyl-amin (WO 00/66585) oder solche CRF1-Antagonisten, wie sie in WO2007105113, WO2007133756, WO2008036541, WO2008036579, WO2008083070 beschrieben sind);

CRF BP-Antagonisten (z.B. Urocortin);

Urocortin-Agonisten;

Modulatoren des beta-3 Adrenoceptors wie z.B. 1-(4-Chloro-3-methanesulfonylmethyl-phenyl)-2-[2-(2,3-dimethyl-1H-indol-6-yloxy)-ethylamino]-ethanol Hydrochlorid (WO 01/83451) oder Solabegron (GW-427353) oder N-5984 (KRP-204) oder solche, wie sie in JP2006111553, WO2002038543, WO2002038544, WO2007048840-843, WO2008015558, EP1947103 beschrieben sind;

MSH (Melanocyt-stimulierendes Hormon)-Agonisten;

MCH (melanin-konzentrierendes Hormon) Rezeptor Antagonisten (wie z. B. NBI-845, A-761, A-665798, A-798, ATC-0175, T-226296, T-71 (AMG-071, AMG-076), GW-856464, NGD-4715, ATC-0453, ATC-0759, GW-803430 oder solche Verbindungen, wie sie in WO2005085200, WO2005019240, WO2004011438, WO2004012648, WO2003015769, WO2004072025, WO2005070898, WO2005070925, WO2004039780, WO2004092181, WO2003033476, WO2002006245, WO2002089729, WO2002002744, WO2003004027, FR2868780, WO2006010446, WO2006038680,

WO2006044293, WO2006044174, JP2006176443, WO2006018280, WO2006018279, WO2006118320, WO2006130075, WO2007018248, WO2007012661, WO2007029847, WO2007024004, WO2007039462, WO2007042660, WO2007042668, WO2007042669, US2007093508, US2007093509, WO2007048802, JP2007091649, WO2007092416; WO2007093363-366, WO2007114902, WO2007114916, WO2007141200, WO2007142217, US2007299062, WO2007146758, WO2007146759, WO2008001160, WO2008016811, WO2008020799, WO2008022979, WO2008038692, WO2008041090, WO2008044632, WO2008047544, WO2008061109, WO2008065021, WO2008068265, WO2008071646, WO2008076562, JP2008088120, WO2008086404, WO2008086409 beschrieben sind);
CCK-A (CCK-1) Agonisten/Modulatoren (wie z.B. {2-[4-(4-Chloro-2,5-dimethoxyphenyl)-5-(2-cyclohexyl-ethyl)-thiazol-2-ylcarbamoyl]-5,7-dimethyl-indol-1-yl}-essigsäure Trifluoressigsäuresalz (WO 99/15525) oder SR-146131 (WO 0244150) oder SSR-125180) oder solchen, wie sie in WO2005116034, WO2007120655, WO2007120688, WO2007120718, WO2008091631 beschrieben sind;
Serotonin-Wiederaufnahme-Inhibitoren (z.B. Dexfenfluramine) oder solchen wie sie in WO2007148341, WO2008034142, WO2008081477, WO2008120761 beschrieben sind;
gemischte Serotonin-/Dopamin-Wiederaufnahme-Inhibitoren (z.B. Bupropion) oder solche wie sie in WO2008063673 beschrieben sind oder feste Kombinationen von Bupropion mit Naltrexon oder Bupropion mit Zonisamid;
gemischte Wiederaufnahmeinhibitoren wie z.B. DOV-21947;
gemischte Sertonin- und noradrenerge Verbindungen (z.B. WO 00/71549);
5-HT-Rezeptor Agonisten z.B. 1-(3-Ethyl-benzofuran-7-yl)-piperazin Oxalsäuresalz (WO 01/09111);
gemischte Dopamin/Norepinephrin/Acetylcholin-Wiederaufnahme-Inhibitoren (z.B. Tesofensine) oder solchen wie sie z.B. in WO2006085118 beschrieben sind;
Dopaminantagonisten wie sie z.B. in WO2008079838, WO2008079839, WO2008079847, WO2008079848 beschrieben sind;
Norepinephrin-Wiederaufnahme-Inhibitoren wie sie z.B. in US2008076724 beschrieben sind;
5-HT2A Rezeptor Antagonisten wie sie z.B. in WO2007138343 beschrieben sind;
5-HT2C Rezeptor Agonisten (wie z.B. Lorcaserin Hydrochlorid (APD-356) oder BVT-933 oder solche, wie sie in WO200077010, WO200077001-02, WO2005019180, WO2003064423, WO200242304, WO2005035533, WO2005082859, WO2006004937, US2006025601, WO2006028961, WO2006077025, WO2006103511, WO2007028132, WO2007084622, US2007249709; WO2007132841, WO2007140213, WO2008007661, WO2008007664, WO2008009125, WO2008010073, WO2008108445 beschrieben sind);
5-HT6 Rezeptor Modulatoren, wie z.B. E-6837, BVT-74316 oder PRX-07034 oder solche wie sie z.B. in WO2005058858, WO2007054257, WO2007107373, WO2007108569, WO2007108742-744, WO2008003703, WO2008027073, WO2008034815, WO2008054288, EP1947085, WO2008084491, WO2008084492, WO2008092665, WO2008092666, WO2008101247, WO2008110598, WO2008116831, WO2008116833 beschrieben sind;
Agonisten des Estrogenrezeptors gamma (ERRγ Agonisten), wie sie z.B. in WO2007131005, WO2008052709 beschrieben sind;
Agonisten des Estrogenrezeptors alpha (ERRα / ERR1 Agonisten), wie sie z.B. in WO2008109727 beschrieben sind;
Sigma-1 Rezeptorantagonisten, wie sie z.B. in WO2007098953, WO2007098961, WO2008015266, WO2008055932, WO2008055933 beschrieben sind;
Muscarin 3 Rezeptor (M3R) Antagonisten, wie sie z.B. in WO2007110782, WO2008041184 beschrieben sind;
Bombesin-Rezeptor Agonisten (BRS-3 Agonisten), wie sie z.B. in WO2008051404, WO2008051405, WO2008051406, WO2008073311 beschrieben sind;
Galanin-Rezeptor Antagonisten;
Wachstumshormon (z.B. humanes Wachstumshormon oder AOD-9604);
Wachstumshormon freisetzende Verbindungen (6-Benzyloxy-1-(2-diisopropylamino-ethylcarbamoyl)-3,4-dihydro-1H-isochinolin-2-carbonsäuretertiärbutylester (WO 01/85695));
Growth Hormone Secretagogue Receptor Antagonisten (Ghrelin Antagonisten) wie z. B. A-778193 oder solchen, wie sie in WO2005030734, WO2007127457, WO2008008286 beschrieben sind;
Growth Hormone Secretagogue Receptor Modulatoren (Ghrelin-Modulatoren) wie z.B. JMV-2959, JMV-3002, JMV-2810, JMV-2951 oder solchen, wie sie in WO2006012577 (z.B. YIL-781 oder YIL-870), WO2007079239, WO2008092681 beschrieben sind;
TRH-Agonisten (siehe z.B. EP 0 462 884);
entkoppelnde Protein 2- oder 3-Modulatoren;
chemische Entkoppler (z.B. WO2008059023, WO2008059024, WO2008059025, WO2008059026);
Leptinagonisten (siehe z.B. Lee, Daniel W.; Leinung, Matthew C.; Rozhavskaya-Arena, Marina; Grasso, Patricia. Leptin agonists as a potential approach to the treatment of obesity. Drugs of the Future (2001), 26(9), 873-881);
DA-Agonisten (Bromocriptin, Doprexin);
Lipase/Amylase-Inhibitoren (z.B. WO 00/40569, WO2008107184);

Inhibitoren der Diacylglycerol O-Acyltransferasen (DGATs) wie z. B. BAY-74-4113 oder wie z. B. in US2004/0224997, WO2004094618, WO200058491, WO2005044250, WO2005072740, JP2005206492, WO2005013907, WO2006004200, WO2006019020, WO2006064189, WO2006082952, WO2006120125, WO2006113919, WO2006134317, WO2007016538, WO2007060140, JP2007131584, WO2007071966, WO2007126957, WO2007137103, WO2007137107, WO2007138304, WO2007138311, WO2007141502, WO2007141517, WO2007141538, WO2007141545, WO2007144571, WO2008011130, WO2008011131, WO2008039007, WO2008048991, WO2008067257, WO2008099221 beschrieben;
Inhibitoren der Monoacylglycerolacyltransferase (2-Acylglycerol-O-Acyltransferase; MGAT) wie sie z.B. in WO2008038768 beschrieben sind;
Inhibitoren der Fettsäuresynthase (FAS) wie z.B. C75 oder solchen, wie in WO2004005277, WO2008006113 beschrieben;
Inhibitoren der Stearoyl-CoA delta9 Desaturase (SCD1) wie sie z.B. in WO2007009236, WO2007044085, WO2007046867, WO2007046868, WO20070501124, WO2007056846, WO2007071023, WO2007130075, WO2007134457, WO2007136746, WO2007143597, WO2007143823, WO2007143824, WO2008003753, WO2008017161, WO2008024390, WO2008029266, WO2008036715, WO2008043087, WO2008044767, WO2008046226, WO2008056687, WO2008062276, WO2008064474, WO2008074824, WO2008074832, WO2008074833, WO2008074834, WO2008074835, WO2008089580, WO2008096746, WO2008104524, WO2008116898, US2008249100, WO2008120744, WO2008120759, WO2008123469, WO2008127349 beschrieben sind;
Inhibitoren der Fatty-Acid-Desaturase-1 (delta5 Desaturase) wie sie z.B. in WO2008089310 beschrieben sind;
hypoglykämische/hypertriglyceridämische Indolinverbindungen wie sie in WO2008039087 beschrieben sind;
Inhibitoren des "Adipocyte fatty acid-binding protein aP2" wie z.B. BMS-309403;
Aktivatoren der Adiponectinsekretion, wie z.B. in WO2006082978, WO2008105533 beschrieben;
Promotoren der Adiponectinproduktion, wie z.B. in WO2007125946, WO2008038712 beschrieben;
modifizierte Adiponectine wie z.B. in WO2008121009 beschrieben;
Oxyntomodulin oder Analoga davon;
Oleoyl-Estron;
oder Agonisten oder partiellen Agonisten des Schilddrüsenhormonrezeptors (thyroid hormone receptor agonists) wie z. B: KB-2115 (Eprotirome), QRX-431 (Sobetirome) oder DITPA oder solche, wie in WO20058279, WO200172692, WO200194293, WO2003084915, WO2004018421, WO2005092316, WO2007003419, WO2007009913, WO2007039125, WO2007110225, WO2007110226, WO2007128492, WO2007132475, WO2007134864, WO2008001959, WO2008106213 beschrieben;
oder Agonisten des Schilddrüsenhormonrezeptors beta (TR-beta) wie z. B. MB-07811 oder MB-07344, oder solchen wie in WO2008062469 beschrieben, verabreicht.

Bei einer Ausführungsform der Erfindung wird die Verbindung der Formel I in Kombination mit einer Kombination von Eprotirome mit Ezetimibe verabreicht.

Bei einer Ausführungsform der Erfindung wird die Verbindung der Formel I in Kombination mit einem Inhibitor der Site-1 Protease (S1 P), wie z.B. PF-429242, verabreicht.

Bei einer weiteren Ausführungsform der Erfindung wird die Verbindung der Formel I in Kombination mit einem Modulator des "Trace-Amine-Associated-Receptor-1" (TAAR1), wie sie z.B. in US2008146523, WO2008092785 beschrieben sind, verabreicht.

Bei einer Ausführungsform der Erfindung wird die Verbindung der Formel I in Kombination mit einem Inhibitor des Growth-Factor-Receptor-Bound-Protein-2 (GRB2), wie z.B. in WO2008067270 beschrieben, verabreicht.

Bei einer weiteren Ausführungsform der Erfindung wird die Verbindung der Formel I in Kombination mit einem RNAi (siRNA) Therapeutikum, welches gegen PCSK9 (Proprotein Convertase Subtilisin/Kexin Typ 9) gerichtet ist, verabreicht.

Bei einer Ausführungsform wird die Verbindung der Formel I in Kombination mit Omacor® oder Lovaza™ (Omega-3-Fettsäureester; hochkonzentrierte Ethylester der Eicosapentaensäure und der Docosahexaensäure) verabreicht.

Bei einer Ausführungsform wird die Verbindung der Formel I in Kombination mit Lycopin verabreicht.

Bei einer Ausführungsform der Erfindung wird die Verbindung der Formel I in Kombination mit einem Antioxidans, wie z.B. OPC-14117, AGI-1067 (Succinobucol), Probucol, Tocopherol, Ascorbinsäure, β-Caroten oder Selen verabreicht.

Bei einer Ausführungsform der Erfindung wird die Verbindung der Formel I in Kombination mit einem Vitamin, wie z. B. Vitamin B6 oder Vitamin B12 verabreicht.

Bei einer Ausführungsform wird die Verbindung der Formel I in Kombination mit mehr als einer der vorstehend genannten Verbindungen, z.B. in Kombination mit einem Sulfonylharnstoff und Metformin, einem Sulfonylharnstoff und Acarbose, Repaglinide und Metformin (PrandiMet (TM)), Insulin und einem Sulfonylharnstoff, Insulin und Metformin,

Insulin und Troglitazon, Insulin und Lovastatin, etc. verabreicht.

Bei einer anderen Ausführungsform wird die Verbindung der Formel I in Kombination mit einem Inhibitor der Carboanhydrase Typ 2 (Carbonic anhydrase type 2), wie z.B. solchen, wie in WO2007065948 beschrieben, verabreicht.

Bei einer anderen Ausführungsform wird die Verbindung der Formel I in Kombination mit Topiramat oder einem Derivat davon, wie es in WO2008027557 beschrieben ist, verabreicht.

Bei einer weiteren Ausführungsform wird die Verbindung der Formel I in Kombination mit einer festen Kombination von Topiramat mit Phentermin (Qnexa™) verabreicht.

Bei einer weiteren Ausführungsform wird die Verbindung der Formel I in Kombination mit einer Antisense-Verbindung, z.B. ISIS-377131, verabreicht, welche die Produktion des Glukokortikoidrezeptors inhibiert.

Bei einer anderen Ausführungsform wird die Verbindung der Formel I in Kombination mit einem Aldosteronsynthaseinhibitor und einem Antagonisten des Glucocorticoidrezeptors, einem Cortisolsyntheseinhibitor und/oder einem Antagonisten des Corticotropin-freisetzenden Faktors (corticotropin releasing factor), wie z.B. in EP1886695, WO2008119744 beschrieben, verabreicht.

Bei einer Ausführungsform wird die Verbindung der Formel I in Kombination mit einem Agonisten des RUP3 Rezeptors, wie z. B. in WO2007035355, WO2008005576 beschrieben, verabreicht.

Bei einer anderen Ausführungsform wird die Verbindung der Formel I in Kombination mit einem Aktivator des Gens, welches für die Ataxia Telangiectasia Mutated (ATM) Proteinkinase kodiert, wie z. B. Chloroquin, verabreicht.

Bei einer Ausführungsform wird die Verbindung der Formel I in Kombination mit einem Tau-Protein-Kinase-1-Inhibitor (TPK1 Inhibitor), wie z. B. in WO2007119463 beschrieben, verabreicht.

Bei einer Ausführungsform wird die Verbindung der Formel I in Kombination mit einem "c-Jun N-terminal kinase" Inhibitor (JNK-Inhibitor), wie z. B. in WO2007125405, WO2008028860, WO2008118626 beschrieben, verabreicht.

Bei einer Ausführungsform wird die Verbindung der Formel I in Kombination mit einem Endothelin-A-Rezeptor Antagonisten, wie z. B. Avosentan (SPP-301), verabreicht.

Bei einer Ausführungsform wird die Verbindung der Formel I in Kombination mit Modulatoren des Glukokortikoidrezeptors (GR), wie z.B. KB-3305 oder solchen Verbindungen wie sie z. B. in WO2005090336, WO2006071609, WO2006135826, WO2007105766, WO2008120661 beschrieben sind, verabreicht.

Bei einer Ausführungsform ist der weitere Wirkstoff Varenicline Tartrate, ein partieller Agonist des alpha 4-beta 2 nikotinischen Acetylcholinrezeptors.

Bei einer Ausführungsform ist der weitere Wirkstoff Trodusquemine.

Bei einer Ausführungsform ist der weitere Wirkstoff ein Modulator des Enzyms SIRT1 und/oder SIRT3 (einer $NAD^+$-abhängigen Proteindeacetylase); dieser Wirkstoff kann z.B. Resveratrol in geeigneten Formulierungen sein, oder solche Verbindungen wie sie in WO2007019416 (z.B. SRT-1720), WO2008073451 genannt sind.

Bei einer Ausführungsform der Erfindung ist der weitere Wirkstoff DM-71 (N-Acetyl-L-Cystein mit Bethanechol).

Bei einer Ausführungsform wird die Verbindung der Formel I in Kombination mit antihypercholesterolemisch wirkenden Verbindungen, wie sie z.B. in WO2007107587, WO2007111994, WO2008106600, WO2008113796 beschrieben sind, verabreicht.

Bei einer weiteren Ausführungsform wird die Verbindung der Formel I in Kombination mit Inhibitoren des SREBP (sterol regulatory element-binding protein), wie sie z.B. in WO2008097835 beschrieben sind, verabreicht.

Bei einer anderen Ausführungsform wird die Verbindung der Formel I in Kombination mit einem cyclischen Peptidagonisten des VPAC2 Rezeptors, wie sie z.B. in WO2007101146, WO2007133828 beschrieben sind, verabreicht.

Bei einer weiteren Ausführungsform wird die Verbindung der Formel I in Kombination mit einem Agonisten des Endothelinrezeptors, wie sie z.B. in WO2007112069 beschrieben sind, verabreicht.

Bei einer weiteren Ausführungsform wird die Verbindung der Formel I in Kombination mit AKP-020 (Bis(ethylmaltolato)oxovanadium-IV) verabreicht.

Bei einer anderen Ausführungsform wird die Verbindung der Formel I in Kombination mit gewebe-selektiven Androgenrezeptor Modulatoren ("tissue-selective androgen receptor modulators"; SARM), wie sie z.B. in WO2007099200, WO2007137874 beschrieben sind, verabreicht.

Bei einer weiteren Ausführungsform wird die Verbindung der Formel I in Kombination mit einem AGE (advanced glycation endproduct) Inhibitor, wie sie z.B. in JP2008024673 beschrieben sind, verabreicht.

Bei einer Ausführungsform der Erfindung ist der weitere Wirkstoff Leptin;
siehe z.B. "Perspectives in the therapeutic use of leptin", Salvador, Javier; Gomez-Ambrosi, Javier; Fruhbeck, Gema, Expert Opinion on Pharmacotherapy (2001), 2(10), 1615-1622.

Bei einer anderen Ausführungsform der Erfindung ist der weitere Wirkstoff Metreleptin (rekombinantes Methionyl-Leptin) kombiniert mit Pramlintide.

Bei einer weiteren Ausführungsform der Erfindung ist der weitere Wirkstoff das Tetrapeptid ISF-402.

Bei einer Ausführungsform ist der weitere Wirkstoff Dexamphetamin oder Amphetamin.

Bei einer Ausführungsform ist der weitere Wirkstoff Fenfluramin oder Dexfenfluramin.

Bei noch einer Ausführungsform ist der weitere Wirkstoff Sibutramin oder solche Derivate wie sie in

WO2008034142 beschrieben sind.

Bei einer Ausführungsform ist der weitere Wirkstoff Mazindol oder Phentermin.

Bei einer weiteren Ausführungsform ist der weitere Wirkstoff Geniposidinsäure (geniposidic acid; WO2007100104) oder Derivate davon (JP2008106008).

Bei einer Ausführungsform ist der weitere Wirkstoff ein nasal verabreichter Calciumkanalblocker wie z.B. Diltiazem oder solche, wie sie in US 7,138,107 beschrieben sind.

Bei einer Ausführungsform ist der weitere Wirkstoff ein Inhibitor des Natrium-Calcium-Ionen-Austausches wie z.B. solche, wie sie in WO2008028958, WO2008085711 beschrieben sind.

Bei einer weiteren Ausführungsform ist der weitere Wirkstoff ein Blocker von Calciumkanälen wie z.B. des CaV3.2 oder CaV2.2 wie sie in WO2008033431, WO2008033447, WO2008033356, WO2008033460, WO2008033464, WO2008033465, WO2008033468, WO2008073461 beschrieben sind.

Bei einer Ausführungsform ist der weitere Wirkstoff ein Modulator eines Calciumkanals wie z.B. solche, wie sie in WO2008073934, WO2008073936 beschrieben sind.

Bei einer Ausführungsform ist der weitere Wirkstoff ein Blocker des "T-type calcium channel" wie sie z.B. in WO2008033431, WO2008110008 beschrieben sind.

Bei einer Ausführungsform ist der weitere Wirkstoff ein Inhibitor des KCNQ-Kaliumkanal-2 bzw. -3 wie z.B. solche, wie sie in US2008027049, US2008027090 beschrieben sind.

Bei einer Ausführungsform ist der weitere Wirkstoff ein Inhibitor des Kalium Kv1.3 Ionenkanals wie z.B. solchen, wie sie in WO2008040057, WO2008040058, WO2008046065 beschrieben sind.

Bei einer anderen Ausführungsform ist der weitere Wirkstoff ein Modulator des MCP-1 Rezeptors (monocyte chemoattractant protein-1 (MCP-1)) wie z.B. solche, wie sie in WO2008014360, WO2008014381 beschrieben sind.

Bei einer Ausführungsform ist der weitere Wirkstoff ein Modulator des Somatostatinrezeptors 5 (SSTR5) wie z.B. solche, wie sie in WO2008019967, US2008064697, US2008249101, WO2008000692 beschrieben sind.

Bei einer Ausführungsform ist der weitere Wirkstoff ein Modulator des Somatostatinrezeptors 2 (SSTR2) wie z.B. solche, wie sie in WO2008051272 beschrieben sind.

Bei einer Ausführungsform ist der weitere Wirkstoff ein Erythropoietin-mimetisches Peptid, welches als Erythropoietin (EPO) Rezeptoragonist agiert. Solche Moleküle sind z.B. in WO2008042800 beschrieben.

Bei einer weiteren Ausführungsform ist der weitere Wirkstoff ein Anorektikum/eine hypoglykämische Verbindung wie z.B. solche, wie sie in WO2008035305, WO2008035306, WO2008035686 beschrieben sind.

Bei einer Ausführungsform ist der weitere Wirkstoff ein Induktor der Liponsäuresynthetase wie z.B. solche, wie sie in WO2008036966, WO2008036967 beschrieben sind.

Bei einer Ausführungsform ist der weitere Wirkstoff ein Stimulator der endothelialen Nitric-Oxid-Synthase (eNOS) wie z.B. solche, wie sie in WO2008058641, WO2008074413 beschrieben sind.

Bei einer Ausführungsform ist der weitere Wirkstoff ein Modulator des Kohlenhydrat- und/oder Lipidstoffwechsels wie z.B. solche, wie sie in WO2008059023, WO2008059024, WO2008059025, WO2008059026 beschrieben sind.

Bei einer weiteren Ausführungsform ist der weitere Wirkstoff ein Angiotensin II Rezeptorantagonist wie z.B. solche, wie sie in WO2008062905, WO2008067378, WO2008062905 beschrieben sind.

Bei einer Ausführungsform ist der weitere Wirkstoff ein Agonist des Sphingosin-1-Phosphatrezeptors (S1 P) wie z.B. solche, wie sie in WO2008064315, WO2008074820. WO2008074821 beschrieben sind.

Bei einer Ausführungsform ist der weitere Wirkstoff ein Mittel, welches die Magenentleerung retardiert wie z.B. 4-Hydroxyisoleucin (WO2008044770).

Bei einer Ausführungsform ist der weitere Wirkstoff eine Muskel-relaxierende Substanz wie sie z.B. in WO2008090200 beschrieben ist.

Bei einer weiteren Ausführungsform ist der weitere Wirkstoff ein Inhibitor der Monoaminoxidase B (MAO-B) wie z.B. solche, wie sie in WO2008092091 beschrieben sind.

Bei einer anderen Ausführungsform ist der weitere Wirkstoff ein Inhibitor der Bindung von Cholesterol und/oder Triglyceriden an das SCP-2 Protein (sterol carrier protein-2) wie z.B. solche, wie sie in US2008194658 beschrieben sind.

Bei einer anderen Ausführungsform ist der weitere Wirkstoff Lisofylline, welcher Autoimmunschäden an insulinproduzierenden Zellen verhindert.

Bei einer Ausführungsform wird die Verbindung der Formel I in Kombination mit Ballaststoffen, vorzugsweise unlöslichen Ballaststoffen (siehe z.B. Carob/ Caromax® (Zunft H J; et al., Carob pulp preparation for treatment of hypercholesterolemia, ADVANCES IN THERAPY (2001 Sep-Oct), 18(5), 230-6.) Caromax ist ein Carob enthaltendes Produkt der Fa. Nutrinova, Nutrition Specialties & Food Ingredients GmbH, Industriepark Höchst, 65926 Frankfurt / Main)) verabreicht. Die Kombination mit Caromax® kann in einer Zubereitung erfolgen, oder durch getrennte Gabe von Verbindungen der Formel I und Caromax®. Caromax® kann dabei auch in Form von Lebensmitteln, wie z.B. in Backwaren oder Müsliriegeln, verabreicht werden.

Es versteht sich, dass jede geeignete Kombination der erfindungsgemäßen Verbindungen mit einer oder mehreren der vorstehend genannten Verbindungen und wahlweise einer oder mehreren weiteren pharmakologisch wirksamen Substanzen als unter den Schutzbereich der vorliegenden Erfindung fallend angesehen wird.

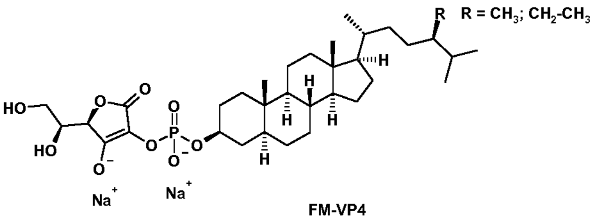

FM-VP4

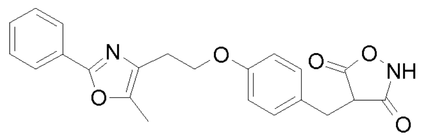

JTT-501

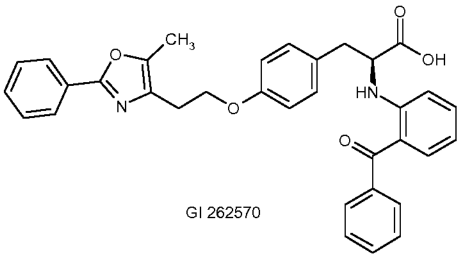

GI 262570

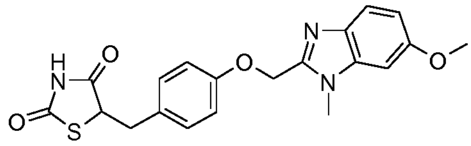

CS-011
Rivoglitazone

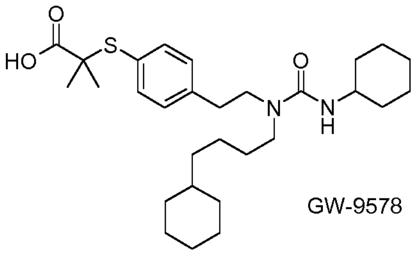

GW-9578

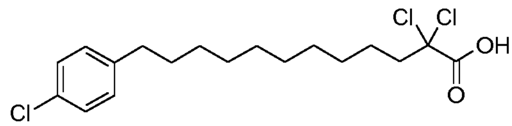

K-111

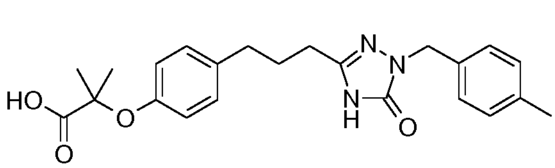

LY-518674

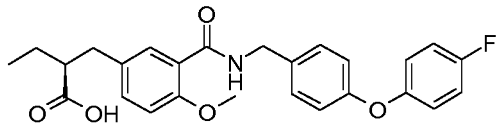

KRP-101

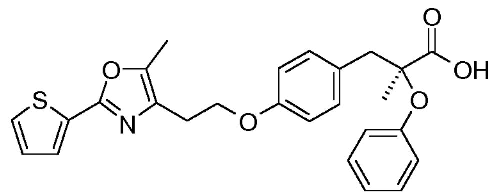

LY-510929

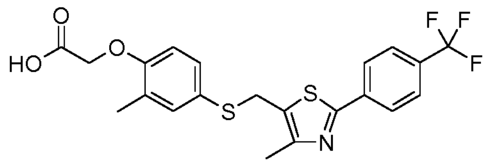

GW-501516

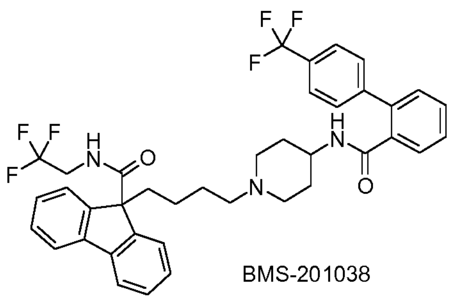

BMS-201038

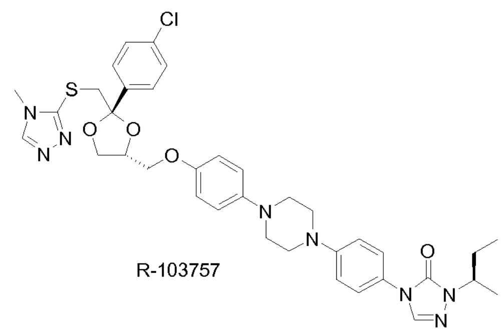

R-103757

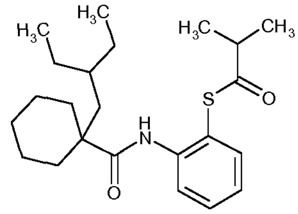

JTT-705

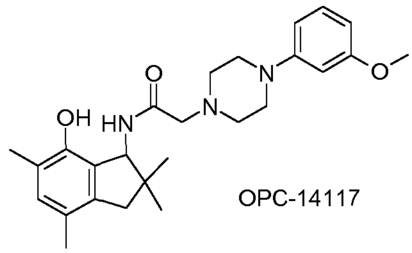

OPC-14117

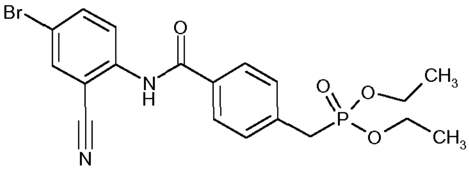

NO-1886

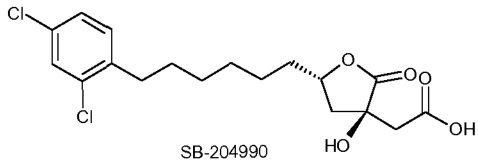

SB-204990

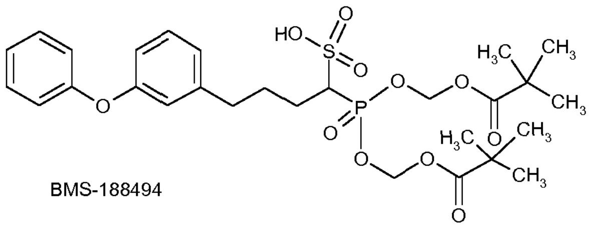

BMS-188494

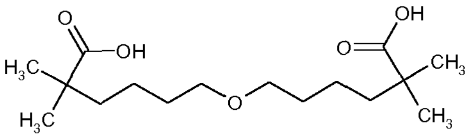

CI-1027

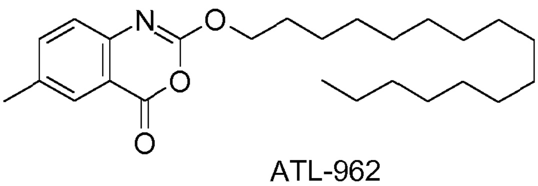

ATL-962

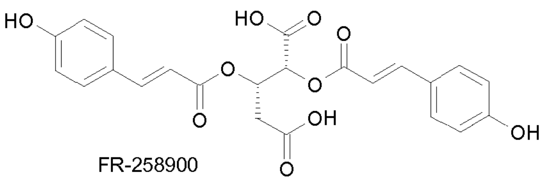

FR-258900

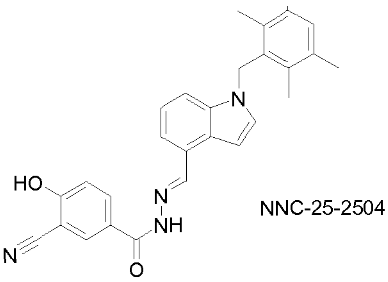

NNC-25-2504

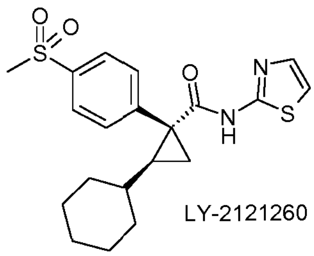

LY-2121260

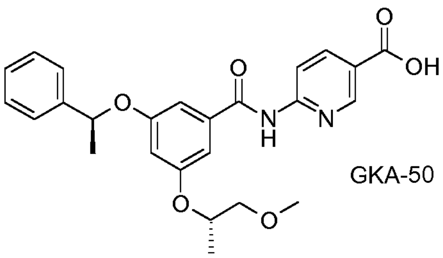

GKA-50

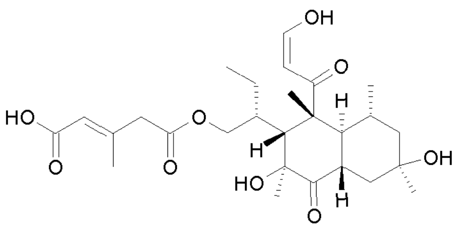

FR-225654

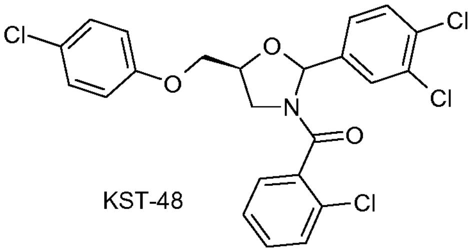

KST-48

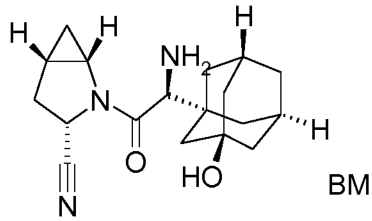

BMS-477118

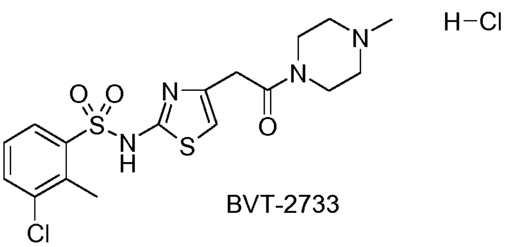

BVT-2733

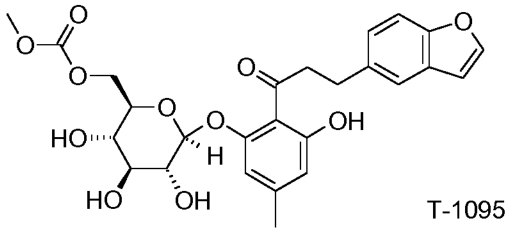

T-1095

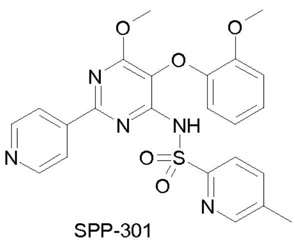

SPP-301

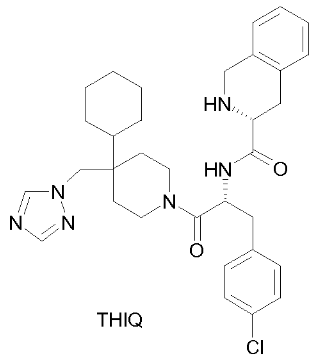

THIQ

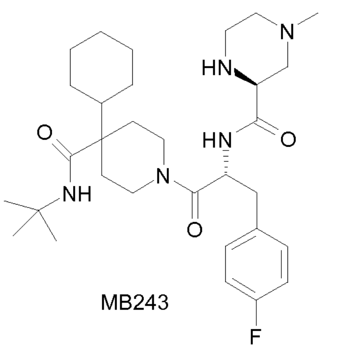

MB243

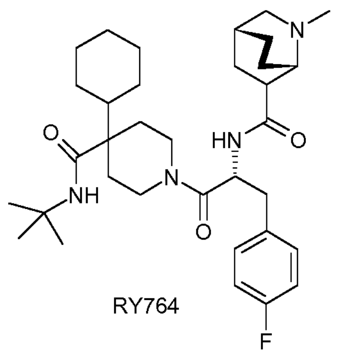

RY764

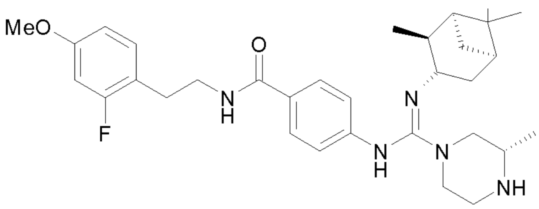

CHIR-785

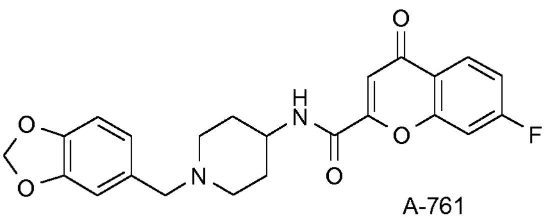

A-761

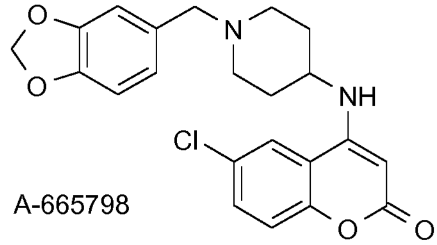

A-665798

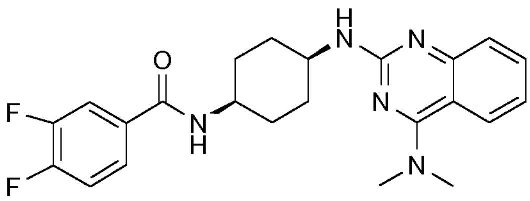

ATC-0175

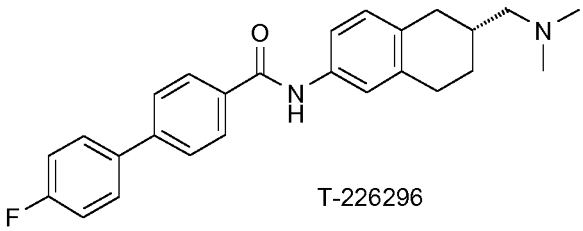

T-226296

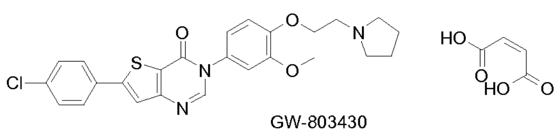

GW-803430

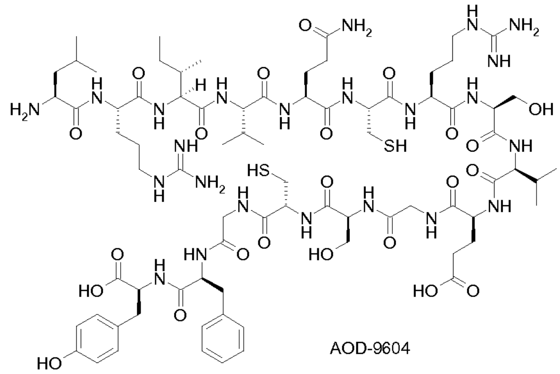

AOD-9604

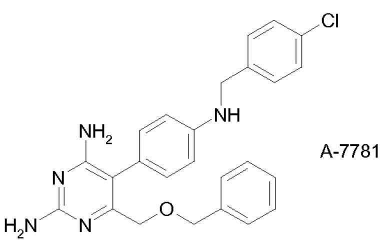

A-778193

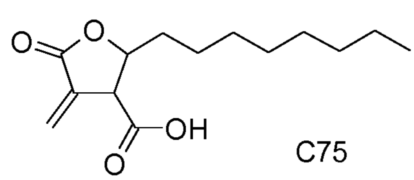

C75

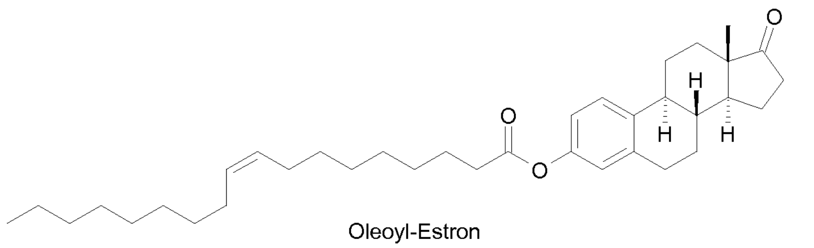

Oleoyl-Estron

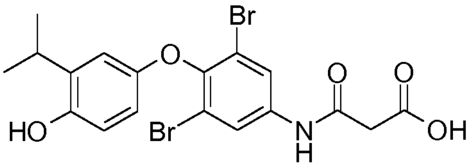

KB-2115

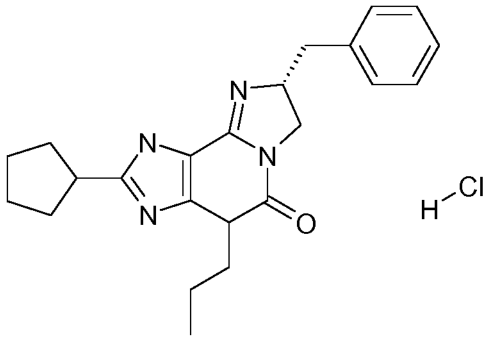

KCP-265

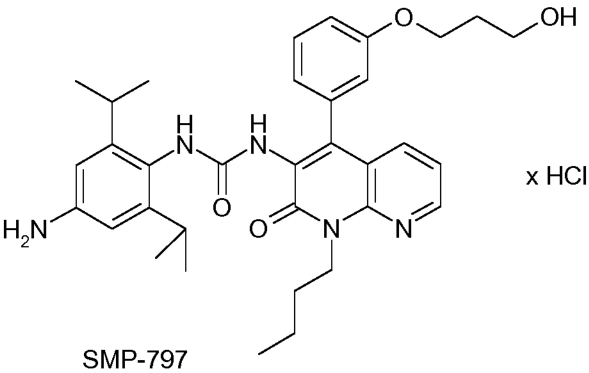

x HCl

SMP-797

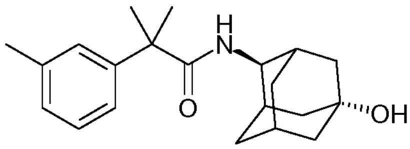

JNJ-25918646

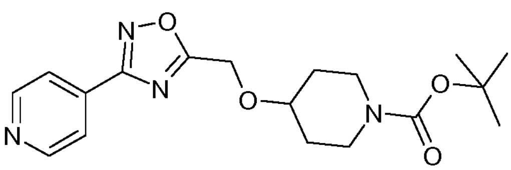

PSN-632408

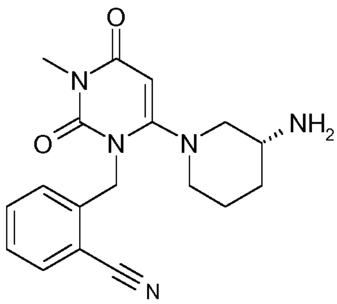

SYR-322

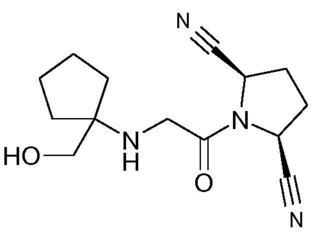

x HCl

DP-893

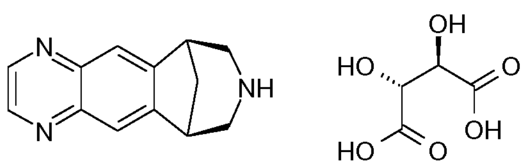

Varenicline Tartrat

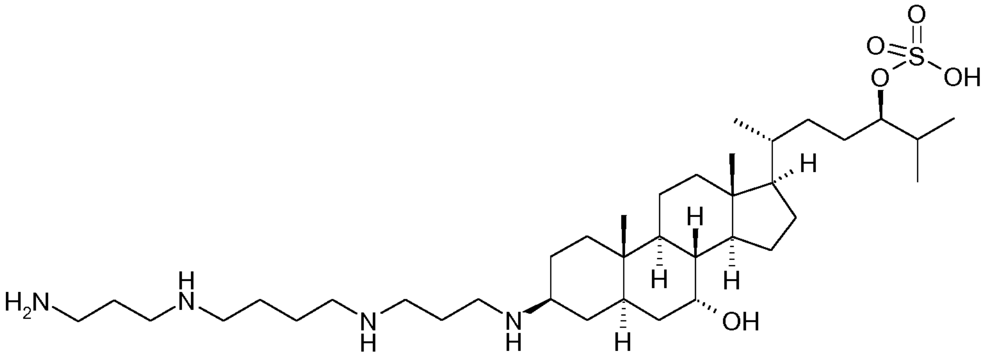

Trodusquemine

x HCl

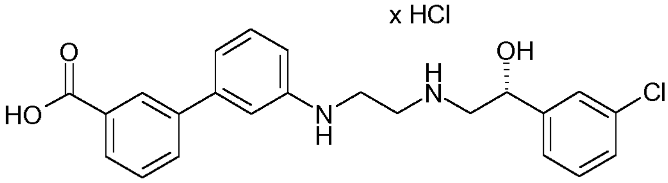

Solabegron

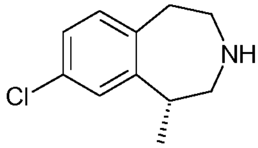

x HCl

Lorcaserin Hydrochlorid

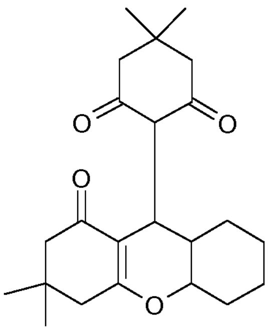

L-152804

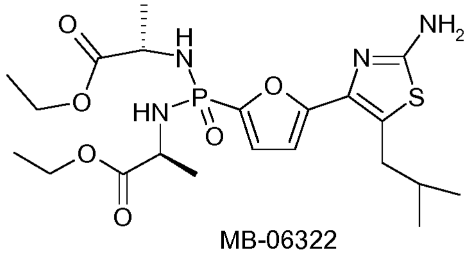

MB-06322
CS-917

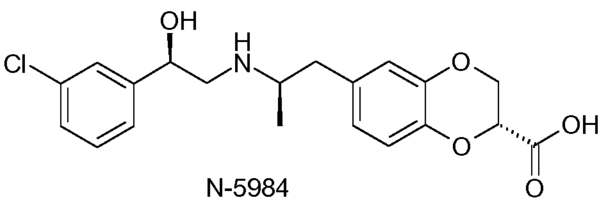

N-5984

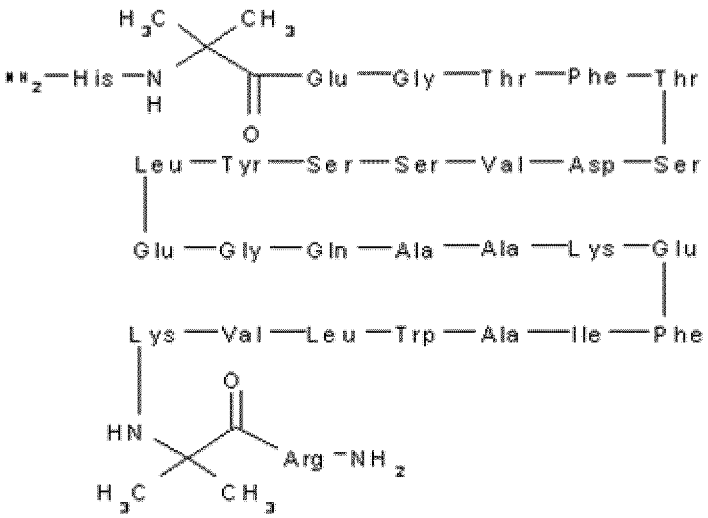

BIM-51077

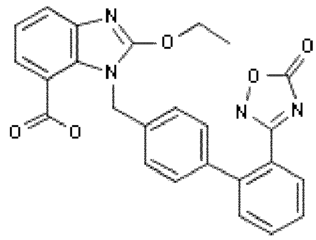

TAK-536

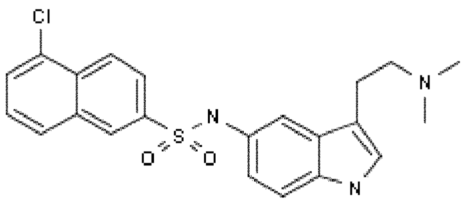

E-6837

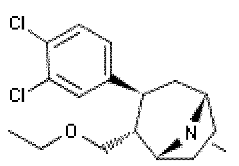

Tesofensine

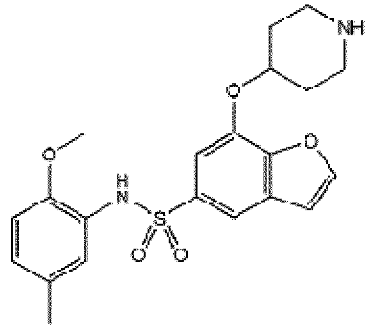

BVT-74316

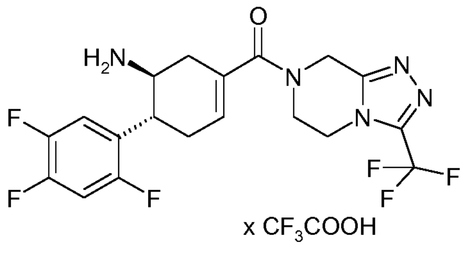

x $CF_3COOH$

ABT-341

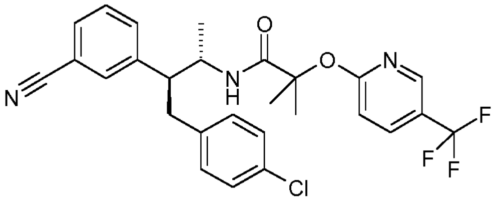

MK-0364

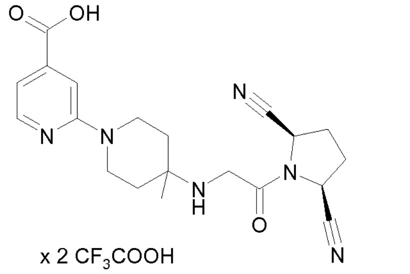

x 2 $CF_3COOH$

ABT-279

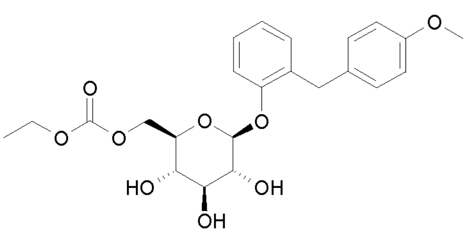

Sergliflozin

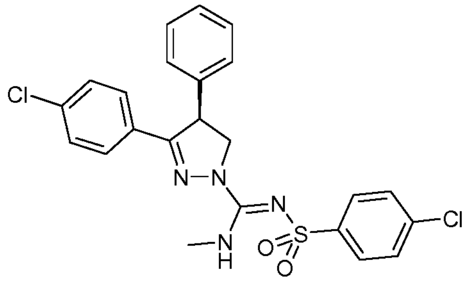

SLV-319

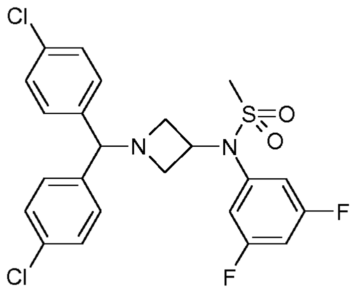

AVE 1625 (proposed INN: drinabant)

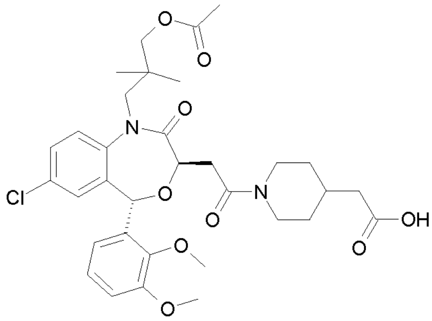

TAK-475 (Lapaquistat Acetat)

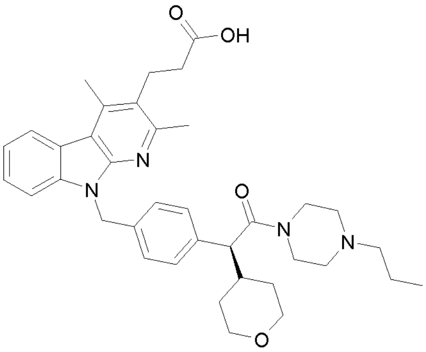

AS-1552133

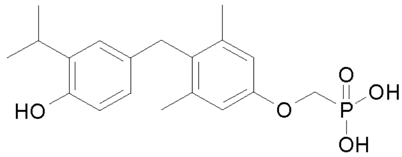

MB-07344

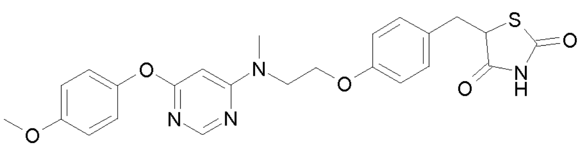

x $H_2SO_4$

CKD-501 (Lobeglitazon Sulfat)

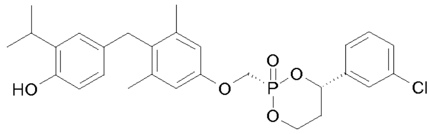

MB-07811

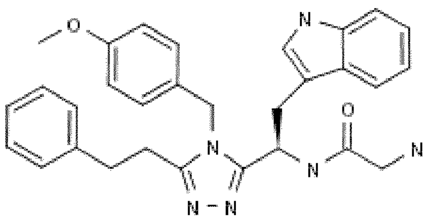

JMV-2959

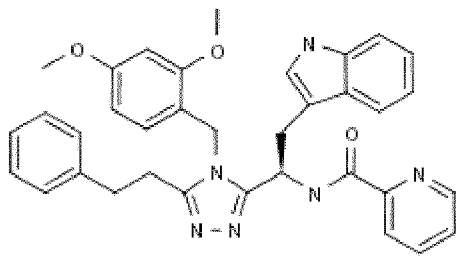

JMV-3002

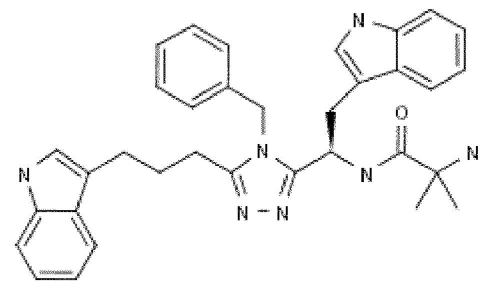

JMV-2810

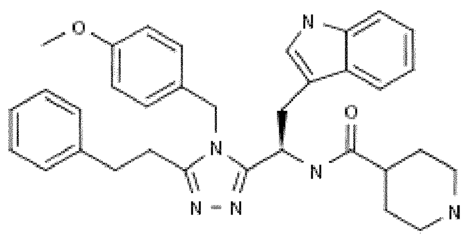

JMV-2951

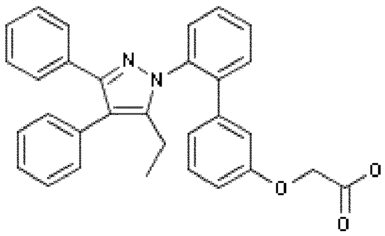

BMS-309403

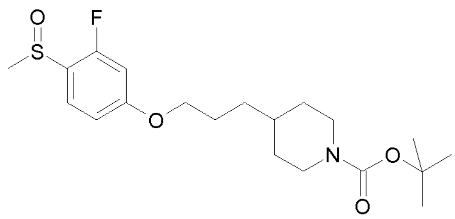

PSN-119-1

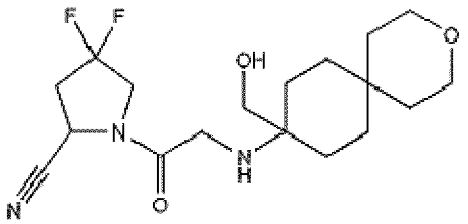

S-40755

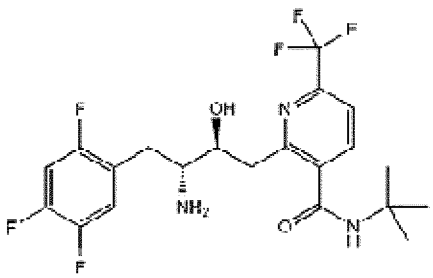

LY-2463665

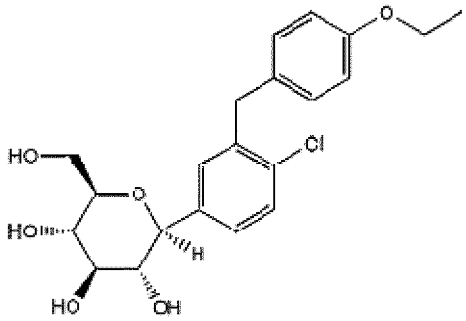

Dapagliflozin, BMS-512148

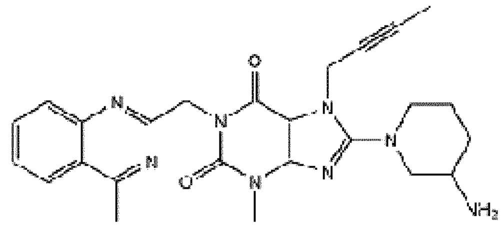

BI-1356

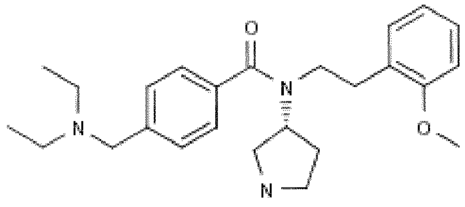

PF-429242

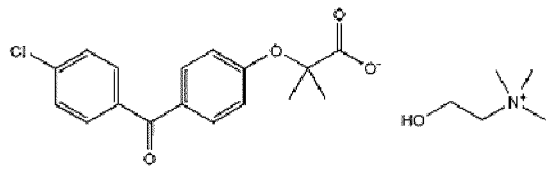

SLV-348

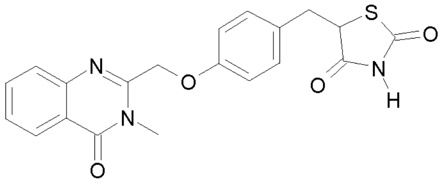

Balaglitazon

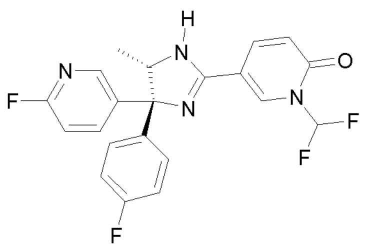

"NPY-5-BY"

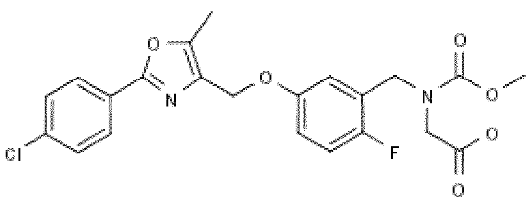

BMS-711939

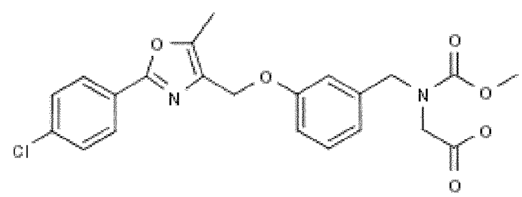

BMS-687453

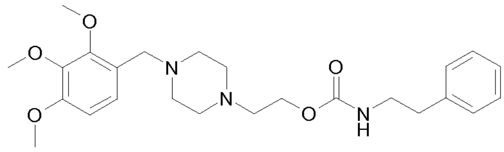

ST-3473

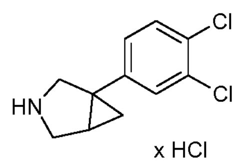

x HCl

DOV-21947

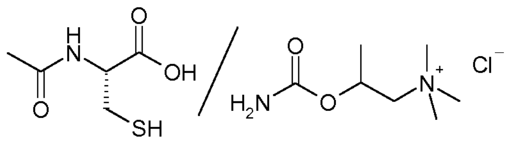

DM-71

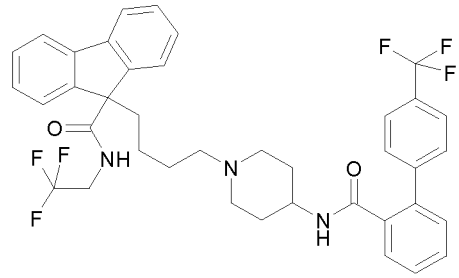

AEGR-733

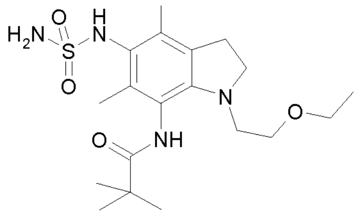

KY-382

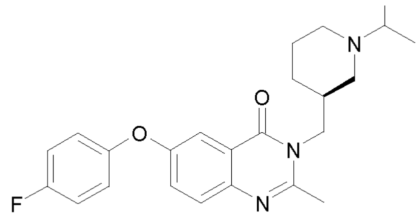

YIL-781

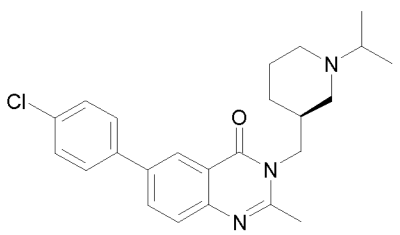

YIL-870

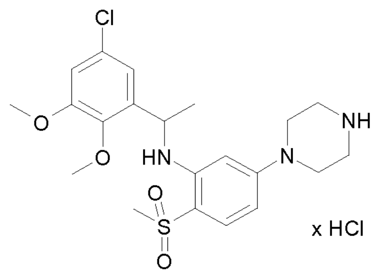

PRX-07034

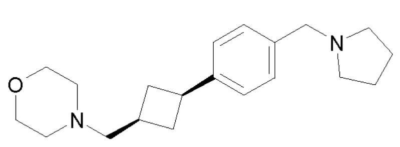

PF-00389027

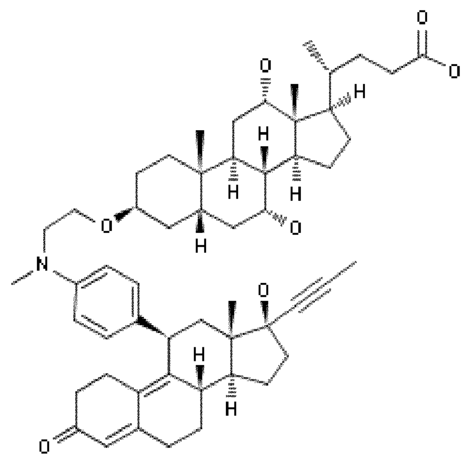

KB-3305

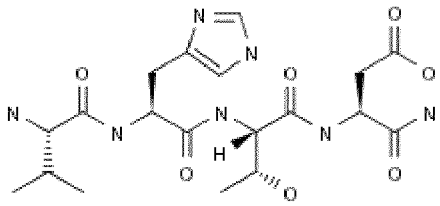

ISF-402

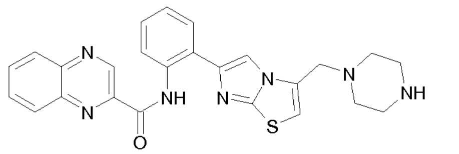

SRT-1720

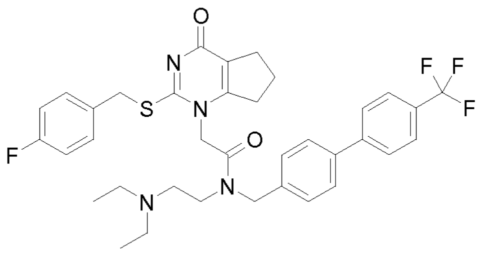

darapladib

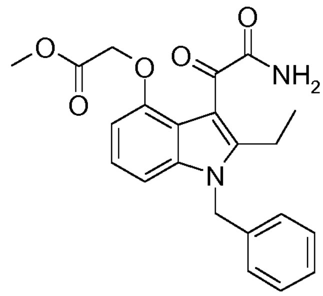

A-002

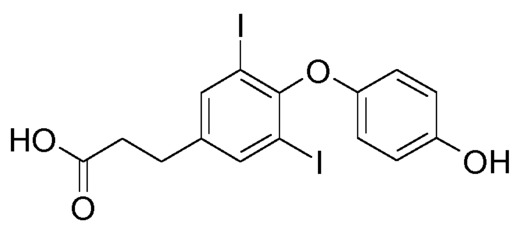

DITPA

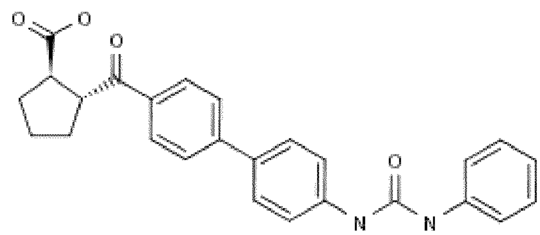

DGAT-1 Inhibitor aus WO2007137103

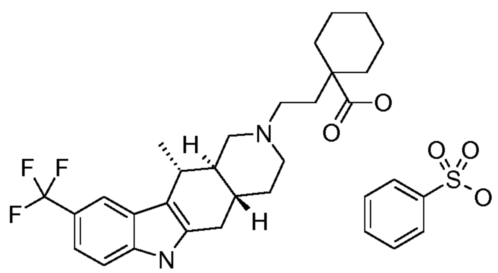

AMG-071

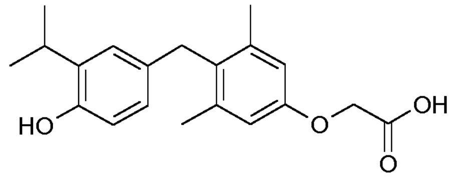

sobetirome

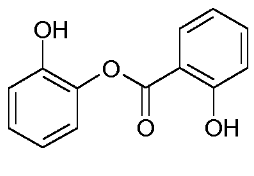

salsalate

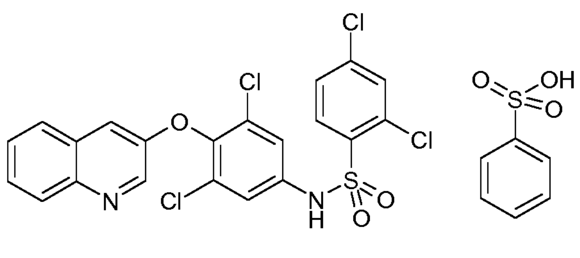

INT-131

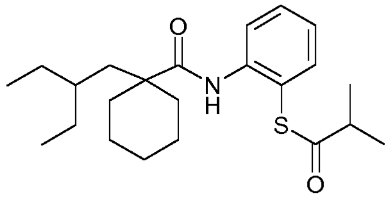

dalcetrapib

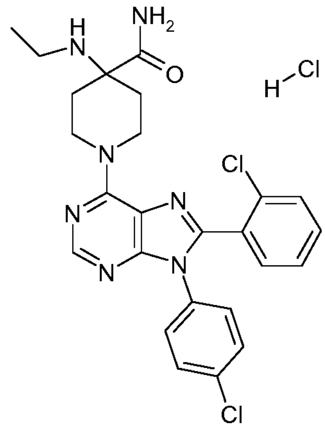

otenabant

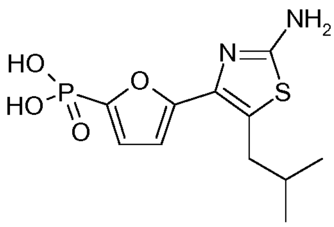

MB-07229

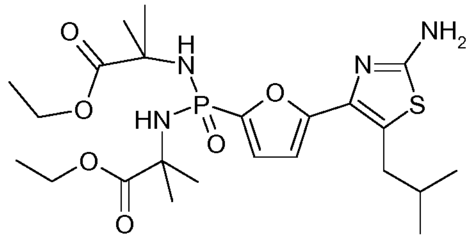

MB-07803

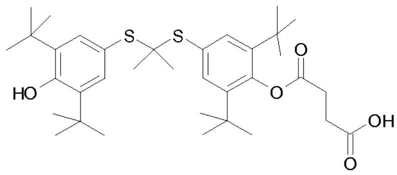

Succinobucol

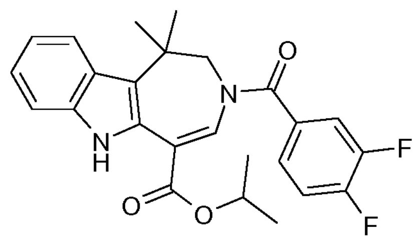

WAY-362450

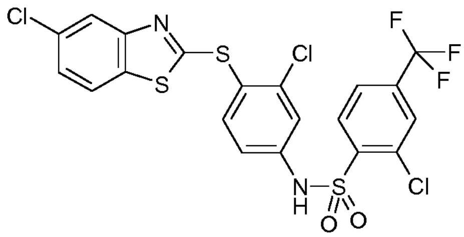

T-2384

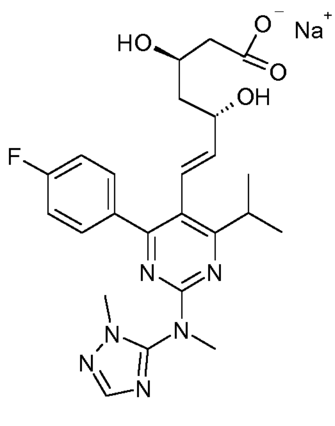

BMS-644950

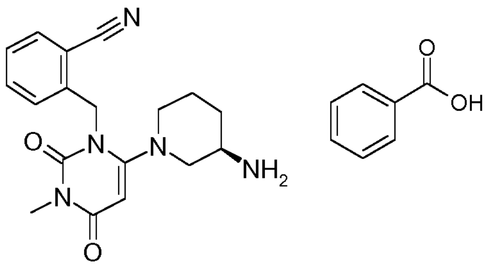

alogliptin benzoate

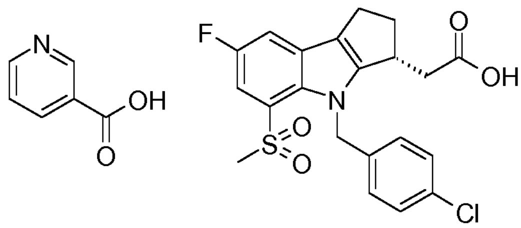

Nicotinsäure / Laropiprant

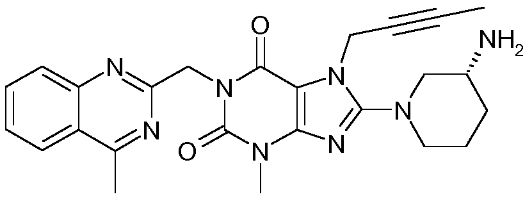

linagliptin

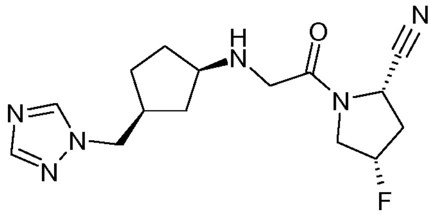

melogliptin

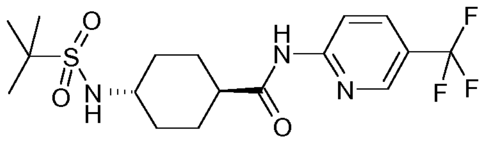

velneperit

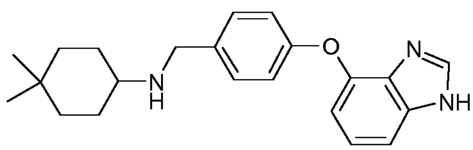

GSK-982

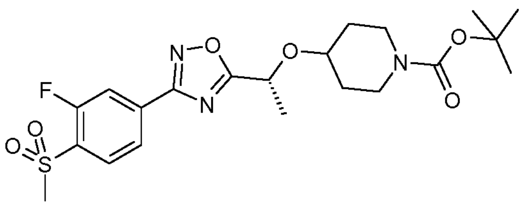

PSN-119-2

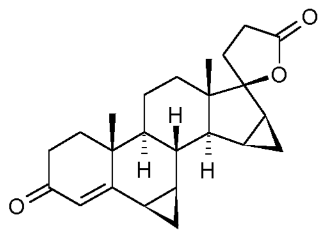

drospirenone

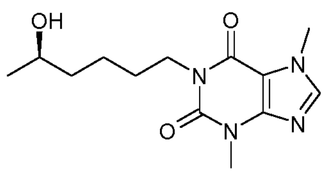

lisofylline

Weiterhin sind folgende Wirkstoffe für Kombinationspräparate geeignet:

Alle Antiepileptika, die in der Roten Liste 2007, Kapitel 15 genannt sind;
alle Antihypertonika, die in der Roten Liste 2007, Kapitel 17 genannt sind;
alle Hypotonika, die in der Roten Liste 2007, Kapitel 19 genannt sind;
alle Antikoagulantia, die in der Roten Liste 2007, Kapitel 20 genannt sind;
alle Arteriosklerosemittel, die in der Roten Liste 2007, Kapitel 25 genannt sind;
alle Betarezeptoren-, Calciumkanalblocker und Hemmstoffe des Renin-Angiotensin-Systems, die in der Roten Liste

2007, Kapitel 27 genannt sind;
alle Diuretika und Durchblutungsfördernde Mittel, die in der Roten Liste 2007, Kapitel 36 und 37 genannt sind;
alle Entwöhnungsmittel/Mittel zur Behandlung von Suchterkrankungen, die in der Roten Liste 2007, Kapitel 39 genannt sind;
alle Koronarmittel und Magen-Darm-Mittel, die in der Roten Liste 2007, Kapitel 55 und 60 genannt sind;
alle Migränemittel, Neuropathiepräparate und Parkinsonmittel, die in der Roten Liste 2007, Kapitel 61, 66 und 70 genannt sind.

Die Wirksamkeit der erfindungsgemäßen Verbindungen der Formel I wurde an folgenden Enzymtestsystemen geprüft:

Test auf Hemmung der EL:

Präparation der EL

EL wird als sekretorisches Protein von rekombinanten Zell-Linien (CHO, HEK293) in hoher Konzentration in Zellkulturmedium abgegeben (konditioniertes Medium). Dieses wurde nach Aufkonzentration als Enzymlösung eingesetzt.

Assay auf EL-Aktivität

Zur Charakterisierung der enzymatischen Aktivität von endothelialer Lipase und der Wirkung von Inhibitoren wurde das Phospholipase-spezifische Substrat 1,2-bis-(4,4-difluoro-5,7-dimethyl-4-bora-3a,4a-diaza-s-indacene-3-undecanoyl)-sn-glycero-3-phosphocholine, (Hersteller Molecular Probes) verwendet. Durch Hydrolyse der A1 Esterbindung dieses Phospholipids durch das Enzym wird der Fluoreszenzfarbstoff Bodipy freigesetzt, der nach Trennung durch Dünnschichtchromatographie auf einer HPTLC-Platte (Kieselgel 60, Merck) oder direkt im Reaktionsgefäß durch Messen der Fluoreszenz nachgewiesen werden kann.
Zur Herstellung der Substratlösung wurden 100 μg 1,2-bis-(4,4-difluoro-5,7-dimethyl-4-bora-3a,4a-diaza-s-indacene-3-undecanoyl)-sn-glycero-3-phospho-choline (Hersteller Molecular Probes) in 100μl DMSO gelöst und in 2,4 mg Tripalmitin (Sigma) in 393 μl Chloroform aufgenommen, welches 20mg/ ml DOP - Cholin (1,2-Dioleoyl-sn-glycero-3-phosphocholin) enthielt. 39,3μl dieses Lipidgemisches wurden in ein frisches Reaktionsgefäß überführt und das Lösungsmittel abgedampft. Das Lipidgemisch wurde in 4 ml 200 mM TRIS-HCl, 150 mM Natriumchlorid, pH = 7,4 durch zweimaliges Sonifizieren gelöst. Die anschließende Enzymreaktion erfolgte für 90 Minuten bei 37°C. Hierzu wurden 20 μl der Substratlösung mit 2 μl Inhibitor entsprechender Konzentration (gelöst in 10% DMSO, zur Kontrolle wurde 10%ige DMSO-Lösung verwendet) und 2 μl Enzymlösung (konditioniertes Medium) inkubiert. Im Anschluß wurden 4 μl des Testansatzes auf eine HPTLC-Platte (Kieselgel 60, Merck) aufgetragen und der freigesetzte Fluoreszenzfarbstoff zum Nachweis mit einem Fließmittel (Diethylether : Petroleumbenzin : Essigsäure [78:22:1]) getrennt. Nach dem Abdampfen des Fließmittels wurde die Platte in einen Fluoreszenz-Scanner eingelesen. Als Maß für die Enzymaktivität war eine gesteigerte Freisetzung des Fluoreszenzfarbstoffes in der ungehemmten Reaktion zu beobachten.
In Abhängigkeit der verwendeten Inhibitorkonzentration ergab sich eine Reduktion der enzymatischen Aktivität. Die Inhibitiorkonzentration, bei welcher eine halbmaximale Enzymaktivität beobachtet wird, wird als $IC_{50}$ bezeichnet.
In diesem Test zeigten die Verbindungen der Beispiele folgende von $IC_{50}$ -Werte:

| Beispiel | $IC_{50}$ [nM] EL | Beispiel | $IC_{50}$ [nM] EL |
|---|---|---|---|
| 3 | 926 | 114 | 216 |
| 4 | 40 | 115 | 1380 |
| 5 | 200 | 116 | 1590 |
| 18 | 69 | 129 | 230 |
| 28 | 107 | 130 | 85 |
| 29 | 4290 | 134 | 391 |
| 37 | 148 | 142 | 517 |
| 38 | 182 | 156 | 1580 |
| 39 | 120 | 161 | 80 |

(fortgesetzt)

| Beispiel | $IC_{50}$ [nM] EL | Beispiel | $IC_{50}$ [nM] EL |
|---|---|---|---|
| 46 | 28 | 162 | 453 |
| 50 | 138 | | |
| 55 | 116 | | |
| 59 | 264 | | |

Andere Prüfmodelle

Anhand verschiedener Prüfmodelle kann die Eignung der erfindungsgemäßen Verbindungen als pharmazeutischer Wirkstoff getestet werden. Im Folgenden werden beispielhaft Beschreibungen solcher Prüfmodelle gegeben.

Löslichkeiten in wässrigen Systemen

Ausreichende Löslichkeit einer Substanz in wässrigen Lösungsmittelsystemen ist eine wichtige Vorraussetzung für eine (reproduzierbare) pharmakologische Wirkung.

Löslichkeiten in wässrigen Systemen können nach verschiedenen Verfahren bestimmt werden. Geeignet sind z. B. Fällungsverfahren aus Lösungen ("kinetische Löslichkeit") und Verfahren, die die Auflösung einer festen Probe bis zur Gleichgewichtseinstellung untersuchen ("thermodynamische Löslichkeit").

a) Kinetische Löslichkeit

Auf einer 96-well Mikrotiterplatte wird eine DMSO-Lösung der Testverbindung (2,5 mM; 0,5 µL) zu 200 µL einer wässrigen Testlösung (z. B. Phosphate buffered Saline, 10x, 1M, Sigma eingestellt auf 10 mM, pH 7,4) pipettiert und die Trübung bei der so erhaltenen theoretischen Konzentration der Testverbindung von 6,25 µM mittels eines Nephelometers (z. B. Nephelostar Galaxy, BMG Labtech) gemessen. Danach wird die Konzentration der Testverbindung in der wässrigen Testlösung durch Zugabe von weiterer DMSO-Lösung (2,5 mM; 0,5 µL) auf theoretisch 12,5 µM erhöht und die Trübungsmessung erneut durchgeführt. Weitere Zugaben von DMSO-Lösungen (1 µL, 2,5 mM; 0,5 µL, 10 mM; dann 9-mal 1 µL, 10 mM ergebend theoretische Konzentrationen von 25 µM, 50 µM, 100 µM, 150 µM, 200 µM, 250 µM, 300 µM, 350 µM, 400 µM, 450 µM und 500 µM) mit zwischenzeitlicher Trübungsmessung komplettieren den Messprozess.

Auswertung: Die Trübungswerte des Nephelometers werden gegen die theoretische Konzentration der Testverbindung in der wässrigen Testlösung aufgetragen. Sobald bei einer theoretischen Konzentration eine signifikante Trübung detektiert wird (z. B. 5-fach über dem Kontrollwert der wässrigen Testlösung), wird der darunter liegende Konzentrationswert als Löslichkeitsgrenze der Testverbindung in der Testlösung angegeben. Als maximal möglicher Messbereich ergeben sich damit die Werte <6,25 µM, 6,25 - 500 µM und >500 µM.

Bevorzugte erfindungsgemäße Verbindungen zeigen eine kinetische Löslichkeit im Phosphatpuffer (pH 7,4) von mindestens 12,5 µM; bevorzugter von mindestens 50 µM und noch stärker bevorzugt von mindestens 250 µM.

b) Thermodynamische Löslichkeit

Mittels HPLC-UV-Messung einer Verdünnungsreihe der Testverbindung in DMSO (500 µM, 100 µM, 50 µM, 10 µM und 1 µM) wird die integrierte UV-Absorption in einer Kalibiergeraden linear mit der Konzentration korreliert. Die Testverbindung (500 µg) wird zusammen mit der wässrigen Testlösung (250 µL) in einem geschlossenen Gefäß (Fassungsvolumen: 1,5 mL) für 16 Stunden geschüttelt (Eppendorf Thermoschüttler, 1400 rpm, 25°C, Abdeckung als Lichtschutz). Anschließend wird die Probe bei maximaler Drehzahl zentrifugiert und der Überstand abschließend noch filtriert. Eine Probe des filtrierten Überstandes wird direkt mittels HPLC-UV-Messung (siehe oben) analysiert. Eine weitere Probe wird nach Verdünnen (1 Volumenteil Überstand, 39 Volumenteile Testlösung) analysiert.

Auswertung: Anhand der erstellten Kalibiergeraden wird aus den erhaltenen integrierten UV-Absorptionen der Überstandsproben die Konzentration der Testverbindung im unverdünnten Überstand berechnet und als Löslichkeit der Testverbindung in der jeweiligen wässrigen Testlösung angegeben.

Beispiele für wässrige Testlösungen sind entsalztes Wasser oder wässrige Phosphatpuffer mit verschiedenen pH-Werten (z. B. pH 1,2; pH 4,0; pH 6,8; pH 7,4; pH 9,0), die nach Standardverfahren aus der kommerziellen Lösung (Phosphate buffered saline, 10x, Sigma) hergestellt werden können durch Verdünnen bzw. Einstellen mit Phosphorsäure oder Natronlauge.

Bevorzugte erfindungsgemäße Verbindungen zeigen eine Löslichkeit im Phosphatpuffer (pH 7,4) von mindestens 12,5 μM; bevorzugter von mindestens 50 μM und noch stärker bevorzugt von mindestens 250 μM.

Permeabilität

Der Test auf Permeabilität wird in CACO-2/TC7 Zellen durchgeführt, die für 21 Tage auf Becton Dickinson-Filtern (24-well, nicht beschichtet) kultiviert wurden (DMEM / Glutamax I / Gibco mit hohem Glukoseanteil, HEPES 25 mM, 1% NEAA, 10% FBS, 40 μg/mL Gentamycin; 37°C Umgebungstemperatur; 95% Luftfeuchtigkeit und 10% CO2-Gehalt). Die Permeabilität wird bei einer Konzentration der Testverbindung von 20 μM (1% DMSO in HBSS) mit einem pH-Gradienten (apical: pH 6,5 und 0,5% BSA; basolateral: pH 7.4 und 5% BSA) geprüft. Die Analyse erfolgt mittels LCMS/MS. Weitere Beschreibungen des Testsystems und Referenzen zur experimentellen Durchführung finden sich bei Balimane, P.V.; Drug Discovery Today 2005, 10(5), 335-343.

Inhibition von CYP-Enzymen

Die Inhibition von CYP-Enzymen wird an rekombinanten Enzymen (erhalten von Becton Dickinson) und fluoreszierenden Substraten (BD/Gentest) nach den Empfehlungen des Herstellers bestimmt (siehe Website http://www.bdbiosciences.com). Weitere Beschreibungen des Testsystems und Referenzen zur experimentellen Durchführung finden sich bei Zlokarnik, G.; Drug Discovery Today 2005, 10(21), 1443-1450.

Metabolische Stabilität

Die metabolische Stabilität wird bestimmt durch Inkubation der Testverbindung (5 μM) bei 37°C mit microsomalen Leberfraktionen (1 mg/mL Protein mit 0,1 % w/v BSA; 1 mM NADPH, 0,5% DMSO). Die Analyse bei 0 und 20 Minuten Inkubationszeit erfolgt mittels LCMS/MS. Weitere Beschreibungen des Testsystems und Referenzen zur experimentellen Durchführung finden sich bei Plant, N.; Drug Discovery Today 2004, 9(7), 328-336 und Lau, Y.Y. et al.; Pharmaceutical Res. 2002, 19(11), 1606-1610.

Plasma Stabilität

Die Plasma Stabilität wird bestimmt durch Inkubation der Testverbindung bei 37°C mit humanem Plasma (Stammlösung der Testverbindung 10mM in DMSO). Arbeitslösung: Konzentration 1000 ng/L verdünnt in Wasser/Acetonitril/DMSO 79/20/1 v/v/v).

Die Analyse bei 0 h, 1 h und 4 h Inkubationszeit erfolgt mittels LCMS/MS.

Die Inkubation wird in Multititerplatten (MTP) mit 0,8 mL Töpfcheninhalt durchgeführt.

Die 1 h und 4 h Proben: 5μl der Arbeitslösung (10-fach) werden mit 45μL Plasma gemischt und während der Inkubation mit einem Deckel abgedeckt.

Die 0 h Probe: 5μl der Arbeitslösung (10-fach) werden mit 300 μL Acetonitril gemischt und danach 45 μL Plasma zugegeben und die Platte mit einer wiederabziehbaren Folie verschlossen und gemischt.

Die Reaktion wird durch schnelle Abkühlung (Eisbad, 0°C, 1 min) und Zugabe von 300 μL vorgekühltem Acetonitril abgestoppt.

Messung:

Die Platten werden zentrifugiert 20 min bei 10°C und 1734 g zentrifugiert. 220 μL des Überstands wird in ein MTP überführt (0,3 mL).

LC/MS-MS Messungen werden in folgender Reihenfolge vorgenommen: 4h - 1h - 0h Probe.

Berechnung des prozentuellen Anteil an hydrolisierter Verbindung:

$$[\%\ \text{Hydrolyse}] = [1 - (\frac{\text{Peak Fläche 1h or 4h}}{\text{Peak Fläche 0h}})] \times 100$$

Chemische Stabilität

Die chemische Stablität der Verbindungen wurde in Phosphatpuffer über 5 Stunden gestet.

Probenpräparation und Durchführung

Standard:

5 µL einer 10 mM DMSO Stammlösung wurden mit 995 µL Acetonitril verdünnt zu einer Endkonzentration von 50 µM. Diese Lösung wurde auf einer Flüssigchromatographiesäule (LC) gemessen wie unten beschrieben.

Belastungsprobe:

5 µL einer 10 mM DMSO Stammlösung der zu prüfenden Verbindung wurden mit 995 µL Phosphatpuffer pH 7.4 in 50% Acetonitril verdünnt zu einer Endkonzentration von 50 µM. Diese Lösung wurde 5 h bei 25°C gehalten und dann der Gehalt an Ausgangsverbindung über LC bestimmt.
Experimentelle Bedingungen:

| | |
|---|---|
| Instrument | : Waters Acquity Ultra Performance LC |
| Detektor | : Waters Acquity Ultra Performance LC PDA Detektor |
| Software | : Empower2 |
| Säule | : Waters Acquity BEH C18 1,7µm 1 x 50mm |
| Eluent A | : Wasser / 0.05 % Trifluoressigsäure |
| Eluent B | : Acetonitril / 0.035 % Trifluoressigsäure |

Gradienten :

| Zeit [min] | %A | %B |
|---|---|---|
| 0.0 | 98 | 2 |
| 0.05 | 98 | 2 |
| 1.8 | 2 | 98 |
| 2.5 | 2 | 98 |
| 2.55 | 98 | 2 |

| | |
|---|---|
| Flußrate | : 0.3 mL/min |
| Detection | : 210-450 nm; Ex. 220/254 nm |
| Säulentemperatur | : 40°C |
| Autosamplertemperatur | : 25°C |
| Injektionsvolumen | : 2 µL |

Verfahren zur Herstellung

Die erfindungsgemäßen Verbindungen der Formel I werden nach an sich bekannten Methoden synthetisiert.
Die Azolopyridinole II können mit Isocyanaten III umgesetzt werden (Methode A), oder mit Carbamoylchloriden acyliert werden (Methode B). Sie können aber auch in zwei Stufen durch Acylierung mit Phosgen oder Phosgenäquivalenten wie Trichlormethylchlorocarbonat, ditrichlormethylcarbonat, 4-Nitrophenylchlorformat und weitere Umsetzung der erhaltenen Azolcarbonsäure-Derivate mit Aminen (Methode C).

Methode A:

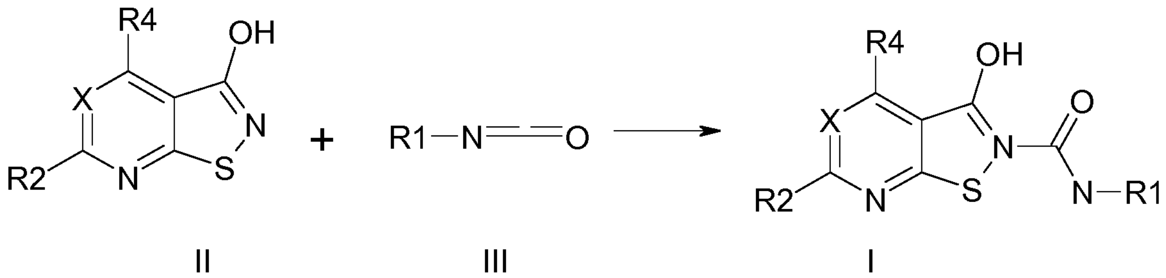

Methode B:

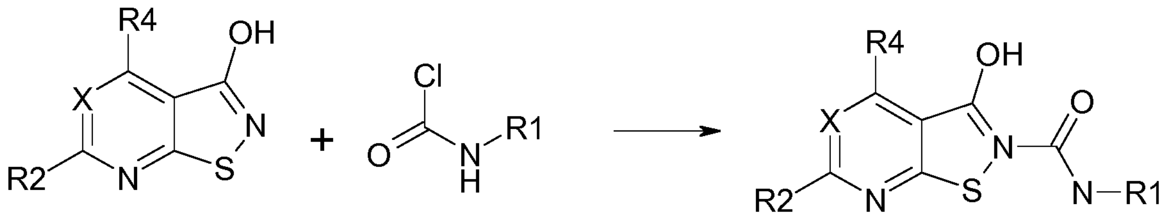

Methode C:

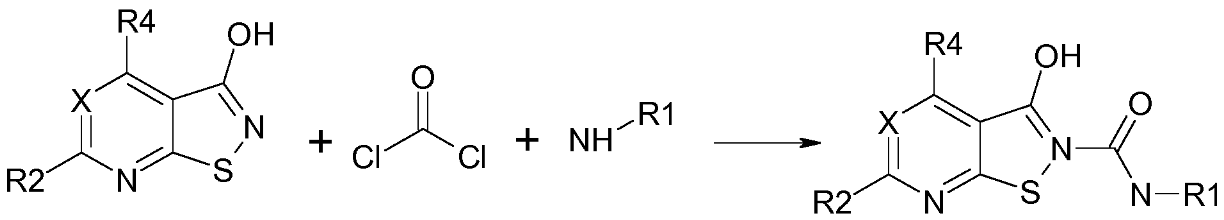

Da bei diesen Reaktionen in der Regel Säuren freigesetzt werden, empfiehlt es sich zur Beschleunigung Basen wie Pyridin, Triethylamin, Natronlauge oder Alkalicarbonate zu zusetzen. Die Reaktionen können in weiten Temperaturbereichen durchgeführt werden. In der Regel hat es sich als vorteilhaft herausgestellt, bei 0°C bis zum Siedepunkt des verwendeten Lösungsmittels zu arbeiten. Als Lösemittel kommen beispielsweise Methylenchlorid, THF, DMF, Toluol, Essigester, n-Heptan, Dioxan, Diethylether oder Pyridin zum Einsatz. Wenn unter wasserfreien Bedingungen gearbeitet wird, haben sich auch starke Basen wie Lithiumhydrid, Natriumhydrid oder Kalium-tert.-butylat in aprotischen Lösungsmitteln wie THF oder DMF bewährt.

Die nachfolgend aufgeführten Beispiele dienen zur Erläuterung der Erfindung, ohne diese jedoch einzuschränken.

Synthese der Azolopyridinol-Bausteine.

Die Azolopyridinole der allgemeinen Formel II (X=CH, N) können aus entprechend substiuierten Mercaptonicotinsäuren (X=CH) bzw. 4-Mercaptopyrimidin-5-carbonsäuren (X=N) durch Umsetzung mit Diphenylphosphorylazid erhalten werden.

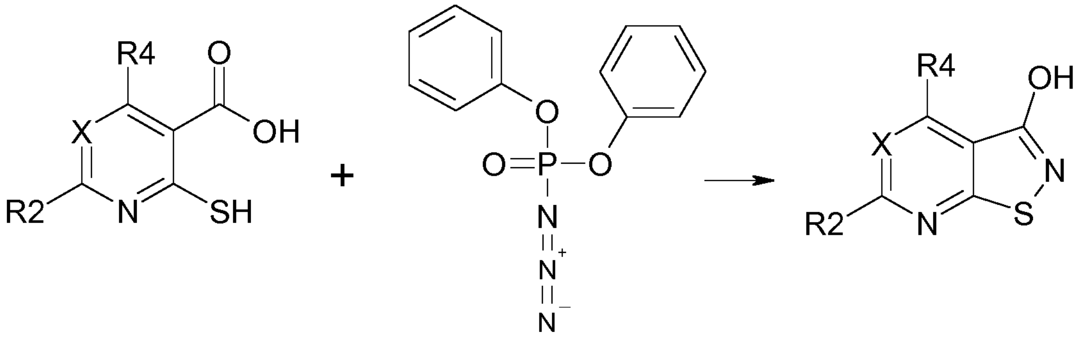

Alternativ erhält man Azolopyridinole auch durch Erhitzen von 3-Cyano-2-mercaptopyridinen der allgemeinen Formel IV in 98%iger Schwefelsäure.

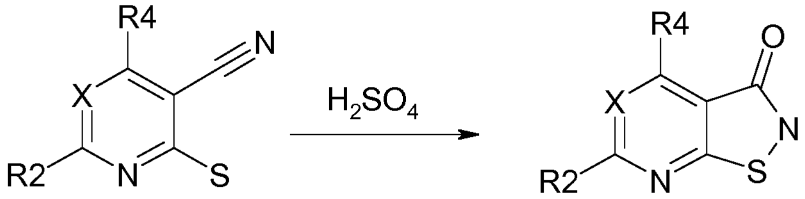


Auch sind Azolopyridin-3-ol-derivate II kommerziell erhältlich oder lassen sich nach Literatur bekannten Verfahren herstellen (z.B. L. Baiocchi, G. Corsi Synthesis (1978) 633-648; I. Sekikawa et al. J. Het. Chem. (1973) 931-932; A.Dornow, M. Siebrecht, Chem. Ber. (1960) 1106-1110; M. Tilser, B. Stanovnik, Z. Zrimsek, Heterocycles (1979) 217-219; K. Bowden, G. Crank, W, J. Roos, J.Chem.Soc. 1968, 172-185).

Synthese der Isocyanate

Die verwendeten Isocyanate sind entweder kommerziell erhältlich, oder können aus entsprechenden Aminen durch Umsetzung mit Phosgen in Toluol, THF oder Acetonitril unter Rückfluß hergestellt werden.

Die dabei eingesetzten Amine können wiederum kommerziell erhältlich sein, oder durch Reduktion aus Cyaniden, oder durch Reduktive Aminierung aus Ketonen erhalten werden.

Umsetzung der Isocyanate mit den Azolopyridinolen zu den Azolopyridinonharnstoffen.

Die erfindungsgemäßen 3-Oxo-3H-isothiazolo[5,4-b]pyridin-2-carbonsäueramide und 3-Oxo-3H-isothiazolo[5,4-d]pyrimidin-2-carbonsäueramide erhält man durch Umsetzung der Azolopyridinone der allgemeinen Formel II mit Isocyanaten.

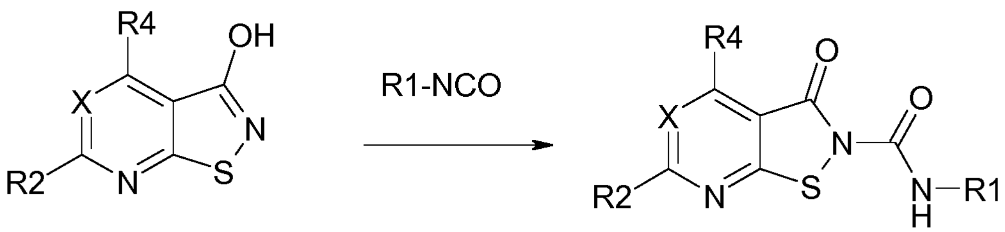


Azolopyridinonbausteine:

Methode A

Verbindung 1: Isothiazolo[5,4-b]pyridin-3-ol (1)

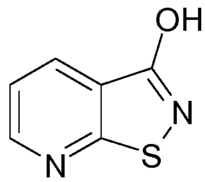

Zu einer Lösung von Diphenylphosphorylazid (6,9 mL, 32 mmol) in Pyridin (50 mL) und Triethylamin (4,5 m,L) wird bei 0° C 2-Mercaptonicotinsäure (5 g, 32 mmol) portionsweise zugegeben. Nach 30 min wird die Eiskühlung entfernt und der Ansatz für 16 h bei 25° C weiter gerührt. Das Lösungsmittel wird im Vakuum abdestilliert und der Rückstand, ein viskoses Öl mit Methyl-tert.Butylether versetzt. Unter Rühren wird EtOAc/$H_2O$ zugegeben und noch 15 Min nachgerührt. Der entstandene leicht gelbe Feststoff wird abgesaugt und getrocknet.
Ausbeute: 2,57 g

Methode B

Verbindung 2: 4,6-Dimethyl-isothiazolo[5,4-b]pyridin-3-ol (2)

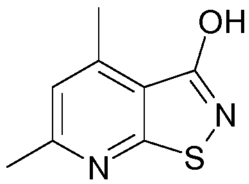

3-Cyano-4,6-dimethyl-2-mercaptopyridin (4,7 g, 28,6 mmol) wird in $H_2SO_4$ (60 mL, 98%ig) gelöst und anschließend für 3 h bei 105° C gerührt. Die Reaktion wird durch HPLC-MS verfolgt. Die Reaktionslösung wird auf 25° C abgekühlt und anschließend auf Eis gegossen. Das Produkt fällt verzögert (15 min) als voluminöser Niederschlag aus und wird abgesaugt, und mit $H_2O$ gewaschen. Der Rückstand wird mit wässriger $Na_2CO_3$-Lösung (pH 8-9) aufgenommen und das Produkt mit EtOAc extrahiert. Das Lösungsmittel wird im Vakuum abdestilliert und der Rückstand (weißer Feststoff) getrocknet ($Na_2SO_4$).
Ausbeute: 1,16 g (22,5%)

Verbindung 3: 6-Cyclopropyl-4-methyl-isothiazolo[5,4-d]pyrimidin-3-ol (3)

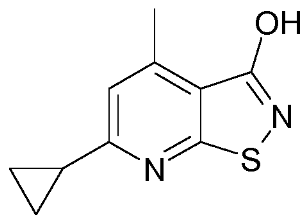

Verbindung 3 wird nach Methode A aus 2-Cyclopropyl-4-mercapto-6-methyl-pyrimidincarbonsäure hergestellt.

Verbindung 4: 6-Phenyl-isothiazolo[5,4-b]pyridin-3-ol (4)

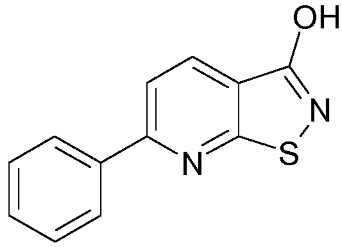

Verbindung 4 wird nach Methode A aus 2-Mercapto-6-phenyl-nicotinsäure hergestellt.

Verbindung 5: 6-Methyl-3-oxo-2,3-dihydro-isothiazolo[5,4-b]pyridin-4-carbonsäure (5)

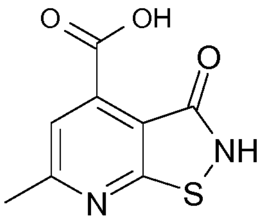

Verbindung 5 wird nach Methode B aus 3-Cyano-2-mercapto-6-methylisonicotin-säureethylester hergestellt.
Umsetzung der Isocyanate mit den Azolopyridinonen zu den Azolopyridinonharnstoffen.

Beispiel 1: 3-Oxo-3H-isothiazolo[5,4-b]pyridin-2-carbonsäure3-(furan-2-ylmethoxymethyl)-benzylamid (6)

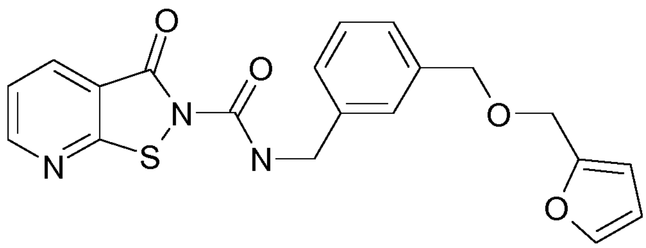

Eine Lösung von 3-(Furan-2-ylmethoxymethyl)benzylamin (218 mg, 1 mmol) in Toluol (10 mL) wird bei 25° C mit Phosgen (5 mL, 10 mmol); 20%ige Lösung in Toluol) versetzt und zum Rückfluß erhitzt. Nach 3 h wird der Ansatz filtriert und das Filtrat eingeengt. Der Rückstand wird in THF (25 mL) aufgenommen und mit Verbindung 1 (122 mg, 0,8 mmol) versetzt. Der Ansatz wird für 3 h bei 60° C gerührt. Die Reaktion wird per HPLC-MS verfolgt. Das Lösungsmittel wird reduziert, wobei das Produkt kristallisiert. C20H17N3O4S, Mw 395,44.
Ausbeute: 239 mg (75,7%).
Die im Folgenden aufgeführten Beispiele wurden analog hergestellt. In Fällen, in denen das Produkt nicht direkt kristallisierte, wurde das Produkt chromatographisch aufgereinigt.
Mit * gekennzeichnete Beispiele sind Vergleichsverbindungen

| Beispiel Nr. | Struktur | Name Summenformel Molgewicht [g/mol] |
|---|---|---|
| 2* | 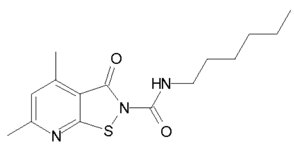 | 4,6-Dimethyl-3-oxo-3H-isothiazolo[5 ,4-b]pyridine-2-carboxylic acid hex ylamide<br>C15 H21 N3 02 307,416 |

(fortgesetzt)

| Beispiel Nr. | Struktur | Name Summenformel Molgewicht [g/mol] |
|---|---|---|
| 3 | 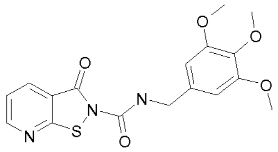 | 3-Oxo-3H-isothiazolo[5,4-b]pyridine -2-carboxylic acid 3,4,5-trimethoxy -benzylamide<br>C17H17N3O5S 375,406 |
| 4 | 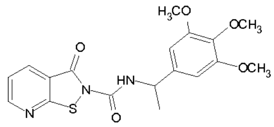 | 3-Oxo-3H-isothiazolo[5,4-b]pyridine -2-carboxylic acid [1-(3,4,5-trimet hoxy-phenyl)-ethyl]-amide<br>C18H19N3O5S 389,433 |
| 5 | 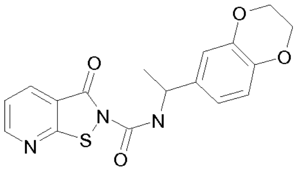 | 3-Oxo-3H-isothiazolo[5,4-b]pyridine -2-carboxylic acid [1-(2,3-dihydro-benzo[1,4]dioxin-6-yl)-ethyl]-amide<br>C17H15N3O4S 357,391 |
| 6 | 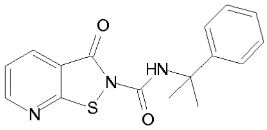 | 3-Oxo-3H-isothiazolo[5,4-b]pyridine -2-carboxylic acid (1-methyl-1-phen yl-ethyl)-amide<br>C16H15N3O2S 313,381 |
| 7 | 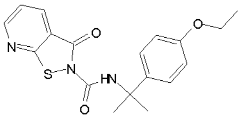 | 3-Oxo-3H-isothiazolo[5,4-b]pyridine -2-carboxylic acid (1-methyl-1-phen yl-ethyl)-amide<br>C18H19N3O3S 357,434 |
| 8 | 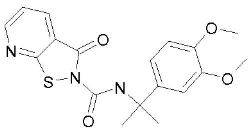 | 3-Oxo-3H-isothiazolo[5,4-b]pyridine -2-carboxylic acid [1-(3,4-dimethox y-phenyl)-1-methyl-ethyl]-amide<br>C18H19N3O4S 373,434 |
| 9 | 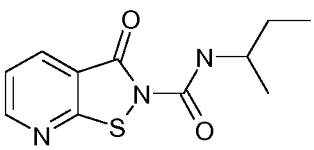 | 3-Oxo-3H-isothiazolo[5,4-b]pyridine -2-carboxylic acid sec-butylamide<br>C11H13N3O2S 251,309 |
| 10 * | 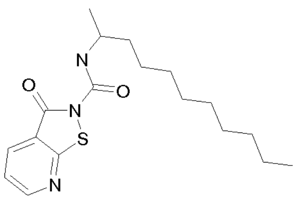 | 3-Oxo-3H-isothiazolo[5,4-b]pyridine -2-carboxylic acid (1-methyl-decyl) -amide<br>C18H27N3O2S 349,499 |
| 11 * | 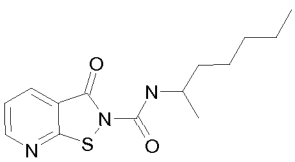 | 3-Oxo-3H-isothiazolo[5,4-b]pyridine -2-carboxylic acid (1-methyl-hexyl) -amide<br>C14H19N3O2S 293,39 |

(fortgesetzt)

| Beispiel Nr. | Struktur | Name Summenformel Molgewicht [g/mol] |
|---|---|---|
| 12 | 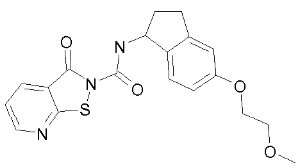 | 3-Oxo-3H-isothiazolo[5,4-b]pyridine -2-carboxylic acid [5-(2-methoxy-et hoxy)-indan-1-yl]-amide C19H19N3O4S 385,445 |
| 13 | 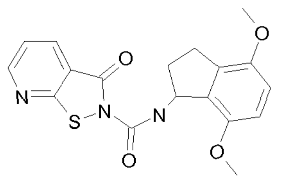 | 3-Oxo-3H-isothiazolo[5,4-b]pyridine -2-carboxylic acid (4,7-dimethoxy-i ndan-1-yl)-amide C18H17N3O4S 371,418 |
| 14* | 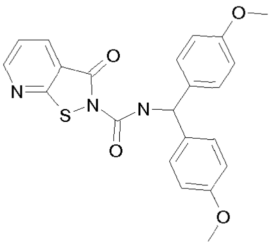 | 3-Oxo-3H-isothiazolo[5,4-b]pyridine -2-carboxylic acid [bis-(4-methoxyphenyl)-methyl]-amide C22H19N3O4S 421,478 |
| 15 | 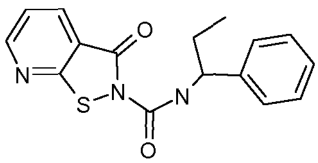 | 3-Oxo-3H-isothiazolo[5,4-b]pyridine -2-carboxylic acid (1-phenyl-propyl )-amide C16H15N3O2S 313,381 |
| 16 | 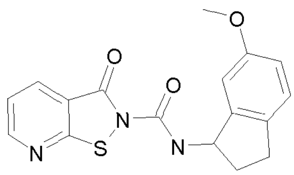 | 3-Oxo-3H-isothiazolo[5,4-b]pyridine -2-carboxylic acid (6-methoxy-indan -1-yl)-amide C17H15N3O3S 341,391 |
| 17 | 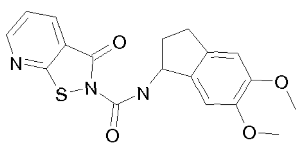 | 3-Oxo-3H-isothiazolo[5,4-b]pyridine -2-carboxylic acid (5,6-dimethoxy-i ndan-1-yl)-amide C18H17N3O4S 371,418 |
| 18 | 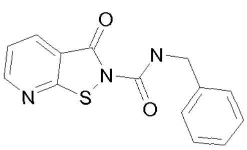 | 3-Oxo-3H-isothiazolo[5,4-b]pyridine -2-carboxylic acid benzylamide C14H11N3O2S 285,327 |
| 19 | 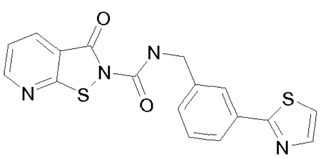 | 3-Oxo-3H-isothiazolo[5,4-b]pyridine -2-carboxylic acid 3-thiazol-2-yl-b enzylamide C17H12N4O2S2 368,439 |

(fortgesetzt)

| Beispiel Nr. | Struktur | Name Summenformel Molgewicht [g/mol] |
|---|---|---|
| 20 | 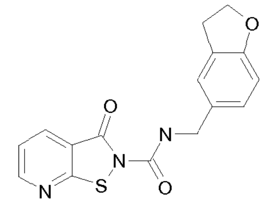 | 3-Oxo-3H-isothiazolo[5,4-b]pyridine -2-carboxylic acid (2,3-dihydro-ben zofuran-5-ylmethyl)-amide C16H13N3O3S 327,364 |
| 21 | 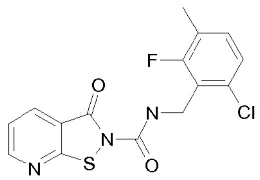 | 3-Oxo-3H-isothiazolo[5,4-b]pyridine -2-carboxylic acid 6-chloro-2-fluor o-3-methyl-benzylamide C15H11ClFN3O2 351,789 |
| 22 | 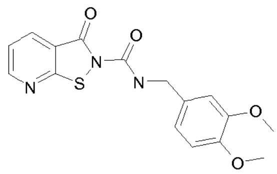 | 3-Oxo-3H-isothiazolo[5,4-b]pyridine -2-carboxylic acid 3,4-dimethoxy-be nzylamide C16H15N3O4S 345,38 |
| 23 | 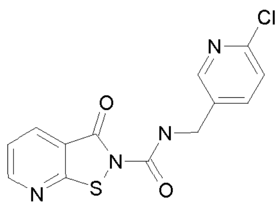 | 3-Oxo-3H-isothiazolo[5,4-b]pyridine -2-carboxylic acid (6-chloro-pyridi n-3-ylmethyl)-amide C13H9ClN4O2S 320,759 |
| 24 | 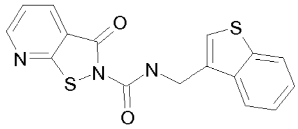 | 3-Oxo-3H-isothiazolo[5,4-b]pyridine -2-carboxylic acid (benzo[b]thiophe n-3-ylmethyl)-amide C16H11N3O2S2 341,413 |
| 25 | 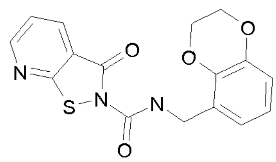 | 3-Oxo-3H-isothiazolo[5,4-b]pyridine -2-carboxylic acid (2,3-dihydro-ben zo[1,4]dioxin-5-ylmethyl)-amide C16H13N3O4S 343,364 |
| 26 | 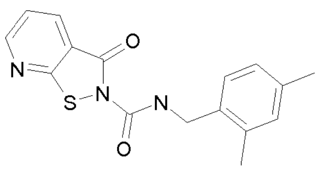 | 3-Oxo-3H-isothiazolo[5,4-b]pyridine -2-carboxylic acid 2,4-dimethyl-ben zylamide C16H15N3O2S 313,381 |
| 27 | 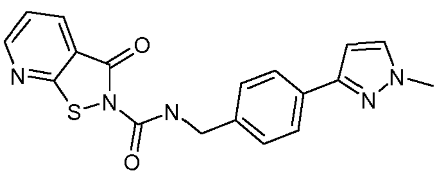 | 3-Oxo-3H-isothiazolo[5,4-b]pyridine -2-carboxylic acid 4-(1-methyl-1H-p yrazol-3-yl)-benzylamide C18H15N5O2S 365,417 |

(fortgesetzt)

| Beispiel Nr. | Struktur | Name Summenformel Molgewicht [g/mol] |
|---|---|---|
| 28 | 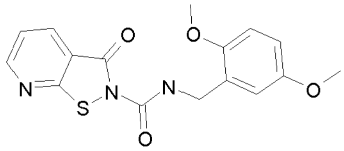 | 3-Oxo-3H-isothiazolo[5,4-b]pyridine -2-carboxylic acid 2,5-dimethoxy-be nzylamide<br>C16H15N3O4S 345,38 |
| 29 | 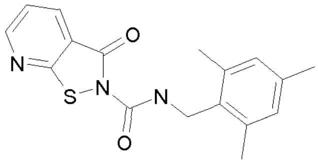 | 3-Oxo-3H-isothiazolo[5,4-b]pyridine -2-carboxylic acid 2,4,6-trimethylbenzylamide<br>C17H17N3O2S 327,408 |
| 30 | 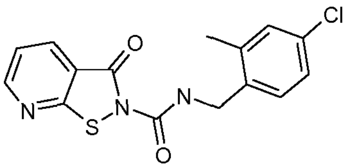 | 3-Oxo-3H-isothiazolo[5,4-b]pyridine -2-carboxylic acid 4-chloro-2-methy l-benzylamide<br>C15H12ClN3O2 333,799 |
| 31 | 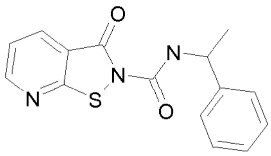 | 3-Oxo-3H-isothiazolo[5,4-b]pyridine -2-carboxylic acid (1-phenyl-ethyl) -amide<br>C15H13N3O2S 299,354 |
| 32 | 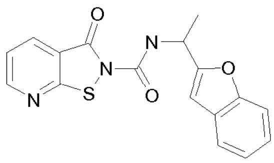 | 3-Oxo-3H-isothiazolo[5,4-b]pyridine -2-carboxylic acid (1-benzofuran-2-yl-ethyl)-amide<br>C17H13N3O3S 339,375 |
| 33 | 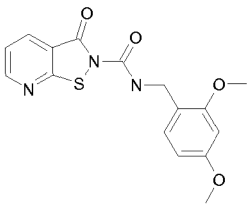 | 3-Oxo-3H-isothiazolo[5,4-b]pyridine -2-carboxylic acid 2,4-dimethoxy-be nzylamide<br>C16H15N3O4S 345,38 |
| 34 | 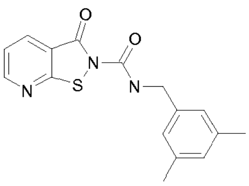 | 3-Oxo-3H-isothiazolo[5,4-b]pyridine -2-carboxylic acid 3,5-dimethyl-ben zylamide<br>C16H15N3O2S 313,381 |
| 35 | 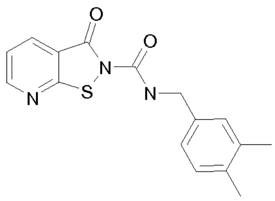 | 3-Oxo-3H-isothiazolo[5,4-b]pyridine -2-carboxylic acid 3,4-dimethyl-ben zylamide<br>C16H15N3O2S 313,381 |

(fortgesetzt)

| Beispiel Nr. | Struktur | Name Summenformel Molgewicht [g/mol] |
|---|---|---|
| 36 | 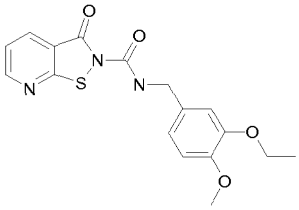 | 3-Oxo-3H-isothiazolo[5,4-b]pyridine -2-carboxylic acid 3-ethoxy-4-metho xy-benzylamide<br>C17H17N3O4S 359,407 |
| 37 | 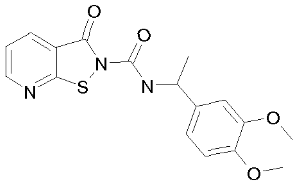 | 3-Oxo-3H-isothiazolo[5,4-b]pyridine -2-carboxylic acid [1-(3,4-dimethox y-phenyl)-ethyl]-amide<br>C17H17N3O4S 359,407 |
| 38 | 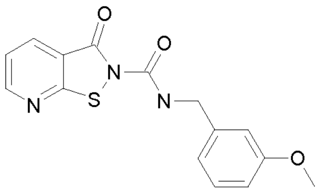 | 3-Oxo-3H-isothiazolo[5,4-b]pyridine -2-carboxylic acid 3-methoxy-benzyl amide<br>C15H13N3O3S 315,353 |
| 39 | 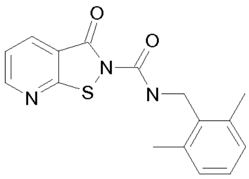 | 3-Oxo-3H-isothiazolo[5,4-b]pyridine -2-carboxylic acid 2,6-dimethyl-ben zylamide<br>C16H15N3O2S 313,381 |
| 40 | 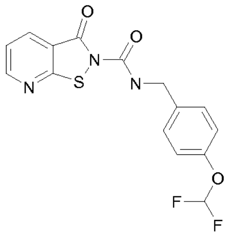 | 3-Oxo-3H-isothiazolo[5,4-b]pyridine -2-carboxylic acid 4-difluoromethox y-benzylamide<br>C15H11F2N3O3 351,334 |
| 41 | 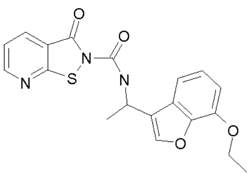 | 3-Oxo-3H-isothiazolo[5,4-b]pyridine -2-carboxylic acid [1-(7-ethoxy-ben zofuran-3-yl)-ethyl]-amide<br>C19H17N3O4S 383,429 |
| 42 | 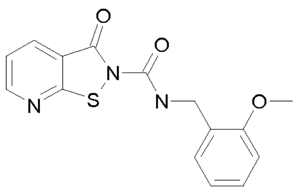 | 3-Oxo-3H-isothiazolo[5,4-b]pyridine -2-carboxylic acid 2-methoxy-benzyl amide<br>C15H13N3O3S 315,353 |

(fortgesetzt)

| Beispiel Nr. | Struktur | Name Summenformel Molgewicht [g/mol] |
|---|---|---|
| 43 | 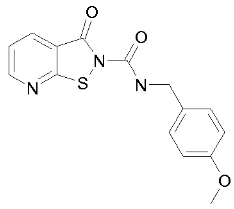 | 3-Oxo-3H-isothiazolo[5,4-b]pyridine -2-carboxylic acid 4-methoxy-benzyl amide<br>C15H13N3O3S 315,353 |
| 44 | 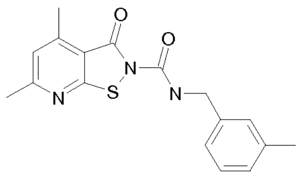 | 4,6-Dimethyl-3-oxo-3H-isothiazolo[5 ,4-b]pyridine-2-carboxylic acid 3-m ethyl-benzylamide<br>C17H17N3O2S 327,408 |
| 45 | 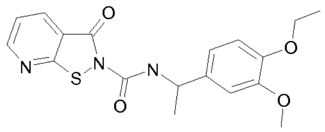 | 3-Oxo-3H-isothiazolo[5,4-b]pyridine -2-carboxylic acid [1-(4-ethoxy-3-m ethoxy-phenyl)-ethyl]-amide<br>C18H19N3O4S 373,434 |
| 46 | 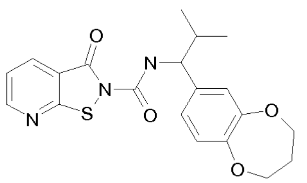 | 3-Oxo-3H-isothiazolo[5,4-b]pyridine -2-carboxylic acid [1-(3,4-dihydro-2H-benzo[b][1,4]dioxepin-7-yl)-2-me thyl-propyl]-amide<br>C20H21N3O4S 399,472 |
| 47 | 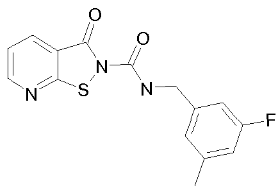 | 3-Oxo-3H-isothiazolo[5,4-b]pyridine -2-carboxylic acid 3-fluoro-5-methy l-benzylamide<br>C15H12FN3O2S 317,344 |
| 48 | 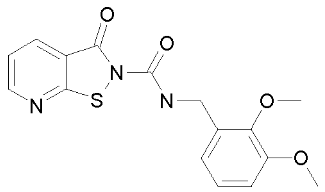 | 3-Oxo-3H-isothiazolo[5,4-b]pyridine -2-carboxylic acid 2,3-dimethoxy-be nzylamide<br>C16H15N3O4S 345,38 |
| 49 | 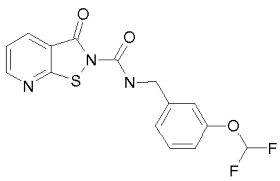 | 3-Oxo-3H-isothiazolo[5,4-b]pyridine -2-carboxylic acid 3-difluoromethox y-benzylamide<br>C15H11F2N3O3 351,334 |
| 50 | 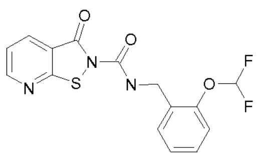 | 3-Oxo-3H-isothiazolo[5,4-b]pyridine -2-carboxylic acid 2-difluoromethox y-benzylamide<br>C15H11F2N3O3 351,334 |

(fortgesetzt)

| Beispiel Nr. | Struktur | Name Summenformel Molgewicht [g/mol] |
|---|---|---|
| 51 | 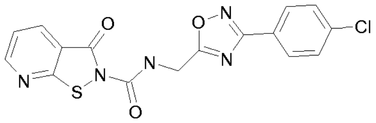 | 3-Oxo-3H-isothiazolo[5,4-b]pyridine -2-carboxylic acid [3-(4-chloro-phe nyl)-[1,2,4]oxadiazol-5-ylmethyl]-a mide C16H10ClN5O3S 387,807 |
| 52 | 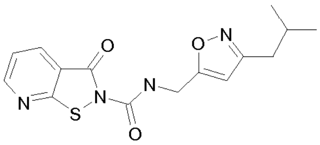 | 3-Oxo-3H-isothiazolo[5,4-b]pyridine -2-carboxylic acid (3-isobutyl-isox azol-5-ylmethyl)-amide C15H16N4O3S 332,384 |
| 53 | 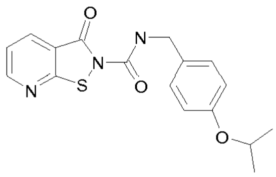 | 3-Oxo-3H-isothiazolo[5,4-b]pyridine -2-carboxylic acid 4-isopropoxy-ben zylamide C17H17N3O3S 343,407 |
| 54 | 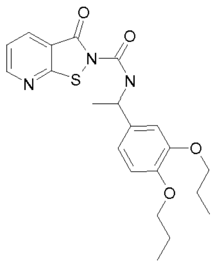 | 3-Oxo-3H-isothiazolo[5,4-b]pyridine -2-carboxylic acid [1-(3,4-dipropox y-phenyl)-ethyl]-amide C21H25N3O4S 415,515 |
| 55 | 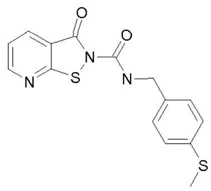 | 3-Oxo-3H-isothiazolo[5,4-b]pyridine -2-carboxylic acid 4-methylsulfanyl -benzylamide C15H13N3O2S2 331,418 |
| 56 | 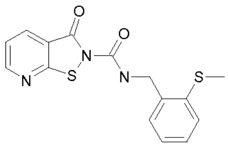 | 3-Oxo-3H-isothiazolo[5,4-b]pyridine -2-carboxylic acid 2-methylsulfanyl -benzylamide C15H13N3O2S2 331,418 |
| 57 | 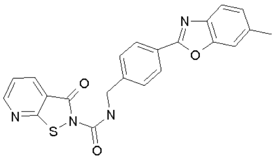 | 3-Oxo-3H-isothiazolo[5,4-b]pyridine -2-carboxylic acid 4-(6-methyl-benz ooxazol-2-yl)-benzylamide C22H16N4O3S 416,462 |
| 58 | 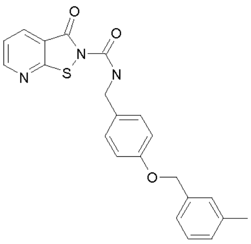 | 3-Oxo-3H-isothiazolo[5,4-b]pyridine -2-carboxylic acid 4-(3-methyl-benz yloxy)-benzylamide C22H19N3O3S 405,479 |

(fortgesetzt)

| Beispiel Nr. | Struktur | Name Summenformel Molgewicht [g/mol] |
|---|---|---|
| 59 | 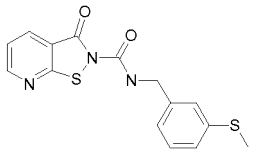 | 3-Oxo-3H-isothiazolo[5,4-b]pyridine -2-carboxylic acid 3-methylsulfanyl -benzylamide<br>C15H13N3O2S2 331,418 |
| 60 | 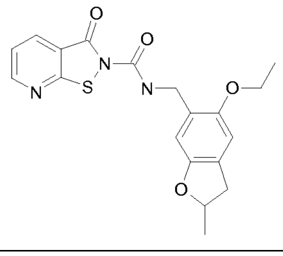 | 3-Oxo-3H-isothiazolo[5,4-b]pyridine -2-carboxylic acid (5-ethoxy-2-meth yl-2,3-dihydro-benzofuran-6-ylmethy l)-amide<br>C19H19N3O4S 385,445 |
| 61 | 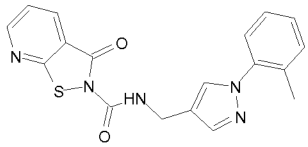 | 3-Oxo-3H-isothiazolo[5,4-b]pyridine -2-carboxylic acid (1-o-tolyl-1H-py razol-4-ylmethyl)-amide<br>C18H15N5O2S 365,417 |
| 62 | 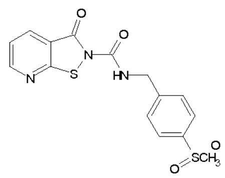 | 3-Oxo-3H-isothiazolo[5,4-b]pyridine -2-carboxylic acid 4-methanesulfony l-benzylamide<br>C15H13N3O4S2 363,417 |
| 63 | 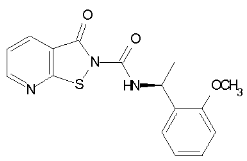 | 3-Oxo-3H-isothiazolo[5,4-b]pyridine -2-carboxylic acid [(S)-1-(2-methox y-phenyl)-ethyl]-amide<br>C16H15N3O3S 329,38 |
| 64 | 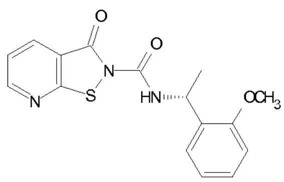 | 3-Oxo-3H-isothiazolo[5,4-b]pyridine -2-carboxylic acid [(R)-1-(2-methox y-phenyl)-ethyl]-amide<br>C16H15N3O3S 329,38 |
| 65 | 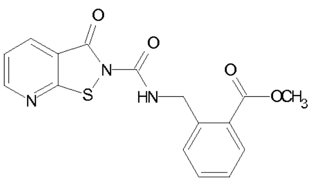 | 2-{[(3-Oxo-3H-isothiazolo[5,4-b]pyr idine-2-carbonyl)-amino]-methyl}-be nzoic acid methyl ester<br>C16H13N3O4S 343,364 |
| 66 | 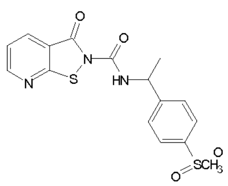 | 3-Oxo-3H-isothiazolo[5,4-b]pyridine -2-carboxylic acid [1-(4-methanesul fonyl-phenyl)-ethyl]-amide<br>C16H15N3O4S2 377,444 |

(fortgesetzt)

| Beispiel Nr. | Struktur | Name Summenformel Molgewicht [g/mol] |
|---|---|---|
| 67 | 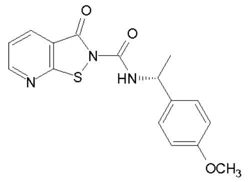 | 3-Oxo-3H-isothiazolo[5,4-b]pyridine -2-carboxylic acid [(R)-1-(4-methox y-phenyl)-ethyl]-amide C16H15N3O3S 329,38 |
| 68 | 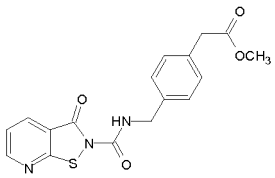 | (4-{[(3-Oxo-3H-isothiazolo[5,4-b]py ridine-2-carbonyl)-amino]-methyl}-p henyl)-acetic acid methyl ester C17H15N3O4S 357,391 |
| 69 | 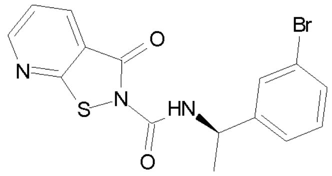 | 3-Oxo-3H-isothiazolo[5,4-b]pyridine -2-carboxylic acid [(R)-1-(3-bromo-phenyl)-ethyl]-amide C15H12BrN3O2 378,25 |
| 70 | 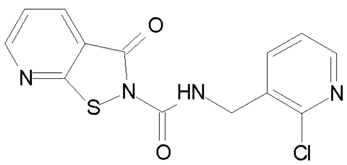 | 3-Oxo-3H-isothiazolo[5,4-b]pyridine -2-carboxylic acid (2-chloro-pyridi n-3-ylmethyl)-amide C13H9ClN4O2S 320,759 |
| 71 | 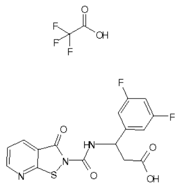 | 3-(3,5-Difluoro-phenyl)-3-[(3-oxo-3 H-isothiazolo[5,4-b] pyridine-2-carb onyl)-amino]-propionic acid; compou nd with trifluoro-acetic acid C16H11F2N3O4S. C2HF3O2 493,369 |
| 72 | 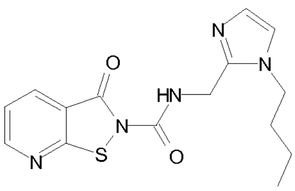 | 3-Oxo-3H-isothiazolo[5,4-b]pyridine -2-carboxylic acid (1-butyl-1H-imid azol-2-ylmethyl)-amide C15H17N5O2S 331,399 |
| 73 | 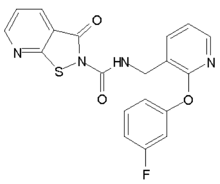 | 3-Oxo-3H-isothiazolo[5,4-b]pyridine -2-carboxylic acid [2-(3-fluoro-phe noxy)-pyridin-3-ylmethyl]-amide C19H13FN4O3S 396,403 |
| 74 | 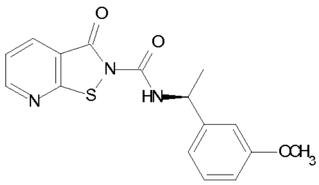 | 3-Oxo-3H-isothiazolo[5,4-b]pyridine -2-carboxylic acid [(S)-1-(3-methox y-phenyl)-ethyl]-amide C16H15N3O3S 329,38 |

(fortgesetzt)

| Beispiel Nr. | Struktur | Name Summenformel Molgewicht [g/mol] |
|---|---|---|
| 75 | 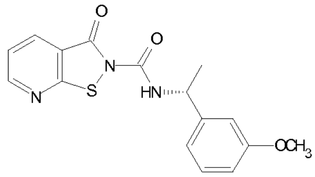 | 3-Oxo-3H-isothiazolo[5,4-b]pyridine -2-carboxylic acid [(R)-1-(3-methox y-phenyl)-ethyl]-amide<br>C16H15N3O3S 329,38 |
| 76 | 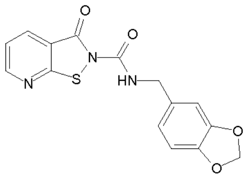 | 3-Oxo-3H-isothiazolo[5,4-b]pyridine -2-carboxylic acid (benzo[1,3]dioxo l-5-ylmethyl)-amide<br>C15H11N3O4S 329,337 |
| 77 | 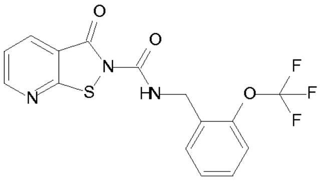 | 3-Oxo-3H-isothiazolo[5,4-b]pyridine -2-carboxylic acid 2-trifluorometho xy-benzylamide<br>C15H10F3N3O3 369,324 |
| 78 | 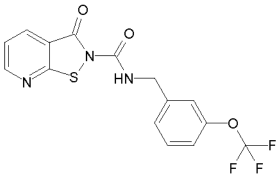 | 3-Oxo-3H-isothiazolo[5,4-b]pyridine -2-carboxylic acid 3-trifluorometho xy-benzylamide<br>C15H10F3N3O3 369,324 |
| 79 | 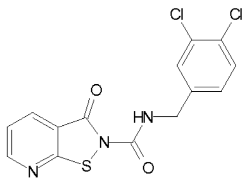 | 3-Oxo-3H-isothiazolo[5,4-b]pyridine -2-carboxylic acid 3,4-dichloro-ben zylamide<br>C14H9Cl2N3O2 354,217 |
| 80 | 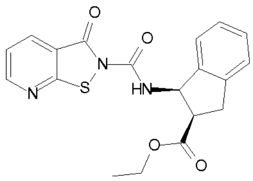 | (1R,2R)-1-[(3-Oxo-3H-isothiazolo[5, 4-b]pyridine-2-carbonyl)-amino]-ind an-2-carboxylic acid ethyl ester<br>C19H17N3O4S 383,429 |
| 81 | 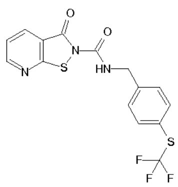 | 3-Oxo-3H-isothiazolo[5,4-b]pyridine -2-carboxylic acid 4-trifluoromethy lsulfanyl-benzylamide<br>C15H10F3N3O2 385,389 |
| 82 | 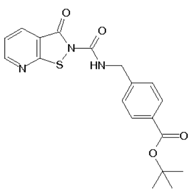 | 4-{[(3-Oxo-3H-isothiazolo[5,4-b]pyr idine-2-carbonyl)-amino]-methyl}-be nzoic acid tert-butyl ester<br>C19H19N3O4S 385,445 |

(fortgesetzt)

| Beispiel Nr. | Struktur | Name Summenformel Molgewicht [g/mol] |
|---|---|---|
| 83 | 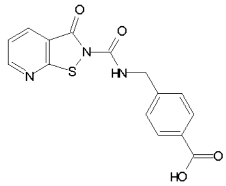 | 4-{[(3-Oxo-3H-isothiazolo[5,4-b]pyr idine-2-carbonyl)-amino]-methyl}-be nzoic acid<br>C15H11N3O4S 329,337 |
| 84 | 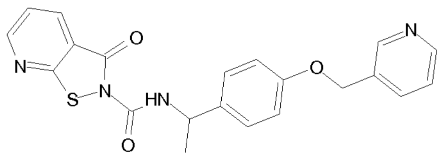 | 3-Oxo-3H-isothiazolo[5,4-b]pyridine -2-carboxylic acid {1-[4-(pyridin-3 -ylmethoxy)-phenyl]-ethyl}-amide<br>C21H18N4O3S 406,467 |
| 85 | 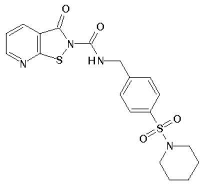 | 3-Oxo-3H-isothiazolo[5,4-b]pyridine -2-carboxylic acid 4-(piperidine-1-sulfonyl)-benzylamide<br>C19H20N4O4S2 432,524 |
| 86 | 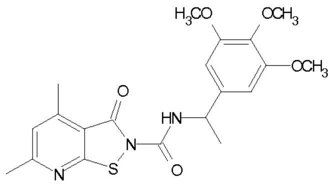 | 4,6-Dimethyl-3-oxo-3H-isothiazolo[5 ,4-b]pyridine-2-carboxylic acid [1-(3,4,5-trimethoxy-phenyl)-ethyl]-am ide<br>C20H23N3O5S 417,487 |
| 87 | 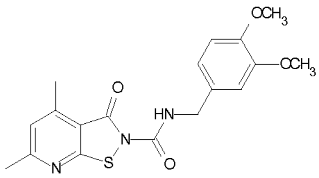 | 4,6-Dimethyl-3-oxo-3H-isothiazolo[5 ,4-b]pyridine-2-carboxylic acid 3,4 -dimethoxy-benzylamide<br>C18H19N3O4S 373,434 |
| 88 | 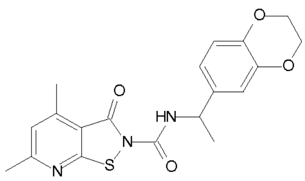 | 4,6-Dimethyl-3-oxo-3H-isothiazolo[5 ,4-b]pyridine-2-carboxylic acid [1-(2,3-dihydro-benzo[1,4]dioxin-6-yl) -ethyl]-amide<br>C19H19N3O4S 385,445 |
| 89 | 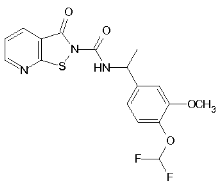 | 3-Oxo-3H-isothiazolo[5,4-b]pyridine -2-carboxylic acid [1-(4-difluorome thoxy-3-methoxy-phenyl)-ethyl]-amid e<br>C17H15F2N3O4 395,388 |
| 90 | 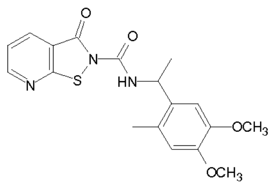 | 3-Oxo-3H-isothiazolo[5,4-b]pyridine -2-carboxylic acid [1-(4,5-dimethox y-2-methyl-phenyl)-ethyl]-amide<br>C18H19N3O4S 373,434 |

(fortgesetzt)

| Beispiel Nr. | Struktur | Name Summenformel Molgewicht [g/mol] |
|---|---|---|
| 91 | 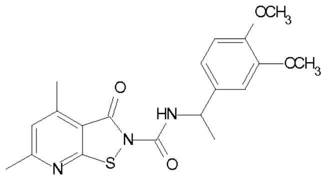 | 4,6-Dimethyl-3-oxo-3H-isothiazolo[5 ,4-b]pyridine-2-carboxylic acid [1-(3,4-dimethoxy-phenyl)-ethyl]-amide C19H21N3O4S 387,461 |
| 92 | 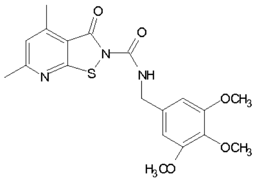 | 4,6-Dimethyl-3-oxo-3H-isothiazolo[5 ,4-b]pyridine-2-carboxylic acid 3,4 ,5-trimethoxy-benzylamide C19H21N3O5S 403,46 |
| 93 | 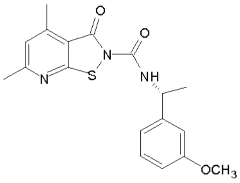 | 4,6-Dimethyl-3-oxo-3H-isothiazolo[5 ,4-b]pyridine-2-carboxylic acid [(R )-1-(3-methoxy-phenyl)-ethyl]-amide C18H19N3O3S 357,434 |
| 94 | 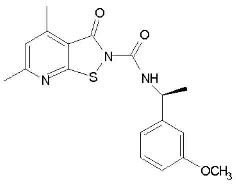 | 4,6-Dimethyl-3-oxo-3H-isothiazolo[5 ,4-b]pyridine-2-carboxylic acid [(S )-1-(3-methoxy-phenyl)-ethyl]-amide C18H19N3O3S 357,434 |
| 95 | 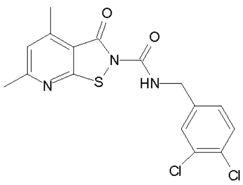 | 4,6-Dimethyl-3-oxo-3H-isothiazolo[5 ,4-b]pyridine-2-carboxylic acid 3,4 -dichloro-benzylamide C16H13Cl2N3O 382,271 |
| 96 | 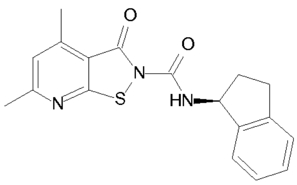 | 4,6-Dimethyl-3-oxo-3H-isothiazolo[5 ,4-b]pyridine-2-carboxylic acid (S) -indan-1-ylamide C18H17N3O2S 339,419 |
| 97 | 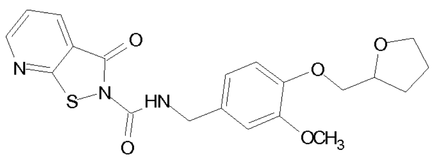 | 3-Oxo-3H-isothiazolo[5,4-b]pyridine -2-carboxylic acid 3-methoxy-4-(tet rahydro-furan-2-ylmethoxy)-benzylam ide C20H21N3O5S 415,471 |
| 98 | 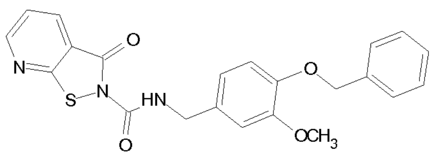 | 3-Oxo-3H-isothiazolo[5,4-b]pyridine -2-carboxylic acid 4-benzyloxy-3-me thoxy-benzylamide C22H19N3O4S 421,478 |

(fortgesetzt)

| Beispiel Nr. | Struktur | Name Summenformel Molgewicht [g/mol] |
|---|---|---|
| 99 | 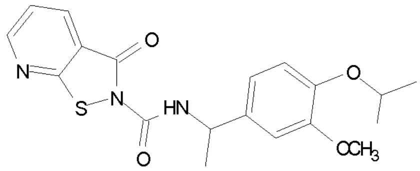 | 3-Oxo-3H-isothiazolo[5,4-b]pyridine -2-carboxylic acid [1-(4-isopropoxy -3-methoxy-phenyl)-ethyl]-amide C19H21N3O4S 387,461 |
| 100 | 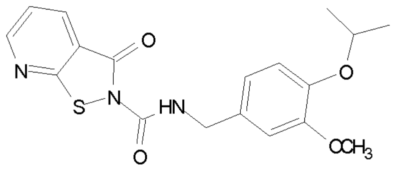 | 3-Oxo-3H-isothiazolo[5,4-b]pyridine -2-carboxylic acid 4-isopropoxy-3-m ethoxy-benzylamide C18H19N3O4S 373,434 |
| 101 | 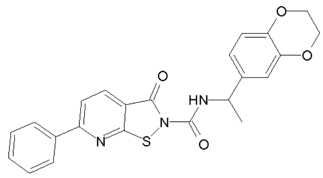 | 3-Oxo-6-phenyl-3H-isothiazolo[5,4-b ]pyridine-2-carboxylic acid [1-(2,3 -dihydro-benzo[1,4]dioxin-6-yl)-eth yl]-amide C23H19N3O4S 433,49 |
| 102 | 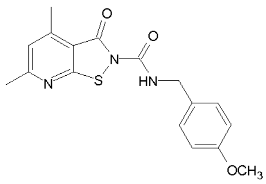 | 4,6-Dimethyl-3-oxo-3H-isothiazolo[5 ,4-b]pyridine-2-carboxylic acid 4-m ethoxy-benzylamide C17H17N3O3S 343,407 |
| 103 | 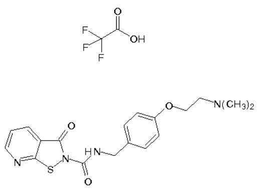 | 3-Oxo-3H-isothiazolo[5,4-b]pyridine -2-carboxylic acid 4-(2-dimethylami no-ethoxy)-benzylamide; compound wi th trifluoro-acetic acid C18H20N4O3S. 2C2HF3O2 600,498 |
| 104 | 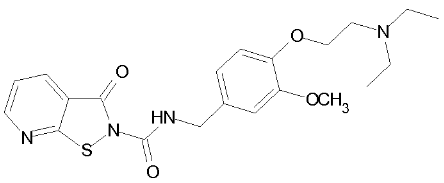 | 3-Oxo-3H-isothiazolo[5,4-b]pyridine -2-carboxylic acid 4-(2-diethylamin o-ethoxy)-3-methoxy-benzylamide C21H26N4O4S 430,53 |
| 105 | 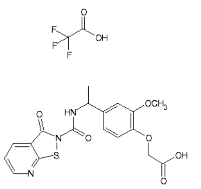 | (2-Methoxy-4-{1-[(3-oxo-3H-isothiaz olo[5,4-b]pyridine-2-carbonyl)-amin o]-ethyl}-phenoxy)-acetic acid; com pound with trifluoro-acetic acid C18H17N3O6S. |
| | | C2HF3O2 517,441 |
| 106 | 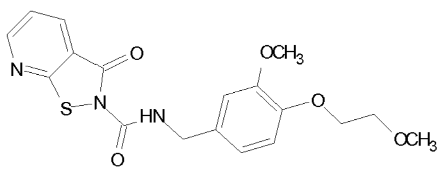 | 3-Oxo-3H-isothiazolo[5,4-b]pyridine -2-carboxylic acid 3-methoxy-4-(2-m ethoxy-ethoxy)-benzylamide C18H19N3O5S 389,433 |

(fortgesetzt)

| Beispiel Nr. | Struktur | Name Summenformel Molgewicht [g/mol] |
|---|---|---|
| 107 | 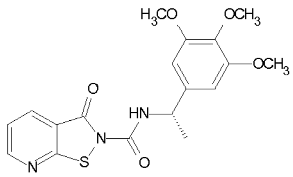 | 3-Oxo-3H-isothiazolo[5,4-b]pyridine -2-carboxylic acid [(S)-1-(3,4,5-tr imethoxy-phenyl)-ethyl]-amide C18H19N3O5S 389,433 |
| 108 | 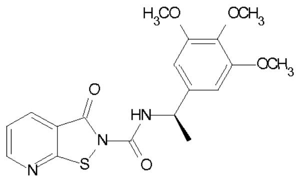 | 3-Oxo-3H-isothiazolo[5,4-b]pyridine -2-carboxylic acid [(R)-1-(3,4,5-tr imethoxy-phenyl)-ethyl]-amide C18H19N3O5S 389,433 |
| 109 | 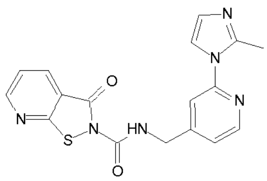 | 3-Oxo-3H-isothiazolo[5,4-b]pyridine -2-carboxylic acid [2-(2-methyl-imi dazol-1-yl)-pyridin-4-ylmethyl]-ami de C17H14N6O2S 366,404 |
| 110 | 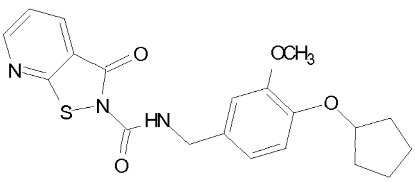 | 3-Oxo-3H-isothiazolo[5,4-b]pyridine -2-carboxylic acid 4-cyclopentyloxy -3-methoxy-benzylamide C20H21N3O4S 399,472 |
| 111 | 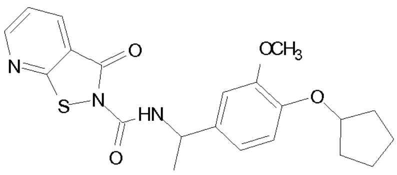 | 3-Oxo-3H-isothiazolo[5,4-b]pyridine -2-carboxylic acid [1-(4-cyclopenty loxy-3-methoxy-phenyl)-ethyl]-amide C21H23N3O4S 413,499 |
| 112 | 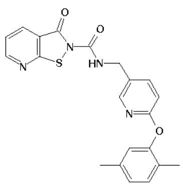 | 3-Oxo-3H-isothiazolo[5,4-b]pyridine -2-carboxylic acid [6-(2,5-dimethyl -phenoxy)-pyridin-3-ylmethyl]-amide C21H18N4O3S 406,467 |
| 113 | 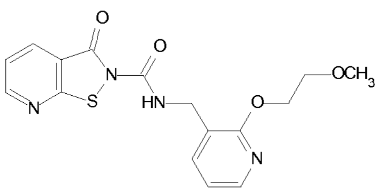 | 3-Oxo-3H-isothiazolo[5,4-b]pyridine -2-carboxylic acid [2-(2-methoxy-et hoxy)-pyridin-3-ylmethyl]-amide C16H16N4O4S 360,394 |
| 114 | 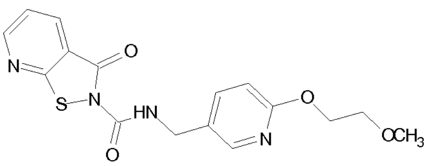 | 3-Oxo-3H-isothiazolo[5,4-b]pyridine -2-carboxylic acid [6-(2-methoxy-et hoxy)-pyridin-3-ylmethyl]-amide C16H16N4O4S 360,394 |

(fortgesetzt)

| Beispiel Nr. | Struktur | Name Summenformel Molgewicht [g/mol] |
|---|---|---|
| 115 | 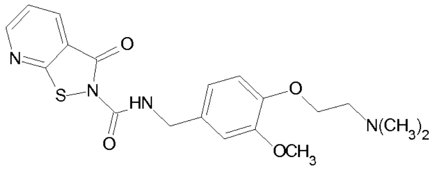 | 3-Oxo-3H-isothiazolo[5,4-b]pyridine -2-carboxylic acid 4-(2-dimethylami no-ethoxy)-3-methoxy-benzylamide C19H22N4O4S 402,476 |
| 116 | 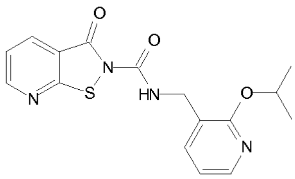 | 3-Oxo-3H-isothiazolo[5,4-b]pyridine -2-carboxylic acid (2-isopropoxy-py ridin-3-ylmethyl)-amide C16H16N4O3S 344,395 |
| 117 | 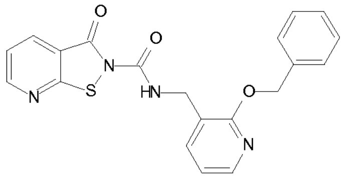 | 3-Oxo-3H-isothiazolo[5,4-b]pyridine -2-carboxylic acid (2-benzyloxy-pyr idin-3-ylmethyl)-amide C20H16N4O3S 392,44 |
| 118 | 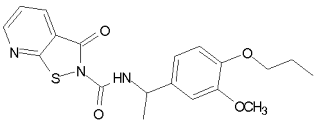 | 3-Oxo-3H-isothiazolo[5,4-b]pyridine -2-carboxylic acid [1-(3-methoxy-4-propoxy-phenyl)-ethyl]-amide C19H21N3O4S 387,461 |
| 119 | 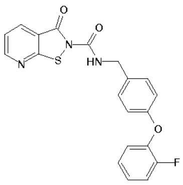 | 3-Oxo-3H-isothiazolo[5,4-b]pyridine -2-carboxylic acid 4-(2-fluoro-phen oxy)-benzylamide C20H14FN3O3S 395,415 |
| 120 | 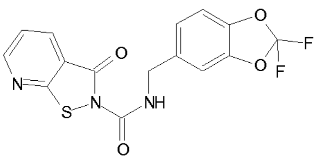 | 3-Oxo-3H-isothiazolo[5,4-b]pyridine -2-carboxylic acid (2,2-difluoro-be nzo[1,3]dioxol-5-ylmethyl)-amide C15H9F2N3O4S 365,317 |
| 121 | HCl 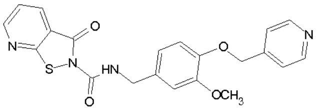 | 3-Oxo-3H-isothiazolo[5,4-b]pyridine -2-carboxylic acid 3-methoxy-4-(pyr idin-4-ylmethoxy)-benzylamide C21H18N4O4S 458,927 |
| 122 | 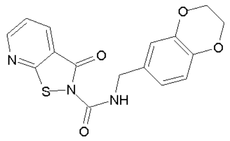 | 3-Oxo-3H-isothiazolo[5,4-b]pyridine -2-carboxylic acid (2,3-dihydro-ben zo[1,4]dioxin-6-ylmethyl)-amide C16H13N3O4S 343,364 |
| 123 | 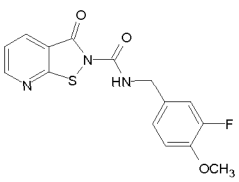 | 3-Oxo-3H-isothiazolo[5,4-b]pyridine -2-carboxylic acid 3-fluoro-4-metho xy-benzylamide C15H12FN3O3S 333,344 |

(fortgesetzt)

| Beispiel Nr. | Struktur | Name Summenformel Molgewicht [g/mol] |
|---|---|---|
| 124 | 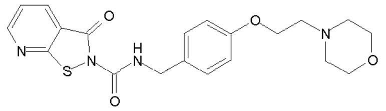 | 3-Oxo-3H-isothiazolo[5,4-b]pyridine -2-carboxylic acid 4-(2-morpholin-4 -yl-ethoxy)-benzylamide C20H22N4O4S 414,487 |
| 125 | 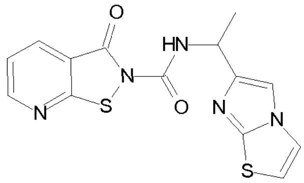 | 3-Oxo-3H-isothiazolo[5,4-b]pyridine -2-carboxylic acid (1-imidazo[2,1-b ]thiazol-6-yl-ethyl)-amide C14H11N5O2S2 345,404 |
| 126 | 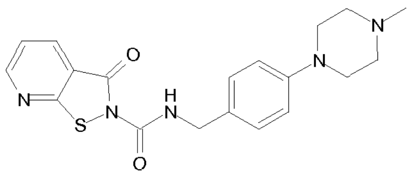 | 3-Oxo-3H-isothiazolo[5,4-b]pyridine -2-carboxylic acid 4-(4-methyl-pipe razin-1-yl)-benzylamide C19H21N5O2S 383,476 |
| 127 | 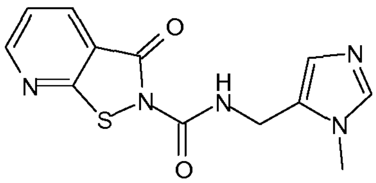 | 3-Oxo-3H-isothiazolo[5,4-b]pyridine -2-carboxylic acid (3-methyl-3H-imi dazol-4-ylmethyl)-amide C12H11N5O2S 289,318 |
| 128 | 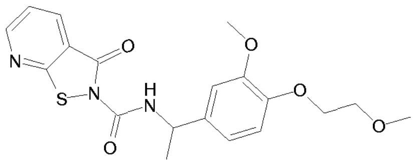 | 3-Oxo-3H-isothiazolo[5,4-b]pyridine -2-carboxylic acid {1-[3-methoxy-4-(2-methoxy-ethoxy)-phenyl]-ethyl}-a mide C19H21N3O5S 403,46 |
| 129 | 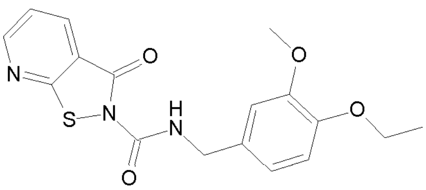 | 3-Oxo-3H-isothiazolo[5,4-b]pyridine -2-carboxylic acid 4-ethoxy-3-metho xy-benzylamide C17H17N3O4S 359,407 |
| 130 | 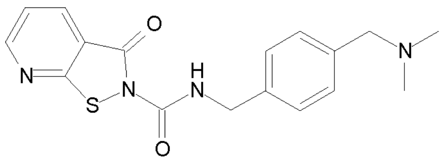 | 3-Oxo-3H-isothiazolo[5,4-b]pyridine -2-carboxylic acid 4-dimethylaminom ethyl-benzylamide C17H18N4O2S 342,423 |
| 131 | 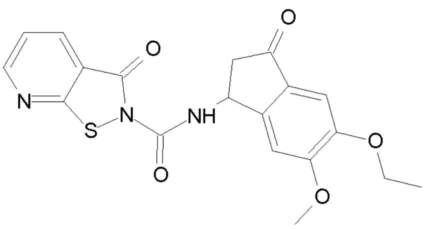 | 3-Oxo-3H-isothiazolo[5,4-b]pyridine -2-carboxylic acid (5-ethoxy-6-meth oxy-3-oxo-indan-1-yl)-amide C19H17N3O5S 399,42 |

(fortgesetzt)

| Beispiel Nr. | Struktur | Name Summenformel Molgewicht [g/mol] |
|---|---|---|
| 132 | 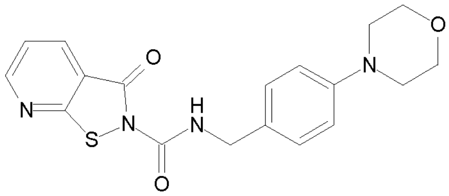 | 3-Oxo-3H-isothiazolo[5,4-b]pyridine -2-carboxylic acid 4-morpholin-4-yl -benzylamide<br>C18H18N4O3S<br>370,433 |
| 133 | 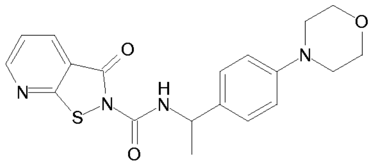 | 3-Oxo-3H-isothiazolo[5,4-b]pyridine -2-carboxylic acid [1-(4-morpholin-4-yl-phenyl)-ethyl]-amide<br>C19H20N4O3S<br>384,46 |
| 134 | 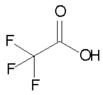<br>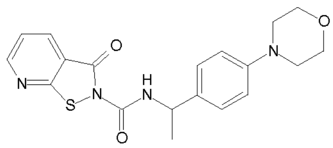 | 3-Oxo-3H-isothiazolo[5,4-b]pyridine -2-carboxylic acid [1-(4-morpholin-4-yl-phenyl)-ethyl]-amide<br>C19H20N4O3S.2C2HF3O2<br>612,509 |
| 135 | 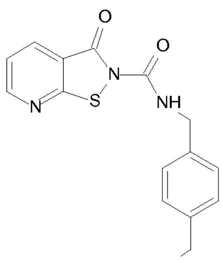 | 3-Oxo-3H-isothiazolo[5,4-b]pyridine -2-carboxylic acid 4-pyrrolidin-1-y lmethyl-benzylamide<br>C19H20N4O2S<br>368,461 |
| 136 | 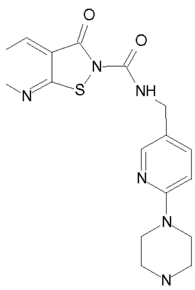 | 3-Oxo-3H-isothiazolo[5,4-b]pyridine -2-carboxylic acid [6-(4-methyl-pip erazin-1-yl)-pyridin-3-ylmethyl]-am ide<br>C18H20N6O2S<br>384,463 |
| 137 | 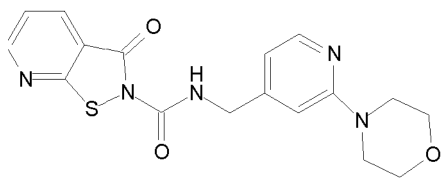 | 3-Oxo-3H-isothiazolo[5,4-b]pyridine -2-carboxylic acid (2-morpholin-4-y l-pyridin-4-ylmethyl)-amide<br>C17H17N5O3S<br>371,421 |

(fortgesetzt)

| Beispiel Nr. | Struktur | Name Summenformel Molgewicht [g/mol] |
|---|---|---|
| 138 | 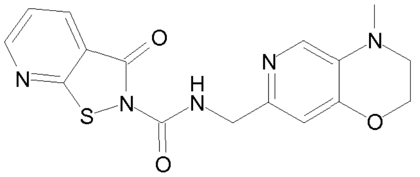 | 3-Oxo-3H-isothiazolo[5,4-b]pyridine -2-carboxylic acid (4-methyl-3,4-di hydro-2H-pyrido[4,3-b][1,4]oxazin-7 -ylmethyl)-amide<br>C16H15N5O3S<br>357,394 |
| 139 * | 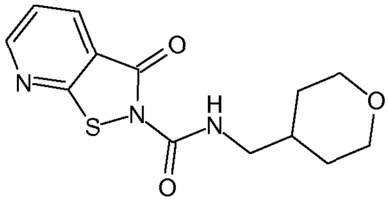 | 3-Oxo-3H-isothiazolo[5,4-b]pyridine -2-carboxylic acid (tetrahydro-pyra n-4-ylmethyl)-amide<br>C13H15N3O3S 293,347 |
| 140 * | 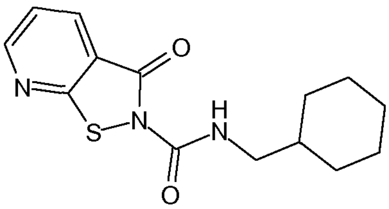 | 3-Oxo-3H-isothiazolo[5,4-b]pyridine -2-carboxylic acid cyclohexylmethyl -amide<br>C14H17N3O2S 291,374 |
| 141 | 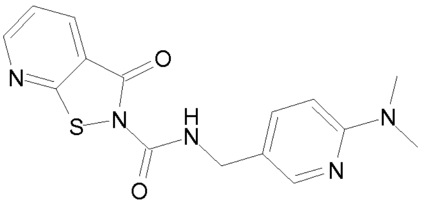 | 3-Oxo-3H-isothiazolo[5,4-b]pyridine -2-carboxylic acid (6-dimethylamino -pyridin-3-ylmethyl)-amide<br>C15H15N5O2S 329,383 |
| 142 * | 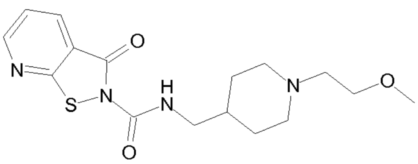 | 3-Oxo-3H-isothiazolo[5,4-b]pyridine -2-carboxylic acid [1-(2-methoxy-et hyl)-piperidin-4-ylmethyl]-amide<br>C16H22N4O3S 350,443 |
| 143 | 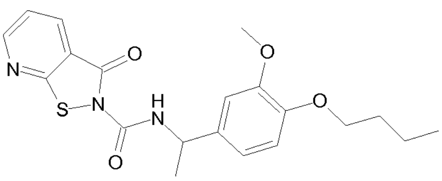 | 3-Oxo-3H-isothiazolo[5,4-b]pyridine -2-carboxylic acid [1-(4-butoxy-3-m ethoxy-phenyl)-ethyl]-amide<br>C20H23N3O4S 401,488 |
| 144 | 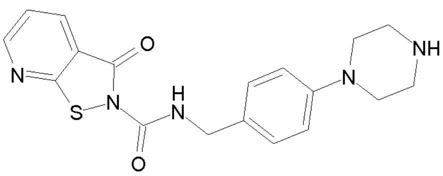 | 3-Oxo-3H-isothiazolo[5,4-b]pyridine -2-carboxylic acid 4-piperazin-1-yl -benzylamide<br>C18H19N5O2S 369,448 |

(fortgesetzt)

| Beispiel Nr. | Struktur | Name Summenformel Molgewicht [g/mol] |
|---|---|---|
| 145 | 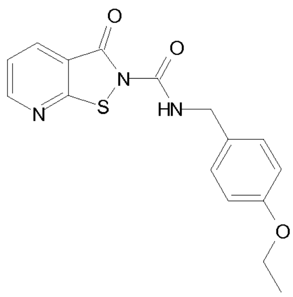 | 3-Oxo-3H-isothiazolo[5,4-b]pyridine -2-carboxylic acid 4-ethoxy-benzyla mide<br>C16H15N3O3S 329,38 |
| 146 | 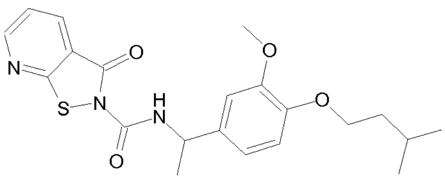 | 3-Oxo-3H-isothiazolo[5,4-b]pyridine -2-carboxylic acid {1-[3-methoxy-4-(3-methyl-butoxy)-phenyl]-ethyl}-am ide<br>C21H25N3O4S 415,515 |
| 147 | 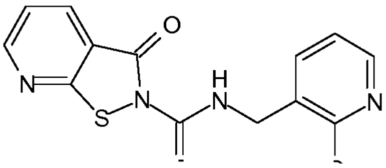 | 3-Oxo-3H-isothiazolo[5,4-b]pyridine -2-carboxylic acid (2-ethoxy-pyridi n-3-ylmethyl)-amide<br>C15H14N4O3S<br>330,368 |
| 148 | 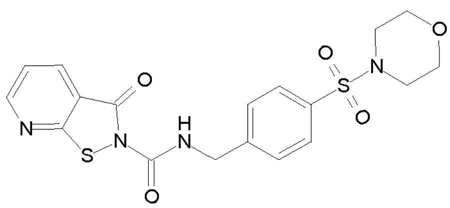 | 3-Oxo-3H-isothiazolo[5,4-b]pyridine -2-carboxylic acid 4-(morpholine-4-sulfonyl)-benzylamide<br>C18H18N4O5S2<br>434,496 |
| 149 | 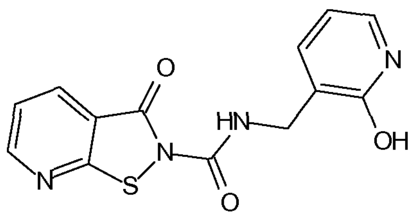 | 3-Oxo-3H-isothiazolo[5,4-b]pyridine -2-carboxylic acid (2-hydroxy-pyrid in-3-ylmethyl)-amide<br>C13H10N4O3S<br>302,314 |
| 150 | 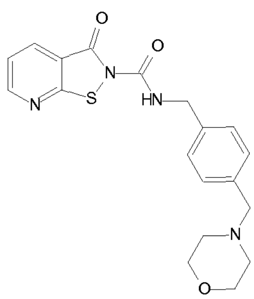 | 3-Oxo-3H-isothiazolo[5,4-b]pyridine -2-carboxylic acid 4-morpholin-4-yl methyl-benzylamide<br>C19H20N4O3S<br>384,46 |

(fortgesetzt)

| Beispiel Nr. | Struktur | Name Summenformel Molgewicht [g/mol] |
|---|---|---|
| 151 | 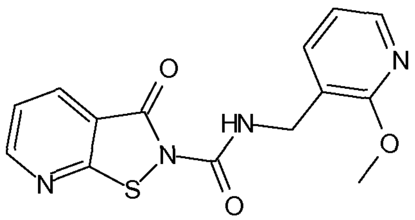 | 3-Oxo-3H-isothiazolo[5,4-b]pyridine -2-carboxylic acid (2-methoxy-pyrid in-3-ylmethyl)-amide C14H12N4O3S 316,34 |
| 152 | 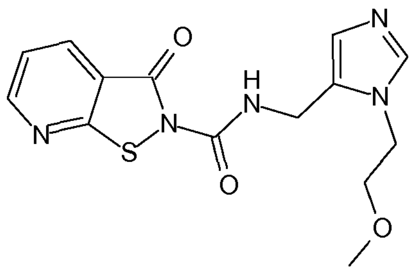 | 3-Oxo-3H-isothiazolo[5,4-b]pyridine -2-carboxylic acid [3-(2-methoxy-et hyl)-3H-imidazol-4-ylmethyl]-amide C14H15N5O3S 333,371 |
| 153 | 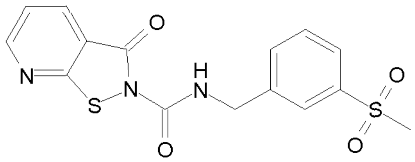 | 3-Oxo-3H-isothiazolo[5,4-b]pyridine -2-carboxylic acid 3-methanesulfony l-benzylamide C15H13N3O4S2 363,417 |
| 154 | 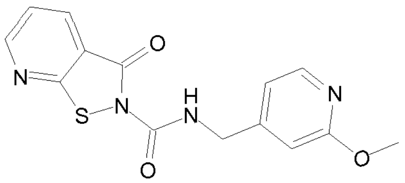 | 3-Oxo-3H-isothiazolo[5,4-b]pyridine -2-carboxylic acid (2-methoxy-pyrid in-4-ylmethyl)-amide C14H12N4O3S 316,341 |
| 155 | 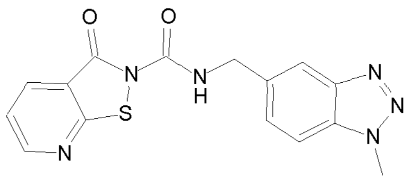 | 3-Oxo-3H-isothiazolo[5,4-b]pyridine -2-carboxylic acid (1-methyl-1H-ben zotriazol-5-ylmethyl)-amide C15H12N6O2S 340,366 |
| 156 | 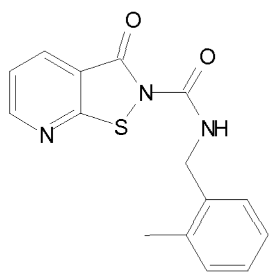 | 3-Oxo-3H-isothiazolo[5,4-b]pyridine -2-carboxylic acid 2-methyl-benzyla mide C15 H13 N3 O2 S 299,353 |
| 157 * | 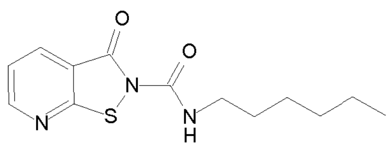 | 3-Oxo-3H-isothiazolo[5,4-b]pyridine -2-carboxylic acid hexylamide C13 H17 N3 O2 S 279,362 |

(fortgesetzt)

| Beispiel Nr. | Struktur | Name Summenformel Molgewicht [g/mol] |
|---|---|---|
| 158 | 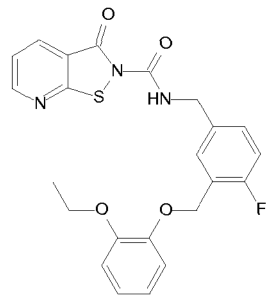 | 3-Oxo-3H-isothiazolo[5,4-b]pyridine -2-carboxylic acid 3-(2-ethoxy-phen oxymethyl)-4-fluoro-benzylamide<br>C23H20FN3O4S 453,496 |
| 159 | 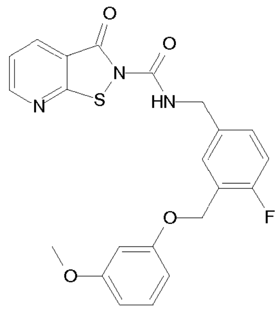 | 3-Oxo-3H-isothiazolo[5,4-b]pyridine -2-carboxylic acid 4-fluoro-3-(3-me thoxy-phenoxymethyl)-benzylamide<br>C22H18FN3O4S 333,371 |
| 160 | 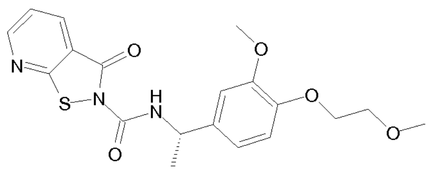 | 3-Oxo-3H-isothiazolo[5,4-b]pyridine -2-carboxylic acid {(S)-1-[3-methox y-4-(2-methoxy-ethoxy)-phenyl]-ethy l}-amide<br>C19H21N3O5S<br>403,46 |
| 161 | 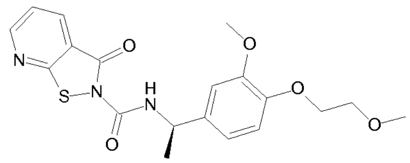 | 3-Oxo-3H-isothiazolo[5,4-b]pyridine -2-carboxylic acid {(R)-1-[3-methox y-4-(2-methoxy-ethoxy)-phenyl]-ethy l}-amide<br>C19H21N3O5S<br>403,46 |

## Patentansprüche

**1.** Verbindung der Formel I

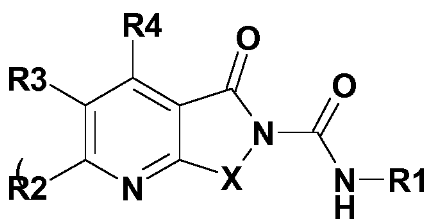

(I)

wobei bedeuten:

X S oder $SO_2$;
R1

ein Rest der Formel Ia

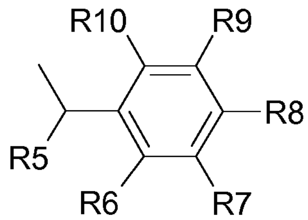

,

worin

R5 Wasserstoff, $(C_1-C_3)$-Alkyl;
R6, R7, R8, R9, R10
unabhängig voneinander Wasserstoff, OH, CN, $OCF_3$, $OCHF_2$, O-$(C_1-C_6)$-Alkyl, O-$(C_1-C_4)$-Alkoxy-$(C_1-C_4)$-alkyl, S-$(C_1-C_6)$-Alkyl, $(C_2-C_4)$-Haloalkyl, O-$(C_2-C_4)$-Haloalkyl, $(C_2-C_6)$-Alkenyl, $(C_2-C_6)$-Alkinyl, N(R11)(R12), $SO_2$-$CH_3$, $SO_2$-N(R13)(R14), $SF_5$, $SCF_3$, COOH, COO-$(C_1-C_6)$-Alkyl, CON(R15)(R16), N(R17)CO(R18), N(R19)$SO_2$(R20), CO(R21), $(CR22R23)_x$-O(R24), $(CR22R23)_x$-CO-O(R24), O-$(CR22R23)_x$-CO-O(R24), $(CR22R23)_x$-N(R25)(R26), O-$(CR22R23)_x$-N(R25)(R26), $(CR22R23)_x$-CON(R25)(R26), O-$(CR22R23)_x$-CON(R25)(R26), O-CO-N(R25)(R26), O-CO-$(C_1-C_6)$-Alkylen-CO-O-$(C_1-C_6)$-Alkyl, O-CO-$(C_1-C_6)$-Alkylen-CO-OH, O-CO-$(C_1-C_6)$-Alkylen-CO-N(R27)(R28);
mit der Massgabe dass mindestens ein Rest R6, R7, R8, R9, R10 von Wasserstoff verschieden ist;
x unabhängig voneinander 0, 1, 2, 3, 4, 5, 6;
oder
R7 oder R8 ist
$(O)_y$-$(CH_2-)_{y'}$-$(O)_{y''}$-$(CH_2)_{y'''}$-R100;
y, y" unabhängig voneinander 0,1;
y', y''' unabhängig voneinander 1, 2, 3, 4, 5, 6;
R100 ein 4 bis 10 gliedriger mono- oder bicyclischer gesättigter, teilweise ungesättigter oder aromatischer Ring welcher 1 bis 4 Heteroatome, ausgewählt aus der Gruppe Sauerstoff, Stickstoff oder Schwefel enthalten kann, wobei das Ringsystem zusätzlich ein oder mehrfach durch F, Cl, Br, I, OH, $CF_3$, $NO_2$, CN, $OCF_3$, O-$(C_1-C_6)$-Alkyl, O-$(C_1-C_4)$-Alkoxy-$(C_1-C_4)$-alkyl, S-$(C_1-C_6)$-Alkyl, $(C_1-C_6)$-Alkyl, $(C_2-C_4)$-Haloalkyl, $(C_2-C_6)$-Alkenyl, $(C_3-C_8)$-Cycloalkyl, O-$(C_3-C_8)$-Cycloalkyl, $(C_2-C_6)$-Alkinyl, N(R29)(R30), $SO_2$-$CH_3$, $SF_5$, COOH, COO-$(C_1-C_6)$-Alkyl, CON(R31)(R32), N(R33)CO(R34), N(R35)$SO_2$(R36), CO(R37), $(CR38R39)_{x'}$-O(R40), $(CR38R39)_{x'}$-CO-O(R40), O-$(CR22R23)_{x'}$-CO-O(R40), $(CR22R23)_{x'}$-N(R41)(R42), O-$(CR38R39)_{x'}$-N(R41)(R42), $(CR38R39)_{x'}$-CON(R41)(R42), O-$(CR38R39)_{x'}$-CON(R41)(R42), O-CO-N(R41)(R42), O-CO-$(C_1-C_6)$-Alkylen-CO-O-$(C_1-C_6)$-Alkyl, O-CO-$(C_1-C_6)$-Alkylen-CO-OH, O-CO-$(C_1-C_6)$-Alkylen-CO-N(R43)(R44) substituiert sein kann;
x' 0, 1, 2, 3, 4, 5, 6;
oder
R7 und R8 oder R8 und R9 oder R9 und R10
zusammen mit dem sie tragenden Kohlenstoffatomen bilden ein monocyclisches, 5 bis 7 gliederiges gesättigtes, teilweise ungesättigtes oder aromatisches Ringsystem, dessen einzelne Glieder durch -CHR45-, -CR46R47-, =(C-R46)-, O, N oder S substituiert sein können; mit der Maßgabe, dass zwei Einheiten aus der Reihe -O-, N oder -S- nicht benachbart sein dürfen;
R11, R12, R13, R14, R15, R16, R17, R18, R19, R20, R21, R22, R23, R24, R25, R26, R27, R28, R29, R30, R31, R32, R33, R34, R35, R36, R37, R38, R39, R40, R41, R42, R43, R44

unabhängig voneinander Wasserstoff, $(C_1-C_6)$-Alkyl;

bedeuten;
oder
R11 und R12, R13 und R14, R15 und R16, R25 und 26, R27 und R28, R29 und R30, R31 und R32,

R41 und 42, R43 und 44 bilden unabhängig voneinander optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-($C_1$-$C_6$)-Alkyl, Sauerstoff und Schwefel beinhalten kann;

R45, R46, R47 gleich oder verschieden, F, Cl, Br, I, OH, $CF_3$, $NO_2$, CN, $OCHF_2$, $OCF_3$, $SF_5$, O-($C_1$-$C_6$)-Alkyl, O-($C_1$-$C_4$)-Alkoxy-($C_1$-$C_4$)-Alkyl, S-($C_1$-$C_6$)-Alkyl, ($C_1$-$C_6$)-Alkyl, ($C_2$-$C_4$)-Haloalkyl, O-($C_2$-$C_4$)-Haloalkyl, ($C_2$-$C_6$)-Alkenyl, ($C_3$-$C_8$)-Cycloalkyl, O-($C_3$-$C_8$)-Cycloalkyl, ($C_2$-$C_6$)-Alkinyl, N(R134)(R135), $SO_2$-$CH_3$, COOH, COO-($C_1$-$C_6$)-Alkyl, CON(R136)(R137), N(R138)CO(R139), N(R140)$SO_2$(R141), CO(R142), $(CR143R144)_{x''}$-O(R145), $(CR143R144)_{x''}$-CO-O(R145), O-$(CR143R144)_{x''}$-CO-O(R145), $(CR143R144)_{x''}$-N(R146)(R147), O-$(CR143R144)_{x''}$-N(R146)(R147), $(CR143R144)_{x''}$-CON(R146)(R147), O-$(CR143R144)_{x''}$-CON(R146)(R147), O-CO-N(R146)(R147), O-CO-($C_1$-$C_6$)-Alkylen-CO-O-($C_1$-$C_6$)-Alkyl, O-CO-($C_1$-$C_6$)-Alkylen-CO-OH, O-CO-($C_1$-$C_6$)-Alkylen-CO-N(R148)(R149),

x'' unabhängig voneinander 1, 2, 3, 4, 5, 6;

R134, R135, R136, R137, R138, R139, R140, R141, R142, R143, R144, R145, R146, R147, R148, R149

gleich oder verschieden Wasserstoff, ($C_1$-$C_6$)-Alkyl,

ein Rest der Formel Ib

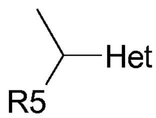

Ib,

worin

R5 Wasserstoff, ($C_1$-$C_3$)-Alkyl;

Het ein 4 bis 10 gliedriger mono oder bicyclischer aromatischer Ring welcher 1 bis 4 Heteroatome, ausgewählt aus der Gruppe Sauerstoff, Stickstoff oder Schwefel enthält, wobei das Ringsystem zusätzlich ein oder mehrfach durch unabhängig voneinander F, Cl, Br, I, OH, $CF_3$, $NO_2$, CN, $OCF_3$, $OCHF_2$, O-($C_1$-$C_6$)-Alkyl, O-($C_1$-$C_4$)-Alkoxy-($C_1$-$C_4$)-alkyl, S-($C_1$-$C_6$)-Alkyl, ($C_1$-$C_6$)-Alkyl, ($C_2$-$C_4$)-Haloalkyl, O-($C_2$-$C_4$)-Haloalkyl, ($C_2$-$C_6$)-Alkenyl, ($C_2$-$C_6$)-Alkinyl, N(R48)(R49), $SO_2$-$CH_3$, $SO_2$-N(R50)(R51), $SF_5$, $SCF_3$, COOH, COO-($C_1$-$C_6$)-Alkyl, CON(R52)(R53), N(R54)CO(R55), N(R56)$SO_2$(R57), CO(R58), $(CR59R60)_{x'''}$-O(R61), $(CR59R60)_{x'''}$-CO-O(R61), O-$(CR59R60)_{x'''}$-CO-O(R61), $(CR59R60)_{x'''}$-N(R62)(R63), O-$(CR59R60)_{x'''}$-N(R62)(R63), $(CR59R60)_{x'''}$-CON(R62)(R63), O-$(CR59R60)_{x'''}$-CON(R62)(R63), O-CO-N(R62)(R63), O-CO-($C_1$-$C_6$)-Alkylen-CO-O-($C_1$-$C_6$)-Alkyl, O-CO-($C_1$-$C_6$)-Alkylen-CO-OH, O-CO-($C_1$-$C_6$)-Alkylen-CO-N(R64)(R65); oder $(O)_y$-$(CH_2-)_{y'}$-$(O)_{y''}$-$(CH_2)_{y'''}$-R101 substituiert sein kann,

x''' unabhängig voneinander 1, 2, 3, 4, 5, 6;

y, y'' unabhängig voneinander 0,1;

y', y''' unabhängig voneinander 0, 1, 2, 3, 4, 5, 6;

R101 ein 4 bis 10 gliedriger mono oder bicyclischer gesättigter, teilweise ungesättigter oder aromatischer Ring welcher 1 bis 4 Heteroatome, ausgewählt aus der Gruppe Sauerstoff, Stickstoff oder Schwefel enthalten kann, wobei das Ringsystem zusätzlich ein oder mehrfach durch, F, Cl, Br, I, OH, $CF_3$, $NO_2$, CN, $OCHF_2$, $OCF_3$, O-($C_1$-$C_6$)-Alkyl, O-($C_1$-$C_4$)-Alkoxy-($C_1$-$C_4$)-alkyl, S-($C_1$-$C_6$)-Alkyl, ($C_1$-$C_6$)-Alkyl, ($C_2$-$C_4$)-Haloalkyl, ($C_2$-$C_6$)-Alkenyl, ($C_3$-$C_8$)-Cycloalkyl, O-($C_3$-$C_8$)-Cycloalkyl, ($C_2$-$C_6$)-Alkinyl, N(R66)(R67), $SO_2$-$CH_3$, $SF_5$, COOH, COO-($C_1$-$C_6$)-Alkyl, CON(R68)(R69), (C), N(R70)CO(R71), N(R72)$SO_2$(R73), CO(R74), $(CR75R76)_{x''''}$-O$(R77R75R76)_{x''''}$-CO-O(R77), O-$(CR75R76)_{x''''}$-CO-O(R77), $(CR75R76)_{x''''}$-N(R78)(R79), O-$(CR75R76)_{x''''}$-N(R78)(R79), $(CR75R76)_{x''''}$-CON(R78)(R79), O-$(CR75R76)_{x''''}$-CON(R78)(R79), O-CO-N(R78)(R79), O-CO-($C_1$-$C_6$)-Alkylen-CO-O-($C_1$-$C_6$)-Alkyl, O-CO-($C_1$-$C_6$)-Alkylen-CO-OH, O-CO-($C_1$-$C_6$)-Alkylen-CO-N(R80)(R81) substituiert sein kann;

x'''' unabhängig voneinander 1, 2, 3, 4, 5, 6;

R48, R49, R50, R51, R52, R53, R54, R55, R56, R57, R58, R59, R60, R61, R62, R63, R64, R65, R66, R67, R68, R69, R70, R71, R72, R73, R74, R75, R76, R77, R78, R79, R80, R81

unabhängig voneinander Wasserstoff, ($C_1$-$C_6$)-Alkyl;

oder

R48 und R49, R50 und R51, R52 und R53, R62 und R63, R64 und R65, R66 und R67, R68 und R69, R78 und R79, R80 und R81 bilden unabhängig voneinander optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-($C_1$-$C_6$)-Alkyl, Sauerstoff und Schwefel beinhalten kann;

ein Rest der Formel Ic

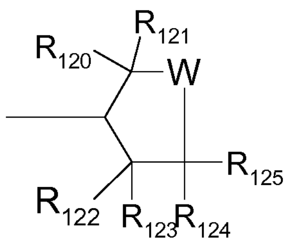

Ic,

worin

W, -C(R126)(R127)-, -C(R126)(R127)-C(R128)(R129)-, -C(R126)(R127)-O-;
R120, R121, R123, R125, R126, R127, R128, R129

gleich oder verschieden Wasserstoff, F, Cl, Br, I, OH, $CF_3$, $NO_2$, CN, $OCHF_2$, $OCF_3$, $SF_5$, O-($C_1$-$C_6$)-Alkyl, O-($C_1$-$C_4$)-Alkoxy-($C_1$-$C_4$)-alkyl, S-($C_1$-$C_6$)-Alkyl, ($C_1$-$C_6$)-Alkyl, ($C_2$-$C_4$)-Haloalkyl, O-($C_2$-$C_4$)-Haloalkyl, ($C_2$-$C_6$)-Alkenyl, ($C_3$-$C_8$)-Cycloalkenyl, ($C_2$-$C_6$)-Alkinyl, N(R90)(R91), $SO_2$-$CH_3$, COOH, COO-($C_1$-$C_6$)-Alkyl, CON(R92)(R93), N(R94)CO(R95), N(R96)$SO_2$(R97), CO(R98), $(CR99R102)_z$-O(R103), $(CR99R76)_z$-CO-O(R103), O-$(CR99R102)_z$-CO-O(R103), $(CR99R102)_z$-N(R104)(R105), O-$(CR99R102)_z$-N(R104)(R105), $(CR99R102)_z$-CON(R104)(R105), O-$(CR99R102)_z$-CON(R104)(R105), O-CO-N(R104)(R105), O-CO-($C_1$-$C_6$)-Alkylen-CO-O-($C_1$-$C_6$)-Alkyl, O-CO-($C_1$-$C_6$)-Alkylen-CO-OH, O-CO-($C_1$-$C_6$)-Alkylen-CO-N(R106)(R107);

z unabhängig voneinander 1, 2, 3, 4, 5, 6;
R90, R91, R92, R93, R94, R95, R96, R97, R98, R99, R102, R103, R104, R105, R106, R107

gleich oder verschieden Wasserstoff, ($C_1$-$C_6$)-Alkyl;

R122 und R124, zusammen mit dem sie tragenden Kohlenstoffatomen bilden ein monocyclisches, 5 oder 6 gliederiges gesättigtes, teilweise ungesättigtes oder ein aromatisches Ringsystem, dessen einzelne Glieder durch -CHR130-, - CR131 R132-, =(C-R133)- substituiert sein können;
R130, R131, R132, R133 gleich oder verschieden, F, Cl, Br, I, OH, $CF_3$, $NO_2$, CN, $OCHF_2$, $OCF_3$, $SF_5$, O-($C_1$-$C_6$)-Alkyl, O-($C_1$-$C_4$)-Alkoxy-($C_1$-$C_4$)-Alkyl, S-($C_1$-$C_6$)-Alkyl, ($C_1$-$C_6$)-Alkyl, ($C_2$-$C_4$)-Haloalkyl, O-($C_2$-$C_4$)-Haloalkyl, ($C_2$-$C_6$)-Alkenyl, ($C_2$-$C_6$)-Alkinyl, N(R160)(R161), $SO_2$-$CH_3$, COOH, COO-($C_1$-$C_6$)-Alkyl, CON(R162)(R163), N(R164)CO(R165), N(R166)$SO_2$(R167), CO(R168), $(CR169R170)_{z'}$-O(R171), $(CR169R170)_{z'}$-CO-O(R77), O-$(CR169R170)_{z'}$-CO-O(R171), $(CR169R170)_{z'}$-N(R172)(R173), O-$(CR169R170)_{z'}$-N(R172)(R173), $(CR169R170)_{z'}$-CON(R172)(R173), O-$(CR169R170)_{z'}$-CON(R172)(R173), O-CO-N(R172)(R173), O-CO-($C_1$-$C_6$)-Alkylen-CO-O-($C_1$-$C_6$)-Alkyl, O-CO-($C_1$-$C_6$)-Alkylen-CO-OH, O-CO-($C_1$-$C_6$)-Alkylen-CO-N(R172)(R173);
z' unabhängig voneinander 1, 2, 3, 4, 5, 6;
R160, R161, R162, R163, R164, R165, R166, R167, R168, R169, R170, R171, R172, R173

gleich oder verschieden Wasserstoff, ($C_1$-$C_6$)-Alkyl;

oder
R160 und R161, R162 und R163, R172 und R173 bilden unabhängig voneinander optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-($C_1$-$C_6$)-Alkyl, Sauerstoff und Schwefel beinhalten kann;
R2, R3, R4 gleich oder verschieden Wasserstoff, F, Cl, Br, I, OH, $CF_3$, $NO_2$, CN, $OCHF_2$, $OCF_3$, $SF_5$,

O-($C_1$-$C_6$)-Alkyl, O-($C_1$-$C_4$)-Alkoxy-($C_1$-$C_4$)-Alkyl, S-($C_1$-$C_6$)-Alkyl, ($C_1$-$C_6$)-Alkyl, ($C_2$-$C_4$)-Haloalkyl, O-($C_2$-$C_4$)-Haloalkyl, ($C_2$-$C_6$)-Alkenyl, ($C_3$-$C_8$)-Cycloalkyl, O-($C_3$-$C_8$)-Cycloalkyl, ($C_3$-$C_8$)-Cycloalkenyl, Aryl, ($C_2$-$C_6$)-Alkinyl, N(R200)(R201), $SO_2$-$CH_3$, COOH, COO-($C_1$-$C_6$)-Alkyl, CON(R202)(R203), N(R204)CO(R205), N(R206)$SO_2$(R207), CO(R208), $(CR209R210)_{z''}$-O(R211), $(CR209R210)_{z''}$-CO-O(R211), O-$(CR209R210)_{z''}$-CO-O(R211), $(CR209R210)_{z''}$-N(R212)(R213), O-$(CR209R210)_{z''}$-N(R212)(R213), $(CR209R210)_{z''}$-CON(R212)(R213), O-$(CR209R210)_{z''}$-CON(R212)(R213), O-CO-N(R212)(R213), O-CO-($C_1$-$C_6$)-Alkylen-CO-O-($C_1$-$C_6$)-Alkyl, O-CO-($C_1$-$C_6$)-Alkylen-CO-OH, O-CO-($C_1$-$C_6$)-Alkylen-CO-N(R212)(R213);
z" unabhängig voneinander 1, 2, 3, 4, 5, 6;
R200, R201, R202, R203, R204R205, R206, R207, R208, R209, R210, R211, R212, R213

gleich oder verschieden Wasserstoff, ($C_1$-$C_6$)-Alkyl;

oder
R200 und R201, R202 und R203, R212 und R213

bilden unabhängig voneinander optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-($C_1$-$C_6$)-Alkyl, Sauerstoff und Schwefel beinhalten kann;

die tautomeren Formen der Verbindung sowie deren physiologisch verträgliche Salze und N-Oxide; mit der Maßgabe, dass die Verbindungen mit R2, R3, R4 = Wasserstoff, X = S und R1 = Heptyl, Nonyl oder Cyclohexyl ausgenommen sind.

**2.** Eine Verbindung der Formel I gemäß Anspruch 1, worin

R1 ein Rest der Formel Ia

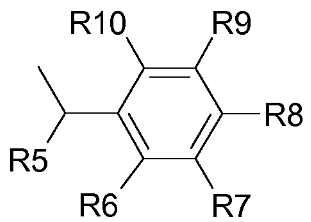

,

worin bedeuten

R5 Wasserstoff, $CH_3$;
R6, R7, R8, R9, R10
unabhängig voneinander Wasserstoff, $OCF_3$, $SCF_3$, $OCHF_2$, O-($C_1$-$C_6$)-Alkyl, O-($C_1$-$C_4$)-Alkoxy-($C_1$-$C_4$)-alkyl, S-($C_1$-$C_6$)-Alkyl, $SO_2$-$CH_3$, $SO_2$-N(R13)(R14), COOH, COO-($C_1$-$C_6$)-Alkyl, $(CR22R23)_x$-O(R24), $(CR22R23)_x$-CO-O(R24), O-$(CR22R23)_x$-CO-O(R24), $(CR22R23)_x$-N(R25)(R26), O-$(CR22R23)_x$-N(R25)(R26), $(CR22R23)_x$-CON(R25)(R26);
mit der Massgabe dass mindestens ein Rest R6, R7, R8, R9, R10 von Wasserstoff verschieden ist;
x unabhängig voneinander 0, 1, 2, 3, 4;
oder
R7 oder R8 ist
R100, -$CH_2$-R100, -$OCH_2$-R100, -$CH_2$-O-R100, -$CH_2$-O-$CH_2$-R100 oder - O-$CH_2CH_2$-R100;
R100 ein 4 bis 7 gliedriger monocyclischer gesättigter, teilweise ungesättigter oder aromatischer Ring welcher 1 bis 3 Heteroatome, ausgewählt aus der Gruppe Sauerstoff, Stickstoff oder Schwefel enthalten kann, wobei das Ringsystem zusätzlich ein oder mehrfach durch F, Cl, Br, OH, $CF_3$, $OCF_3$, O-($C_1$-$C_6$)-Alkyl, O-($C_1$-$C_4$)-Alkoxy-($C_1$-$C_4$)-alkyl, S-($C_1$-$C_6$)-Alkyl, ($C_1$-$C_6$)-Alkyl, $SO_2$-$CH_3$, COOH, COO-($C_1$-$C_6$)-Alkyl, N(R35)$SO_2$(R36), CO(R37), $(CR38R39)_{x'}$-O(R40), $(CR38R39)_{x'}$-CO-O(R40), O-$(CR22R23)_{x'}$-CO-O(R40), $(CR22R23)_{x'}$-N(R41)(R42), O-$(CR38R39)_{x'}$-N(R41)(R42), $(CR38R39)_{x'}$-CON(R41)(R42), O-$(CR38R39)_{x'}$-CON(R41)(R42) substituiert sein kann;
x' unabhängig voneinander 1, 2, 3, 4;

oder
R7 und R8 oder R8 und R9 oder R9 und R10
zusammen mit dem sie tragenden Kohlenstoffatomen bilden $-O-CH_2-O-$, $-O-CH_2-CH_2-O-$, $-O-CH_2-CH_2-CH_2-O-$, $-O-CF_2-O-$ oder - $N(CH_3)-N=N-$;
R11, R12, R13, R14, R15, R16, R17, R18, R19, R20, R21, R22, R23, R24, R25, R26, R27, R28, R29, R30, R31, R32, R33, R34, R35, R36, R37, R38, R39, R40, R41, R42

unabhängig voneinander Wasserstoff, $(C_1-C_6)$-Alkyl;

bedeuten;
oder
R11 und R12, R13 und R14, R15 und R16, R25 und 26, R27 und R28, R29 und R30, R31 und R32, R41 und 42, R43 und 44 bilden unabhängig voneinander optional zusammen mit dem Stickstoffatom, an das sie gebunden sind, einen 5-6 gliedrigen Ring, welcher außer dem Stickstoffatom noch 0-1 weitere Heteroatome aus der Gruppe NH, $N-(C_1-C_6)$-Alkyl, Sauerstoff und Schwefel beinhalten kann;
bedeutet.

**3.** Eine Verbindung der Formel I gemäß Anspruch 1, worin

R1 ein Rest der Formel Ib

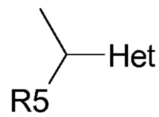

Ib,

worin bedeuten:

R5 Wasserstoff, $CH_3$;
Het ausgewählt ist aus der Gruppe Pyridin, Pyrazol, Imidazol, Oxazol, Oxadiazol, Benzothiophen, Imidazo[2,1,b]thiazol, wobei Het zusätzlich ein oder mehrfach durch unabhängig voneinander F, Cl, Br, $CF_3$, $OCF_3$, $OCHF_2$, $O-(C_1-C_6)$-Alkyl, $O-(C_1-C_4)$-Alkoxy-$(C_1-C_4)$-alkyl, $S-(C_1-C_6)$-Alkyl, $(C_1-C_6)$-Alkyl, $(C_2-C_6)$-Alkenyl, $SO_2-CH_3$, $SO_2$-N(R50)(R51), COOH, COO-$(C_1-C_6)$-Alkyl, N(R56)$SO_2$(R57), CO(R58), $(CR59R60)_{x'''}$-O(R61), $(CR59R60)_{x'''}$-CO-O(R61), O-$(CR59R60)_{x'''}$-CO-O(R61), $(CR59R60)_{x'''}$-N(R62)(R63), O-$(CR59R60)_{x'''}$-N(R62)(R63), $(CR59R60)_{x'''}$-CON(R62)(R63), O-$(CR59R60)_{x'''}$-CON(R62)(R63), O-CO-N(R62)(R63), O-CO-$(C_1-C_6)$-Alkylen-CO-O-$(C_1-C_6)$-Alkyl, O-CO-$(C_1-C_6)$-Alkylen-CO-OH, O-CO-$(C_1-C_6)$-Alkylen-CO-N(R64)(R65);
oder $(O)_y-(CH_2-)_{y'}-(O)_{y''}-(CH_2)_{y'''}$-R101 substituiert sein kann,
x''' unabhängig voneinander 1, 2, 3, 4;
y,y" unabhängig voneinander 0,1;
y', y''' unabhängig voneinander 0, 1, 2;
R101 ein 4 bis 10 gliedriger mono oder bicyclischer gesättigter, teilweise ungesättigter oder aromatischer Ring welcher 1 bis 4 Heteroatome, ausgewählt aus der Gruppe Sauerstoff, Stickstoff oder Schwefel enthalten kann, wobei das Ringsystem zusätzlich ein oder mehrfach durch, F, Cl, Br, I, OH, $CF_3$, $NO_2$, CN, $OCHF_2$, $OCF_3$, $O-(C_1-C_6)$-Alkyl, $O-(C_1-C_4)$-Alkoxy-$(C_1-C_4)$-alkyl, $S-(C_1-C_6)$-Alkyl, $(C_1-C_6)$-Alkyl, $(C_2-C_4)$-Haloalkyl, $(C_2-C_6)$-Alkenyl, $(C_3-C_8)$-Cycloalkyl, $O-(C_3-C_8)$-Cycloalkyl, $(C_2-C_6)$-Alkinyl, N(R66)(R67), $SO_2-CH_3$, $SF_5$, COOH, COO-$(C_1-C_6)$-Alkyl, CON(R68)(R69), (C), N(R70)CO(R71), N(R72)$SO_2$(R73), CO(R74), $(CR75R76)_{x''''}$-O$(R77R75R76)_{x''''}$-CO-O(R77), O-$(CR75R76)_{x''''}$-CO-O(R77), $(CR75R76)_{x''''}$-N(R78)(R79), O-$(CR75R76)_{x''''}$-N(R78)(R79), $(CR75R76)_{x''''}$-CON(R78)(R79), O-$(CR75R76)_{x''''}$-CON(R78)(R79), O-CO-N(R78)(R79), O-CO-$(C_1-C_6)$-Alkylen-CO-O-$(C_1-C_6)$-Alkyl, O-CO-$(C_1-C_6)$-Alkylen-CO-OH, O-CO-$(C_1-C_6)$-Alkylen-CO-N(R80)(R81) substituiert sein kann;
x'''' unabhängig voneinander 1, 2, 3, 4, 5, 6;
R48, R49, R50, R51, R52, R53, R54, R55, R56, R57, R58, R59, R60, R61, R62, R63, R64, R65, R66, R67, R68, R69, R70, R71, R72, R73, R74, R75, R76, R77, R78, R79, R80, R81

unabhängig voneinander Wasserstoff, $(C_1-C_6)$-Alkyl;

oder

R48 und R49, R50 und R51, R52 und R53, R62 und R63, R64 und R65, R66 und R67, R68 und R69, R78 und R79, R80 und R81 bilden unabhängig voneinander optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-($C_1$-$C_6$)-Alkyl, Sauerstoff und Schwefel beinhalten kann;

bedeutet.

**4.** Eine Verbindung der Formel I gemäß Anspruch 1, worin

R1 ein Rest der Formel Ic:

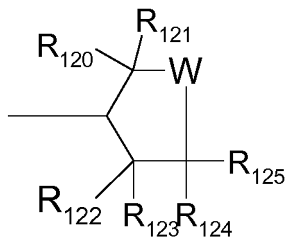

Ic,

worin bedeuten

W -C(R126)(R127)-, -C(R126)(R127)-C(R128)(R129)-, -C(R126)(R127)-O-;

R120, R121, R123, R125, R126, R127, R128, R129

R120, R121, R126, R127, R128, R129 gleich oder verschieden Wasserstoff, ($C_1$-$C_6$)-Alkyl, oxo, COO-($C_1$-$C_6$)-Alkyl;

R122 und R124 zusammen mit dem sie tragenden Kohlenstoffatomen bilden ein monocyclisches, 6 gliederiges aromatisches Ringsystem, dessen einzelne Glieder durch =(C-R133)- substituiert sein können;

R133 gleich oder verschieden, F, Cl, Br, I, OH, $CF_3$, $NO_2$, CN, $OCHF_2$, $OCF_3$, $SF_5$, O-($C_1$-$C_6$)-Alkyl, O-($C_1$-$C_4$)-Alkoxy-($C_1$-$C_4$)-Alkyl, S-($C_1$-$C_6$)-Alkyl, ($C_1$-$C_6$)-Alkyl, ($C_2$-$C_4$)-Haloalkyl, O-($C_2$-$C_4$)-Haloalkyl, ($C_2$-$C_6$)-Alkenyl, ($C_2$-$C_6$)-Alkinyl, N(R160)(R161), $SO_2$-$CH_3$, COOH, COO-($C_1$-$C_6$)-Alkyl, CON(R162)(R163), N(R164)CO(R165), N(R166)$SO_2$(R167), CO(R168), $(CR169R170)_{z'}$-O(R171), $(CR169R170)_{z'}$-CO-O(R77), O-$(CR169R170)_{z'}$-CO-O(R171), $(CR169R170)_{z'}$-N(R172)(R173), O-$(CR169R170)_{z'}$-N(R172)(R173), $(CR169R170)_{z'}$-CON(R172)(R173), O-$(CR169R170)_{z'}$-CON(R172)(R173), O-CO-N(R172)(R173), O-CO-($C_1$-$C_6$)-Alkylen-CO-O-($C_1$-$C_6$)-Alkyl, O-CO-($C_1$-$C_6$)-Alkylen-CO-OH, O-CO-($C_1$-$C_6$)-Alkylen-CO-N(R172)(R173);

z' unabhängig voneinander 1, 2, 3, 4, 5, 6;

R160, R161, R162, R163, R164, R165, R166, R167, R168, R169, R170, R171, R172, R173

gleich oder verschieden Wasserstoff, ($C_1$-$C_6$)-Alkyl;

oder

R160 und R161, R162 und R163, R172 und R173 bilden unabhängig voneinander optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-($C_1$-$C_6$)-Alkyl, Sauerstoff und Schwefel beinhalten kann;

bedeutet.

**5.** Eine Verbindung der Formel I gemäß Ansprüchen 1 bis 4, worin

R2 Wasserstoff, -($C_1$-$C_6$)-Alkyl, ($C_3$-$C_8$)-Cycloalkyl oder Phenyl;

R3 Wasserstoff;

R4 Wasserstoff oder -($C_1$-$C_6$)-Alkyl;

bedeuten.

**6.** Arzneimittel enthaltend eine oder mehrere Verbindungen der Formel I gemäß den Ansprüchen 1 bis 5.

**7.** Arzneimittel, gemäß Anspruch 6, **dadurch gekennzeichnet, dass** es als weiteren Wirkstoff Metformin, Arcabose, Glibenclamid, Glimepirid, Gliclazid,

Gliquidon, Pioglitazon, Rosiglitazon, Exenatid, Miglitol, Vildagliptin, Sitagliptin, Repaglinid, Nateglinid oder Mitiglinid enthält.

**8.** Arzneimittel, gemäß Anspruch 6, **dadurch gekennzeichnet, dass** es als weiteren Wirkstoff eine oder mehrere Antidiabetika, hypoglykämischen Wirkstoffe, HMGCoA-Reduktase Inhibitoren, Cholesterinresorptionsinhibitoren, PPAR gamma Agonisten, PPAR alpha Agonisten, PPAR alpha/gamma Agonisten, Fibrate, MTP-Inhibitoren, Gallensäureresorptionsinhibitoren, CETP-Inhibitoren, polymere Gallensäureadsorber, LDL-Rezeptorinducer, ACAT-Inhibitoren, Antioxidantien, Lipoprotein-Lipase Inhibitoren, ATP-Citrat-Lyase Inhibitoren, Squalen synthetase inhibitoren, Lipoprotein(a) antagonisten, Lipase Inhibitoren, Insuline, Sulphonylharnstoffe, Biguanide, Meglitinide, Thiazolidindione, $\alpha$-Glukosidase-Inhibitoren, auf den ATPabhängigen Kaliumkanal der Betazellen wirkende Wirkstoffe, CART-Agonisten, NPY-Agonisten, MC4-Agonisten, Orexin-Antgonisten, H3-Agonisten, TNF-Agonisten, CRF-Antagonisten, CRF BP-Antagonisten, Urocortin-Agonisten, $\beta$3-Agonisten, MSH (Melanocyt-stimulierendes Hormon)-Agonisten, CCK-Agonisten, Serotonin-Wiederaufnahme-Inhibitoren, gemischte Serotonin- und noradrenerge Verbindungen, 5HT-Agonisten, Bombesin-Agonisten, Galanin-Antagonisten, Wachstumshormone, Wachstumshormon freisetzende Verbindungen, TRH-Agonisten, entkoppelnde Protein 2- oder 3-Modulatoren, Leptinagonisten, DA-Agonisten (Bromocriptin, Doprexin), Lipase/Amylase-Inhibitoren, PPAR-Modulatoren, RXR-Modulatoren oder TR-$\beta$-Agonisten oder Amphetamine enthält.

**9.** Verbindung der Formel I gemäß den Ansprüchen 1 bis 5 zur Behandlung und/oder Prävention von Störungen des Fettsäurestoffwechsels und Glucoseverwertungsstörungen.

**10.** Verbindung der Formel I gemäß den Ansprüchen 1 bis 5 zur Behandlung und/oder Prävention von Dyslipidämien und deren Folgen.

**11.** Verbindung der Formel I gemäß den Ansprüchen 1 bis 5 zur Behandlung und/oder Prävention von Zuständen, die mit dem Metabolischen Syndrom assoziert sind, sowie bei Störungen, bei denen Insulin Resistenz eine Rolle spielt, oder zur Behandlung und/oder Prävention von Diabetes mellitus und der damit verbundenen Folgeerkrankungen.

**12.** Verbindung der Formel I gemäß den Ansprüchen 1 bis 5 zur Behandlung und/oder Prävention von Zuständen, die mit erniedrigtem HDL-Spiegel verbunden sind.

**13.** Verbindung der Formel I gemäß den Ansprüchen 1 bis 5 zur Behandlung und/oder Prävention von atherosklerotischen Erkrankungen.

**14.** Verbindung der Formel I gemäß den Ansprüchen 1 bis 5 in Kombination mit mindestens einem weiteren Wirkstoff zur Behandlung und/oder Prävention von Störungen, bei denen Insulin Resistenz eine Rolle spielt.

**15.** Verfahren zur Herstellung eines Arzneimittels enthaltend eine oder mehrere der Verbindungen der Formel I gemäß den Ansprüchen 1 bis 5, **dadurch gekennzeichnet, dass** diese mit einem pharmazeutisch geeigneten Träger vermischt wird und diese Mischung in eine für die Verabreichung geeignete Form gebracht wird.

**16.** Verfahren zur Herstellung von Verbindungen der allgemeinen Formel I gemäß den Ansprüchen 1 bis 5 **dadurch gekennzeichnet, dass**
substituierte, bzw. unsubstituierte Azolopyridin-3-ol-derivate II mit Isocyanaten III zu Verbindungen der Formel I umgesetzt werden;

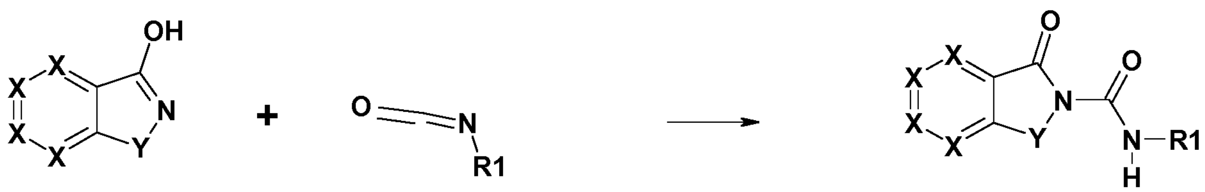

II III IV

oder
Azolopyridin-3-ol-derivate II werden

a) mit Carbamoylchloriden der Formel VI acyliert;
oder
b) in zwei Stufen zunächst mit Phosgen oder Äquivalenten wie Chlorcarbonsäuretrichlormethylester, Carbonsäureditrichlormethylester oder Chlorameisensäure-4-nitrophenylester und einem zweiten Schritt mit Aminen der Formel VII umgesetzt,

worin die Substituenten die oben genannten Bedeutungen haben.

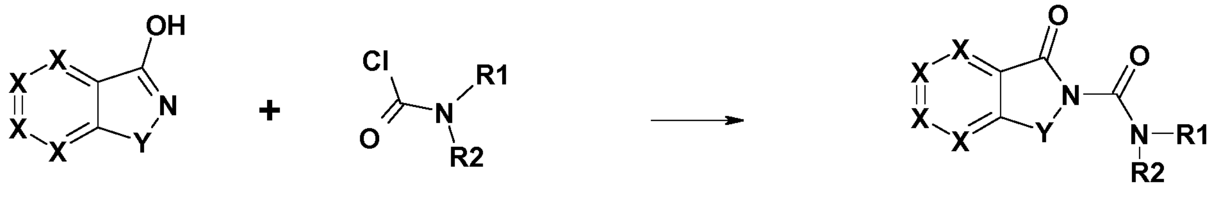

II VI I

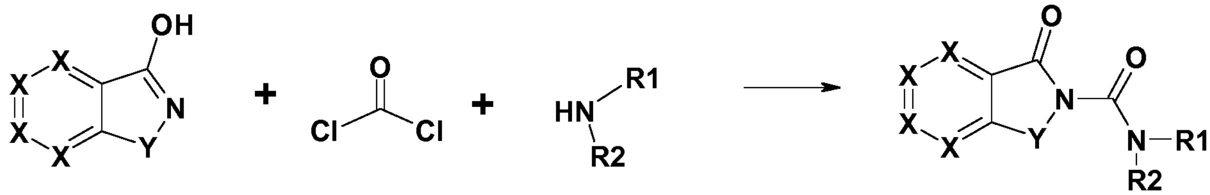

II VII I

**Claims**

**1.** A compound of the formula I

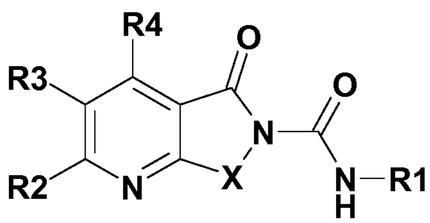

(I)

where:

X is S or $SO_2$;
R1

is a radical of the formula Ia

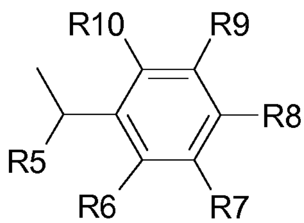

in which
R5 is hydrogen, $(C_1-C_3)$-alkyl;
R6, R7, R8, R9, R10

are each independently hydrogen, OH, CN, $OCF_3$, $OCHF_2$, O-($C_1$-$C_6$)-alkyl, O-($C_1$-$C_4$)-alkoxy-($C_1$-$C_4$)-alkyl, S-($C_1$-$C_6$)-alkyl, ($C_2$-$C_4$)-haloalkyl, O-($C_2$-$C_4$)-haloalkyl, ($C_2$-$C_6$)-alkenyl, ($C_2$-$C_6$)-alkynyl, N(R11)(R12), $SO_2$-$CH_3$, $SO_2$-N(R13)(R14), $SF_5$, $SCF_3$, COOH, COO-($C_1$-$C_6$)-alkyl, CON(R15)(R16), N(R17)CO(R18), N(R19)$SO_2$(R20), CO(R21), $(CR22R23)_x$-0(R24), $(CR22R23)_x$-CO-O(R24), O-$(CR22R23)_x$-COO(R24), $(CR22R23)_x$-N(R25)(R26), O-$(CR22R23)_x$-N(R25)(R26), $(CR22R23)_x$-CON(R25)(R26), 0-$(CR22R23)_x$-CON(R25)(R26), O-CO-N(R25)(R26), O-CO-($C_1$-$C_6$)-alkylene-CO-O-($C_1$-$C_6$)-alkyl, O-CO-($C_1$-$C_6$)-alkylene-CO-OH, O-CO-($C_1$-$C_6$)-alkylene-CON(R27)(R28);
with the proviso that at least one R6, R7, R8, R9, R10 radical is not hydrogen;
x is independently 0, 1, 2, 3, 4, 5, 6;
or
R7 or R8 is
$(O)_{y}$-$(CH_2\text{-})_{y'}$-$(O)_{y''}$-$(CH_2)_{y'''}$-R100;
y, y" are each independently 0,1;
y', y''' are each independently 1, 2, 3, 4, 5, 6;
R100 is a 4- to 10-membered mono- or bicyclic saturated, partly unsaturated or aromatic ring which may contain 1 to 4 heteroatoms selected from the group of oxygen, nitrogen and sulfur, where the ring system may additionally be mono- or polysubstituted by F, Cl, Br, I, OH, $CF_3$, $NO_2$, CN, $OCF_3$, O-($C_1$-$C_6$)-alkyl, O-($C_1$-$C_4$)-alkoxy-($C_1$-$C_4$)-alkyl, S-($C_1$-$C_6$)-alkyl, ($C_1$-$C_6$)-alkyl, ($C_2$-$C_4$)-haloalkyl, ($C_2$-$C_6$)-alkenyl, ($C_3$-$C_8$)-cycloalkyl, 0-($C_3$-$C_8$)-cycloalkyl, ($C_2$-$C_6$)-alkynyl, N(R29)(R30), $SO_2$-$CH_3$, $SF_5$, COOH, COO-($C_1$-$C_6$)-alkyl, CON(R31)(R32), N(R33)CO(R34), N(R35)$SO_2$(R36), CO(R37), $(CR38R39)_{x'}$-O(R40), $(CR38R39)_{x'}$-CO-O(R40), O-$(CR22R23)_{x'}$-CO-O(R40), $(CR22R23)_{x'}$-N(R41)(R42), 0-$(CR38R39)_{x'}$-N(R41)(R42), $(CR38R39)_{x'}$-CON(R41) (R42), O-$(CR38R39)_{x'}$-CON(R41) (R42), O-CO-N(R41) (R42), 0-CO-($C_1$-$C_6$)-alkylene-CO-O-($C_1$-$C_6$)-alkyl, O-CO-($C_1$-$C_6$)-alkylene-CO-OH, O-CO-($C_1$-$C_6$)-alkylene-CO-N(R43)(R44);
x' is 0, 1, 2, 3, 4, 5, 6;
or
R7 and R8 or R8 and R9 or R9 and R10 together with the carbon atom which bears them form a monocyclic, 5- to 7-membered saturated, partly unsaturated or aromatic ring system whose individual members may be substituted by -CHR45-, -CR46R47-, =(C-R46)- , 0, N or S; with the proviso that no two units from the group of -O-, N and -S- may be adjacent;
R11, R12, R13, R14, R15, R16, R17, R18, R19, R20, R21, R22, R23, R24, R25, R26, R27, R28, R29, R30, R31, R32, R33, R34, R35, R36, R37, R38, R39, R40, R41, R42, R43, R44

are each independently hydrogen, ($C_1$-$C_6$)-alkyl;

or
R11 and R12, R13 and R14, R15 and R16, R25 and 26, R27 and R28, R29 and R30, R31 and R32, R41 and 42, R43 and 44
each independently form, optionally together with the nitrogen atom to which they are bonded, a 5-6-membered ring which, apart from the nitrogen atom, may also include 0-1 further heteroatom from the group of NH, N-($C_1$-$C_6$)-alkyl, oxygen and sulfur;
R45, R46, R47 are the same or different and are each F, Cl, Br, I, OH, $CF_3$, $NO_2$, CN, $OCHF_2$, $OCF_3$, $SF_5$, O-($C_1$-$C_6$)-alkyl, O-($C_1$-$C_4$)-alkoxy-($C_1$-$C_4$)-alkyl, S-($C_1$-$C_6$)-alkyl, ($C_1$-$C_6$)-alkyl, ($C_2$-$C_4$)-haloalkyl, O-($C_2$-$C_4$)-haloalkyl, ($C_2$-$C_6$)-alkenyl, ($C_3$-$C_8$)-cycloalkyl, O-($C_3$-$C_8$)-cycloalkyl, ($C_2$-$C_6$)-alkynyl, N(R134)(R135), $SO_2$-$CH_3$, COOH, COO- ($C_1$-$C_6$)-alkyl, CON(R136)(R137), N(R138)CO(R139), N(R140)$SO_2$(R141), CO(R142), $(CR143R144)_{x''}$-O(R145), $(CR143R144)_{x''}$-CO-O(R145), O-$(CR143R144)_{x''}$-CO-O(R145), $(CR143R144)_{x''}$-N(R146)(R147), O-$(CR143R144)_{x''}$-N(R146)(R147), $(CR143R144)_{x''}$-CON(R146)(R147), O-$(CR143R144)_{x''}$-CON(R146)(R147), O-CO-N(R146)(R147), O-CO-($C_1$-$C_6$)-alkylene-CO-O-($C_1$-$C_6$)-alkyl, O-CO-($C_1$-$C_6$)-alkylene-CO-OH, O-CO-($C_1$-$C_6$)-alkylene-CO-N(R148)(R149);
x" is independently 1, 2, 3, 4, 5, 6;
R134, R135, R136, R137, R138, R139, R140, R141, R142, R143, R144, R145, R146, R147, R148, R149 are the same or different and are each hydrogen, ($C_1$-$C_6$)-alkyl;

a radical of the formula Ib

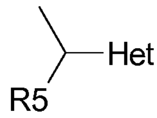

Ib

in which

R5 is hydrogen, $(C_1-C_3)$-alkyl;
Het is a 4- to 10-membered mono- or bicyclic aromatic ring containing 1 to 4 heteroatoms selected from the group of oxygen, nitrogen and sulfur, where the ring system may additionally be mono- or polysubstituted independently by F, Cl, Br, I, OH, $CF_3$, $NO_2$, CN, $OCF_3$, $OCHF_2$, O-$(C_1-C_6)$-alkyl, O-$(C_1-C_4)$-alkoxy-$(C_1-C_4)$-alkyl, S-$(C_1-C_6)$-alkyl, $(C_1-C_6)$-alkyl, $(C_2-C_4)$-haloalkyl, O-$(C_2-C_4)$-haloalkyl, $(C_2-C_6)$-alkenyl, $(C_2-C_6)$-alkynyl, N(R48)(R49), $SO_2$-$CH_3$, $SO_2$-N(R50)(R51), $SF_5$, $SCF_3$, COOH, COO-$(C_1-C_6)$-alkyl, CON(R52)(R53), N(R54)CO(R55), N(R56)$SO_2$(R57), CO(R58), $(CR59R60)_{x'''}$-O(R61), $(CR59R60)_{x'''}$-CO-O(R61), 0-$(CR59R60)_{x'''}$-CO-O(R61), $(CR59R60)_{x'''}$-N(R62)(R63), O-$(CR59R60)_{x'''}$-N(R62)(R63), $(CR59R60)_{x'''}$-CON(R62)(R63), O-$(CR59R60)_{x'''}$-CON(R62)(R63), O-CO-N(R62)(R63), O-CO-$(C_1-C_6)$-alkylene-CO-O-$(C_1-C_6)$-alkyl, O-CO-$(C_1-C_6)$-alkylene-CO-OH, O-CO-$(C_1-C_6)$-alkylene-CO-N(R64)(R65);
or $(O)_y$-$(CH_2\text{-})_{y'}$-$(O)_{y''}$-$(CH_2)_{y'''}$-R101,
x''' is independently 1, 2, 3, 4, 5, 6;
y, y" are each independently 0,1;
y', y''' are each independently 0, 1, 2, 3, 4, 5, 6;
R101 is a 4- to 10-membered mono- or bicyclic saturated, partly unsaturated or aromatic ring which may contain 1 to 4 heteroatoms selected from the group of oxygen, nitrogen and sulfur, where the ring system may additionally be mono- or polysubstituted by F, Cl, Br, I, OH, $CF_3$, $NO_2$, CN, $OCHF_2$, $OCF_3$, O-$(C_1-C_6)$-alkyl, O-$(C_1-C_4)$-alkoxy-$(C_1-C_4)$-alkyl, S-$(C_1-C_6)$-alkyl, $(C_1-C_6)$-alkyl, $(C_2-C_4)$-haloalkyl, $(C_2-C_6)$-alkenyl, $(C_3-C_8)$-cycloalkyl, 0-$(C_3-C_8)$-cycloalkyl, $(C_2-C_6)$-alkynyl, N(R66)(R67), $SO_2$-$CH_3$, $SF_5$, COOH, COO-$(C_1-C_6)$-alkyl, CON(R68)(R69), (C), N(R70)CO(R71), N(R72)$SO_2$(R73), CO(R74), $(CR75R76)_{x''''}$-O(R77R75R76)$_{x''''}$-CO-O(R77), O-$(CR75R76)_{x''''}$-CO-O(R77), $(CR75R76)_{x''''}$-N(R78)(R79), O-$(CR75R76)_{x''''}$-N(R78)(R79), $(CR75R76)_{x''''}$-CON(R78)(R79), O-$(CR75R76)_{x''''}$-CON(R78)(R79), O-CO-N(R78)(R79), O-CO-$(C_1-C_6)$-alkylene-CO-O-$(C_1-C_6)$-alkyl, O-CO-$(C_1-C_6)$-alkylene-CO-OH, O-CO-$(C_1-C_6)$-alkylene-CO-N(R80)(R81);
x'''' is independently 1, 2, 3, 4, 5, 6;
R48, R49, R50, R51, R52, R53, R54, R55, R56, R57, R58, R59, R60, R61, R62, R63, R64, R65, R66, R67, R68, R69, R70, R71, R72, R73, R74, R75, R76, R77, R78, R79, R80, R81

are each independently hydrogen, $(C_1-C_6)$-alkyl;

or
R48 and R49, R50 and R51, R52 and R53, R62 and R63, R64 and R65, R66 and R67, R68 and R69, R78 and R79, R80 and R81
each independently form, optionally together with the nitrogen atom to which they are bonded, a 5-6-membered ring which, apart from the nitrogen atom, may also include 0-1 further heteroatom from the group of NH, N-$(C_1-C_6)$-alkyl, oxygen and sulfur;

a radical of the formula Ic

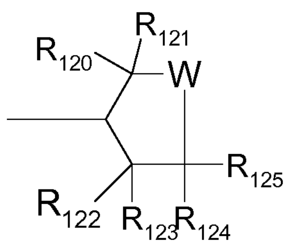

Ic

in which

W is -C(R126)(R127)-, -C(R126)(R127)-C(R128)(R129)-, -C(R126)(R127)-O-;

R120, R121, R123, R125, R126, R127, R128, R129

are the same or different and are each hydrogen, F, Cl, Br, I, OH, $CF_3$, $NO_2$, CN, $OCHF_2$, $OCF_3$, $SF_5$, O-($C_1$-$C_6$)-alkyl, O-($C_1$-$C_4$)-alkoxy-($C_1$-$C_4$)-alkyl, S-($C_1$-$C_6$)-alkyl, ($C_1$-$C_6$)-alkyl, ($C_2$-$C_4$)-haloalkyl, O-($C_2$-$C_4$)-haloalkyl, ($C_2$-$C_6$)-alkenyl, ($C_3$-$C_8$)-cycloalkenyl, ($C_2$-$C_6$)-alkynyl, N(R90)(R91), $SO_2$-$CH_3$, COOH, COO-($C_1$-$C_6$)-alkyl, CON(R92)(R93), N(R94)CO(R95), N(R96)$SO_2$(R97), CO(R98), $(CR99R102)_z$-O(R103), $(CR99R76)_z$-CO-O(R103), O-$(CR99R102)_z$-CO-O(R103), $(CR99R102)_z$-N(R104)(R105), O-$(CR99R102)_z$-N(R104)(R105), $(CR99R102)_z$-CON(R104)(R105), 0-$(CR99R102)_z$-CON(R104)(R105), O-CO-N(R104)(R105), O-CO-($C_1$-$C_6$)-alkylene-CO-O-($C_1$-$C_6$)-alkyl, O-CO-($C_1$-$C_6$)-alkylene-CO-OH, O-CO-($C_1$-$C_6$)-alkylene-CO-N(R106)(R107);
z is independently 1, 2, 3, 4, 5, 6;
R90, R91, R92, R93, R94, R95, R96, R97, R98, R99, R102, R103, R104, R105, R106, R107

are the same or different and are each hydrogen, ($C_1$-$C_6$)-alkyl;

R122 and R124, together with the carbon atom which bears them form a monocyclic, 5- or 6-membered saturated, partly unsaturated or aromatic ring system whose individual members may be substituted by -CHR130-, -CR131R132-, =(C-R133)-;
R130, R131, R132, R133 are the same or different and are each F, Cl, Br, I, OH, $CF_3$, $NO_2$, CN, $OCHF_2$, $OCF_3$, $SF_5$, 0- ($C_1$-$C_6$)-alkyl, 0-($C_1$-$C_4$)-alkoxy-($C_1$-$C_4$)-alkyl, S-($C_1$-$C_6$)-alkyl, ($C_1$-$C_6$)-alkyl, ($C_2$-$C_4$)-haloalkyl, O-($C_2$-$C_4$)-haloalkyl, ($C_2$-$C_6$)-alkenyl, ($C_2$-$C_6$)-alkynyl, N(R160)(R161), $SO_2$-$CH_3$, COOH, COO-($C_1$-$C_6$)-alkyl, CON(R162)(R163), N(R164)CO(R165), N(R166)$SO_2$(R167), CO(R168), $(CR169R170)_{z'}$-0(R171), $(CR169R170)_{z'}$-CO-O(R77), 0-$(CR169R170)_{z'}$-CO-O(R171), $(CR169R170)_{z'}$-N(R172)(R173), O-$(CR169R170)_{z'}$-N(R172)(R173), $(CR169R170)_{z'}$-CON(R172)(R173), O-$(CR169R170)_{z'}$-CON(R172)(R173), O-CO-N(R172)(R173), O-CO-($C_1$-$C_6$) -alkylene-CO-O-($C_1$-$C_6$)-alkyl, O-CO-($C_1$-$C_6$)-alkylene-CO-OH, O-CO-($C_1$-$C_6$)-alkylene-CO-N(R172)(R173);
z' is independently 1, 2, 3, 4, 5, 6;
R160, R161, R162, R163, R164, R165, R166, R167, R168, R169, R170, R171, R172, R173

are the same or different and are each hydrogen, ($C_1$-$C_6$)-alkyl;
or

R160 and R161, R162 and R163, R172 and R173 each independently form, optionally together with the nitrogen atom to which they are bonded, a 5-6-membered ring which, apart from the nitrogen atom, may also include 0-1 further heteroatom from the group of NH, N-($C_1$-$C_6$)-alkyl, oxygen and sulfur;

R2, R3, R4 are the same or different and are each hydrogen, F, Cl, Br, I, OH, $CF_3$, $NO_2$, CN, $OCHF_2$, $OCF_3$, $SF_5$, O-($C_1$-$C_6$)-alkyl, O-($C_1$-$C_4$)-alkoxy-($C_1$-$C_4$)-alkyl, S-($C_1$-$C_6$)-alkyl, ($C_1$-$C_6$)-alkyl, ($C_2$-$C_4$)-haloalkyl, O-($C_2$-$C_4$)-haloalkyl, ($C_2$-$C_6$)-alkenyl, ($C_3$-$C_8$)-cycloalkyl, O-($C_3$-$C_8$)-cycloalkyl, ($C_3$-$C_8$)-cycloalkenyl, aryl, ($C_2$-$C_6$)-alkynyl, N(R200)(R201), $SO_2$-$CH_3$, COOH, COO-($C_1$-$C_6$)-alkyl, CON(R202)(R203), N(R204)CO(R205), N(R206)$SO_2$(R207), CO(R208), $(CR209R210)_{z''}$-O(R211), $(CR209R210)_{z''}$-CO-O(R211), 0-$(CR209R210)_{z''}$-CO-O(R211), $(CR209R210)_{z''}$-N(R212)(R213), O-$(CR209R210)_{z''}$-N(R212)(R213), $(CR209R210)_{z''}$-CON(R212)(R213), O-$(CR209R210)_{z''}$-CON(R212)(R213), O-CO-N(R212)(R213), O-CO-($C_1$-$C_6$)-alkylene-CO-O-($C_1$-$C_6$)-alkyl, O-CO-($C_1$-$C_6$)-alkylene-CO-OH, O-CO-($C_1$-$C_6$)-alkylene-CO-N(R212)(R213);
z" is independently 1, 2, 3, 4, 5, 6;
R200, R201, R202, R203, R204R205, R206, R207, R208, R209, R210, R211, R212, R213

are the same or different and are each hydrogen, ($C_1$-$C_6$)-alkyl;

or
R200 and R201, R202 and R203, R212 and R213

each independently form, optionally together with the nitrogen atom to which they are bonded, a 5-6-membered ring which, apart from the nitrogen atom, may also include 0-1 further heteroatom from the group of NH, N-($C_1$-$C_6$)-alkyl, oxygen and sulfur;

the tautomeric forms of the compound and the physiologically compatible salts and N-oxides thereof; with the proviso that the compounds where R2, R3, R4 = hydrogen, X = S and R1 = heptyl, nonyl or cyclohexyl are excluded.

**2.** A compound of the formula I as claimed in claim 1, in which

R1 is a radical of the formula Ia

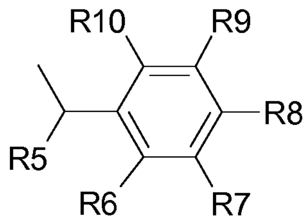

in which

R5 is hydrogen, $CH_3$;
R6, R7, R8, R9, R10
are each independently hydrogen, $OCF_3$, $SCF_3$, $OCHF_2$, O-($C_1$-$C_6$)-alkyl, O-($C_1$-$C_4$)-alkoxy-($C_1$-$C_4$)-alkyl, S-($C_1$-$C_6$)-alkyl, $SO_2$-$CH_3$, $SO_2$-N(R13)(R14), COOH, COO-($C_1$-$C_6$)-alkyl, $(CR22R23)_x$-O(R24), $(CR22R23)_x$-CO-0(R24), O-$(CR22R23)_x$-CO-O(R24), $(CR22R23)_x$-N(R25)(R26), O-$(CR22R23)_x$-N(R25)(R26), $(CR22R23)_x$-CON(R25)(R26);
with the proviso that at least one R6, R7, R8, R9, R10 radical is not hydrogen;
x is independently 0, 1, 2, 3, 4;
or
R7 or R8 is
R100, -$CH_2$-R100, -$OCH_2$-R100, -$CH_2$-O-R100, -$CH_2$-O-$CH_2$-R100 or -O-$CH_2CH_2$-R100;
R100 is a 4- to 7-membered monocyclic saturated, partly unsaturated or aromatic ring which may contain 1 to 3 heteroatoms selected from the group of oxygen, nitrogen and sulfur, where the ring system may additionally be mono- or polysubstituted by F, Cl, Br, OH, $CF_3$, $OCF_3$, O-($C_1$-$C_6$)-alkyl, O-($C_1$-$C_4$)-alkoxy-($C_1$-$C_4$)-alkyl, S-($C_1$-$C_6$)-alkyl, ($C_1$-$C_6$)-alkyl, $SO_2$-$CH_3$, COOH, COO-($C_1$-$C_6$)-alkyl, N(R35)$SO_2$(R36), CO(R37), $(CR38R39)_{x'}$-O(R40), $(CR38R39)_{x'}$-CO-O(R40), 0-$(CR22R23)_{x'}$-CO-O(R40), $(CR22R23)_{x'}$-N(R41) (R42), 0- $(CR38R39)_{x'}$-N(R41) (R42), $(CR38R39)_{x'}$-CON(R41) (R42), O-$(CR38R39)_{x'}$-CON(R41)(R42);
x' is independently 1, 2, 3, 4;
or
R7 and R8 or R8 and R9 or R9 and R10 together with the carbon atom which bears them form
-O-$CH_2$-O-, -O-$CH_2$-$CH_2$-O-, -O-$CH_2$-$CH_2$-$CH_2$-O-, -O-$CF_2$-0- or -N($CH_3$)-N=N-;
R11, R12, R13, R14, R15, R16, R17, R18, R19, R20, R21, R22, R23, R24, R25, R26, R27, R28, R29, R30, R31, R32, R33, R34, R35, R36, R37, R38, R39, R40, R41, R42

are each independently hydrogen, ($C_1$-$C_6$)-alkyl;

or
R11 and R12, R13 and R14, R15 and R16, R25 and 26, R27 and R28, R29 and R30, R31 and R32, R41 and 42, R43 and 44
each independently form, optionally together with the nitrogen atom to which they are bonded, a 5-6-membered ring which, apart from the nitrogen atom, may also include 0-1 further heteroatom from the group of NH, N-($C_1$-$C_6$)-alkyl, oxygen and sulfur.

**3.** A compound of the formula I as claimed in claim 1, in which

R1 is a radical of the formula Ib

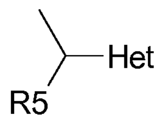

Ib

in which:

R5 is hydrogen, $CH_3$;
Het is selected from the group of pyridine, pyrazole, imidazole, oxazole, oxadiazole, benzothiophene, imidazo[2,1-b]thiazole, where Het may additionally be mono- or polysubstituted independently by F, Cl, Br, $CF_3$, $OCF_3$, $OCHF_2$, O-($C_1$-$C_6$)-alkyl, O-($C_1$-$C_4$)-alkoxy-($C_1$-$C_4$)-alkyl, S-($C_1$-$C_6$)-alkyl, ($C_1$-$C_6$)-alkyl, ($C_2$-$C_6$)-alkenyl, $SO_2$-$CH_3$, $SO_2$-N(R50)(R51), COOH, COO-($C_1$-$C_6$)-alkyl, N(R56)$SO_2$(R57), CO(R58), $(CR59R60)_{x'''}$-O(R61), $(CR59R60)_{x'''}$-CO-O(R61), O-$(CR59R60)_{x'''}$-CO-O(R61), $(CR59R60)_{x'''}$-N(R62)(R63), O-$(CR59R60)_{x'''}$-N(R62)(R63), $(CR59R60)_{x'''}$-CON(R62)(R63), 0-$(CR59R60)_{x'''}$-CON(R62)(R63), O-CO-N(R62)(R63), 0-CO-($C_1$-$C_6$)-alkylene-CO-O-($C_1$-$C_6$)-alkyl, O-CO-($C_1$-$C_6$)-alkylene-CO-OH, O-CO-($C_1$-$C_6$)-alkylene-CO-N(R64)(R65);
or $(O)_y$-$(CH_2-)_{y'}$-$(O)_{y''}$-$(CH_2)_{y'''}$-R101,
x''' is independently 1, 2, 3, 4;
y, y" are each independently 0,1;
y', y''' are each independently 0, 1, 2;
R101 is a 4- to 10-membered mono- or bicyclic saturated, partly unsaturated or aromatic ring which may contain 1 to 4 heteroatoms selected from the group of oxygen, nitrogen and sulfur, where the ring system may additionally be mono- or polysubstituted by F, Cl, Br, I, OH, $CF_3$, $NO_2$, CN, $OCHF_2$, $OCF_3$, O-($C_1$-$C_6$)-alkyl, O-($C_1$-$C_4$)-alkoxy-($C_1$-$C_4$)-alkyl, S-($C_1$-$C_6$)-alkyl, ($C_1$-$C_6$)-alkyl, ($C_2$-$C_4$)-haloalkyl, ($C_2$-$C_6$)-alkenyl, ($C_3$-$C_8$)-cycloalkyl, 0-($C_3$-$C_8$)-cycloalkyl, ($C_2$-$C_6$)-alkynyl, N(R66)(R67), $SO_2$-$CH_3$, $SF_5$, COOH, COO-($C_1$-$C_6$)-alkyl, CON(R68)(R69), (C), N(R70)CO(R71), N(R72)$SO_2$(R73), CO(R74), $(CR75R76)_{x''''}$-O$(R77R75R76)_{x''''}$-CO-O(R77), O-$(CR75R76)_{x''''}$-CO-O(R77), $(CR75R76)_{x''''}$-N(R78)(R79), O-$(CR75R76)_{x''''}$-N(R78)(R79), $(CR75R76)_{x''''}$-CON(R78)(R79), O-$(CR75R76)_{x''''}$-CON(R78)(R79), O-CO-N(R78)(R79), O-CO-($C_1$-$C_6$)-alkylene-CO-O-($C_1$-$C_6$)-alkyl, O-CO-($C_1$-$C_6$)-alkylene-CO-OH, O-CO-($C_1$-$C_6$)-alkylene-CO-N(R80)(R81);
x'''' is independently 1, 2, 3, 4, 5, 6;
R48, R49, R50, R51, R52, R53, R54, R55, R56, R57, R58, R59, R60, R61, R62, R63, R64, R65, R66, R67, R68, R69, R70, R71, R72, R73, R74, R75, R76, R77, R78, R79, R80, R81

are each independently hydrogen, ($C_1$-$C_6$)-alkyl;

or
R48 and R49, R50 and R51, R52 and R53, R62 and R63, R64 and R65, R66 and R67, R68 and R69, R78 and R79, R80 and R81
each independently form, optionally together with the nitrogen atom to which they are bonded, a 5-6-membered ring which, apart from the nitrogen atom, may also include 0-1 further heteroatom from the group of NH, N-($C_1$-$C_6$)-alkyl, oxygen and sulfur.

**4.** A compound of the formula I as claimed in claim 1, in which

R1 is a radical of the formula Ic:

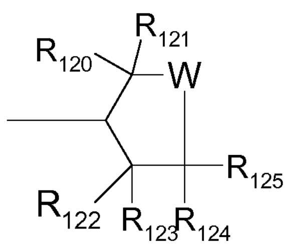

Ic

in which

W is -C(R126)(R127) -, -C(R126)(R127) -C(R128)(R129)-, -C(R126)(R127)-O-;
R120, R121, R123, R125, R126, R127, R128, R129 R120, R121, R126, R127, R128, R129 are the same or different and are each hydrogen, ($C_1$-$C_6$)-alkyl, oxo, COO-($C_1$-$C_6$)-alkyl;
R122 and R124 together with the carbon atom which bears them form a monocyclic, 6-membered aromatic ring system whose individual members may be substituted by = (C-R133)-;
R133 are the same or different and are each F, Cl, Br, I, OH, $CF_3$, $NO_2$, CN, $OCHF_2$, $OCF_3$, $SF_5$, O-($C_1$-$C_6$)-alkyl, O-($C_1$-$C_4$)-alkoxy-($C_1$-$C_4$)-alkyl, S-($C_1$-$C_6$)-alkyl, ($C_1$-$C_6$)-alkyl, ($C_2$-$C_4$)-haloalkyl, O-($C_2$-$C_4$)-haloalkyl, ($C_2$-$C_6$) -alkenyl, ($C_2$-$C_6$)-alkynyl, N(R160)(R161), $SO_2$-$CH_3$, COOH, COO-($C_1$-$C_6$)-alkyl, CON(R162)(R163), N(R164)CO(R165), N(R166)$SO_2$(R167), CO(R168), $(CR169R170)_{z'}$-O(R171), $(CR169R170)_{z'}$-CO-O(R77), O-$(CR169R170)_{z'}$-CO-O(R171), $(CR169R170)_{z'}$-N(R172)(R173), O-$(CR169R170)_{z'}$-N(R172)(R173), $(CR169R170)_{z'}$-CON(R172)(R173), O-$(CR169R170)_{z'}$-CON(R172)(R173), O-CO-N(R172)(R173), O-CO-($C_1$-$C_6$)-alkylene-CO-O-($C_1$-$C_6$)-alkyl, O-CO-($C_1$-$C_6$)-alkylene-CO-OH, O-CO-($C_1$-$C_6$)-alkylene-CO-N(R172)(R173);
z' is independently 1, 2, 3, 4, 5, 6;
R160, R161, R162, R163, R164, R165, R166, R167, R168, R169, R170, R171, R172, R173

are the same or different and are each hydrogen, ($C_1$-$C_6$)-alkyl;

or
R160 and R161, R162 and R163, R172 and R173 each independently form, optionally together with the nitrogen atom to which they are bonded, a 5-6-membered ring which, apart from the nitrogen atom, may also include 0-1 further heteroatom from the group of NH, N-($C_1$-$C_6$)-alkyl, oxygen and sulfur.

**5.** A compound of the formula I as claimed in claims 1 to 4, in which

R2 is hydrogen, -($C_1$-$C_6$)-alkyl, ($C_3$-$C_8$)-cycloalkyl or phenyl;
R3 is hydrogen;
R4 is hydrogen or -($C_1$-$C_6$)-alkyl.

**6.** A medicament comprising one or more of the compounds of the formula I as claimed in claims 1 to 5.

**7.** The medicament as claimed in claim 6, which comprises, as a further active ingredient, metformin, arcabose, glibenclamide, glimepiride, gliclazide, gliquidone, pioglitazone, rosiglitazone, exenatide, miglitol, vildagliptin, sitagliptin, repaglinide, nateglinide or mitiglinide.

**8.** The medicament as claimed in claim 6, which comprises, as a further active ingredient, one or more antidiabetics, active hypoglycemic ingredients, HMG-CoA reductase inhibitors, cholesterol absorption inhibitors, PPAR gamma agonists, PPAR alpha agonists, PPAR alpha/gamma agonists, fibrates, MTP inhibitors, bile acid absorption inhibitors, CETP inhibitors, polymeric bile acid adsorbers, LDL receptor inducers, ACAT inhibitors, antioxidants, lipoprotein lipase inhibitors, ATP citrate lyase inhibitors, squalene synthetase inhibitors, lipoprotein(a) antagonists, lipase inhibitors, insulins, sulfonylureas, biguanides, meglitinides, thiazolidinediones, $\alpha$-glucosidase inhibitors, active ingredients which act on the ATP-dependent potassium channel of the beta cells, CART agonists, NPY agonists, MC4 agonists, orexin antagonists, H3 agonists, TNF agonists, CRF antagonists, CRF BP antagonists, urocortin agonists, $\beta$3 agonists, MSH (melanocyte-stimulating hormone) agonists, CCK agonists, serotonin reuptake inhibitors, mixed serotoninergic and noradrenergic compounds, 5HT agonists, bombesin agonists, galanin antagonists, growth hormones, growth hormone-releasing compounds, TRH agonists, decoupling protein 2 or 3 modulators, leptin agonists, DA agonists (bromocriptine, Doprexin), lipase/amylase inhibitors, PPAR modulators, RXR modulators or TR $\beta$ agonists or amphetamines.

**9.** A compound of the formula I as claimed in claims 1 to 5 for treatment and/or prevention of disorders of fatty acid metabolism and glucose utilization disorders.

**10.** A compound of the formula I as claimed in claims 1 to 5 for treatment and/or prevention of dyslipidemias and sequelae thereof.

**11.** A compound of the formula I as claimed in claims 1 to 5 for treatment and/or prevention of conditions associated with metabolic syndrome, and in the event of disorders involving insulin resistance, or for treatment and/or prevention of diabetes mellitus and the associated sequelae.

**12.** A compound of the formula I as claimed in claims 1 to 5 for treatment and/or prevention of states associated with a lowered HDL level.

**13.** A compound of the formula I as claimed in claims 1 to 5 for treatment and/or prevention of atherosclerotic disorders.

**14.** A compound of the formula I as claimed in claims 1 to 5 in combination with at least one further active ingredient for treatment and/or prevention of disorders involving insulin resistance.

**15.** A process for producing a medicament comprising one or more of the compounds of the formula I as claimed in claims 1 to 5, which comprises mixing said compound(s) with a pharmaceutically suitable carrier and converting this mixture to a form suitable for administration.

**16.** A process for preparing compounds of the formula I as claimed in claims 1 to 5, wherein
substituted or unsubstituted azolopyridin-3-ol derivatives II are reacted with isocyanates III to give compounds of the formula I;

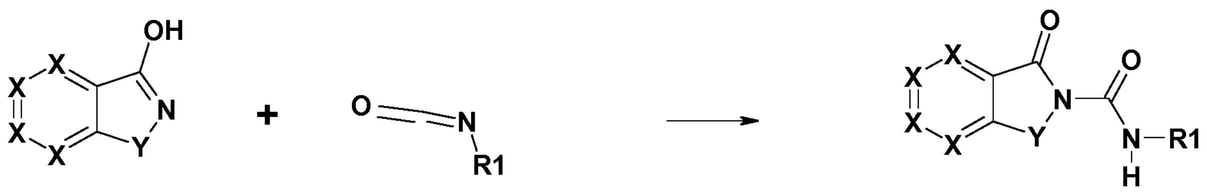

II III IV

or
azolopyridin-3-ol derivatives II

a) are acylated with carbamoyl chlorides of the formula VI;
or
b) are reacted in two stages, first with phosgene or equivalents such as trichloromethyl chlorocarbonate, ditrichloromethyl carbonate or 4-nitrophenyl chloroformate, and in a second step with amines of the formula VII

in which the substituents are each as defined above.

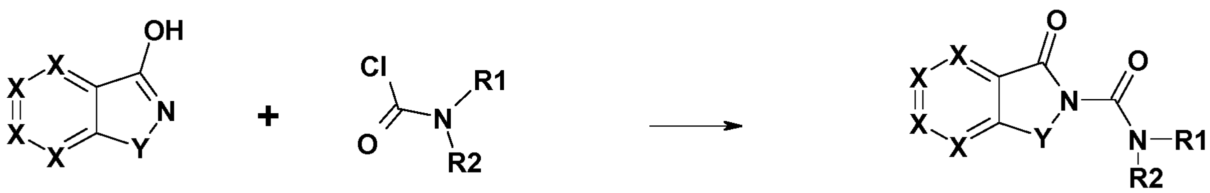

II VI I

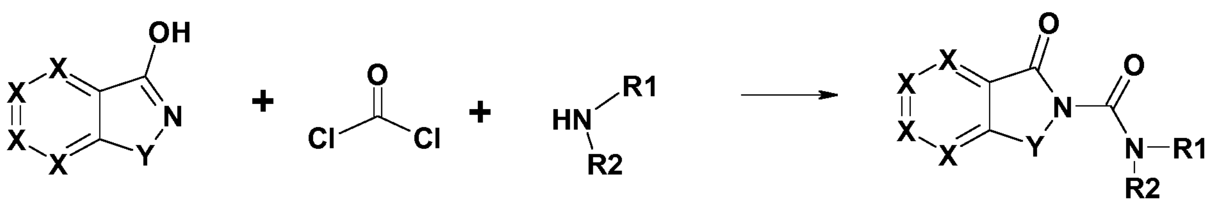

II VII I

## Revendications

**1.** Composé de formule I

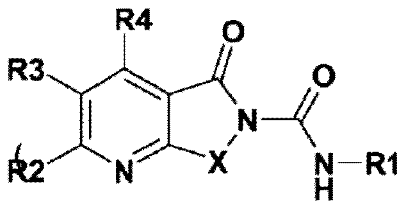

(I)

dans laquelle :

X signifie S ou $SO_2$;
R1 signifie un radical de formule la

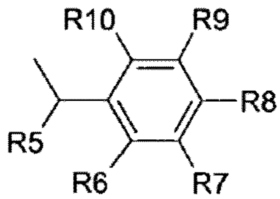

,

dans laquelle
R5 signifie hydrogène, alkyle en ($C_1$-$C_3$),
R6, R7, R8, R9, R10

signifient indépendamment les uns des autres hydrogène, OH, CN, $OCF_3$, $OCHF_2$, O-alkyle en ($C_1$-$C_6$), O-alcoxy en ($C_1$-$C_4$)-alkyle en ($C_1$-$C_4$), S-alkyle en ($C_1$-$C_6$), haloalkyle en ($C_2$-$C_4$), O-haloalkyle en ($C_2$-$C_4$), alcényle en ($C_2$-$C_6$), alcynyle en ($C_2$-$C_6$), N(R11)(R12), $SO_2$-$CH_3$, $SO_2$-N(R13)(R14), $SF_5$, $SCF_3$, COOH, COO-alkyle en ($C_1$-$C_6$), CON(R15)(R16), N(R17)CO(R18), N(R19)$SO_2$(R20), CO(R21), $(CR22R23)_x$-O(R24), $(CR22R23)_x$-CO-O(R24), O-$(CR22R23)_x$-CO-O(R24), $(CR22R23)_x$-N(R25)(R26), O-$(CR22R23)_x$-N(R25)(R26), $(CR22R23)_x$-CON(R25)(R26), O-$(CR22R23)_x$-CON(R25)(R26), O-CO-N(R25)(R26), O-CO-alkylène en ($C_1$-$C_6$)-CO-O-alkyle en ($C_1$-$C_6$), O-CO-alkylène en ($C_1$-$C_6$)-CO-OH, O-CO-alkylène en ($C_1$-$C_6$)-CO-N(R27)(R28);

à condition qu'au moins un radical R6, R7, R8, R9, R10 soit différent de l'hydrogène;
les x signifient indépendamment les uns des autres 0, 1, 2, 3, 4, 5, 6 ;
ou
R7 ou R8 signifie $(O)_y$-$(CH_2)_{y'}$-$(O)_{y''}$ -$(CH_2)_{y'''}$-R100,
y, y" signifient indépendamment l'un de l'autre 0, 1 ;
y', y'" signifient indépendamment l'un de l'autre 1, 2, 3, 4, 5, 6 ;
R100 signifie un cycle de 4 à 10 chaînons, mono- ou bicyclique, saturé, partiellement insaturé ou aromatique, qui peut contenir 1 à 4 hétéroatomes, choisis dans le groupe constitué par oxygène, azote ou soufre, le système cyclique pouvant en outre être substitué une ou plusieurs fois par F, Cl, Br, I, OH, $CF_3$, $NO_2$, CN, $OCF_3$, O-alkyle en ($C_1$-$C_6$), O-alcoxy en ($C_1$-$C_4$)-alkyle en ($C_1$-$C_4$), S-alkyle en ($C_1$-$C_6$), alkyle en ($C_1$-$C_6$), haloalkyle en ($C_2$-$C_4$), alcényle en ($C_2$-$C_6$), cycloalkyle en ($C_3$-$C_8$), O-cycloalkyle en ($C_3$-$C_8$), alcynyle en ($C_2$-$C_6$), N(R29)(R30), $SO_2$-$CH_3$, $SF_5$, COOH, COO-alkyle en ($C_1$-$C_6$), CON(R31)(R32), N(R33)CO(R34), N(R35)$SO_2$(R36), CO(R37), $(CR38R39)_{x'}$-O(R40), $(CR38R39)_{x'}$-CO-O(R40), O-$(CR22R23)_{x'}$-CO-O(R40), $(CR22R23)_{x'}$-N(R41)(R42), O-$(CR38R39)_{x'}$-N(R41)(R42), $(CR38R39)_{x'}$-CON(R41)(R42), O-$(CR38R39)_{x'}$-CON(R41)(R42), O-CO-N(R41)(R42), O-CO-alkylène en ($C_1$-$C_6$)-CO-O-alkyle en ($C_1$-$C_6$), O-CO-alkylène en ($C_1$-$C_6$)-CO-OH, O-CO-alkylène en ($C_1$-$C_6$)-CO-N(R43)(R44) ;
x' signifie 0, 1, 2, 3, 4, 5, 6 ;
ou
R7 et R8 ou R8 et R9 ou R9 et R10

forment ensemble avec les atomes de carbone qui les portent un système cyclique monocyclique de 5 à 7 chaînons, saturé, partiellement insaturé ou aromatique, dont les éléments individuels peuvent être substitués par -CHR45-, -CR46R47-, =(C-R46)-, O, N ou S ; à condition que deux unités de la série -O-, N ou

-S- ne puissent pas être voisines;

R11, R12, R13, R14, R15, R16, R17, R18, R19, R20, R21, R22, R23, R24, R25, R26, R27, R28, R29, R30, R31, R32, R33, R34, R35, R36, R37, R38, R39, R40, R41, R42, R43, R44

signifient indépendamment les uns des autres hydrogène, alkyle en ($C_1$-$C_6$);

ou
R11 et R12, R13 et R14, R15 et R16, R25 et 26, R27 et R28, R29 et R30, R31 et R32, R41 et 42, R43 et 44

forment indépendamment les uns des autres, éventuellement conjointement avec l'atome d'azote auquel ils sont reliés, un cycle de 5 à 6 chaînons, qui peut contenir en plus de l'atome d'azote encore 0 à 1 hétéroatome supplémentaire du groupe constitué par NH, N-alkyle en ($C_1$-$C_6$), oxygène et soufre;

R45, R46, R47 signifient, de manière identique ou différente, F, Cl, Br, I, OH, $CF_3$, $NO_2$, CN, $OCHF_2$, $OCF_3$, $SF_5$, O-alkyle en ($C_1$-$C_6$), O-alcoxy en ($C_1$-$C_4$)-alkyle en ($C_1$-$C_4$), S-alkyle en ($C_1$-$C_6$), alkyle en ($C_1$-$C_6$), haloalkyle en ($C_2$-$C_4$), O-haloalkyle en ($C_2$-$C_4$), alcényle en ($C_2$-$C_6$), cycloalkyle en ($C_3$-$C_8$), O-cycloalkyle en ($C_3$-$C_8$), alcynyle en ($C_2$-$C_6$), N(R134)(R135), $SO_2$-$CH_3$, COOH, COO-alkyle en ($C_1$-$C_6$), CON(R136)(R137), N(R138)CO(R139), N(R140)$SO_2$(R141), CO(R142), $(CR143R144)_{x''}$-O(R145), $(CR143R144)_{x''}$-CO-O(R145), O-$(CR143R144)_{x''}$-CO-O(R145), $(CR143R144)_{x''}$-N(R146)(R147), O-$(CR143R144)_{x''}$-N(R146)(R147), $(CR143R144)_{x''}$-CON(R146)(R147), O-$(CR143R144)_{x''}$-CON(R146)(R147), O-CO-N(R146)(R147), O-CO-alkylène en ($C_1$-$C_6$)-CO-O-alkyle en ($C_1$-$C_6$), O-CO-alkylène en ($C_1$-$C_6$)-CO-OH, O-CO-alkylène en ($C_1$-$C_6$)-CO-N(R148)(R149) ;
les x" signifient indépendamment les uns des autres 1, 2, 3, 4, 5, 6 ;
R134, R135. R136, R137, R138, R139, R140, R141, R142, R143, R144, R145, R146, R147, R148, R149

signifient, de manière identque ou différente, hydrogène, alkyle en ($C_1$-$C_6$);

un radical de formule Ib

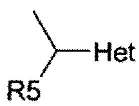

Ib,

dans laquelle

R5 signifie hydrogène, alkyle en ($C_1$-$C_3$);
Het signifie un cycle aromatique mono- ou bicyclique de 4 à 10 chaînons, qui contient 1 à 4 hétéroatomes, choisis dans le groupe constitué par oxygène, azote ou soufre, le système cyclique pouvant en outre être substitué une ou plusieurs fois par, indépendamment les uns des autres, F, Cl, Br, I, OH, $CF_3$, $NO_2$, CN, $OCF_3$, $OCHF_2$, O-alkyle en ($C_1$-$C_6$), O-alcoxy en ($C_1$-$C_4$)-alkyle en ($C_1$-$C_4$), S-alkyle en ($C_1$-$C_6$), alkyle en ($C_1$-$C_6$), haloalkyle en ($C_2$-$C_4$), O-haloalkyle en ($C_2$-$C_4$), alcényle en ($C_2$-$C_6$), alcynyle en ($C_2$-$C_6$), N(R48)(R49), $SO_2$-$CH_3$, $SO_2$-N(R50)(R51), $SF_5$, $SCF_3$, COOH, COO-alkyle en ($C_1$-$C_6$), CON(R52)(R53), N(R54)CO(R55), N(R56)$SO_2$(R57), CO(R58), $(CR59R60)_{x'''}$-O(R61), $(CR59R60)_{x'''}$-CO-O(R61), O-$(CR59R60)_{x'''}$,-CO-O(R61), $(CR59R60)_{x'''}$-N(R62)(R63), O-$(CR59R60)_{x'''}$-N(R62)(R63), $(CR59R60)_{x'''}$-CON(R62)(R63), O-$(CR59R60)_{x'''}$-CON(R62)(R63), O-CO-N(R62)(R63), O-CO-alkylène en ($C_1$-$C_6$)-CO-O-alkyle en ($C_1$-$C_6$), O-CO-alkylène en ($C_1$-$C_6$)-CO-OH, O-CO-alkylène en ($C_1$-$C_6$)-CO-N(R64)(R65) ; ou $(O)_y$-$(CH_2\text{-})_{y'}$-$(O)_{y''}$-$(CH_2)_{y'''}$-R101,
les x'" signifient indépendamment les uns des autres 1, 2, 3, 4, 5, 6 ;
y, y" signifient indépendamment l'un de l'autre 0, 1 ;
y', y'" signifient indépendamment l'un de l'autre 0, 1, 2, 3, 4, 5, 6 ;
R101 signifie un cycle de 4 à 10 chaînons mono- ou bicyclique saturé, partiellement insaturé ou aromatique, qui peut contenir 1 à 4 hétéroatomes, choisis dans le groupe constitué par oxygène, azote ou soufre, le système cyclique pouvant en outre être substitué une ou plusieurs fois par F, Cl, Br, I, OH, $CF_3$, $NO_2$, CN, $OCHF_2$, $OCF_3$, O-alkyle en ($C_1$-$C_6$), O-alcoxy en ($C_1$-$C_4$)-alkyle en ($C_1$-$C_4$), S-alkyle en ($C_1$-$C_6$), alkyle en ($C_1$-$C_6$), haloalkyle en ($C_2$-$C_4$), alcényle en ($C_2$-$C_6$), cycloalkyle en ($C_3$-$C_8$), O-cycloalkyle en ($C_3$-$C_8$), al-

cynyle en ($C_2$-$C_6$), N(R66)(R67), $SO_2$-$CH_3$, $SF_5$, COOH, COO-alkyle en ($C_1$-$C_6$), CON(R68)(R69), (C), N(R70)CO(R71), N(R72)$SO_2$(R73), CO(R74), $(CR75R76)_{x''''}$-O(R77R75R76)$_{x''''}$-CO-O(R77), O-$(CR75R76)_{x''''}$-CO-O(R77), $(CR75R76)_{x''''}$-N(R78)(R79), O-$(CR75R76)_{x''''}$-N(R78)(R79), $(CR75R76)_{x''''}$-CON(R78)(R79), O-$(CR75R76)_{x''''}$-CON(R78)(R79), O-CO-N(R78)(R79), O-CO-alkylène en ($C_1$-$C_6$)-CO-O-alkyle en ($C_1$-$C_6$), O-CO-alkylène en ($C_1$-$C_6$)-CO-OH, O-CO-alkylène en ($C_1$-$C_6$)-CO-N(R80)(R81);

les x''''signifient indépendamment les uns des autres 1, 2, 3, 4, 5, 6 ;

R48, R49, R50, R51, R52, R53, R54, R55, R56, R57, R58, R59, R60, R61, R62, R63, R64, R65, R66, R67, R68, R69, R70, R71, R72, R73, R74, R75, R76, R77, R78, R79, R80, R81

signifient indépendamment les uns des autres hydrogène, alkyle en ($C_1$-$C_6$);

ou

R48 et R49, R50 et R51, R52 et R53, R62 et R63, R64 et R65, R66 et R67, R68 et R69, R78 et R79, R80 et R81

forment indépendamment les uns des autres éventuellement conjointement avec l'atome d'azote auquel ils sont reliés un cycle de 5 à 6 chaînons, qui peut contenir en plus de l'atome d'azote encore 0 à 1 hétéroatome supplémentaire du groupe constitué par NH, N-alkyle en ($C_1$-$C_6$), oxygène et soufre;

un radical de formule Ic

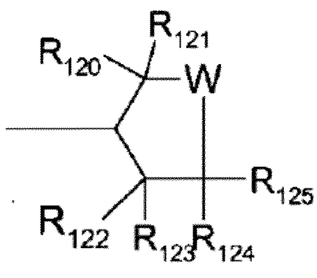

Ic,

dans laquelle

W signifie -C(R126)(R127)-, -C(R126)(R127)-C(R128)(R129)-, - C(R126)(R127)-O-;

R120, R121, R123, R125, R126, R127, R128, R129

signifient, de manière identique ou différente, hydrogène, F, Cl, Br, I, OH, $CF_3$, $NO_2$, CN, $OCHF_2$, $OCF_3$, $SF_5$, O-alkyle en ($C_1$-$C_6$), O-alcoxy en ($C_1$-$C_4$)-alkyle en ($C_1$-$C_4$), S-alkyle en ($C_1$-$C_6$), alkyle en ($C_1$-$C_6$), haloalkyle en ($C_2$-$C_4$), O-haloalkyle en ($C_2$-$C_4$), alcényle en ($C_2$-$C_6$), cycloalcényle en ($C_3$-$C_8$), alcynyle en ($C_2$-$C_6$), N(R90)(R91), $SO_2$-$CH_3$, COOH, COO-alkyle en ($C_1$-$C_6$), CON(R92)(R93), N(R94)CO(R95), N(R96)$SO_2$(R97), CO(R98), $(CR99R102)_z$-O(R103), $(CR99R76)_z$-CO-O(R103), O-$(CR99R102)_z$-CO-O(R103), $(CR99R102)_z$-N(R104)(R105), O-$(CR99R102)_z$-N(R104)(R105), $(CR99R102)_z$-CON(R104)(R105), O-$(CR99R102)_z$-CON(R104)(R105), O-CO-N(R104)(R105), O-CO-alkylène en ($C_1$-$C_6$)-CO-O-alkyle en ($C_1$-$C_6$), O-CO-alkylène en ($C_1$-$C_6$)-CO-OH, O-CO-alkylène en ($C_1$-$C_6$)-CO-N(R106)(R107) ;

les z signifient indépendamment les uns des autres 1, 2, 3, 4, 5, 6 ;

R90, R91, R92, R93, R94, R95, R96, R97, R98, R99, R102, R103, R104, R105, R106, R107

signifient, de manière identique ou différente, hydrogène, alkyle en ($C_1$-$C_6$);

R122 et R124 forment conjointement avec les atomes de carbone qui les portent un système cyclique monocyclique, à 5 ou 6 chaînons, saturé, partiellement insaturé ou aromatique, dont les éléments individuels peuvent être substitués par -CHR130-, -CR131R132-, =(C-R133)- ;

R130, R131, R132, R133 signifient, de manière identique ou différente, F, Cl, Br, I, OH, $CF_3$, $NO_2$, CN, $OCHF_2$, $OCF_3$, $SF_5$, O-alkyle en ($C_1$-$C_6$), O-alcoxy en ($C_1$-$C_4$)-alkyle en ($C_1$-$C_4$), S-alkyle en ($C_1$-$C_6$), alkyle en ($C_1$-$C_6$), haloalkyle en ($C_2$-$C_4$), O-haloalkyle en ($C_2$-$C_4$), alcényle en ($C_2$-$C_6$), alcynyle en ($C_2$-$C_6$),

N(R160)(R161), $SO_2$-$CH_3$, COOH, COO-alkyle en ($C_1$-$C_6$), CON(R162)(R163), N(R164)CO(R165), N(R166)$SO_2$(R167), CO(R168), $(CR169R170)_{z'}$-O(R171), $(CR169R170)_{z'}$-CO-O(R77), O-$(CR169R170)_{z'}$-CO-O(R171), $(CR169R170)_{z'}$-N(R172)(R173), O-$(CR169R170)_{z'}$-N(R172)(R173), $(CR169R170)_{z'}$-CON(R172)(R173), O-$(CR169R170)_{z'}$-CON(R172)(R173), O-CO-N(R172)(R173), O-CO-alkylène en ($C_1$-$C_6$)-CO-O-alkyle en ($C_1$-$C_6$), O-CO-alkylène en ($C_1$-$C_6$)-CO-OH, O-CO-alkylène en ($C_1$-$C_6$)-CO-N(R172)(R173);
les z' signifient indépendamment les uns des autres 1, 2, 3, 4, 5, 6 ;
R160, R161, R162, R163, R164, R165, R166, R167, R168, R169, R170, R171, R172, R173

signifient, de manière identique ou différente, hydrogène, alkyle en ($C_1$-$C_6$);

ou
R160 et R161, R162 et R163, R172 et R173 forment indépendamment les uns des autres éventuellement conjointement avec l'atome d'azote auquel ils sont reliés un cycle de 5 à 6 chaînons, qui peut contenir en plus de l'atome d'azote encore 0 à 1 hétéroatome supplémentaire du groupe constitué par NH, N-alkyle en ($C_1$-$C_6$), oxygène et soufre ;
R2, R3, R4 signifient, de manière identique ou différente, hydrogène, F, Cl, Br, I, OH, $CF_3$, $NO_2$, CN, $OCHF_2$, $OCF_3$, $SF_5$, O-alkyle en ($C_1$-$C_6$), O-alcoxy en ($C_1$-$C_4$)-alkyle en ($C_1$-$C_4$), S-alkyle en ($C_1$-$C_6$), alkyle en ($C_1$-$C_6$), haloalkyle en ($C_2$-$C_4$), O-haloalkyle en ($C_2$-$C_4$), alcényle en ($C_2$-$C_6$), cycloalkyle en ($C_3$-$C_8$), O-cycloalkyle en ($C_3$-$C_8$), cycloalcényle en ($C_3$-$C_8$), aryle, alcynyle en ($C_2$-$C_6$), N(R200)(R201), $SO_2$-$CH_3$, COOH, COO-alkyle en ($C_1$-$C_6$), CON(R202)(R203), N(R204)CO(R205), N(R206)$SO_2$(R207), CO(R208), $(CR209R210)_{z''}$-O(R211), $(CR209R210)_{z''}$-CO-O(R211), O-$(CR209R210)_{z''}$-CO-O(R211), $(CR209R210)_{z''}$-N(R212)(R213), O-$(CR209R210)_{z''}$-N(R212)(R213), $(CR209R210)_{z''}$-CON(R212)(R213), O-$(CR209R210)_{z''}$-CON(R212)(R213), O-CO-N(R212)(R213), O-CO-alkylène en ($C_1$-$C_6$)-CO-O-alkyle en ($C_1$-$C_6$), O-CO-alkylène en ($C_1$-$C_6$)-CO-OH, O-CO-alkylène en ($C_1$-$C_6$)-CO-N(R212)(R213);
les z" signifient indépendamment les uns des autres 1, 2, 3, 4, 5, 6 ;
R200, R201, R202, R203, R204R205, R206, R207, R208, R209, R210, R211, R212, R213

signifient, de manière identique ou différente, hydrogène, alkyle en ($C_1$-$C_6$);

ou
R200 et R201, R202 et R203, R212 et R213

forment indépendamment les uns des autres éventuellement conjointement avec l'atome d'azote auquel ils sont reliés, un cycle de 5 à 6 chaînons, qui peut contenir en plus de l'atome d'azote encore 0 à 1 hétéroatome supplémentaire du groupe constitué par NH, N-alkyle en ($C_1$-$C_6$), oxygène et soufre;

les formes tautomères du composé, ainsi que leurs sels et N-oxydes physiologiquement compatibles;
à condition que les composés avec R2, R3, R4 = hydrogène, X = S et R1 = heptyle, nonyle ou cyclohexyle soient exclus.

**2.** Composé de formule I selon la revendication 1, dans lequel

R1 est un radical de formule la

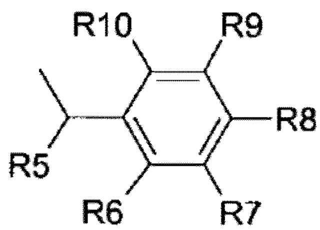

,

dans laquelle

R5 signifie hydrogène, $CH_3$,
R6, R7, R8, R9, R10

signifient indépendamment les uns des autres hydrogène, $OCF_3$, $SCF_3$, $OCHF_2$, O-alkyle en ($C_1$-$C_6$), O-alcoxy en ($C_1$-$C_4$)-alkyle en ($C_1$-$C_4$), S-alkyle en ($C_1$-$C_6$), $SO_2$-$CH_3$, $SO_2$-N(R13)(R14), COOH, COO-alkyle en ($C_1$-$C_6$), $(CR22R23)_x$-O(R24), $(CR22R23)_x$-CO-O(R24), O-$(CR22R23)_x$-CO-O(R24), $(CR22R23)_x$-N(R25)(R26), O-$(CR22R23)_x$-N(R25)(R26), $(CR22R23)_x$-CON(R25)(R26);

à condition qu'au moins un radical R6, R7, R8, R9, R10 soit différent de l'hydrogène ;
les x signifient indépendamment les uns des autres 0, 1, 2, 3, 4 ;
ou
R7 ou R8 signifie R100, -$CH_2$-R1OO, -$OCH_2$-R100, -$CH_2$-O-R100, -$CH_2$-O-$CH_2$-R100 ou -O-$CH_2CH_2$-R100;
R100 signifie un cycle de 4 à 7 chaînons, monocyclique, saturé, partiellement insaturé ou aromatique, qui peut contenir 1 à 3 hétéroatomes, choisis dans le groupe constitué par oxygène, azote ou soufre, le système cyclique pouvant en outre être substitué une ou plusieurs fois par F, Cl, Br, OH, $CF_3$, $OCF_3$, O-alkyle en ($C_1$-$C_6$), O-alcoxy en ($C_1$-$C_4$)-alkyle en ($C_1$-$C_4$), S-alkyle en ($C_1$-$C_6$), alkyle en ($C_1$-$C_6$), $SO_2$-$CH_3$, COOH, COO-alkyle en ($C_1$-$C_6$), N(R35)$SO_2$(R36), CO(R37), $(CR38R39)_{x'}$-O(R40), $(CR38R39)_{x'}$-CO-O(R40), O-$(CR22R23)_{x'}$-CO-O(R40), $(CR22R23)_{x'}$-N(R41)(R42), O-$(CR38R39)_{x'}$-N(R41)(R42), $(CR38R39)_{x'}$-CON(R41)(R42), O-$(CR38R39)_{x'}$-CON(R41)(R42);
les x' signifient indépendamment les uns des autres 1, 2, 3, 4 ;
ou
R7 et R8 ou R8 et R9 ou R9 et R10

forment conjointement avec les atomes de carbone qui les portent-O-$CH_2$-O-, -O-$CH_2$-$CH_2$-O-, -O-$CH_2$-$CH_2$-$CH_2$-O-, -O-$CF_2$-O- ou - N($CH_3$)-N=N- ;

R11, R12, R13, R14, R15, R16, R17, R18, R19, R20, R21, R22, R23, R24, R25, R26, R27, R28, R29, R30, R31, R32, R33, R34, R35, R36, R37, R38, R39, R40, R41. R42

signifient indépendamment les uns des autres hydrogène, alkyle en ($C_1$-$C_6$);

ou
R11 et R12, R13 et R14, R15 et R16, R25 et 26, R27 et R28, R29 et R30, R31 et R32, R41 et 42, R43 et 44

forment indépendamment les uns des autres, éventuellement conjointement avec l'atome d'azote auquel ils sont reliés, un cycle de 5 à 6 chaînons, qui peut contenir en plus de l'atome d'azote encore 0 à 1 hétéroatome supplémentaire du groupe constitué par NH, N-alkyle en ($C_1$-$C_6$), oxygène et soufre.

**3.** Composé de formule I selon la revendication 1, dans lequel

R1 est un radical de formule Ib

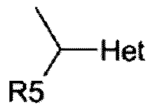 Ib,

dans laquelle

R5 signifie hydrogène, $CH_3$;
Het est choisi dans le groupe constitué par pyridine, pyrazole, imidazole, oxazole, oxadiazole, benzothiophène, imidazo[2,1,b]thiazole, Het pouvant en outre être substitué une ou plusieurs fois par, indépendamment les uns des autres, F, Cl, Br, $CF_3$, $OCF_3$, $OCHF_2$, O-alkyle en ($C_1$-$C_6$), O-alcoxy en ($C_1$-$C_4$)-alkyle en ($C_1$-$C_4$), S-alkyle en ($C_1$-$C_6$), alkyle en ($C_1$-$C_6$), alcényle en ($C_2$-$C_6$), $SO_2$-$CH_3$, $SO_2$-N(R50)(R51), COOH, COO-alkyle en ($C_1$-$C_6$), N(R56)$SO_2$(R57), CO(R58), $(CR59R60)_{x''''}$-O(R61), $(CR59R60)_{x''''}$-CO-O(R61), O-$(CR59R60)_{x''''}$,-CO-O(R61), $(CR59R60)_{x''''}$-N(R62)(R63), O-$(CR59R60)_{x''''}$-N(R62)(R63), $(CR59R60)_{x''''}$-CON(R62)(R63), O-$(CR59R60)_{x''''}$-CON(R62)(R63), O-CO-N(R62)(R63), O-CO-alkylène en ($C_1$-$C_6$)-CO-O-alkyle en ($C_1$-$C_6$), O-CO-alkylène en ($C_1$-$C_6$)-CO-OH, O-CO-alkylène en ($C_1$-$C_6$)-CO-N(R64)(R65); ou $(O)_y$-$(CH_2-)_{y'}$-$(O)_{y''}$-$(CH_2)_{y'''}$-R101, les x'''' signifient indépendamment les uns des autres 1, 2, 3, 4 ;

y, y" signifient indépendamment l'un de l'autre 0, 1 ;
y', y''' signifient indépendamment l'un de l'autre 0, 1, 2 ;
R101 signifie un cycle de 4 à 10 chaînons mono- ou bicyclique saturé, partiellement insaturé ou aromatique, qui peut contenir 1 à 4 hétéroatomes, choisis dans le groupe constitué par oxygène, azote ou soufre, le système cyclique pouvant en outre être substitué une ou plusieurs fois par F, Cl, Br, I, OH, $CF_3$, $NO_2$, CN, $OCHF_2$, $OCF_3$, O-alkyle en ($C_1$-$C_6$), O-alcoxy en ($C_1$-$C_4$)-alkyle en ($C_1$-$C_4$), S-alkyle en ($C_1$-$C_6$), alkyle en ($C_1$-$C_6$), haloalkyle en ($C_2$-$C_4$), alcényle en ($C_2$-$C_6$), cycloalkyle en ($C_3$-$C_8$), O-cycloalkyle en ($C_3$-$C_8$), alcynyle en ($C_2$-$C_6$), N(R66)(R67), $SO_2$-$CH_3$, $SF_5$, COOH, COO-alkyle en ($C_1$-$C_6$), CON(R68)(R69), (C), N(R70)CO(R71), N(R72)$SO_2$(R73), CO(R74), $(CR75R76)_{x''''}$-O(R77R75R76)$_{x''''}$-CO-O(R77), O-$(CR75R76)_{x''''}$-CO-O(R77), $(CR75R76)_{x''''}$-N(R78)(R79), O-$(CR75R76)_{x''''}$-N(R78)(R79), $(CR75R76)_{x''''}$-CON(R78)(R79), O-$(CR75R76)_{x''''}$-CON(R78)(R79), O-CO-N(R78)(R79), O-CO-alkylène en ($C_1$-$C_6$)-CO-O-alkyle en ($C_1$-$C_6$), O-CO-alkylène en ($C_1$-$C_6$)-CO-OH, O-CO-alkylène en ($C_1$-$C_6$)-CO-N(R80)(R81); les x''''signifient indépendamment les uns des autres 1, 2, 3, 4, 5, 6 ;
R48, R49, R50, R51, R52, R53, R54, R55, R56, R57, R58, R59, R60, R61, R62, R63, R64, R65, R66, R67, R68, R69, R70, R71, R72, R73, R74, R75, R76, R77, R78, R79, R80, R81

signifient indépendamment les uns des autres hydrogène, alkyle en ($C_1$-$C_6$);

ou
R48 et R49, R50 et R51, R52 et R53, R62 et R63, R64 et R65, R66 et R67, R68 et R69, R78 et R79, R80 et R81

forment indépendamment les uns des autres éventuellement conjointement avec l'atome d'azote auquel ils sont reliés un cycle de 5 à 6 chaînons, qui peut contenir en plus de l'atome d'azote encore 0 à 1 hétéroatome supplémentaire du groupe constitué par NH, N-alkyle en ($C_1$-$C_6$), oxygène et soufre.

**4.** Composé de formule I selon la revendication 1, dans lequel R1 est un radical de formule Ic :

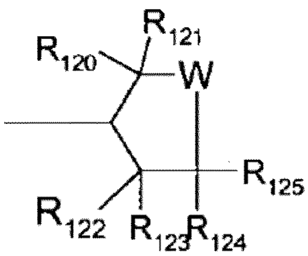
Ic,

dans laquelle

W signifie -C(R126)(R127)-, -C(R126)(R127)-C(R128)(R129)-,-C(R126)(R127)-O-;
R120, R121, R123, R125, R126, R127, R128, R129
R120, R121, R126, R127, R128, R129 signifient, de manière identique ou différente, hydrogène, alkyle en ($C_1$-$C_6$), oxo, COO-alkyle en ($C_1$-$C_6$);
R122 et R124 forment conjointement avec les atomes d'azote qui les portent un système cyclique monocyclique aromatique à 6 chaînons, dont les éléments individuels peuvent être substitués par =(C-R133)- ;
R133 signifie, de manière identique ou différente, F, Cl, Br, I, OH, $CF_3$, $NO_2$, CN, $OCHF_2$, $OCF_3$, $SF_5$, O-alkyle en ($C_1$-$C_6$), O-alcoxy en ($C_1$-$C_4$)-alkyle en ($C_1$-$C_4$), S-alkyle en ($C_1$-$C_6$), alkyle en ($C_1$-$C_6$), haloalkyle en ($C_2$-$C_4$), O-haloalkyle en ($C_2$-$C_4$), alcényle en ($C_2$-$C_6$), alcynyle en ($C_2$-$C_6$), N(R160)(R161), $SO_2$-$CH_3$, COOH, COO-alkyle en ($C_1$-$C_6$), CON(R162)(R163), N(R164)CO(R165), N(R166)$SO_2$(R167), CO(R168), $(CR169R170)_{z'}$-O(R171), $(CR169R170)_{z'}$-CO-O(R77), O-$(CR169R170)_{z'}$-CO-O(R171), $(CR169R170)_{z'}$-N(R172)(R173), O-$(CR169R170)_{z'}$-N(R172)(R173), $(CR169R170)_{z'}$-CON(R172)(R173), O-$(CR169R170)_{z'}$-CON(R172)(R173), O-CO-N(R172)(R173), O-CO-alkylène en ($C_1$-$C_6$)-CO-O-alkyle en ($C_1$-$C_6$), O-CO-alkylène en ($C_1$-$C_6$)-CO-OH, O-CO-alkylène en ($C_1$-$C_6$)-CO-N(R172)(R173);
les z' signifient indépendamment les uns des autres 1, 2, 3, 4, 5, 6 ; R160, R161, R162, R163, R164, R165, R166, R167, R168, R169, R170, R171, R172, R173

signifient, de manière identique ou différente, hydrogène, alkyle en ($C_1$-$C_6$);

ou

R160 et R161, R162 et R163, R172 et R173 forment indépendamment les uns des autres éventuellement conjointement avec l'atome d'azote auquel ils sont reliés un cycle de 5 à 6 chaînons, qui peut contenir en plus de l'atome d'azote encore 0 à 1 hétéroatome supplémentaire du groupe constitué par NH, N-alkyle en ($C_1$-$C_6$), oxygène et soufre.

**5.** Composé de formule I selon les revendications 1 à 4, dans lequel

R2 signifie hydrogène, alkyle en ($C_1$-$C_6$), cycloalkyle en ($C_3$-$C_8$) ou phényle;
R3 signifie hydrogène;
R4 signifie hydrogène ou alkyle en ($C_1$-$C_6$).

**6.** Médicament contenant un ou plusieurs composés de formule I selon les revendications 1 à 5.

**7.** Médicament selon la revendication 6, **caractérisé en ce qu'**il contient en tant qu'agent actif supplémentaire de la metformine, de l'arcabose, du glibenclamide, du glimépiride, du gliclazide, de la gliquidone, de la pioglitazone, de la rosiglitazone, de l'exenatide, du miglitol, de la vildagliptine, de la sitagliptine, du répaglinide, du natéglinide ou du mitiglinide.

**8.** Médicament selon la revendication 6, **caractérisé en ce qu'**il contient en tant qu'agent actif supplémentaire un ou plusieurs antidiabétiques, agents actifs hypoglycémiques, inhibiteurs d'HMGCoA-réductase, inhibiteurs de la résorption de cholestérol, agonistes de PPAR gamma, agonistes de PPAR alpha, agonistes de PPAR alpha/gamma, fibrates, inhibiteurs de MTP, inhibiteurs de la résorption d'acide gallique, inhibiteurs de CETP, adsorbants polymères d'acide gallique, inducteurs des récepteurs de LDL, inhibiteurs d'ACAT, antioxydants, inhibiteurs de lipoprotéine-lipase, inhibiteurs d'ATP-citrate-lyase, inhibiteurs de squalène synthétase, antagonistes de lipoprotéine (a), inhibiteurs de lipase, insulines, sulfonylurées, biguanides, méglitinides, thiazolidine-diones, inhibiteurs d'$\alpha$-glucosidase, agents actifs agissant sur le canal calcique dépendant de l'ATP des cellules bêta, agonistes de CART, agonistes de NPY, agonistes de MC4, antagonistes d'orexine, agonistes d'H3, agonistes de TNF, antagonistes de CRF, antagonistes de CRP BP, agonistes d'urocortine, agonistes de $\beta$3, agonistes de MSH (hormone simulant les mélanocytes), agonistes de CCK, inhibiteurs de la réabsorption de sérotonine, composés de sérotonine et noradrénergiques mixtes, agonistes de 5HT, agonistes de bombésine, antagonistes de galanine, hormones de croissance, composés libérant des hormones de croissance, agonistes de TRH, modulateurs de la protéine découplante 2 ou 3, agonistes de leptine, agonistes de DA (bromocriptine, doprexine), inhibiteurs de lipase/amylase, modulateurs de PPAR, modulateurs de RXR ou agonistes de TR-$\beta$ ou amphétamines.

**9.** Composé de formule I selon les revendications 1 à 5 pour le traitement et/ou la prévention de troubles du métabolisme lipidique et de troubles de l'utilisation de glucose.

**10.** Composé de formule I selon les revendications 1 à 5 pour le traitement et/ou la prévention de dyslipidémies et leurs conséquences.

**11.** Composé de formule I selon les revendications 1 à 5 pour le traitement et/ou la prévention d'états associés avec le syndrome métabolique, ainsi que pour des troubles dans lesquels la résistance à l'insuline joue un rôle, ou pour le traitement et/ou la prévention du diabète sucré et des complications associées avec celui-ci.

**12.** Composé de formule I selon les revendications 1 à 5 pour le traitement et/ou la prévention d'états associés avec un niveau d'HDL réduit.

**13.** Composé de formule I selon les revendications 1 à 5 pour le traitement et/ou la prévention de maladies athérosclérotiques.

**14.** Composé de formule I selon les revendications 1 à 5 en combinaison avec au moins un autre agent actif pour le traitement et/ou la prévention de troubles dans lesquels la résistance à l'insuline joue un rôle.

**15.** Procédé de fabrication d'un médicament contenant un ou plusieurs des composés de formule I selon les revendications 1 à 5, **caractérisé en ce que** celui-ci est mélangé avec un véhicule pharmaceutiquement approprié et ce mélange est mis sous une forme appropriée pour l'administration.

**16.** Procédé de fabrication de composés de formule générale I selon les revendications 1 à 5, **caractérisé en ce que** des dérivés d'azolopyridin-3-ol substitués ou non substitués II sont mis en réaction avec des isocyanates III pour former des composés de formule I ;

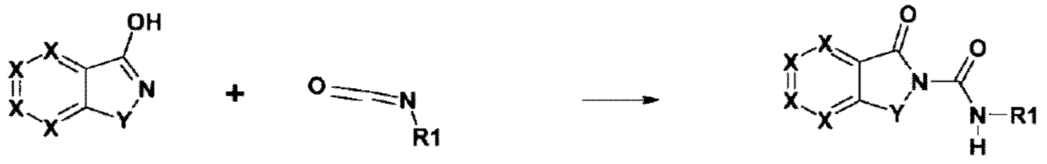

II III IV

ou

des dérivés d'azolopyridin-3-ol II sont

a) acylés avec des chlorures de carbamoyle de formule VI ;

ou

b) mis en réaction en deux étapes tout d'abord avec du phosgène ou des équivalents tels que l'ester trichlorométhylique d'un acide chlorocarboxylique, l'ester ditrichlorométhylique d'un acide carboxylique ou l'ester 4-nitrophénylique de l'acide chloroformique, et lors d'une deuxième étape avec des amines de formule VII, les substituants ayant les significations indiquées précédemment.

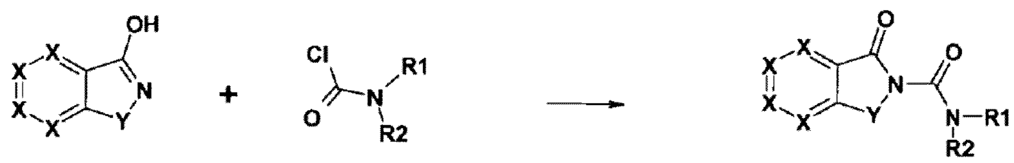

II VI I

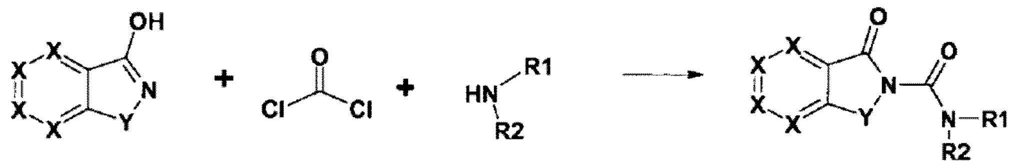

II VII I

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2004093872 A
- WO 2004094393 A
- US 4512985 A
- WO 2007110216 A
- WO 2004094394 A
- WO 2007045393 A
- WO 2007110215 A
- WO 208122352 A
- WO 2008122357 A
- WO 2005005477 A
- US 6221633 B
- WO 2007128815 A
- WO 2007128817 A
- WO 2008034881 A
- WO 2008049711 A
- WO 2008061355 A
- WO 9808871 A
- WO 2005027978 A
- WO 2006037811 A
- WO 2006037810 A
- WO 0104156 A
- WO 0034331 A
- WO 2006124529 A
- WO 2007124461 A
- WO 2008062457 A
- WO 2008082274 A
- WO 2008101017 A
- WO 2008081418 A
- WO 2008112939 A
- WO 2008112941 A
- WO 2008113601 A
- WO 2008116294 A
- WO 2008116648 A
- WO 2008119238 A
- WO 2007104789 A
- WO 2007120899 A
- WO 2008022015 A
- WO 2008056726 A
- WO 2006121860 A
- WO 2008021560 A
- WO 9726265 A
- WO 9903861 A
- US 2007264331 A
- WO 2008050987 A
- WO 2008062273 A
- WO 2007095462 A
- WO 2007101060 A
- WO 2007105650 A
- WO 2007137008 A
- WO 2008020607 A
- WO 9741097 A
- WO 2005086904 A
- WO 2007060992 A
- WO 2007100027 A
- WO 2007103252 A
- WO 2007122970 A
- WO 2007138485 A
- WO 2008006319 A
- WO 2008006969 A
- WO 2008010238 A
- WO 2008017398 A
- WO 2008028188 A
- WO 2008066356 A
- WO 2008084303 A
- WO 2008089461 A
- WO 2008089464 A
- WO 2008093639 A
- WO 2008096769 A
- WO 2008096820 A
- WO 2008096829 A
- US 2008194617 A
- WO 2008099944 A
- WO 2008108602 A
- WO 2008109334 A
- WO 2008126731 A
- WO 2008126732 A
- WO 2001040207 A
- WO 2002096894 A
- WO 2005097076 A
- WO 2007056771 A
- WO 2007087448 A
- WO 2007089667 A
- WO 2007089557 A
- WO 2007102515 A
- JP 2007246474 B
- WO 2007118963 A
- WO 2007118964 A
- WO 2007126043 A
- WO 2008006043 A
- WO 2008006044 A
- WO 2008012470 A
- WO 2008035359 A
- WO 2008087365 A
- WO 2008087366 A
- WO 2008087367 A
- WO 2008117982 A
- WO 0064888 A
- WO 0064876 A

- WO 03020269 A
- WO 2004024726 A
- WO 2007099553 A
- US 2007276041 A
- WO 2007085135 A
- WO 2007085136 A
- WO 2007141423 A
- WO 2008016175 A
- WO 2008053331 A
- WO 2008109697 A
- WO 2008109700 A
- WO 2008108735 A
- WO 2006059744 A
- WO 2006084176 A
- WO 2006029699 A
- WO 2007039172 A
- WO 2007039178 A
- WO 2007071766 A
- WO 2007101864 A
- US 2007244094 A
- WO 2007119887 A
- US 2008004281 A
- WO 2008071311 A
- WO 2008084962 A
- US 2008176861 A
- WO 2007114532 A
- WO 2007140230 A
- US 2007287674 A
- US 2008103201 A
- WO 2008065796 A
- WO 2008082017 A
- FR 258900
- WO 2003084922 A
- WO 2004007455 A
- WO 200507322931 A
- WO 2005067932 A
- WO 2008062739 A
- WO 2008099000 A
- WO 2008113760 A
- WO 2004100875 A
- WO 2005065680 A
- WO 2006086488 A
- WO 2007047177 A
- WO 2007106181 A
- WO 2007111864 A
- WO 2007120270 A
- WO 2007120284 A
- WO 2007123581 A
- WO 2007136577 A
- WO 2008042223 A
- WO 2008098244 A
- WO 2004063179 A
- WO 2004072031 A
- WO 2004072066 A
- WO 2005080360 A
- WO 2005044801 A
- WO 2006016194 A
- WO 2006058923 A
- WO 2006112549 A
- WO 2006125972 A
- WO 2007017549 A
- WO 2007017649 A
- WO 2007007910 A
- WO 200700704042 A
- WO 200700676061 A
- WO 2007006814 A
- WO 2007007886 A
- WO 2007028135 A
- WO 2007031739 A
- WO 2007041365 A
- WO 2007041366 A
- WO 2007037534 A
- WO 2007043638 A
- WO 2007053345 A
- WO 2007051846 A
- WO 2007051845 A
- WO 2007053765 A
- WO 2007051847 A
- WO 2007061923 A
- WO 2007075847 A
- WO 2007089512 A
- WO 2007104034 A
- WO 2007117381 A
- WO 2007122482 A
- WO 2007125103 A
- WO 2007125105 A
- US 2007281942 A
- WO 2008005914 A
- WO 2008005964 A
- WO 2008043701 A
- WO 2008044777 A
- WO 2008047821 A
- US 2008096877 A
- WO 2008050117 A
- WO 2008050101 A
- WO 2008059625 A
- US 2008146625 A
- WO 2008078674 A
- WO 2008079787 A
- WO 2008084043 A
- WO 2008084044 A
- WO 2008084872 A
- WO 2008089892 A
- WO 2008091770 A
- WO 2008075073 A
- WO 2008084873 A
- JP 2008189659 B
- WO 2008104994 A
- WO 2008111473 A
- WO 2008116107 A
- WO 2008118718 A
- WO 2008120754 A
- FR 225654
- WO 2008053446 A
- WO 2006023515 A
- WO 2006104030 A

- WO 2007014619 A
- WO 2007137962 A
- WO 2008019309 A
- WO 2008037628 A
- WO 2004101528 A
- WO 2003074500 A
- WO 2003106456 A
- WO 2004037169 A
- WO 200450658 A
- WO 2005037828 A
- WO 2005058901 A
- WO 2005012312 A
- WO 2005012308 A
- WO 2006039325 A
- WO 2006058064 A
- WO 2006015691 A
- WO 2006015701 A
- WO 2006015699 A
- WO 2006015700 A
- WO 2006018117 A
- WO 2006099943 A
- WO 2006099941 A
- JP 2006160733 B
- WO 2006071752 A
- WO 2006065826 A
- WO 2006078676 A
- WO 2006073167 A
- WO 2006068163 A
- WO 2006085685 A
- WO 2006090915 A
- WO 2006104356 A
- WO 2006127530 A
- WO 2006111261 A
- US 2006890898 A
- US 2006803357 A
- US 2006303661 A
- WO 2007015767 A
- WO 2007024993 A
- WO 2007029086 A
- WO 2007063928 A
- WO 2007070434 A
- WO 2007071738 A
- WO 2007071576 A
- WO 2007077508 A
- WO 2007087231 A
- WO 2007097931 A
- WO 2007099385 A
- WO 2007100374 A
- WO 2007112347 A
- WO 2007112669 A
- WO 2007113226 A
- WO 2007113634 A
- WO 2007115821 A
- WO 2007116092 A
- US 2007259900 A
- EP 1852108 A
- US 2007270492 A
- WO 2007126745 A
- WO 2007136603 A
- WO 2007142253 A
- WO 2007148185 A
- WO 2008017670 A
- US 2008051452 A
- WO 2008027273 A
- WO 2008028662 A
- WO 2008029217 A
- JP 2008031064 B
- JP 2008063256 B
- WO 2008033851 A
- WO 2008040974 A
- WO 2008040995 A
- WO 2008060488 A
- WO 2008064107 A
- WO 2008066070 A
- WO 2008077597 A
- JP 2008156318 B
- WO 2008087560 A
- WO 2008089636 A
- WO 2008093960 A
- WO 2008096841 A
- WO 2008101953 A
- WO 2008118848 A
- WO 2008119005 A
- WO 2008119208 A
- WO 2008120813 A
- WO 2008121506 A
- WO 2007128801 A
- WO 2003097064 A
- WO 2007026761 A
- WO 2008045484 A
- WO 2004007517 A
- WO 200452903 A
- WO 200452902 A
- EP 2005005959 W
- WO 2005085237 A
- JP 2004359630 B
- WO 2005121161 A
- WO 2006018150 A
- WO 2006035796 A
- WO 2006062224 A
- WO 2006058597 A
- WO 2006073197 A
- WO 2006080577 A
- WO 2006087997 A
- WO 2006108842 A
- WO 2007000445 A
- WO 2007014895 A
- WO 2007080170 A
- WO 2007093610 A
- WO 2007126117 A
- WO 2007128480 A
- WO 2007129668 A
- US 2007275907 A
- WO 2007136116 A
- WO 2007143316 A
- WO 2007147478 A

- WO 2008001864 A
- WO 2008002824 A
- WO 2008013277 A
- WO 2008013280 A
- WO 2008013321 A
- WO 2008013322 A
- WO 2008016132 A
- WO 2008020011 A
- JP 2008031161 B
- WO 2008034859 A
- WO 2008042688 A
- WO 2008044762 A
- WO 2008046497 A
- WO 2008049923 A
- WO 2008055870 A
- WO 2008055940 A
- WO 2008069327 A
- WO 2008070609 A
- WO 2008071288 A
- WO 2008072726 A
- WO 2008083200 A
- WO 2008090209 A
- WO 2008090210 A
- WO 2008101586 A
- WO 2008101939 A
- WO 2008116179 A
- WO 2008116195 A
- US 2008242596 A
- WO 20019009094 A
- WO 200343999 A
- WO 2004112782 A
- WO 200344000 A
- WO 200344009 A
- WO 2004112779 A
- WO 2004113310 A
- WO 2004103980 A
- WO 2004112784 A
- WO 2003065983 A
- WO 2003104207 A
- WO 2003104208 A
- WO 2004106294 A
- WO 2004011410 A
- WO 2004033427 A
- WO 2004041264 A
- WO 2004037251 A
- WO 2004056744 A
- WO 2004058730 A
- WO 2004065351 A
- WO 2004089367 A
- WO 2004089380 A
- WO 200408947071 A
- WO 2004089896 A
- WO 2005016877 A
- WO 2005063247 A
- WO 2005097759 A
- WO 2006010546 A
- WO 2006012227 A
- WO 2006012173 A
- WO 2006017542 A
- WO 2006034804 A
- WO 2006040329 A
- WO 2006051662 A
- WO 2006048750 A
- WO 2006049952 A
- WO 2006048331 A
- WO 2006050908 A
- WO 2006024627 A
- WO 2006066109 A
- WO 2006074244 A
- WO 2006078006 A
- WO 2006106423 A
- WO 2006132436 A
- WO 2006134481 A
- WO 2006134467 A
- WO 2006135795 A
- WO 2006136502 A
- WO 2006138508 A
- WO 2006138695 A
- WO 2006133926 A
- WO 2007003521 A
- WO 2007007688 A
- US 2007066584 A
- WO 2007029021 A
- WO 2007047625 A
- WO 2007051811 A
- WO 2007051810 A
- WO 2007057768 A
- WO 2007058346 A
- WO 2007061661 A
- WO 2007068330 A
- WO 2007070506 A
- WO 2007087150 A
- WO 2007092435 A
- WO 2007089683 A
- WO 2007101270 A
- WO 2007105753 A
- WO 2007107470 A
- WO 2007107550 A
- WO 2007111921 A
- US 2007207985 A
- US 2007208001 A
- WO 2007115935 A
- WO 2007118185 A
- WO 2007122411 A
- WO 2007124329 A
- WO 2007124337 A
- WO 2007124254 A
- WO 2007127688 A
- WO 2007127693 A
- WO 2007127704 A
- WO 2007127726 A
- WO 2007127763 A
- WO 2007127765 A
- WO 2007127901 A
- US 2007270424 A
- JP 2007291075 B

- WO 2007130898 A
- WO 2007135427 A
- WO 2007139992 A
- WO 2007144394 A
- WO 2007145834 A
- WO 2007145835 A
- WO 2007146761 A
- WO 2008000950 A
- WO 2008000951 A
- WO 2008003611 A
- WO 2008005910 A
- WO 2008006702 A
- WO 2008006703 A
- WO 2008011453 A
- WO 2008012532 A
- WO 2008024497 A
- WO 2008024892 A
- WO 2008032164 A
- WO 2008034032 A
- WO 2008043544 A
- WO 2008044656 A
- WO 2008046758 A
- WO 2008052638 A
- WO 2008053194 A
- WO 2008071169 A
- WO 2008074384 A
- WO 2008076336 A
- WO 2008076862 A
- WO 2008078725 A
- WO 2008087654 A
- WO 2008088540 A
- WO 2008099145 A
- WO 2008101885 A
- WO 2008101886 A
- WO 2008101907 A
- WO 2008101914 A
- WO 2008106128 A
- WO 2008110196 A
- WO 2008119017 A
- WO 2008120655 A
- WO 2008127924 A
- WO 20011983031 A
- WO 200117516 A
- WO 2004506446 A
- WO 2005012295 A
- WO 2005116003 A
- WO 2006007959 A
- DE 102004060542
- WO 2007009911 A
- WO 2007028145 A
- WO 2007067612615 A
- WO 2007081755 A
- WO 2007115058 A
- US 2008004325 A
- WO 2008033455 A
- WO 2008033931 A
- WO 2008033932 A
- WO 2008033934 A
- WO 2008089581 A
- WO 2004041274 A
- WO 2006045565 A
- WO 2006045564 A
- WO 2006069242 A
- WO 2006085108 A
- WO 2006085112 A
- WO 2006085113 A
- WO 2006124490 A
- WO 2006113150 A
- WO 2007017261 A
- WO 2007017262 A
- WO 2007017265 A
- WO 2007015744 A
- WO 2007027532 A
- WO 2007092364 A
- WO 2007120575 A
- WO 2007134986 A
- WO 2007150025 A
- WO 2007150026 A
- WO 2008016968 A
- WO 2008051403 A
- WO 2008086949 A
- WO 2008091338 A
- WO 2008097535 A
- WO 2008099448 A
- US 2008234277 A
- WO 2008127591 A
- WO 2008039882 A
- WO 2006067531 A
- WO 2006067532 A
- WO 2007013689 A
- WO 2007033002 A
- WO 2007106469 A
- US 2007265332 A
- WO 2007123225 A
- WO 2007131619 A
- WO 2007131620 A
- WO 2007131621 A
- WO 2007131622 A
- WO 2007136572 A
- WO 2008001931 A
- WO 2008030520 A
- WO 2008030618 A
- WO 2008054674 A
- WO 2008054675 A
- WO 2008066097 A
- US 2008176912 A
- WO 2004065380 A
- WO 2005061489 A
- WO 2006083491 A
- WO 200700396062 A
- WO 2007003964 A
- WO 2007035355 A
- WO 2007116229 A
- WO 2007116230 A
- WO 2008005569 A
- WO 2008005576 A

- WO 2008008887 A
- WO 2008008895 A
- WO 2008025798 A
- WO 2008025799 A
- WO 2008025800 A
- WO 2008070692 A
- WO 2008076243 A
- WO 200807692 A
- WO 2008081204 A
- WO 2008081205 A
- WO 2008081206 A
- WO 2008081207 A
- WO 2008081208 A
- WO 2008083238 A
- WO 2008085316 A
- WO 2008109702 A
- EP 1688138 A
- WO 2008066131 A
- WO 2008103500 A
- WO 2008103501 A
- WO 2005073199 A
- WO 2006074957 A
- WO 2006087309 A
- WO 2006111321 A
- WO 2007042178 A
- WO 2007119837 A
- WO 2008122352 A
- WO 2006131233 A
- WO 2006131232 A
- WO 2006131231 A
- WO 2007045392 A
- WO 2008048866 A
- WO 20080488867 A
- WO 2007119827 A
- US 2005222220 A
- WO 2005085230 A
- WO 2005111018 A
- WO 2003078403 A
- WO 2004022544 A
- WO 2003106410 A
- WO 2005058908 A
- US 2005038023 A
- WO 2005009997 A
- US 2005026984 A
- WO 2005000836 A
- WO 2004106343 A
- EP 1460075 A
- WO 2004014910 A
- WO 2003076442 A
- WO 2005087727 A
- WO 2004046117 A
- WO 2007073117 A
- WO 2007083978 A
- WO 2007120102 A
- WO 2007122634 A
- WO 2007125109 A
- WO 2007125110 A
- US 2007281949 A
- WO 2008002244 A
- WO 2008002245 A
- WO 2008016123 A
- WO 2008023239 A
- WO 2008044700 A
- WO 2008056266 A
- WO 2008057940 A
- WO 2008077138 A
- EP 1939191 A
- EP 1939192 A
- WO 2008078196 A
- WO 2008094992 A
- WO 2008112642 A
- WO 2008112651 A
- WO 2008113469 A
- WO 2008121063 A
- WO 2008121064 A
- WO 2004074288 A
- WO 2008027584 A
- WO 2008070150 A
- WO 2008125833 A
- WO 2008125835 A
- WO 2008125839 A
- WO 2006072354 A
- WO 2007093264 A
- WO 2008009335 A
- WO 2008086854 A
- WO 2008057855 A
- WO 2008057856 A
- WO 2008057857 A
- WO 2008057859 A
- WO 2008057862 A
- WO 2008059867 A
- WO 2008059866 A
- WO 2008059865 A
- WO 2008070507 A
- WO 2008124665 A
- WO 2008124745 A
- WO 2008104306 A
- WO 2008119918 A
- WO 2008096260 A
- WO 2008125945 A
- WO 2008088006 A
- WO 2007062568 A
- WO 2008006432 A
- WO 2008016278 A
- WO 2008016730 A
- WO 2008083124 A
- WO 2007112914 A
- WO 2007149865 A
- WO 2007104053 A
- WO 2007115822 A
- WO 2008008547 A
- WO 2008075741 A
- WO 2001000610 A
- WO 2001030774 A
- WO 2004022057 A
- WO 2004022553 A

- WO 2005097129 A
- WO 2005113544 A
- US 2007244140 A
- WO 2008099072 A
- WO 2008099073 A
- WO 2008099074 A
- WO 2008099075 A
- WO 2008016131 A
- US 2007249583 A
- WO 2008083551 A
- WO 2003099821 A
- WO 2005056554 A
- WO 2007052843 A
- WO 2007070796 A
- WO 2007092751 A
- JP 2007230909 B
- WO 2007095174 A
- WO 2007140174 A
- WO 2007140183 A
- WO 2008000643 A
- WO 2008002573 A
- WO 2008025539 A
- WO 2008025540 A
- JP 2008214222 B
- WO 2007092965 A
- WO 2008041003 A
- WO 2008049047 A
- WO 2008065754 A
- WO 2008073825 A
- US 2008242677 A
- WO 2008093655 A
- WO 2005042692 A
- WO 2005005453 A
- WO 2005021497 A
- WO 2005021495 A
- WO 2002066464 A
- WO 2005000353 A
- WO 2005044256 A
- WO 2005062824 A
- WO 2005061451 A
- WO 2005061452 A
- WO 2006017257 A
- WO 2005033100 A
- WO 2002050060 A
- WO 2002050068 A
- WO 2004000803 A
- WO 2004000804 A
- WO 2004000805 A
- WO 2004087655 A
- WO 2004097655 A
- WO 2005047248 A
- WO 2006086562 A
- WO 2006102674 A
- WO 2006116499 A
- WO 2006121861 A
- WO 2006122186 A
- WO 2006122216 A
- WO 2006127893 A
- WO 2006137794 A
- WO 2006137796 A
- WO 2006137782 A
- WO 2006137793 A
- WO 2006137797 A
- WO 2006137795 A
- WO 2006137792 A
- WO 2006138163 A
- WO 2007059871 A
- US 2007232688 A
- WO 2007126358 A
- WO 2008033431 A
- WO 2008033465 A
- WO 2008052658 A
- WO 2008057336 A
- WO 2008085300 A
- WO 2008033464 A
- US 6992067 B
- US 7205290 B
- WO 2006002342 A
- WO 2006010422 A
- WO 2006012093 A
- WO 2006073973 A
- WO 2006072362 A
- WO 2007088996 A
- WO 2007088999 A
- US 2007185058 A
- US 2007185113 A
- US 2007185154 A
- US 2007185182 A
- WO 2006097169 A
- WO 2007041494 A
- WO 2007090752 A
- WO 2007107243 A
- WO 2007120621 A
- US 2007265252 A
- US 2007265304 A
- WO 2007128568 A
- WO 2007132906 A
- WO 2008006257 A
- WO 2008009435 A
- WO 2008018529 A
- WO 2008058961 A
- WO 2008058967 A
- WO 2008059513 A
- WO 2008070496 A
- WO 2008115442 A
- WO 2008111604 A
- US 6245744 B
- US 6221897 B
- WO 0061568 A
- DE 102005033099
- DE 102005033100
- DE 102006053635
- DE 102006053637
- WO 200700965556 A
- WO 2008058628 A
- WO 2008058629 A

- WO 2008058630 A
- WO 2008058631 A
- US 20060199795 A
- WO 2007110237 A
- WO 2007127505 A
- WO 2008009407 A
- WO 2008067219 A
- WO 2008067222 A
- FR 2908310
- WO 2008091540 A
- WO 2008097976 A
- WO 2008097504 A
- WO 2005085226 A
- WO 2005121091 A
- WO 2006010423 A
- WO 2006113910 A
- WO 2007143164 A
- WO 2008049806 A
- WO 2008049808 A
- WO 2008090198 A
- WO 2008100423 A
- WO 2008030382 A
- WO 2008079398 A
- WO 2008032980 A
- WO 2006094682 A
- WO 2008087029 A
- WO 2008087030 A
- WO 2008095189 A
- WO 2007063012 A
- WO 2007096251 A
- WO 2008015081 A
- US 2008103182 A
- WO 2008074692 A
- WO 2008031032 A
- WO 2008046071 A
- WO 2008083280 A
- WO 2008084300 A
- WO 2005077907 A
- JP 2007022943 B
- WO 2008003424 A
- WO 2008092231 A
- US 6342512 B
- WO 2005097738 A
- WO 2008040651 A
- WO 2008099278 A
- WO 2006072393 A
- WO 2008062830 A
- EP 1258247 A
- EP 1375508 A
- WO 2008028590 A
- WO 2008077050 A
- WO 2007111954 A
- WO 2007121918 A
- WO 2007121921 A
- WO 2007121923 A
- WO 2008070661 A
- WO 2008064788 A
- WO 2008064789 A
- US 2007270433 A
- WO 2008027585 A
- WO 2008080461 A
- WO 199946262 A
- WO 200372197 A
- WO 2003072197 A
- WO 2005044814 A
- WO 2005108370 A
- JP 2006131559 B
- WO 2007011809 A
- WO 2007011811 A
- WO 2007013691 A
- WO 2007095601603 A
- WO 2007119833 A
- WO 2008065508 A
- WO 2008069500 A
- WO 2008072850 A
- WO 2008079610 A
- WO 2008088688 A
- WO 2008088689 A
- WO 2008088692 A
- US 2008171761 A
- WO 2008090944 A
- JP 2008179621 B
- US 2008200461 A
- WO 2008102749 A
- WO 2008103382 A
- WO 2008121592 A
- WO 2007100789 A
- WO 2007100833 A
- WO 2008051873 A
- WO 2008051875 A
- WO 2008073623 A
- WO 2008094869 A
- WO 2008112022 A
- WO 2006001318 A
- WO 2007103295 A
- WO 2007125952 A
- WO 2008026563 A
- WO 2008026564 A
- WO 2008052769 A
- WO 2008092887 A
- WO 2008092888 A
- WO 2008092891 A
- WO 2007038942 A
- WO 2007038943 A
- WO 2005080424 A
- WO 2006095166 A
- WO 2008003947 A
- WO 2006096847 A
- EP 0656354 A
- WO 0015609 A
- WO 20016463264634 A
- WO 02076949 A
- WO 2005080345 A
- WO 2005080328 A
- WO 2005080343 A
- WO 2005075450 A

- WO 2005080357 A
- WO 200170700 A
- WO 200302664748 A
- WO 200302776 A
- WO 2003040107 A
- WO 2003007887 A
- WO 2003027069 A
- US 6509367 B
- WO 200132663 A
- WO 2003086288 A
- WO 2003087037 A
- WO 2004048317 A
- WO 2004058145 A
- WO 2003084930 A
- WO 2003084943 A
- WO 2004058744 A
- WO 2004013120 A
- WO 2004029204 A
- WO 2004035566 A
- WO 2004058249 A
- WO 2004058255 A
- WO 2004058727 A
- WO 2004069838 A
- US 20040214837 A
- US 20040214855 A
- US 20040214856 A
- WO 2004096209 A
- WO 2004096763 A
- WO 2004096794 A
- WO 2005000809 A
- WO 2004099157 A
- US 20040266845 A
- WO 2004110453 A
- WO 2004108728 A
- WO 2004000817 A
- WO 2005000820 A
- US 20050009870 A
- WO 200500974 A
- WO 200411103334 A
- WO 20041103839 A
- WO 2005016286 A
- WO 2005007111 A
- WO 2005007628 A
- US 20050054679 A
- WO 2005027837 A
- WO 2005028456 A
- WO 200506376162 A
- WO 2005061509 A
- WO 2005077897 A
- WO 2006018662 A
- WO 2006047516 A
- WO 2006060461 A
- WO 2006067428 A
- WO 2006067443 A
- WO 2006087480 A
- WO 2006087476 A
- WO 2006100208 A
- WO 2006106054 A
- WO 2006111849 A
- WO 2006113704 A
- WO 2007009705 A
- WO 2007017124 A
- WO 2007017126 A
- WO 2007018459 A
- WO 2007018460 A
- WO 2007016460 A
- WO 2007020502 A
- WO 2007026215 A
- WO 2007028849 A
- WO 2007031720 A
- WO 2007031721 A
- WO 2007036945 A
- WO 2007038045 A
- WO 2007039740 A
- US 20070015810 A
- WO 2007046548 A
- WO 2007047737 A
- WO 2007057687 A
- WO 2007062193 A
- WO 2007064272 A
- WO 2007079681 A
- WO 2007084319 A
- WO 2007084450 A
- WO 2007086080 A
- EP 1816125 A
- US 2007213302 A
- WO 2007095513 A
- WO 2007096764 A
- US 2007254863 A
- WO 2007119001 A
- WO 2007120454 A
- WO 2007121687 A
- WO 2007123949 A
- US 2007259934 A
- WO 2007131219 A
- WO 2007133820 A
- WO 2007136571 A
- WO 2007136607 A
- US 7297710 B
- WO 2007138050 A
- WO 2007139464 A
- WO 2007140385 A
- WO 2007140439 A
- WO 2007148061 A
- WO 2007148062 A
- US 2007293509 A
- WO 2008004698 A
- WO 2008017381 A
- US 2008021031 A
- WO 2008024284 A
- WO 2008031734 A
- WO 2008035356 A
- WO 2008036021 A
- WO 2008036022 A
- WO 2008039023 A
- WO 2998043544 A

- WO 2008044111 A
- WO 2008048648 A
- EP 1921072 A1
- WO 2008053341 A
- WO 2008056377 A
- WO 2008059207 A
- WO 2008059335 A
- WO 2008062424 A
- WO 2008068423 A
- WO 2008068424 A
- WO 2008070305 A
- WO 2008070306 A
- WO 2008074816 A
- WO 2008074982 A
- WO 2008075012 A
- WO 2008075013 A
- WO 2008075019 A
- WO 2008075118 A
- WO 2008076754 A
- WO 2008081009 A
- WO 2008084057 A
- EP 1944295 A
- US 2008090809 A
- US 2008090810 A
- WO 2008092816 A
- WO 2008094473 A
- WO 2008094476 A
- WO 2008099076 A
- WO 2008099139 A
- WO 2008101995 A
- US 2008207704 A
- WO 2008107179 A
- WO 2008109027 A
- WO 2008112674 A
- WO 2008115705 A
- WO 2008118414 A
- WO 2008119999 A
- WO 200812000 A
- WO 2008121257 A
- WO 2008127585 A
- WO 2007001939 A
- WO 2007044215 A
- WO 2007112399 A
- WO 2007112402 A
- WO 2008122618 A
- WO 2007140005 A
- WO 2008019357 A
- WO 2008021625 A
- WO 2008023720 A
- WO 2008030532 A
- WO 2008057585 A
- WO 2008059214 A
- WO 2008075064 A
- WO 2008075070 A
- WO 2008075077 A
- WO 2008120653 A
- WO 2007091948 A
- WO 2007129188 A
- WO 2007133637 A
- WO 2008007780 A
- WO 2008010061 A
- WO 2008007211 A
- WO 2008015335 A
- WO 2008018827 A
- WO 2008024433 A
- WO 2008024438 A
- WO 2008032204 A
- WO 2008050199 A
- WO 2008059339 A
- WO 2008059370 A
- WO 2008066664 A
- WO 2008075150 A
- WO 2008090382 A
- WO 2008090434 A
- WO 2008093024 A
- WO 2008107543 A
- WO 2008107544 A
- WO 2008110863 A
- WO 2007047397 A
- WO 2008021849 A
- WO 2008021851 A
- WO 2008032156 A
- US 2008249122 A
- WO 2008089201 A
- WO 2007111806 A
- WO 0191752 A
- WO 2005060985 A
- WO 2005009950 A
- WO 2004087159 A
- WO 2004078717 A
- WO 2004078716 A
- WO 2004024720 A
- US 20050124652 A
- WO 2005051391 A
- WO 2004112793 A
- US 20050222014 A
- US 20050176728 A
- US 20050164914 A
- US 20050124636 A
- US 20050130988 A
- US 20040167201 A
- WO 2004005324 A
- WO 2004037797 A
- WO 2005042516 A
- WO 2005040109 A
- WO 2005030797 A
- US 20040224901 A
- WO 200501921 A
- WO 200509184 A
- WO 2005000339 A
- EP 1460069 A
- WO 2005047253 A
- WO 2005047251 A
- WO 2005118573 A
- EP 1538159 A
- WO 2004072076 A

- WO 2004072077 A
- WO 200602165557 A
- WO 2007009894 A
- WO 2007015162 A
- WO 2007041061 A
- WO 2007041052 A
- JP 2007131570 B
- EP 1842846 A
- WO 2007096186 A
- WO 2007096763 A
- WO 2007141343 A
- WO 2008007930 A
- WO 2008017852 A
- WO 2008039418 A
- WO 2008087186 A
- WO 2008087187 A
- WO 2008087189 A
- WO 2008087190 A
- WO 2008090357 A
- WO 200196302 A
- WO 200185693 A
- WO 2004085403 A
- WO 2005075458 A
- WO 2006067224 A
- WO 2007085718 A
- WO 2007088276 A
- WO 2007116374 A
- WO 2007122591 A
- WO 2007126934 A
- WO 2007126935 A
- WO 2008008517 A
- WO 2008008518 A
- WO 2008008551 A
- WO 2008020405 A
- WO 2008026149 A
- WO 2008038251 A
- US 2008132490 A
- WO 2008065626 A
- WO 2008078291 A
- WO 2008087611 A
- WO 2008081399 A
- WO 2008108991 A
- WO 2008107335 A
- US 2008249125 A
- WO 0063208 A
- WO 200064884 A
- WO 2005082893 A
- US 2005171181 A
- WO 2006107661 A
- WO 2007003804 A
- WO 2007016496 A
- WO 2007020213 A
- WO 2007049798 A
- WO 2007055418 A
- WO 2007057329 A
- WO 2007065820 A
- WO 2007068620 A
- WO 2007068641 A
- WO 2007075629 A
- WO 2007080140 A
- WO 2007082840 A
- WO 2007088450 A
- WO 2007088462 A
- WO 2007094962 A
- WO 2007099423 A
- WO 2007100990 A
- WO 2007105053 A
- WO 2007106349 A
- WO 2007110364 A
- WO 2007115938 A
- WO 2007131907 A
- WO 2007133561 A
- US 2007270440 A
- WO 2007135111 A
- WO 2007137955 A
- US 2007281923 A
- WO 2007137968 A
- WO 2007138431 A
- WO 2007146122 A
- WO 2008005338 A
- WO 2008012010 A
- WO 2008015125 A
- WO 2008045371 A
- EP 1757594 A
- WO 2008068173 A
- WO 2008068174 A
- US 20080171753 A
- WO 2008072703 A
- WO 2008072724 A
- US 2008188484 A
- US 2008188486 A
- US 2008188487 A
- WO 2008109333 A
- WO 2008109336 A
- WO 2008002816 A
- WO 2008002817 A
- WO 2008002818 A
- WO 2008002820 A
- WO 2007117399 A
- WO 0066585 A
- WO 2007105113 A
- WO 2007133756 A
- WO 2008036541 A
- WO 2008036579 A
- WO 2008083070 A
- WO 0183451 A
- JP 2006111553 B
- WO 2002038543 A
- WO 2002038544 A
- WO 2007048840843 A
- WO 2008015558 A
- EP 1947103 A
- WO 2005085200 A
- WO 2005019240 A
- WO 2004011438 A
- WO 2004012648 A

- WO 2003015769 A
- WO 2004072025 A
- WO 2005070898 A
- WO 2005070925 A
- WO 2004039780 A
- WO 2004092181 A
- WO 2003033476 A
- WO 2002006245 A
- WO 2002089729 A
- WO 2002002744 A
- WO 2003004027 A
- FR 2868780
- WO 2006010446 A
- WO 2006038680 A
- WO 2006044293 A
- WO 2006044174 A
- JP 2006176443 B
- WO 2006018280 A
- WO 2006018279 A
- WO 2006118320 A
- WO 2006130075 A
- WO 2007018248 A
- WO 2007012661 A
- WO 2007029847 A
- WO 2007024004 A
- WO 2007039462 A
- WO 2007042660 A
- WO 2007042668 A
- WO 2007042669 A
- US 2007093508 A
- US 2007093509 A
- WO 2007048802 A
- JP 2007091649 B
- WO 2007092416 A
- WO 2007093363366 A
- WO 2007114902 A
- WO 2007114916 A
- WO 2007141200 A
- WO 2007142217 A
- US 2007299062 A
- WO 2007146758 A
- WO 2007146759 A
- WO 2008001160 A
- WO 2008016811 A
- WO 2008020799 A
- WO 2008022979 A
- WO 2008038692 A
- WO 2008041090 A
- WO 2008044632 A
- WO 2008047544 A
- WO 2008061109 A
- WO 2008065021 A
- WO 2008068265 A
- WO 2008071646 A
- WO 2008076562 A
- JP 2008088120 B
- WO 2008086404 A
- WO 2008086409 A
- WO 9915525 A
- WO 0244150 A
- WO 2005116034 A
- WO 2007120655 A
- WO 2007120688 A
- WO 2007120718 A
- WO 2008091631 A
- WO 2007148341 A
- WO 2008034142 A
- WO 2008081477 A
- WO 2008120761 A
- WO 2008063673 A
- WO 0071549 A
- WO 0109111 A
- WO 2006085118 A
- WO 2008079838 A
- WO 2008079839 A
- WO 2008079847 A
- WO 2008079848 A
- US 2008076724 A
- WO 2007138343 A
- WO 200077010 A
- WO 20007700102 A
- WO 2005019180 A
- WO 2003064423 A
- WO 200242304 A
- WO 2005035533 A
- WO 2005082859 A
- WO 2006004937 A
- US 2006025601 A
- WO 2006028961 A
- WO 2006077025 A
- WO 2006103511 A
- WO 2007028132 A
- WO 2007084622 A
- US 2007249709 A
- WO 2007132841 A
- WO 2007140213 A
- WO 2008007661 A
- WO 2008007664 A
- WO 2008009125 A
- WO 2008010073 A
- WO 2008108445 A
- WO 2005058858 A
- WO 2007054257 A
- WO 2007107373 A
- WO 2007108569 A
- WO 2007108742744 A
- WO 2008003703 A
- WO 2008027073 A
- WO 2008034815 A
- WO 2008054288 A
- EP 1947085 A
- WO 2008084491 A
- WO 2008084492 A
- WO 2008092665 A
- WO 2008092666 A
- WO 2008101247 A

- WO 2008110598 A
- WO 2008116831 A
- WO 2008116833 A
- WO 2007131005 A
- WO 2008052709 A
- WO 2008109727 A
- WO 2007098953 A
- WO 2007098961 A
- WO 2008015266 A
- WO 2008055932 A
- WO 2008055933 A
- WO 2007110782 A
- WO 2008041184 A
- WO 2008051404 A
- WO 2008051405 A
- WO 2008051406 A
- WO 2008073311 A
- WO 0185695 A
- WO 2005030734 A
- WO 2007127457 A
- WO 2008008286 A
- WO 2006012577 A
- WO 2007079239 A
- WO 2008092681 A
- EP 0462884 A
- WO 2008059023 A
- WO 2008059024 A
- WO 2008059025 A
- WO 2008059026 A
- WO 0040569 A
- WO 2008107184 A
- US 20040224997 A
- WO 2004094618 A
- WO 200058491 A
- WO 2005044250 A
- WO 2005072740 A
- JP 2005206492 B
- WO 2005013907 A
- WO 2006004200 A
- WO 2006019020 A
- WO 2006064189 A
- WO 2006082952 A
- WO 2006120125 A
- WO 2006113919 A
- WO 2006134317 A
- WO 2007016538 A
- WO 2007060140 A
- JP 2007131584 B
- WO 2007071966 A
- WO 2007126957 A
- WO 2007137103 A
- WO 2007137107 A
- WO 2007138304 A
- WO 2007138311 A
- WO 2007141502 A
- WO 2007141517 A
- WO 2007141538 A
- WO 2007141545 A
- WO 2007144571 A
- WO 2008011130 A
- WO 2008011131 A
- WO 2008039007 A
- WO 2008048991 A
- WO 2008067257 A
- WO 2008099221 A
- WO 2008038768 A
- WO 2004005277 A
- WO 2008006113 A
- WO 2007009236 A
- WO 2007044085 A
- WO 2007046867 A
- WO 2007046868 A
- WO 20070501124 A
- WO 2007056846 A
- WO 2007071023 A
- WO 2007130075 A
- WO 2007134457 A
- WO 2007136746 A
- WO 2007143597 A
- WO 2007143823 A
- WO 2007143824 A
- WO 2008003753 A
- WO 2008017161 A
- WO 2008024390 A
- WO 2008029266 A
- WO 2008036715 A
- WO 2008043087 A
- WO 2008044767 A
- WO 2008046226 A
- WO 2008056687 A
- WO 2008062276 A
- WO 2008064474 A
- WO 2008074824 A
- WO 2008074832 A
- WO 2008074833 A
- WO 2008074834 A
- WO 2008074835 A
- WO 2008089580 A
- WO 2008096746 A
- WO 2008104524 A
- WO 2008116898 A
- US 2008249100 A
- WO 2008120744 A
- WO 2008120759 A
- WO 2008123469 A
- WO 2008127349 A
- WO 2008089310 A
- WO 2008039087 A
- WO 2006082978 A
- WO 2008105533 A
- WO 2007125946 A
- WO 2008038712 A
- WO 2008121009 A
- WO 20058279 A
- WO 200172692 A
- WO 200194293 A

- WO 2003084915 A
- WO 2004018421 A
- WO 2005092316 A
- WO 2007003419 A
- WO 2007009913 A
- WO 2007039125 A
- WO 2007110225 A
- WO 2007110226 A
- WO 2007128492 A
- WO 2007132475 A
- WO 2007134864 A
- WO 2008001959 A
- WO 2008106213 A
- WO 2008062469 A
- US 2008146523 A
- WO 2008092785 A
- WO 2008067270 A
- WO 2007065948 A
- WO 2008027557 A
- EP 1886695 A
- WO 2008119744 A
- WO 2007119463 A
- WO 2007125405 A
- WO 2008028860 A
- WO 2008118626 A
- WO 2005090336 A
- WO 2006071609 A
- WO 2006135826 A
- WO 2007105766 A
- WO 2008120661 A
- WO 2007019416 A
- WO 2008073451 A
- WO 2007107587 A
- WO 2007111994 A
- WO 2008106600 A
- WO 2008113796 A
- WO 2008097835 A
- WO 2007101146 A
- WO 2007133828 A
- WO 2007112069 A
- WO 2007099200 A
- WO 2007137874 A
- JP 2008024673 B
- WO 2007100104 A
- JP 2008106008 B
- US 7138107 B
- WO 2008028958 A
- WO 2008085711 A
- WO 2008033447 A
- WO 2008033356 A
- WO 2008033460 A
- WO 2008033468 A
- WO 2008073461 A
- WO 2008073934 A
- WO 2008073936 A
- WO 2008110008 A
- US 2008027049 A
- US 2008027090 A
- WO 2008040057 A
- WO 2008040058 A
- WO 2008046065 A
- WO 2008014360 A
- WO 2008014381 A
- WO 2008019967 A
- US 2008064697 A
- US 2008249101 A
- WO 2008000692 A
- WO 2008051272 A
- WO 2008042800 A
- WO 2008035305 A
- WO 2008035306 A
- WO 2008035686 A
- WO 2008036966 A
- WO 2008036967 A
- WO 2008058641 A
- WO 2008074413 A
- WO 2008062905 A
- WO 2008067378 A
- WO 2008064315 A
- WO 2008074820 A
- WO 2008074821 A
- WO 2008044770 A
- WO 2008090200 A
- WO 2008092091 A
- US 2008194658 A
- DE 2010120
- EP 2011060122 W
- EP 10305655 A

**In der Beschreibung aufgeführte Nicht-Patentliteratur**


- *J. Pharm. Sci.,* 2005, vol. 94 (10), 2111-2120
- **H. OKADA et al.** *Chem. Pharm. Bull.,* 1994, vol. 42, 57-61
- **D. CHEN et al.** *Proc. Natl. Acad. Sci. USA,* 2007, vol. 104, 943
- **R. D. CARR et al.** *Diabetes,* 2003, vol. 52, 2315-2318
- **J. B. HANSEN et al.** *Current Medicinal Chemistry,* 2004, vol. 11, 1595-1615
- **T. M. TAGMOSE et al.** *J. Med. Chem.,* 2004, vol. 47, 3202-3211
- **M. J. COGHLAN et al.** *J. Med. Chem.,* 2001, vol. 44, 1627-1653
- **J.P.BERGER et al.** *TRENDS in Pharmacological Sciences,* 2005, vol. 28 (5), 244-251
- **A. L. HANDION.** *Expert Opin. Ther. Patents,* 2005, vol. 15 (11), 1531-1540

- **ASAKAWA, A. et al.** Cocaine-amphetamine-regulated transcript influences energy metabolism, anxiety and gastric emptying in mice. *Hormone and Metabolic Research,* 2001, vol. 33 (9), 554-558
- **LEE, DANIEL W. ; LEINUNG, MATTHEW C. ; ROZHAVSKAYA-ARENA, MARINA ; GRASSO, PATRICIA.** Leptin agonists as a potential approach to the treatment of obesity. *Drugs of the Future,* 2001, vol. 26 (9), 873-881
- **SALVADOR, JAVIER ; GOMEZ-AMBROSI, JAVIER.** Perspectives in the therapeutic use of leptin. *Fruhbeck, Gema, Expert Opinion on Pharmacotherapy,* 2001, vol. 2 (10), 1615-1622
- **ZUNFT H J et al.** Carob pulp preparation for treatment of hypercholesterolemia. *ADVANCES IN THERAPY,* September 2001, vol. 18 (5), 230-6
- **BALIMANE, P.V.** *Drug Discovery Today,* 2005, vol. 10 (5), 335-343
- **ZLOKARNIK, G.** *Drug Discovery Today,* 2005, vol. 10 (21), 1443-1450
- **PLANT, N.** *Drug Discovery Today,* 2004, vol. 9 (7), 328-336
- **LAU, Y.Y. et al.** *Pharmaceutical Res.,* 2002, vol. 19 (11), 1606-1610
- **L. BAIOCCHI ; G. CORSI.** *Synthesis,* 1978, 633-648
- **I. SEKIKAWA et al.** *J. Het. Chem.,* 1973, 931-932
- **A.DORNOW ; M. SIEBRECHT.** *Chem. Ber.,* 1960, 1106-1110
- **M. TILSER ; B. STANOVNIK ; Z. ZRIMSEK.** *Heterocycles,* 1979, 217-219
- **K. BOWDEN ; G. CRANK ; W, J. ROOS.** *J.Chem.Soc.,* 1968, 172-185